(19) Europäisches Patentamt
European Patent Office
Office européen des brevets

(11) EP 4 004 992 B1

(12) EUROPEAN PATENT SPECIFICATION

(45) Date of publication and mention of the grant of the patent:
**27.08.2025 Bulletin 2025/35**

(21) Application number: **20746628.5**

(22) Date of filing: **24.07.2020**

(51) International Patent Classification (IPC):
***H10K 85/60*** *(2023.01)* *H10K 50/12* *(2023.01)*

(52) Cooperative Patent Classification (CPC):
**H10K 85/631; H10K 85/654; H10K 85/6572; H10K 85/658;** H10K 50/121; H10K 2101/20; H10K 2101/30; H10K 2101/90

(86) International application number:
**PCT/EP2020/071054**

(87) International publication number:
**WO 2021/014023 (28.01.2021 Gazette 2021/04)**

(54) **ORGANIC ELECTROLUMINESCENT DEVICE EMITTING GREEN LIGHT**

ORGANISCHE MOLEKÜLE FÜR OPTOELEKTRONISCHE VORRICHTUNGEN

MOLÉCULES ORGANIQUES POUR DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**25.07.2019 EP 19188361**
**11.10.2019 EP 19202645**
**20.12.2019 EP 19218997**
**23.12.2019 EP 19219378**
**11.03.2020 EP 20162459**
**16.03.2020 EP 20163321**

(43) Date of publication of application:
**01.06.2022 Bulletin 2022/22**

(73) Proprietor: **Samsung Display Co., Ltd.**
**Gyeonggi-do 17113 (KR)**

(72) Inventors:
- **SHARIFIDEHSARI, Hamed**
  **73614 Schorndorf (DE)**
- **LEGANÉS CARBALLO, Jaime**
  **73430 Aalen (DE)**
- **LIAPTSIS, Georgios**
  **68161 Mannheim (DE)**
- **FLÜGGE, Harald**
  **76135 Karlsruhe (DE)**
- **BONUS, Sandra**
  **50827 Köln (DE)**
- **THIRION, Damien**
  **76689 Karlsdorf-Neuthard (DE)**
- **JOLY, Damien**
  **67930 Beinheim (FR)**

(74) Representative: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

(56) References cited:
**EP-A1- 3 490 023**
**WO-A1-2018/216990**
**US-A1- 2019 207 112**

- **THANH-TUÂN BUI ET AL: "Recent advances on organic blue thermally activated delayed fluorescence (TADF) emitters for organic light-emitting diodes (OLEDs)", BEILSTEIN JOURNAL OF ORGANIC CHEMISTRY, vol. 14, 19 January 2018 (2018-01-19), pages 282 - 308, XP055687894, DOI: 10.3762/bjoc.14.18**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GUOYUN MENG, XING CHEN, XIANG WANG, NAN WANG, TAI PENG, AND SUNING WANG: "Isomeric Bright Sky-Blue TADF Emitters Based on BisacridineDecorated DBNA: Impact of Donor Locations on Luminescentand Electroluminescent Properties", ADV. OPTICAL MATER., vol. 7, 1900130, 25 March 2019 (2019-03-25), XP002800444, DOI: 10.1002/ adom.201900130
- ANTON PERSHIN, ET AL.: "Highly emissive excitons with reduced exchangeenergy in thermally activated delayed fluorescentmolecules", NATURE COMMUNICATIONS, vol. 10, 597, 5 February 2019 (2019-02-05), XP002800445, DOI: 10.1038/ s41467-019-08495-5

## Description

The present invention relates to organic electroluminescent devices comprising at least one light-emitting layer B comprising at least one host material $H^B$, at least one thermally activated delayed fluorescence (TADF) material $E^B$, and at least one small full width at half maximum (FWHM) emitter $S^B$, wherein $E^B$ transfers energy to $S^B$, and $S^B$ emits light. $S^B$ exhibits a narrow - expressed by a small full width at half maximum (FWHM) - green emission with an emission maximum of 500 nm to 560 nm. Furthermore, the present invention relates to a method for generating green light by means of an organic electroluminescent device according to the present invention.

Organic electroluminescent devices containing one or more light-emitting layers based on organics such as, e.g., organic light emitting diodes (OLEDs), light emitting electrochemical cells (LECs) and light-emitting transistors gain increasing importance. In particular, OLEDs are promising devices for electronic products such as screens, displays and illumination devices. In contrast to most electroluminescent devices essentially based on inorganics, organic electroluminescent devices based on organics are often rather flexible and producible in particularly thin layers. The OLED-based screens and displays already available today bear either good efficiencies and long lifetimes or good color purity and long lifetimes, but do not combine all three properties, i.e. good efficiency, long lifetime, and good color purity.

EP3490023 (A1) discloses an OLED device comprising a TADF material and an emitter.

Thus, there is still an unmet technical need for organic electroluminescent devices which have a high quantum yield, a long lifetime, and good color purity.

The color purity or color point of an OLED is typically provided by CIEx and CIEy coordinates, whereas the color gamut for the next display generation is provided by so-called BT-2020 and DCPI3 values. Generally, in order to achieve these color coordinates, top emitting devices are needed to adjust the color coordinates by changing the cavity. In order to achieve high efficiency in top emitting devices while targeting these color gamut, a narrow emission spectrum in bottom emitting devices is typically desired.

Recently, some fluorescent *near-range-charge-transfer* (NRCT) emitters have been developed that display a rather narrow emission spectrum with a FWHM that is smaller than or equal to 0.25 eV, and therefore more suitable to achieve the BT-2020 and DCPI3 color gamut.

A central element of an organic electroluminescent device for generating light typically is the at least one light-emitting layer placed between an anode and a cathode. When a voltage (and current) is applied to an organic electroluminescent device, holes and electrons are injected from an anode and a cathode, respectively. Typically, a hole transport layer is typically located between the light-emitting layer and the anode, and an electron transport layer is typically located between the light-emitting layer and the cathode. The different layers are sequentially disposed. Excitons of high energy are then generated by recombination of the holes and the electrons in the light-emitting layer. The decay of such excited states (e.g., singlet states such as S1 and/or triplet states such as T1) to the ground state (S0) desirably leads to the emission of light.

Surprisingly, it has been found that an organic electroluminescent device's light-emitting layer comprising at least one TADF material, at least one small full width at half maximum (FWHM) emitter, and at least one host material provides an organic electroluminescent device having a long lifetime, a high quantum yield and exhibiting narrow emission, ideally suitable to achieve the BT-2020 and DCPI3 color gamut.

Herein, at least one TADF material transfers energy to at least one small full width at half maximum (FWHM) emitter displaying green emission with an emission maximum of 500 nm to 560 nm.

Accordingly, one aspect of the present invention relates to an organic electroluminescent device according to the claims which comprises one or more light-emitting layers B. each independently of each other comprising:

(i) at least one host material $H^B$, which has a lowermost excited singlet state energy level $E(S1^H)$ and a lowermost excited triplet state energy level $E(T1^H)$;
(ii) at least one TADF material $E^B$, which has a lowermost excited singlet state energy level $E(S1^E)$ and a lowermost excited triplet state energy level $E(T1^E)$; and
(iii) at least one small FWHM emitter $S^B$, which has a lowermost excited singlet state energy level $E(S1^S)$ and a lowermost excited triplet state energy level $E(T1^S)$,

wherein (each) $E^B$ transfers energy to (at least one) $S^B$ and (each) $S^B$ emits green light with an emission maximum (in the wavelength range) between 500 nm and 560 nm; and wherein the relations expressed by the following formulas (1) to (5) apply:

$$E(S1^H) > E(S1^E) \qquad (1)$$

$$E(S1^H) > E(S1^S) \quad (2)$$

$$E(S1^E) > E(S1^S) \quad (3)$$

$$E(T1^H) > E(T1^S) \quad (4)$$

$$E(T1^H) > E(T1^E) \quad (5).$$

In a preferred embodiment, at least one small full width at half maximum (FWHM) emitter $S^B$ is a boron containing emitter. In a preferred embodiment, each small full width at half maximum (FWHM) emitter $S^B$ is a boron containing emitter.

Accordingly, the lowermost excited singlet state $S1^H$ of a host material $H^B$ is higher in energy than the lowermost excited singlet state $S1^E$ of a TADF material $E^B$. The lowermost excited singlet state $S1^H$ of a host material $H^B$ is higher in energy than the lowermost excited singlet state $S1^S$ of any small FWHM emitter $S^B$. The lowermost excited singlet state $S1^E$ of a TADF material $E^B$ is higher in energy than the lowermost excited singlet state $S1^S$ of any small FWHM emitter $S^B$. The lowermost excited triplet state $T1^H$ of a host material $H^B$ is higher in energy than the lowermost excited triplet state $T1^S$ of any small FWHM emitter $S^B$. The lowermost excited triplet state $T1^H$ of a host material $H^B$ is higher in energy than the lowermost excited triplet state $T1^E$ of a TADF material $E^B$.

In a preferred embodiment of the invention, the lowermost excited triplet state $T1^E$ of a TADF material $E^B$ is higher in energy than the lowermost excited triplet state $T1^S$ of any small FWHM emitter $S^B$: $E(T1^E) > E(T1^S)$.

In a preferred embodiment of the invention, an electroluminescent device according to the invention comprises exactly one light-emitting layer B.

In another embodiment of the invention, an electroluminescent device according to the invention comprises exactly two light-emitting layers B.

In another embodiment of the invention, an electroluminescent device according to the invention comprises more than two light-emitting layers B.

It is understood that different light-emitting layers B optionally comprised in the same organic electroluminescent device according to the invention do not necessarily all comprise the same materials or even the same materials in the same ratios.

In one embodiment of the invention, at least one light-emitting layer B comprises at least one host material $H^B$, exactly one TADF material $E^B$, and exactly one small FWHM emitter $S^B$.

In a preferred embodiment of the invention, at least one light-emitting layer B comprises exactly one host material $H^B$. In a preferred embodiment of the invention, at least one light-emitting layer B comprises exactly one TADF material $E^B$. In a preferred embodiment of the invention, at least one light-emitting layer B comprises exactly one small FWHM emitter $S^B$. In a preferred embodiment of the invention, at least one light-emitting layer B comprises exactly one host material $H^B$ and exactly one TADF material $E^B$. In a preferred embodiment of the invention, at least one light-emitting layer B comprises exactly one host material $H^B$ and exactly one FWHM emitter $S^B$. In a preferred embodiment of the invention, at least one light-emitting layer B comprises exactly one TADF material $E^B$ and exactly one FWHM emitter $S^B$. In a preferred embodiment of the invention, at least one light-emitting layer B comprises exactly one host material $H^B$, exactly one TADF material $E^B$, and exactly one small FWHM emitter $S^B$.

In one embodiment, a small FWHM emitter $S^B$ displays thermally activated delayed fluorescence (TADF). In one embodiment, a TADF material $E^B$ displays a small FWHM. A TADF material $E^B$ may optionally emit light in the visible wavelength range, for example with an emission maximum within the wavelength range of 500 nm to 560 nm. In one embodiment, a TADF material $E^B$ displays a small FWHM (e.g., less than or equal to 0.30 eV, less than or equal to 0.25 eV, less than or equal to 0.20 eV, less than or equal to 0.15 eV, or less than or equal to 0.13 eV).

Surprisingly, it was found that the main contribution to the emission band of the optoelectronic device according to the invention can typically be attributed to the emission of $S^B$ indicating a sufficient transfer of energy from $E^B$ to $S^B$ as well as preferably from the at least one host material $H^B$ to $E^B$ and/or $S^B$. This indicates that at least one TADF material $E^B$ may act as energy pump for the at least one small FWHM emitter $S^B$, whose main function may be the emission of green light.

The emission of devices according to the invention shows longer lifetimes and/ or higher efficiencies compared to that of devices with similar device architecture, wherein all of the at least one light-emitting layers comprise at least one host material $H^B$ in combination with either at least one TADF material $E^B$ or at least one small FWHM emitter $S^B$ (but not both).

Particularly interesting is that, depending on the combinations of $E^B$ and $S^B$ of the present invention, energy from lower energy states can also be transferred to higher energy states of the other compound. Also taking the reverse

intersystem crossing (RISC) occurring in TADF materials into account, the combinations of $E^B$ and $S^B$ of the present invention may lead to particularly efficient emission of the small FWHM emitter $S^B$.

A further embodiment of the present invention relates to an electroluminescent device (e.g., an OLED), which exhibits an external quantum efficiency at 1000 cd/m$^2$ of more than 10%, more preferably of more than 13%, more preferably of more than 15%, even more preferably of more than 18% or even more than 20 % and exhibits an emission maximum between 500 nm and 560 nm.

A further embodiment of the present invention relates to an electroluminescent device (e.g., an OLED), which exhibits an external quantum efficiency at 1000 cd/m$^2$ of more than 10%, more preferably of more than 13%, more preferably of more than 15%, even more preferably of more than 18 % or even more than 20% and exhibits an emission maximum between 510 nm and 550 nm.

A further embodiment of the present invention relates to an electroluminescent device (e.g., an OLED) which exhibits an external quantum efficiency at 1000 cd/m$^2$ of more than 10%, more preferably of more than 13%, more preferably of more than 15%, even more preferably of more than 18 % or even more than 20% and exhibits an emission maximum between 520 nm and 540 nm.

In a preferred embodiment, the electroluminescent device (e.g., an OLED) exhibits a LT95 value at constant current density $J_0$ = 15 mA/cm$^2$ of more than 100 h, preferably more than 200 h, more preferably more than 400 h, even more preferably more than 750 h or even more than 1000 h.

A further embodiment of the present invention relates to an electroluminescent device (e.g., an OLED), which emits light at a distinct color point. According to the present invention, the electroluminescent device (e.g., OLED) emits light with a narrow emission band (small full width at half maximum (FWHM)). In a preferred embodiment, the electroluminescent device (e.g., OLED) according to the invention emits light with a FWHM of the main emission peak of below 0.25 eV, more preferably of below 0.20 eV, even more preferably of below 0.15 eV or even below 0.13 eV.

A further embodiment of the present invention relates to an electroluminescent device (e.g., an OLED), which emits light with CIEx and CIEy color coordinates close to the CIEx (= 0.170) and CIEy (= 0.797) color coordinates of the primary color green (CIEx = 0.170 and CIEy = 0.797) as defined by ITU-R Recommendation BT.2020 (Rec. 2020) and thus may be suited for the use in Ultra High Definition (UHD) displays, e.g. UHD-TVs. In this context, the term "close to" refers to the ranges of CIEx and CIEy coordinates provided at the end of this paragraph. In commercial applications, typically top-emitting (top-electrode is typically transparent) devices are used, whereas test devices as used throughout the present application represent bottom-emitting devices (bottom-electrode and substrate are transparent). Accordingly, a further aspect of the present invention relates to an electroluminescent device (e.g., an OLED), whose emission exhibits a CIEx color coordinate of between 0.15 and 0.45 preferably between 0.15 and 0.35, more preferably between 0.15 and 0.30 or even more preferably between 0.15 and 0.25 or even between 0.15 and 0.20 and/ or a CIEy color coordinate of between 0.60 and 0.92, preferably between 0.65 and 0.90, more preferably between 0.70 and 0.88 or even more preferably between 0.75 and 0.86 or even between 0.79 and 0.84.

A further embodiment of the present invention relates to an OLED, which emits light with CIEx and CIEy color coordinates close to the CIEx (= 0.265) and CIEy (= 0.65) color coordinates of the primary color green (CIEx = 0.265 and CIEy = 0.65) as defined by DCIP3. In this context, the term "close to" refers to the ranges of CIEx and CIEy coordinates provided at the end of this paragraph. In commercial applications, typically top-emitting (top-electrode is typically transparent) devices are used, whereas test devices as used throughout the present application represent bottom-emitting devices (bottom-electrode and substrate are transparent). Accordingly, a further aspect of the present invention relates to an OLED, whose bottom emission exhibits a CIEx color coordinate of between 0.2 and 0.45 preferably between 0.2 and 0.35 or more preferably between 0.2 and 0.30 or even more preferably between 0.24 and 0.28 or even between 0.25 and 0.27 and/ or a CIEy color coordinate of between 0.60 and 0.9, preferably between 0.6 and 0.8, more preferably between 0.60 and 0.70 or even more preferably between 0.62 and 0.68 or even between 0.64 and 0.66.

One of the purposes of interest of an organic electroluminescent device may be the generation of light. Thus, the present invention further relates to a method for generating light of a desired wavelength range, comprising the step of providing an organic electroluminescent device according to any the present invention.

Accordingly, a further aspect of the present invention relates to a method for generating light of a desired wavelength range, comprising the steps of

(i) providing an organic electroluminescent device according to the present invention; and
(ii) applying an electrical current to said organic electroluminescent device.

A further aspect of the present invention relates to a process of making the organic electroluminescent devices by assembling the elements described above. The present invention also relates to a method for generating green light, in particular by using said organic electroluminescent device.

The examples and claims further illustrate the invention.

*Host material(s) $H^B$*

According to the invention, any of the one or more host materials $H^B$ comprised in any of the at least one light-emitting layers B may be a p-host $H^P$ exhibiting high hole mobility, an n-host $H^N$ exhibiting high electron mobility, or a bipolar host material $H^{BP}$ exhibiting both, high hole mobility and high electron mobility.

In one embodiment of the invention, the at least one light-emitting layer B of an organic electroluminescent device according to the invention comprises one or more p-hosts $H^P$. In one embodiment of the invention, the at least one light-emitting layer B of an organic electroluminescent device according to the invention comprises only one p-host $H^P$.

In one embodiment of the invention, the at least one light-emitting layer B of an organic electroluminescent device according to the invention comprises one or more n-hosts $H^N$. In another embodiment of the invention, the at least one light-emitting layer B of an organic electroluminescent device according to the invention comprises only one n-host $H^N$.

In one embodiment of the invention, the at least one light-emitting layer B of an organic electroluminescent device according to the invention comprises one or more bipolar hosts $H^{BP}$. In one embodiment of the invention, the at least one light-emitting layer B of an organic electroluminescent device according to the invention comprises only one bipolar host $H^{BP}$.

In another embodiment of the invention, the at least one light-emitting layer B of an organic electroluminescent device according to the invention comprises at least two different host materials. In this case, the more than one host materials present in the at least one light-emitting layer B may either all be p-hosts or all be n-hosts, or all be bipolar hosts, but may also be a combination thereof.

It is understood that, if an organic electroluminescent device according to the invention comprises more than one light-emitting layer B, any of them may independently of the other light-emitting layers comprise either one host material $H^B$ or more than one host materials $H^B$ for which the above-mentioned definitions apply. It is further understood that different light-emitting layers B comprised in an organic electroluminescent device according to the invention do not necessarily all comprise the same materials or even the same materials in the same concentrations.

If comprised in the same light-emitting layer B of an organic electroluminescent device according to the invention, at least one p-host $H^P$ and at least one n-host $H^N$ may optionally form an exciplex. The person skilled in the art knows how to choose pairs of $H^P$ and $H^N$, which form an exciplex and the selection criteria, including HOMO- and/or LUMO-energy level requirements of $H^P$ and $H^N$. This is to say that, in case exciplex formation may be aspired, the highest occupied molecular orbital (HOMO) of the p-host material $H^P$ may be at least 0.20 eV higher in energy than the HOMO of the n-host material $H^N$ and the lowest unoccupied molecular orbital (LUMO) of the p-host material $H^P$ may be at least 0.20 eV higher in energy than the LUMO of the n-host material $H^N$.

In a preferred embodiment of the invention, at least one host material $H^B$ (e.g., $H^P$, $H^N$, and/or bipolar host $H^{BP}$) is an organic host material, which, in the context of the invention, means that it does not contain any transition metals. In a preferred embodiment of the invention, all host materials $H^B$ ($H^P$, $H^N$, and/or bipolar host $H^{BP}$) in the electroluminescent device of the present invention are organic host materials, which, in the context of the invention, means that they do not contain any transition metals. Preferably, at least one host material $H^B$, preferably all host materials $H^B$ ($H^P$, $H^N$ and/or bipolar host $H^{BP}$) predominantly consist of the elements hydrogen (H), carbon (C), and nitrogen (N), but may for example also comprise oxygen (O), boron (B), silicon (Si), fluorine (F), and bromine (Br).

According to the invention, a p-host $H^P$ optionally comprised in any of the at least one light-emitting layers B of an organic electroluminescent device according to the invention has a highest occupied molecular orbital HOMO($H^P$) having an energy $E^{HOMO}(H^P)$, wherein preferably: $-6.1 \text{ eV} \leq E^{HOMO}(H^P) \leq -5.6 \text{ eV}$.

According to the invention, a p-host $H^P$ optionally comprised in any of the at least one light-emitting layers B of an organic electroluminescent device according to the invention has a lowest unoccupied molecular orbital LUMO($H^P$) having an energy $E^{LUMO}(H^P)$, wherein preferably: $-2.6 \text{ eV} \leq E^{LUMO}(H^P)$.

According to the invention, a p-host $H^P$ optionally comprised in any of the at least one light-emitting layers B of an organic electroluminescent device according to the invention has a lowermost excited singlet state energy level $E(S1^{p\text{-}H})$, wherein preferably: $E(S1^{p\text{-}H}) \geq 3.0$ eV.

According to the invention, a p-host $H^P$ optionally comprised in any of the at least one light-emitting layers B of an organic electroluminescent device according to the invention has a lowermost excited triplet state energy level $E(T1^{p\text{-}H})$, wherein preferably: $E(T1^{p\text{-}H}) \geq 2.7$ eV.

It is understood that any requirements or preferred features previously defined for a host material $H^B$ comprised in any of the at least one light-emitting layers B of an organic electroluminescent device according to the invention are preferably also valid for a p-host $H^P$ according to the invention. Thus, in a preferred embodiment, the relations expressed by the following formulas (6) to (9) apply:

$$E(S1^{p\text{-}H}) > E(S1^E) \qquad (6)$$

$$E(S1^{p\text{-}H}) > E(S1^{S}) \quad (7)$$

$$E(T1^{p\text{-}H}) > E(T1^{S}) \quad (8)$$

$$E(T1^{p\text{-}H}) > E(T1^{E}) \quad (9).$$

Accordingly, the lowermost excited singlet state $S1^{p\text{-}H}$ of a p-host $H^P$ is preferably higher in energy than the lowermost excited singlet state $S1^E$ of a TADF material $E^B$. The lowermost excited singlet state $S1^{p\text{-}H}$ of a p-host $H^P$ is preferably higher in energy than the lowermost excited singlet state $S1^S$ of any small FWHM emitter $S^B$. The lowermost excited triplet state $T1^{p\text{-}H}$ of a p-host $H^P$ is preferably higher in energy than the lowermost excited triplet state $T1^S$ of any small FWHM emitter $S^B$. The lowermost excited triplet state $T1^{p\text{-}H}$ of a p-host $H^P$ is preferably higher in energy than the lowermost excited triplet state $T1^E$ of a TADF material $E^B$.

In a preferred embodiment of the invention, a p-host $H^P$, optionally comprised in any of the at least one light-emitting layers B, optionally comprises or consists of:

- one first chemical moiety, comprising or consisting of a structure according to any of the formulas $H^P$-I, $H^P$-II, $H^P$-III, $H^P$-IV, $H^P$-V, $H^P$-VI, $H^P$-VII, $H^P$-VIII, $H^P$-IX, and $H^P$-X:

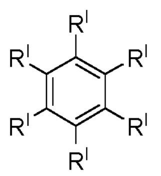

Formula $H^P$-I

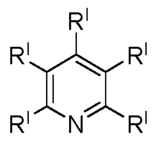

Formula $H^P$-II

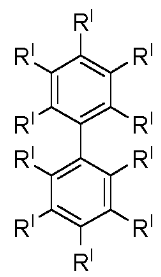

Formula $H^P$-III

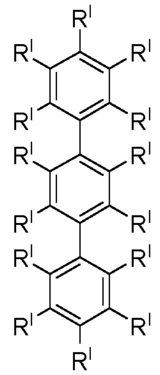

Formula $H^P$-IV

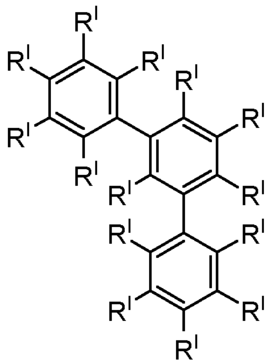

Formula $H^P$-V

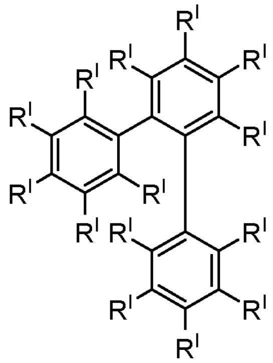

Formula $H^P$-VI

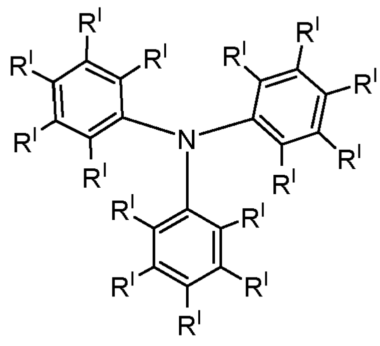

Formula $H^P$-VII

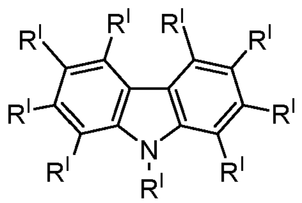

Formula $H^P$-VIII

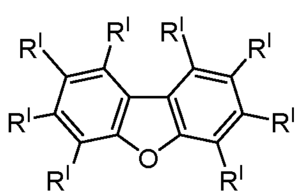

Formula $H^P$-IX

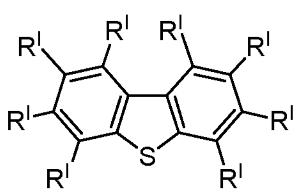

Formula $H^P$-X

and

- one or more second chemical moiety comprising or consisting of a structure according to any of formulas $H^P$-XI, $H^P$-XII, $H^P$-XIII, $H^P$-XIV, $H^P$-XV, $H^P$-XVI, $H^P$-XVII, $H^P$-XVIII, and $H^P$-XIX:

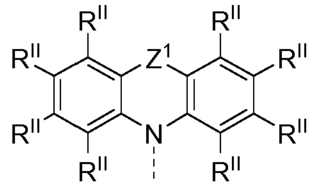

Formula $H^P$-XI

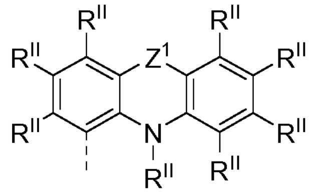

Formula $H^P$-XII

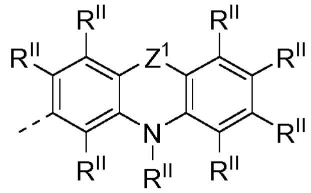

Formula $H^P$-XIII

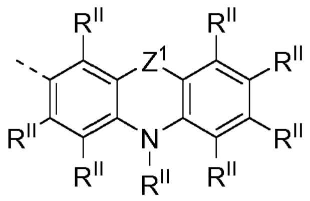

Formula $H^P$-XIV

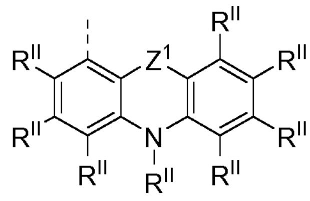

Formula $H^P$-XV

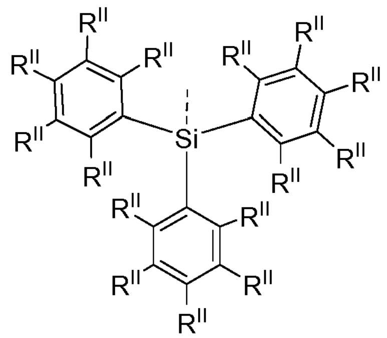

Formula $H^P$-XVI

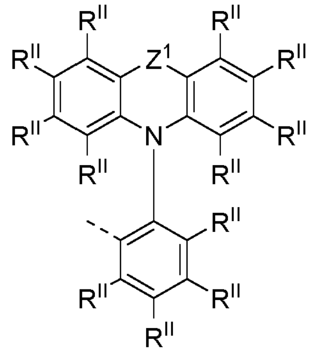

Formula $H^P$-XVII

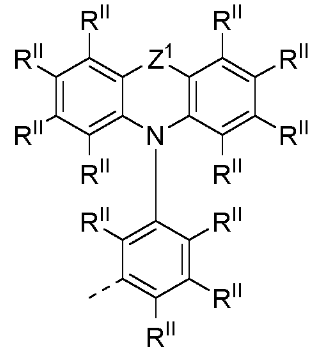

Formula $H^P$-XVIII

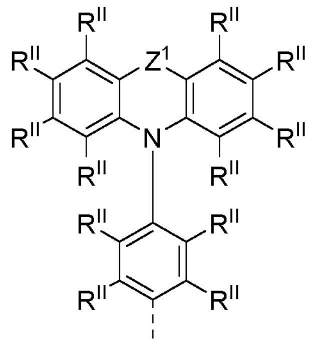

Formula $H^P$-XIX

wherein each of the at least one second chemical moieties which is present in the p-host material $H^P$ is linked to the first chemical moiety via a single bond which is represented in the formulas above by a dashed line;
wherein
$Z^1$ is at each occurrence independently of each other selected from the group consisting of a direct bond, $C(R^{II})2$, $C=C(R^{II})_2$, C=O, $C=NR^{II}$, $NR^{II}$, O, $Si(R^{II})_2$, S, S(O) and $S(O)_2$;
$R^I$ is at each occurrence independently of each other a binding site of a single bond linking the first chemical moiety to a second chemical moiety or is selected from the group consisting of: hydrogen, deuterium, Me, $^i$Pr, $^t$Bu, wherein at least one $R^I$ is a binding site of a single bond linking the first chemical moiety to a second chemical moiety, and
Ph, which is optionally substituted with one or more substituents independently of each other selected from the group consisting of: Me, $^i$Pr, $^t$Bu, and Ph;
$R^{II}$ is at each occurrence independently of each other selected from the group consisting of: hydrogen, deuterium, Me, $^i$Pr, $^t$Bu, and
Ph, which is optionally substituted with one or more substituents independently of each other selected from the group consisting of: Me, $^i$Pr, $^t$Bu, and Ph;
wherein two or more adjacent substituents $R^{II}$ may optionally form an aromatic or heteroaromatic ring system with 3-18 carbon atoms.

In an even more preferred embodiment of the invention, $Z^1$ is at each occurrence a direct bond and adjacent substituents $R^{II}$ do not combine to form an additional ring system.

In a still even more preferred embodiment of the invention, the one or more p-host $H^P$ optionally comprised in an organic electroluminescent device according to the invention is selected from the group consisting of the following structures:

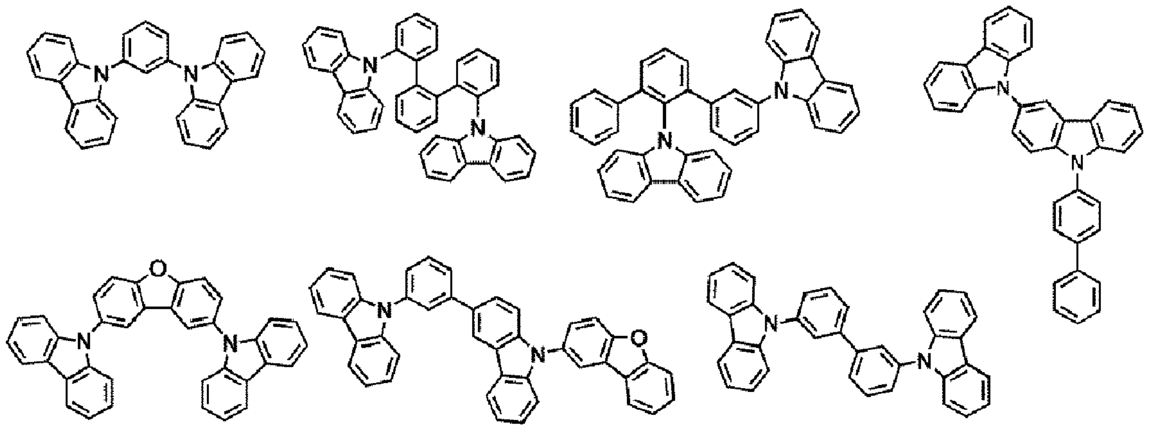

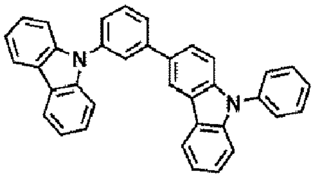

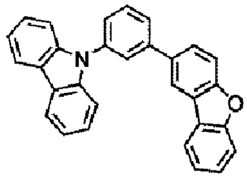

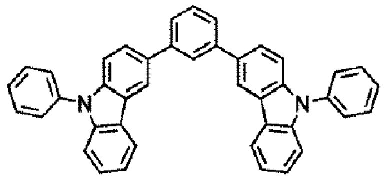

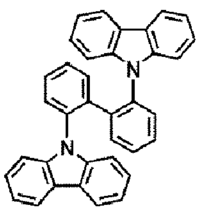

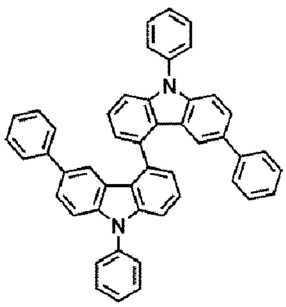

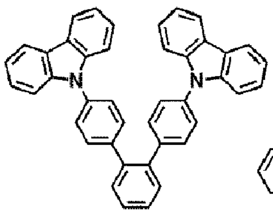

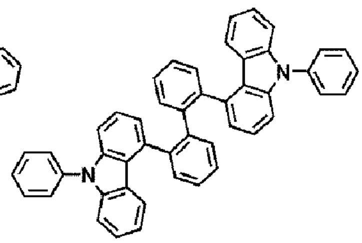

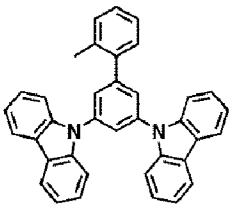

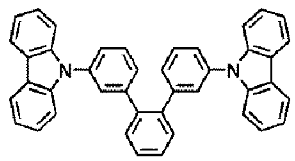

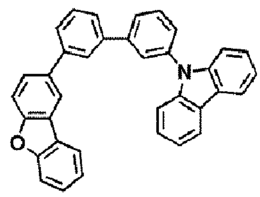

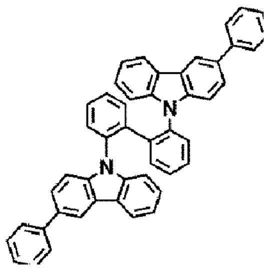

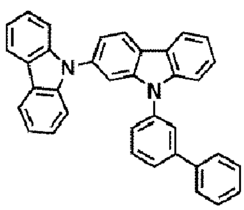

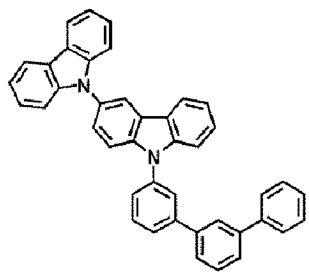

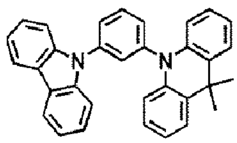

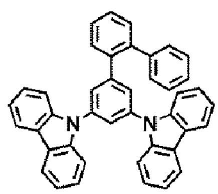

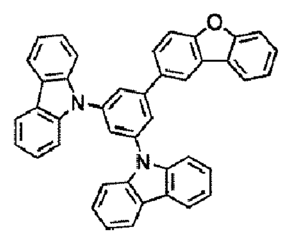
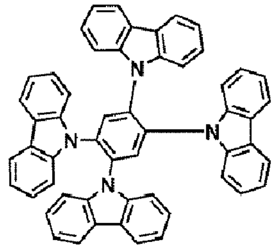
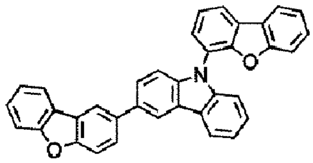
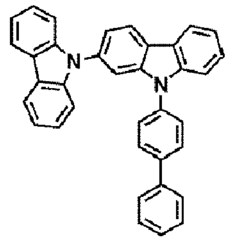

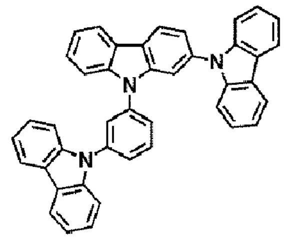
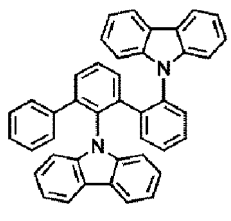
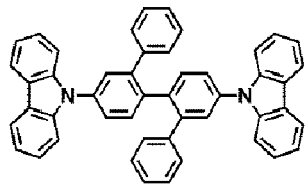
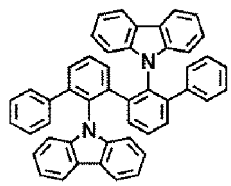

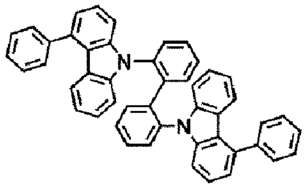
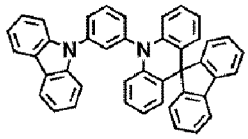
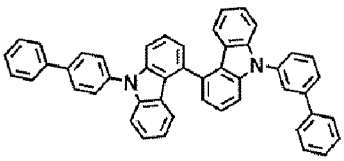

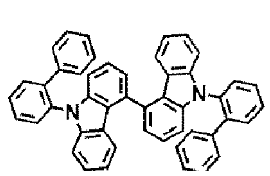
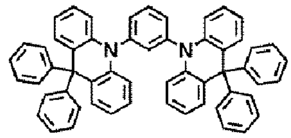
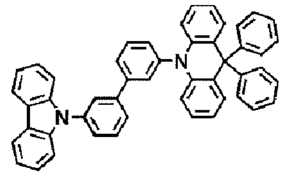

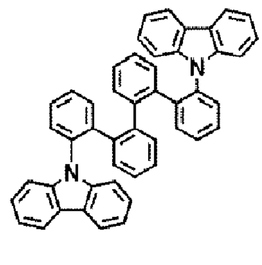
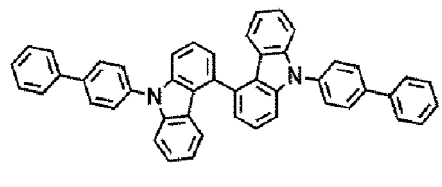
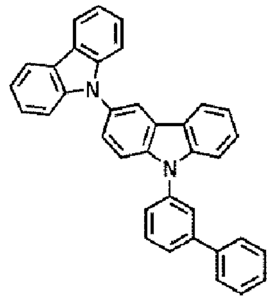

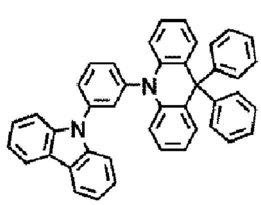
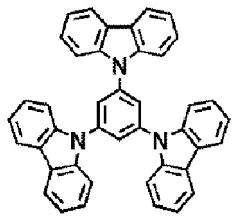
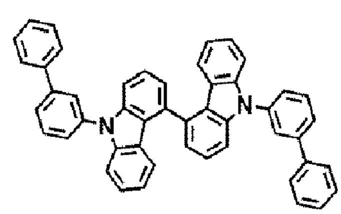

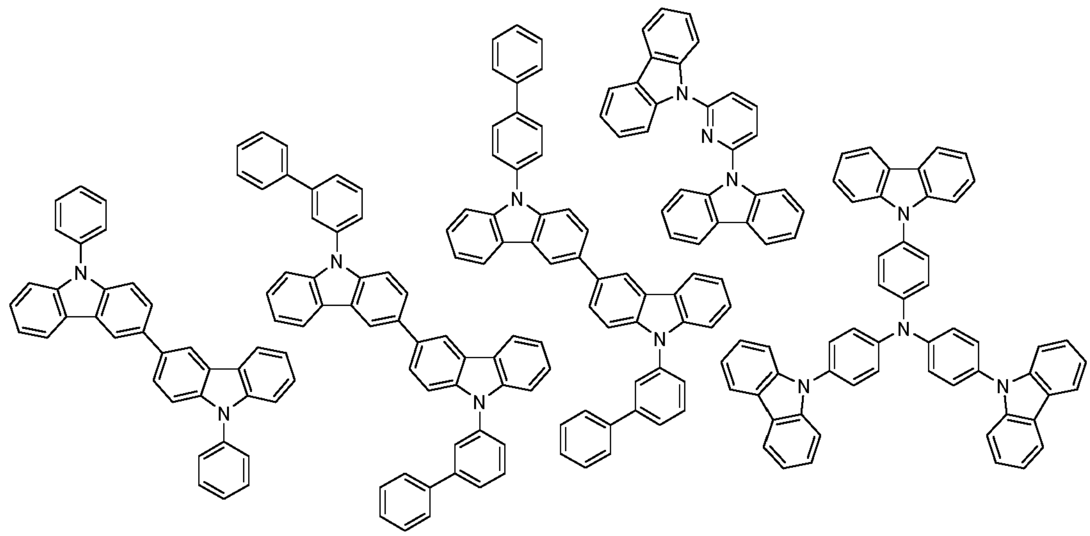

According to the invention, an n-host $H^N$ optionally comprised in any of the at least one light-emitting layers B of an organic electroluminescent device according to the invention has a highest occupied molecular orbital HOMO($H^N$) having an energy $E^{HOMO}(H^N)$, wherein preferably: $E^{HOMO}(H^N) \leq -5.9$ eV.

According to the invention, an n-host $H^N$ optionally comprised in any of the at least one light-emitting layers B of an organic electroluminescent device according to the invention has a lowest unoccupied molecular orbital LUMO($H^N$) having an energy $E^{LUMO}(H^N)$, wherein preferably: $-3.5 \text{ eV} \leq E^{LUMO}(H^N) \leq -2.9$ eV.

According to the invention, an n-host $H^N$ optionally comprised in any of the at least one light-emitting layers B of an organic electroluminescent device according to the invention has a lowermost excited singlet state energy level $E(S1^{n-H})$, wherein preferably: $E(S1^{n-H}) \geq 3.0$ eV.

According to the invention, an n-host $H^N$ optionally comprised in any of the at least one light-emitting layers B of an organic electroluminescent device according to the invention has a lowermost excited triplet state energy level $E(T1^{n-H})$, wherein preferably: $E(T1^{n-H}) \geq 2.7$ eV.

It is understood that any requirements or preferred properties previously defined for a host material $H^B$ comprised in any of the at least one light-emitting layers B of an organic electroluminescent device according to the invention are preferably also valid for an n-host $H^N$ according to the invention. Thus, in a preferred embodiment, the relations expressed by the following formulas (10) to (13) apply:

$$E(S1^{n-H}) > E(S1^{E}) \qquad (10)$$

$$E(S1^{n-H}) > E(S1^{S}) \qquad (11)$$

$$E(T1^{n-H}) > E(T1^{S}) \qquad (12)$$

$$E(T1^{n-H}) > E(T1^{E}) \qquad (13).$$

Accordingly, the lowermost excited singlet state $S1^{n-H}$ of an n-host $H^N$ is preferably higher in energy than the lowermost excited singlet state $S1^E$ of a TADF material $E^B$. The lowermost excited singlet state $S1^{n-H}$ of an n-host $H^N$ is preferably higher in energy than the lowermost excited singlet state $S1^S$ of any small FWHM emitter $S^B$. The lowermost excited triplet state $T1^{n-H}$ of an n-host $H^N$ is preferably higher in energy than the lowermost excited triplet state $T1^S$ of any small FWHM emitter $S^B$. Preferably, the lowermost excited triplet state $T1^{n-H}$ of any n-host $H^N$ is higher in energy than the lowermost excited triplet state $T1^E$ of any TADF material $E^B$.

In a preferred embodiment of the invention, an n-host $H^N$ optionally comprised in any of the at least one light-emitting layers B comprises or consists of a structure according to any of the formulas $H^N$-I, $H^N$-II, and $H^N$-III:

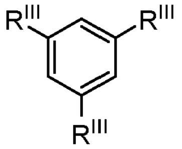

Formula $H^N$-I

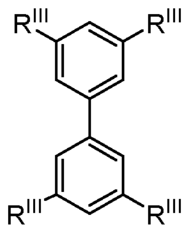

Formula $H^N$-II

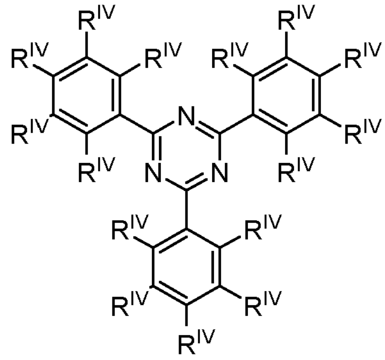

Formula $H^N$-III

wherein $R^{III}$ and $R^{IV}$ are at each occurrence independently of each other selected from the group consisting of: hydrogen, deuterium, Me, $^iPr$, $^tBu$, CN, $CF_3$,

Ph, which is optionally substituted with one or more substituents independently of each other selected from the group consisting of: Me, $^iPr$, $^tBu$, and Ph; and

a structure represented by any of the formulas $H^N$-IV, $H^N$-V, $H^N$-VI, $H^N$-VII, $H^N$-VIII, $H^N$-IX, $H^N$-X, $H^N$-XI, $H^N$-XII, $H^N$-XIII, and $H^N$-XIV:

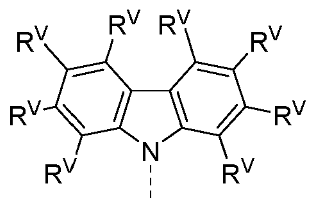

Formula $H^N$-IV

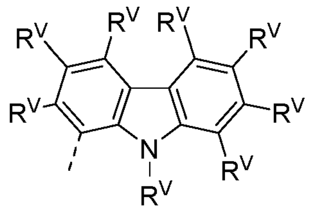

Formula $H^N$-V

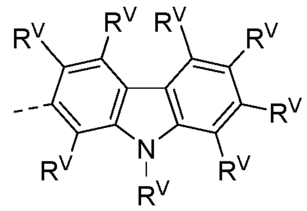

Formula $H^N$-VI

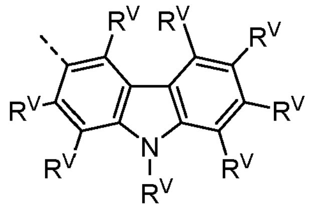

Formula $H^N$-VII

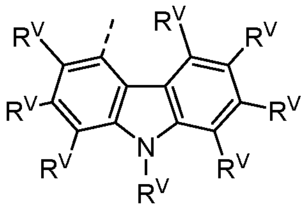

Formula $H^N$-VIII

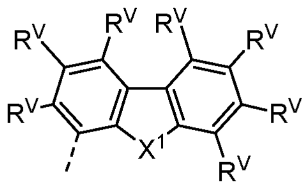

Formula $H^N$-IX

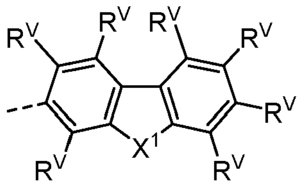

Formula $H^N$-X

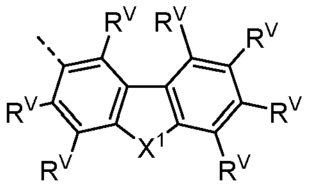

Formula $H^N$-XI

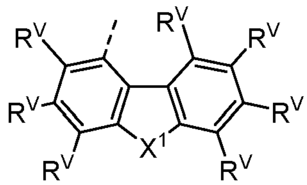

Formula $H^N$-XII

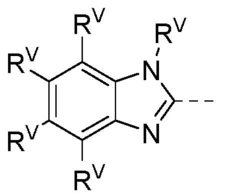

Formula $H^N$-XIII

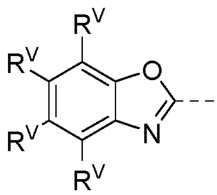

Formula $H^N$-XIV

wherein $X^1$ is oxygen (O), sulfur (S) or $C(R^V)_2$;

$R^V$ is at each occurrence independently of each other selected from the group consisting of: hydrogen, deuterium, Me, $^i$Pr, $^t$Bu, and

Ph, which is optionally substituted with one or more substituents independently of each other selected from the group consisting of: Me, $^i$Pr, $^t$Bu, and Ph;

wherein two or more adjacent substituents $R^V$ may optionally form an aromatic or heteroaromatic ring system with 3-18 carbon atoms; and

wherein in formulas $H^N$-I and $H^N$-II, at least one substituent $R^{III}$ is CN. ; and

the dashed line indicates the binding site to any of Formulas $H^N$-I, $H^N$-II, $H^N$-III.

In an even more preferred embodiment of the invention, the one or more n-host $H^N$ optionally comprised in an organic electroluminescent device according to the invention is selected from the group consisting of the following structures:

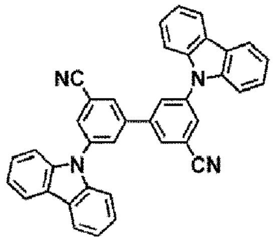
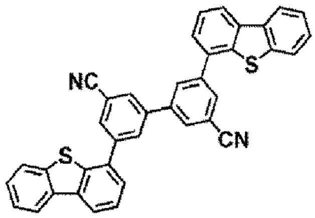
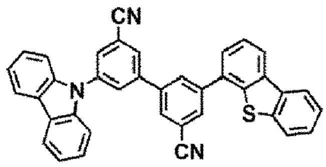

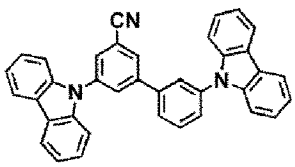
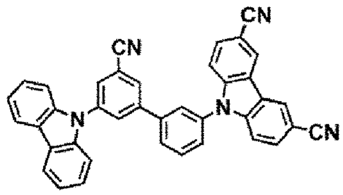
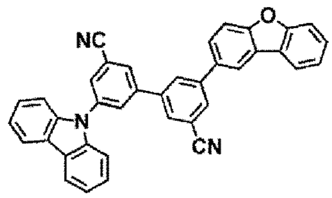

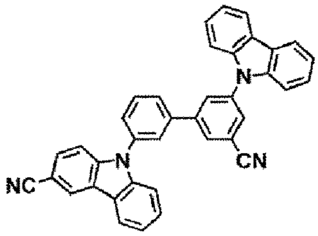
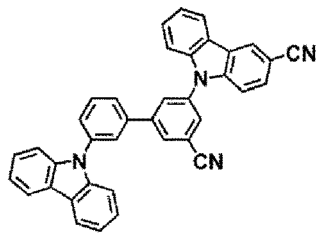

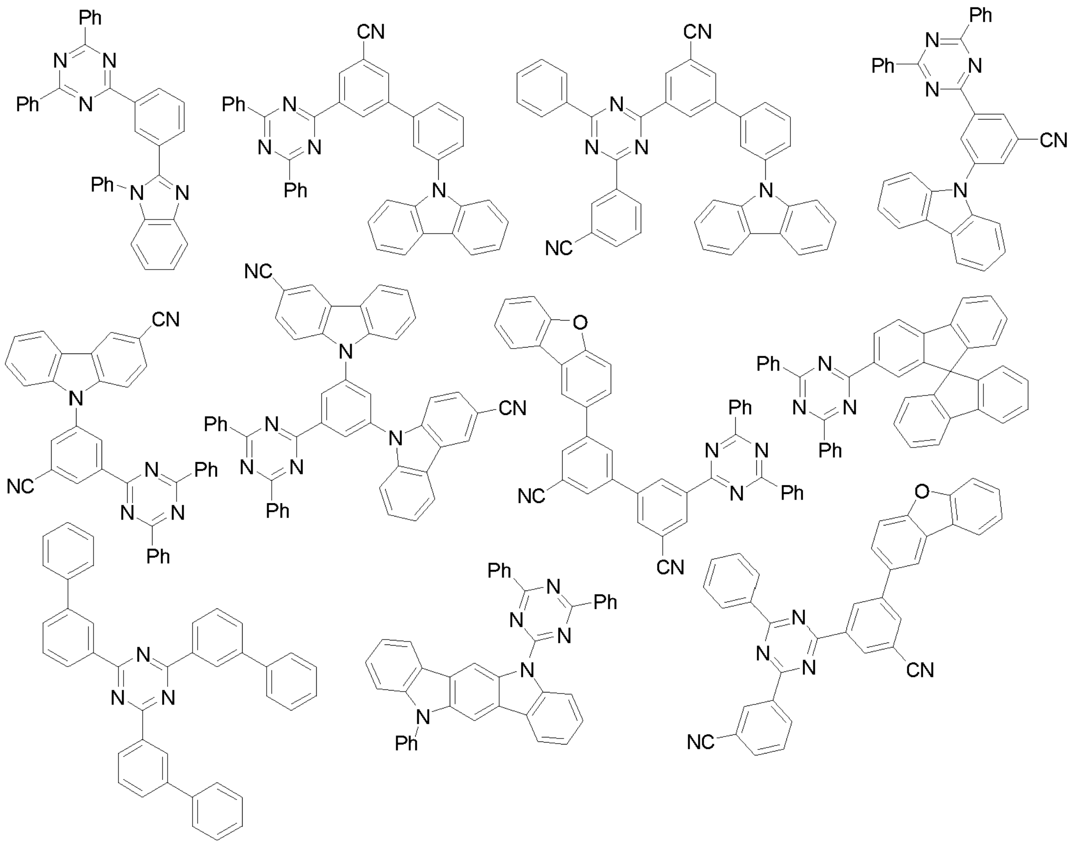

In one embodiment of the invention, no n-host $H^N$ comprised in at least one light-emitting layer B of an organic electroluminescent device according to the invention contains any phosphine oxide groups and, in particular, no n-host $H^N$ is bis[2-(diphenylphosphino)phenyl] ether oxide (DPEPO).

According to the invention, a bipolar host $H^{BP}$ optionally comprised in any of the at least one light-emitting layers B of an organic electroluminescent device according to the invention has a highest occupied molecular orbital HOMO($H^{BP}$) having an energy $E^{HOMO}(H^{BP})$, wherein preferably: -6.1 eV ≤ $E^{HOMO}(H^{BP})$ ≤ -5.6 eV.

According to the invention, a bipolar host $H^{BP}$ optionally comprised in any of the at least one light-emitting layers B of an organic electroluminescent device according to the invention has a lowest unoccupied molecular orbital LUMO($H^{BP}$) having an energy $E^{LUMO}(H^{BP})$, wherein preferably: -3.5 eV ≤ $E^{LUMO}(H^{BP})$ ≤ -2.9 eV.

According to the invention, a bipolar host $H^{BP}$ optionally comprised in any of the at least one light-emitting layers B of an organic electroluminescent device according to the invention has a lowermost excited singlet state energy level $E(S1^{bp-H})$, wherein preferably: $E(S1^{bp-H}) \geq 3.0$ eV.

According to the invention, abipolar host $H^{BP}$ optionally comprised in any of the at least one light-emitting layers B of an organic electroluminescent device according to the invention has a lowermost excited triplet state energy level $E(T1^{bP-H})$, wherein preferably: $E(T1^{bP-H}) \geq 2.7$ eV.

It is understood that any requirements or preferred properties previously defined for a host material $H^B$ comprised in any of the at least one light-emitting layers B of an organic electroluminescent device according to the invention are preferably also valid for a bipolar host $H^{BP}$ according to the invention. Thus, in a preferred embodiment, the relations expressed by the following formulas (14) to (17) apply:

$$E(S1^{bp-H}) > E(S1^{E}) \quad (14)$$

$$E(S1^{bp-H}) > E(S1^{S}) \quad (15)$$

$$E(T1^{bp-H}) > E(T1^{S}) \quad (16)$$

$$E(T1^{bp\text{-}H}) > E(T1^{E}) \qquad (17).$$

Accordingly, the lowermost excited singlet state $S1^{bp\text{-}H}$ of a bipolar host $H^{BP}$ is preferably higher in energy than the lowermost excited singlet state $S1^{E}$ of a TADF material $E^{B}$. The lowermost excited singlet state $S1^{bp\text{-}H}$ of a bipolar host $H^{BP}$ is preferably higher in energy than the lowermost excited singlet state $S1^{S}$ of any small FWHM emitter $S^{B}$. The lowermost excited triplet state $T1^{bp\text{-}H}$ of a bipolar host $H^{BP}$ is preferably higher in energy than the lowermost excited triplet state $T1^{S}$ of any small FWHM emitter $S^{B}$. Preferably, the lowermost excited triplet state $T1^{bp\text{-}H}$ of any bipolar host $H^{BP}$ is higher in energy than the lowermost excited triplet state $T1^{E}$ of any TADF material $E^{B}$.

*TADF material(s) $E^B$*

According to the invention, any of the one or more thermally activated delayed fluorescence (TADF) materials $E^{B}$ is preferably characterized by exhibiting a $\Delta E_{ST}$ value, which corresponds to the energy difference between the lowermost excited singlet state $S1^{E}$ and the lowermost excited triplet state $T1^{E}$, of less than 0.4 eV, preferably of less than 0.3 eV, more preferably of less than 0.2 eV, even more preferably of less than 0.1 eV, or even of less than 0.05 eV. Thus, $\Delta E_{ST}$ of a TADF material $E^{B}$ according to the invention is preferably sufficiently small to allow for thermal repopulation of the lowermost excited singlet state $S1^{E}$ to the lowermost excited triplet state $T1^{E}$ (also referred to as up-intersystem crossing or reverse intersystem crossing) at room temperature (RT).

It is understood that a small FWHM emitter $S^{B}$ comprised in the at least one light-emitting layer B of an organic electroluminescent device according to the invention may optionally also have a $\Delta E_{ST}$ value of less than 0.4 eV and exhibit thermally activated delayed fluorescence (TADF). However, for any small FWHM emitter $S^{B}$ in the context of the invention, this is only an optional feature. Additionally, a TADF material $E^{B}$ in the context of the invention preferably differs from a small FWHM emitter $S^{B}$ in the context of the invention in that a TADF material $E^{B}$ mainly functions as energy pump transferring energy to at least one small FWHM emitter $S^{B}$ while the main contribution to the emission band of the optoelectronic device according to the invention can preferably be attributed to the emission of at least one small FWHM emitter $S^{B}$.

According to the invention, a TADF material $E^{B}$ comprised in any of the at least one light-emitting layers B of an organic electroluminescent device according to the invention has a highest occupied molecular orbital HOMO($E^{B}$) having an energy $E^{HOMO}(E^{B})$, wherein preferably: $-6.0\ eV \leq E^{HOMO}(E^{B}) \leq -5.8\ eV$.

According to the invention, a TADF material $E^{B}$ comprised in any of the at least one light-emitting layers B of an organic electroluminescent device according to the invention has a lowest unoccupied molecular orbital LUMO($E^{B}$) having an energy $E^{LUMO}(E^{B})$, wherein preferably: $-3.4\ eV \leq E^{LUMO}(E^{B}) \leq -3.0\ eV$.

According to the invention, a TADF material $E^{B}$ comprised in any of the at least one light-emitting layers B of an organic electroluminescent device according to the invention has a lowermost excited singlet state energy level $E(S1^{E})$, wherein preferably: $2.5\ eV \leq E(S1^{E}) \leq 2.8\ eV$.

According to the invention, a TADF material $E^{B}$ comprised in any of the at least one light-emitting layers B of an organic electroluminescent device according to the invention has a lowermost excited triplet state energy level $E(T1^{E})$, whose preferred range may be defined by the above-mentioned preferred range for the singlet state energy level $E(S1^{E})$ in combination with the above-mentioned preferred range for $\Delta E_{ST}$.

In a preferred embodiment of the invention, a TADF material $E^{B}$ has an emission maximum in the wavelength range of 480 nm to 560 nm, preferably of 500 nm to 540 nm.

In a preferred embodiment of the invention, a TADF material $E^{B}$ is an organic TADF material, which, in the context of the invention, means that it does not contain any transition metals. Preferably, a TADF material $E^{B}$ according to the invention predominantly consists of the elements hydrogen (H), carbon (C), and nitrogen (N), but may for example also comprise oxygen (O), boron (B), silicon (Si), fluorine (F), and bromine (Br).

In one embodiment of the invention, each of the at least one TADF materials $E^{B}$ comprises at least one electron-donating moiety D (i.e., donor) and at least one electron-withdrawing moiety A (i.e., acceptor), wherein the at least one donor D and the at least one acceptor A are covalently attached to the same linker; wherein this linker is an aromatic or heteroaromatic group with 3 to 30 carbon atoms, most preferably benzene or biphenyl.

In a preferred embodiment of the invention, each moiety D each comprises or consist of a structure represented by any of the formulas shown below:

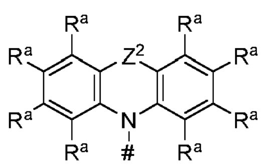 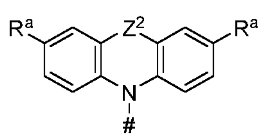 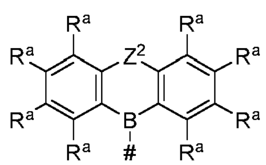 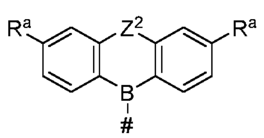

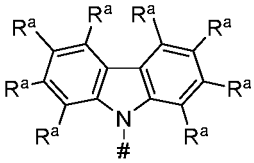 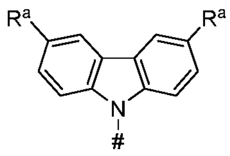 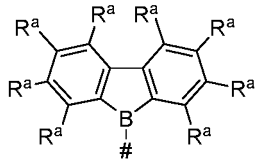 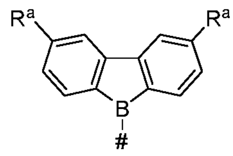

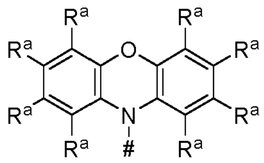 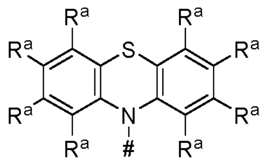 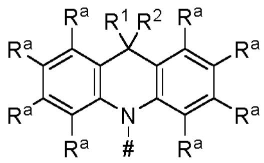 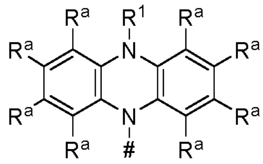

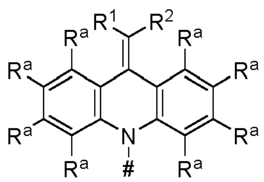 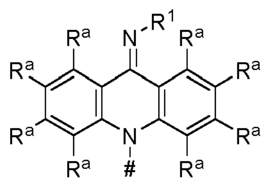 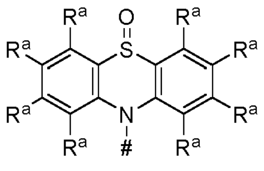 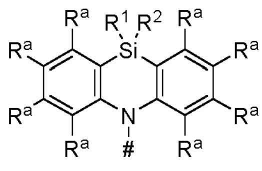

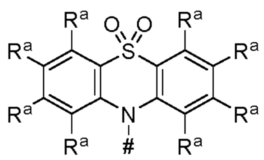 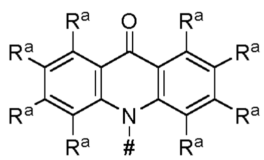 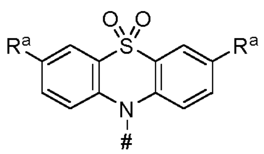 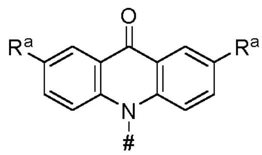

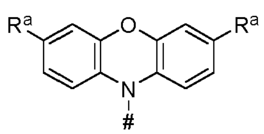 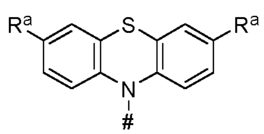 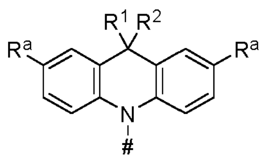 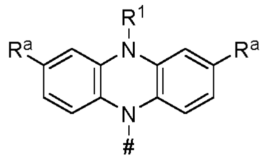

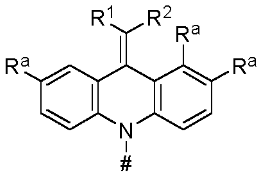 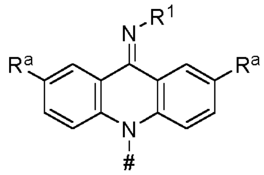 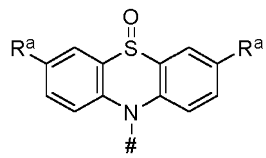 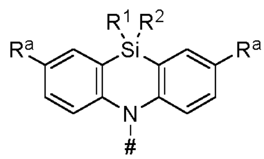

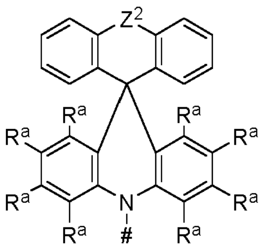 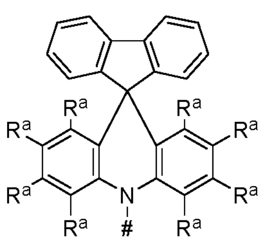 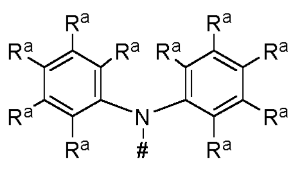 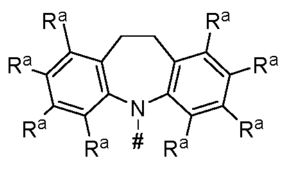

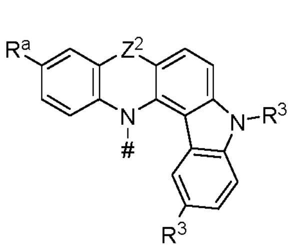 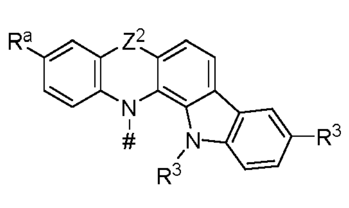 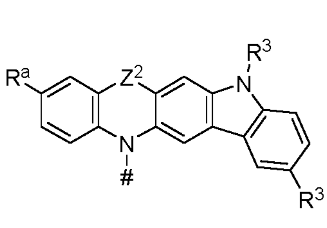

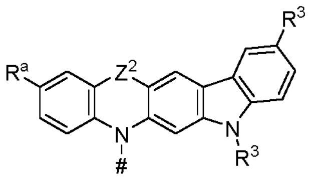
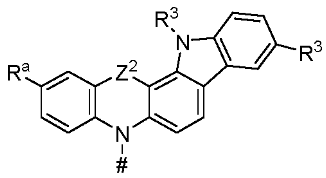
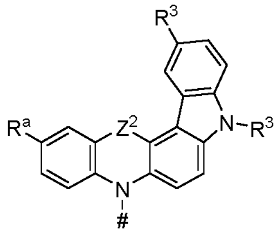

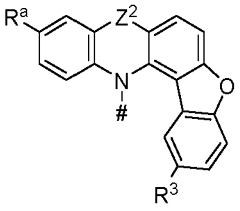
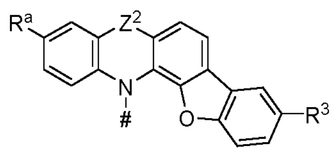
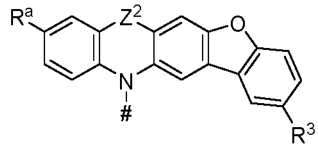

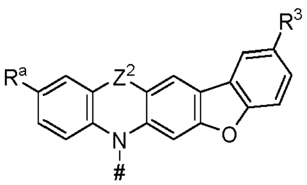
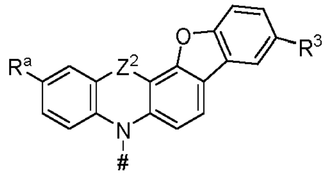
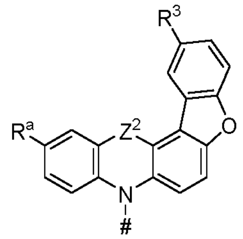

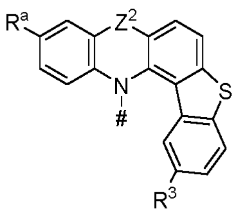
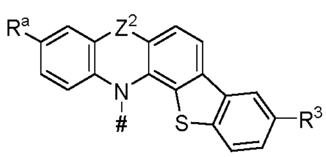
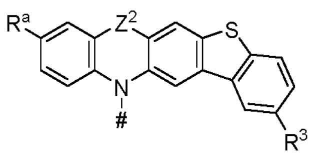

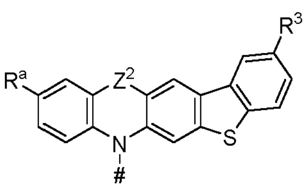
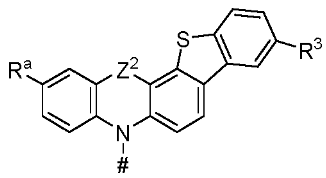
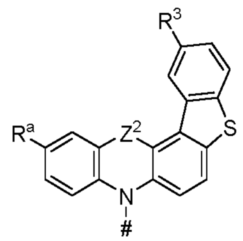

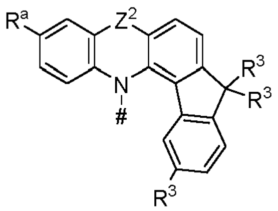
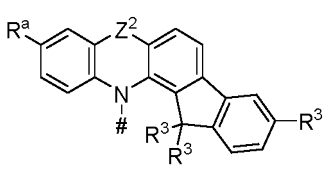
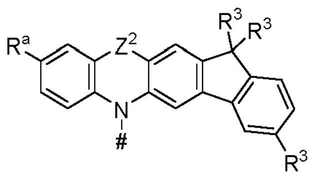

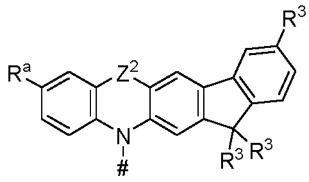
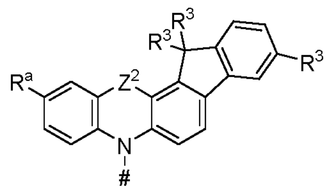
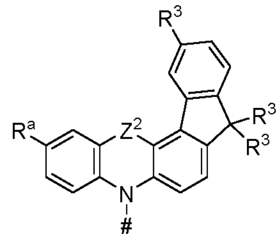

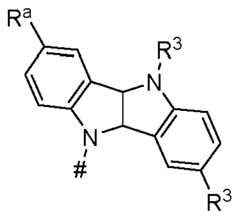
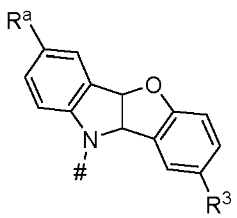
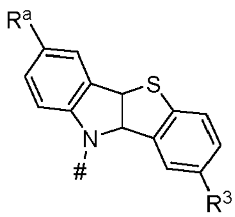
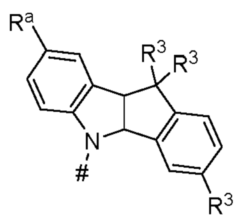

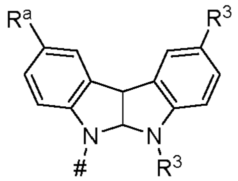
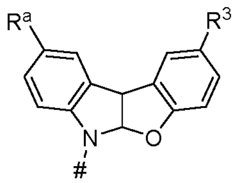
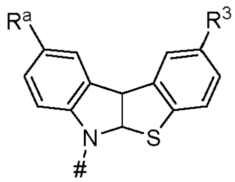
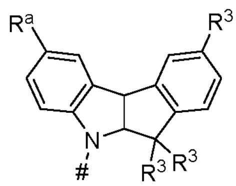

wherein

$Z^2$ is at each occurrence independently of each other selected from the group consisting of a direct bond, $CR^1R^2$, $C=CR^1R^2$, C=O, $C=NR^1$, $NR^1$, O, $SiR^1R^2$, S, S(O) and $S(O)_2$;
# represents the binding site of a donor moiety D to the aforementioned linker;
$R^a$, $R^1$, and $R^2$ are at each occurrence independently of each other selected from the group consisting of: hydrogen, deuterium, $N(R^3)_2$, $OR^3$, $Si(R^3)_3$, $B(OR^3)_2$, $OSO_2R^3$, $CF_3$, CN, F, Cl, Br, I,
$C_1$-$C_{40}$-alkyl,

which is optionally substituted with one or more substituents $R^3$ and
wherein one or more non-adjacent $CH_2$-groups are optionally substituted by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$;

$C_1$-$C_{40}$-alkoxy,

which is optionally substituted with one or more substituents $R^3$ and
wherein one or more non-adjacent $CH_2$-groups are optionally substituted by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$;

$C_1$-$C_{40}$-thioalkoxy,

which is optionally substituted with one or more substituents $R^3$ and
wherein one or more non-adjacent $CH_2$-groups are optionally substituted by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$;

$C_2$-$C_{40}$-alkenyl,

which is optionally substituted with one or more substituents $R^3$ and
wherein one or more non-adjacent $CH_2$-groups are optionally substituted by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$;

$C_2$-$C_{40}$-alkynyl,

which is optionally substituted with one or more substituents $R^3$ and

wherein one or more non-adjacent $CH_2$-groups are optionally substituted by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$;

$C_6$-$C_{60}$-aryl,
which is optionally substituted with one or more substituents $R^3$; and
$C_3$-$C_{57}$-heteroaryl,
which is optionally substituted with one or more substituents $R^3$;
$R^3$ is at each occurrence independently of each other selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$, and
$N(C_3$-$C_{17}$-heteroaryl$)(C_6$-$C_{18}$-aryl$)$;
wherein, optionally, any of the substituents $R^a$, $R^1$, and $R^2$ may independently of each other form a mono- or polycyclic, aliphatic, aromatic and/or benzo-fused ring system with one or more adjacent substituents $R^a$, $R^1$, and $R^2$, wherein one or more hydrogen atoms of the optionally so formed ring system may be substituted by $R^3$.

In a preferred embodiment of the invention, the donor moiety D is not an unsubsituted carbazole.

In a preferred embodiment of the invention, each acceptor moiety A each comprises or consists of a structure represented by any of the formulas shown below:

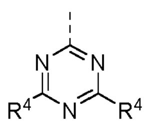
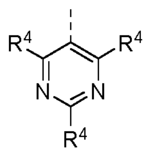
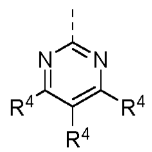
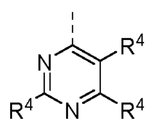
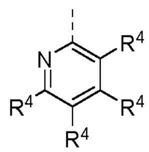
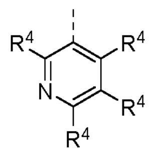

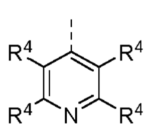
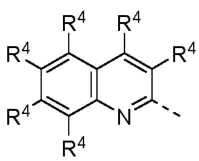
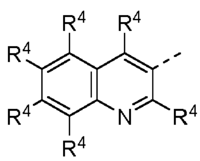
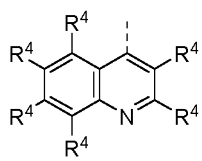

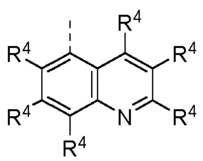
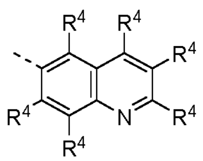
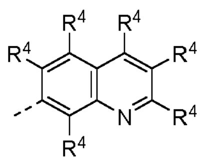
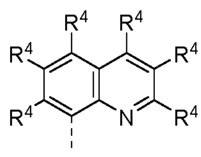

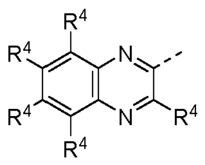
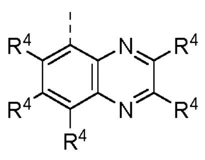
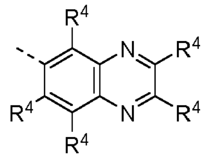
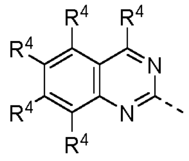
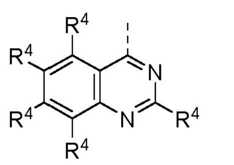
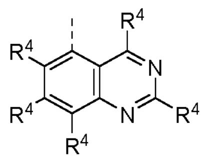
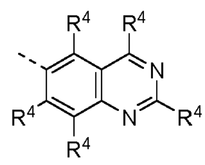
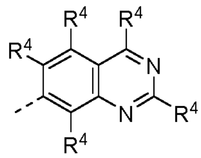
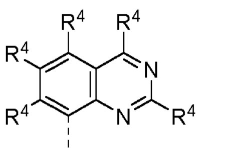
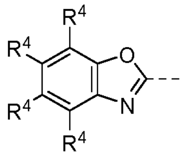
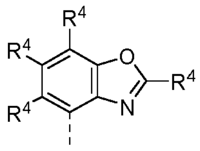
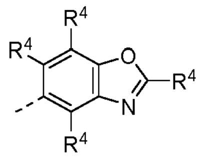
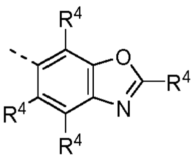
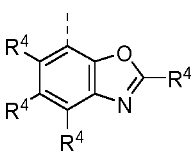
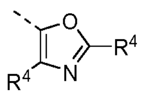
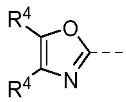
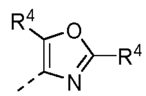
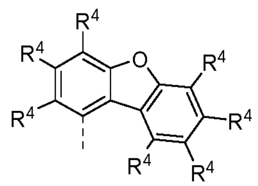
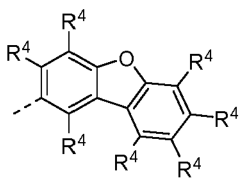
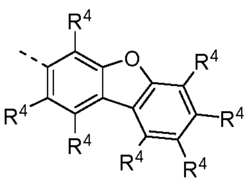
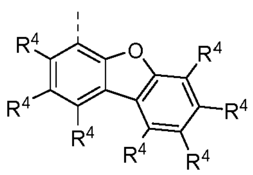
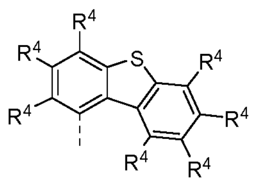
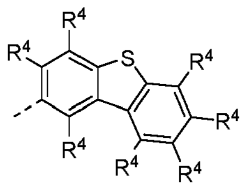
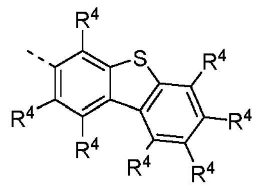
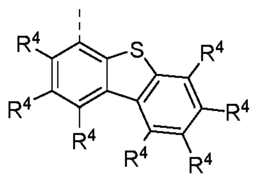
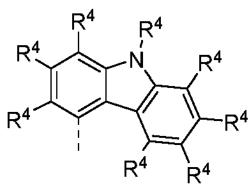
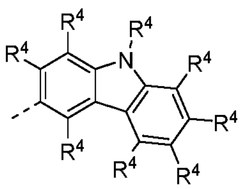
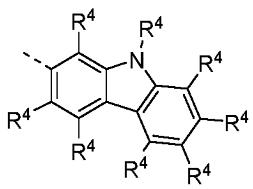
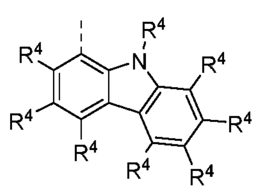
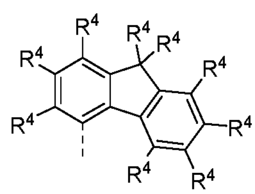

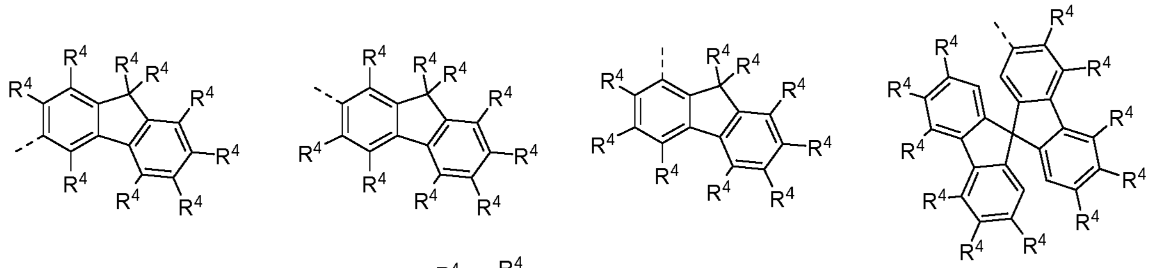

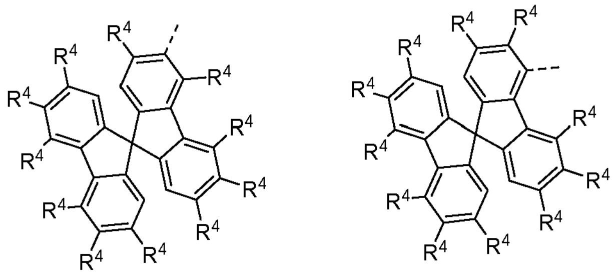

-CN

-Cl

$-CF_3$

-F

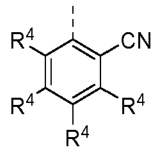 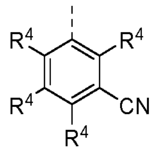 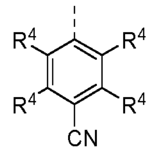 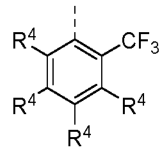 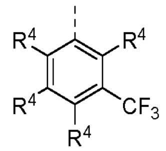 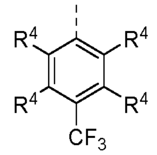

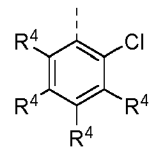 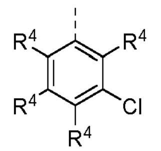 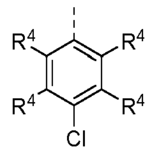 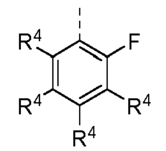 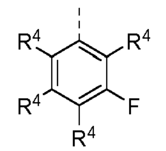 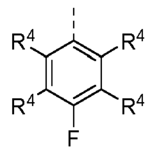

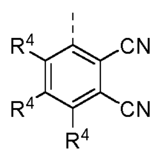 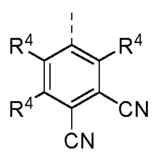 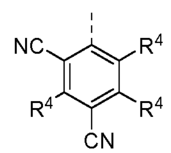 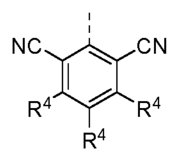 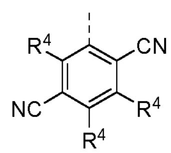 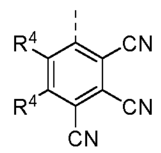

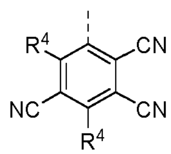 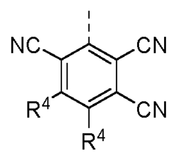 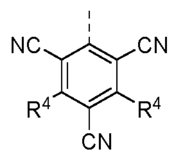 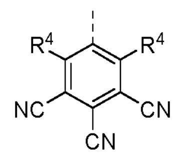 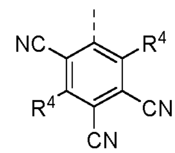 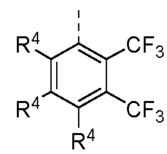

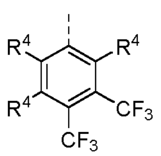 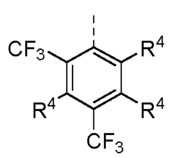 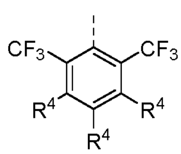 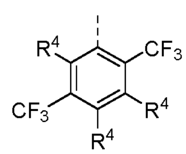 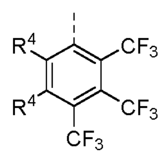 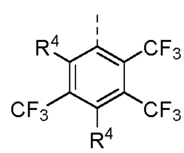

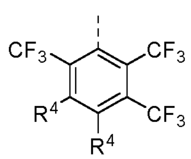 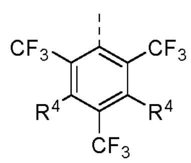 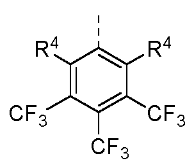 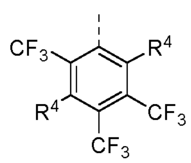 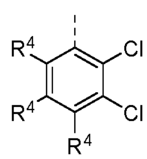 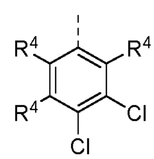

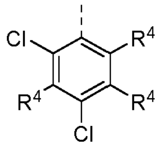 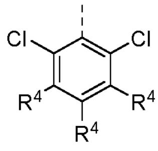 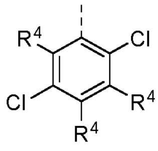 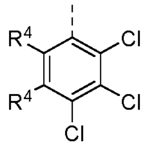 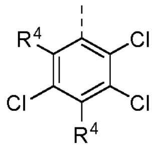 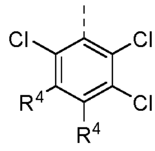

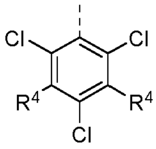 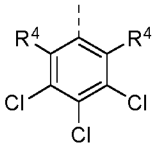 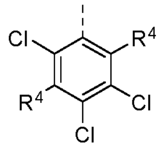 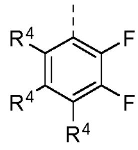 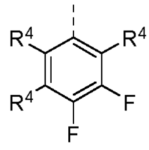 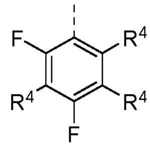

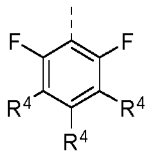 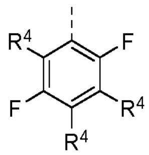 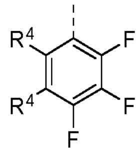 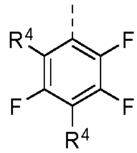 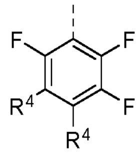 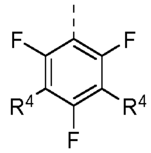

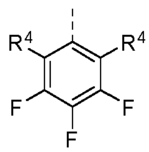 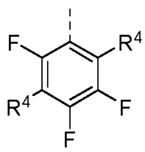

wherein

the dashed line represents a single bond linking the acceptor moiety A to the aforementioned linker;
$R^4$ is at each occurrence independently of each other selected from the group consisting of: hydrogen, deuterium, $N(R^5)_2$, $OR^5$, $Si(R^5)_3$, $B(OR^5)_2$, $OSO_2R^5$, $CF_3$, CN, F, Cl, Br, I,
$C_1$-$C_{40}$-alkyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
$R^5$ is at each occurrence independently of each other selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy, wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents; $N(C_6$-$C_{18}$-aryl$)_2$;
$N(C_3$-$C_{17}$-heteroaryl$)_2$, and
$N(C_3$-$C_{17}$-heteroaryl$)(C_6$-$C_{18}$-aryl$)$;
wherein, optionally, two or more adjacent substituents $R^4$ may independently of each other form a mono- or polycyclic, aliphatic, aromatic and/or benzo-fused ring system, wherein one or more hydrogen atoms of the optionally so formed ring system may be substituted by $R^5$.

In an even more preferred embodiment of the invention, each of the at least one TADF materials $E^B$ comprises at least one electron-donating moiety D (i.e., donor) and at least one electron-withdrawing moiety A (i.e., acceptor), wherein all donors D and all acceptors A are covalently attached to the same same linker;

wherein this linker is an aromatic or heteroaromatic group with 3 to 30 carbon atoms, most preferably benzene or biphenyl;
wherein, the above-mentioned donor moieties D each comprise or consist of a structure represented by any of the formulas shown above; and
wherein, the above-mentioned acceptor moieties A each comprise or consist of a structure represented by any of the formulas shown above; and
wherein $R^1$ and $R^2$ are at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^3$; and
$C_3$-$C_{17}$-heteroaryl,
which is optionally substituted with one or more substituents $R^3$;
$R^4$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^5$; and
$C_3$-$C_{17}$-heteroaryl,
which is optionally substituted with one or more substituents $R^5$;
$R^3$ and $R^5$ are at each occurrence independently of each other selected from the group consisting of: hydrogen, deuterium, Me, $^i$Pr, $^t$Bu,CN, $CF_3$, and

phenyl, which is optionally substituted with one or more substituents independently of each other selected from the group consisting of Me, $^iPr$, $^tBu$, CN, $CF_3$ and Ph; and

$R^a$ is at each occurrence independently of each other selected from the group consisting of: hydrogen, Me, $^iPr$, $^tBu$, CN, $CF_3$,

Ph, which is optionally substituted with one or more substituents independently from each other selected from the group consisting of Me, 'Pr, $^tBu$, CN, $CF_3$, and Ph,

pyridinyl, which is optionally substituted with one or more substituents independently from each other selected from the group consisting of Me, $^iPr$, $^tBu$, CN, $CF_3$, and Ph,

pyrimidinyl, which is optionally substituted with one or more substituents independently from each other selected from the group consisting of Me, $^iPr$, $^tBu$, CN, $CF_3$, and Ph,

carbazolyl, which is optionally substituted with one or more substituents independently from each other selected from the group consisting of Me, $^iPr$, $^tBu$, CN, $CF_3$, and Ph,

triazinyl, which is optionally substituted with one or more substituents independently from each other selected from the group consisting of Me, $^iPr$, $^tBu$, CN, $CF_3$, and Ph;

and $N(Ph)_2$;

In a still even more preferred embodiment of the invention, each of the at least one TADF materials $E^B$ comprises at least one electron-donating moiety D (i.e., donor) and at least one electron-withdrawing moiety A (i.e., acceptor), wherein all donors D and all acceptors A are covalently attached to the same same linker;

wherein this linker is an aromatic or heteroaromatic group with 3 to 30 carbon atoms, most preferably benzene or biphenyl;

wherein, the above-mentioned donor moieties D each comprise or consist of a structure represented by any of the formulas shown above; and

wherein, the above-mentioned acceptor moieties A each comprise or consist of a structure represented by any of the formulas shown above; and

wherein $R^1$, $R^2$, $R^3$, and $R^4$ are at each occurrence independently of each other selected from the group consisting of: hydrogen, deuterium, Me, 'Pr, $^tBu$,CN, $CF_3$, and

phenyl, which is optionally substituted with one or more substituents independently of each other selected from the group consisting of Me, $^iPr$, $^tBu$, CN, $CF_3$ and Ph;

$R^a$ is at each occurrence independently of each other selected from the group consisting of: hydrogen, Me, $^iPr$, $^tBu$, CN, $CF_3$,

Ph, which is optionally substituted with one or more substituents independently of each other selected from the group consisting of Me, $^iPr$, $^tBu$, CN, $CF_3$, and Ph; and

triazinyl, which is optionally substituted with one or more substituents independently of each other selected from the group consisting of Me, $^iPr$, $^tBu$, CN, $CF_3$, and Ph.

In one embodiment of the invention, each of the at least one TADF materials $E^B$ is an organic TADF material comprising:

- one or more first chemical moieties $R^6$, each independently comprising or consisting of a structure of formula $E^B$-I,

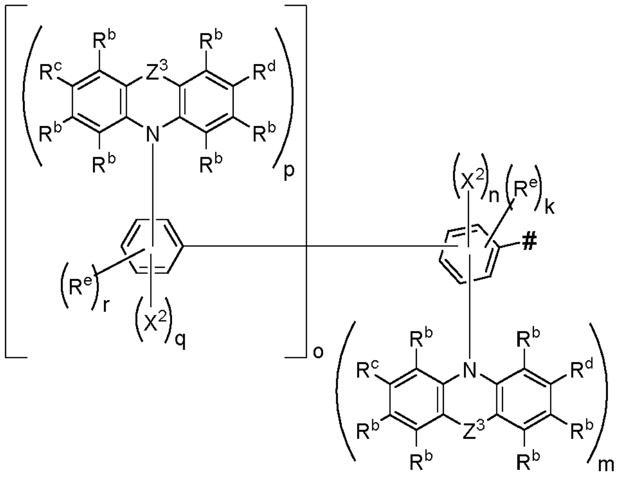

Formula $E^B$-I

and

- optionally one second chemical moiety comprising or consisting of a structure according to any of formulas $E^B$-II and $E^B$-III,

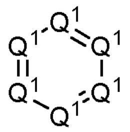

Formula $E^B$-II

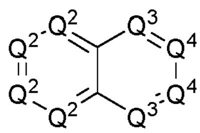

Formula $E^B$-III

wherein each second chemical moiety (if present) is linked to a first chemical moiety $R^6$ via a single bond;
wherein
# represents the binding site of a first chemical moiety $R^6$ to a second chemical moiety or is hydrogen;
k is at each occurrence independently of each other 0, 1, 2, 3, or 4;
m is at each occurrence independently of each other 0, 1 or 2;
n is at each occurrence independently of each other 0, 1 or 2;
o is at each occurrence independently of each other 0 or 1;
p is at each occurrence independently of each other 0, 1 or 2;
q is at each occurrence independently of each other 0, 1 or 2;
r is at each occurrence independently of each other 0, 1, 2, 3, 4 or 5;
$Q^1$ is at each occurrence independently of each other selected from the group consisting of N, $CR^6$, and $CR^7$;
$Q^2$ is at each occurrence independently of each other selected from the group consisting of C-$Ar^{EWG}$ and $CR^{Q2}$;
$Q^3$ is at each occurrence independently of each other selected from the group consisting of N, C-$Ar^{EWG}$ and $CR^{Q2}$;
$Q^4$ is at each occurrence independently of each other selected from the group consisting of $CR^6$, C-$Ar^{EWG}$ and $CR^{Q2}$;
$X^2$ is at each occurrence independently of each other selected from the group consisting of $Ar^{EWG}$, CN, and $CF_3$;
$Ar^{EWG}$ is at each occurrence independently of each other represented by a structure according to any of the formulas $Ar^{EWG}$-I, $Ar^{EWG}$-II, $Ar^{EWG}$-III, $Ar^{EWG}$-IV, $Ar^{EWG}$-V, $Ar^{EWG}$-VI, $Ar^{EWG}$-VII, $Ar^{EWG}$-VIII, $Ar^{EWG}$-IX, $Ar^{EWG}$-X, $Ar^{EWG}$-XI, $Ar^{EWG}$-XII, $Ar^{EWG}$-XIII, and $Ar^{EWG}$-XIV,

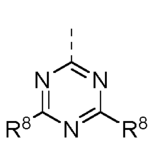

Formula $Ar^{EWG}$-I

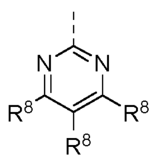

Formula $Ar^{EWG}$-II

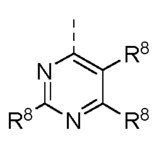

Formula $Ar^{EWG}$-III

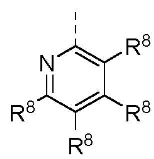

Formula $Ar^{EWG}$-IV

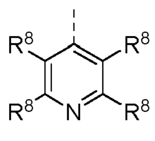
Formula $Ar^{EWG}$-V

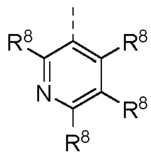
Formula $Ar^{EWG}$-VI

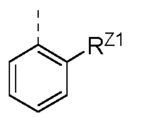
Formula $Ar^{EWG}$-VII

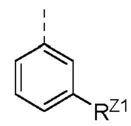
Formula $Ar^{EWG}$-VIII

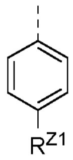
Formula $Ar^{EWG}$-IX

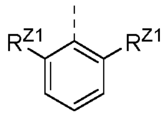
Formula $Ar^{EWG}$-X

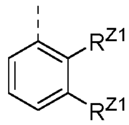
Formula $Ar^{EWG}$-XI

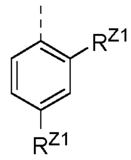
Formula $Ar^{EWG}$-XII

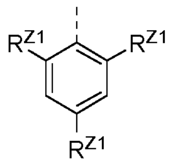
Formula $Ar^{EWG}$-XIII

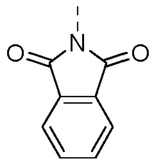
Formula $Ar^{EWG}$-XIV

which are bonded to the core structure (here preferably formula $E^B$-I) via the position marked by the dashed line;
$R^{Z1}$ is at each occurrence independently of each other selected from the group consisting of CN and $CF_3$;
$Z^3$ is at each occurrence independently of each other selected from the group consisting of a direct bond, $CR^9R^{10}$, $C=CR^9R^{10}$, C=O, $C=NR^9$, $NR^9$, O, $SiR^9R^{10}$, S, S(O) and $S(O)_2$;
$R^e$ is at each occurrence independently of each other selected from the group consisting of: hydrogen, deuterium, F, Cl, Br, and I,
$C_1$-$C_5$-alkyl,
wherein one or more hydrogen atoms are optionally substituted by deuterium;
$C_6$-$C_{18}$-aryl,
which is optionally substituted with one or more substituents independently of each other selected from the group consisting of: deuterium, $C_1$-$C_5$-alkyl groups, $C_6$-$C_{18}$-aryl groups, F, Cl, Br, and I;
$R^7$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^{11}$; and
$C_3$-$C_{17}$-heteroaryl,
which is optionally substituted with one or more substituents $R^{11}$;
$R^8$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^{11}$; and
$C_3$-$C_{17}$-heteroaryl,
which is optionally substituted with one or more substituents $R^{11}$;
$R^{Q2}$ is at is at each occurrence independently of each other selected from the group consisting of: hydrogen, deuterium, Me, $^i$Pr, $^t$Bu, CN, $CF_3$, Ph, and carbazolyl, which is optionally substituted with one or more substituents independently from each other selected from the group consisting of Me, $^i$Pr, $^t$Bu, CN, $CF_3$, Ph, and $N(Ph)_2$;
$R^b$, $R^c$, $R^d$, $R^9$, and $R^{10}$ are at each occurrence independently of each other selected from the group consisting of: hydrogen, deuterium, $N(R^{12})_2$, $OR^{12}$, $Si(R^{12})_3$, $B(OR^{12})_2$, $OSO_2R^{12}$, $CF_3$, CN, F, Br, I,
$C_1$-$C_{40}$-alkyl,

which is optionally substituted with one or more substituents $R^{12}$ and

wherein one or more non-adjacent $CH_2$-groups are optionally substituted by $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_1$-$C_{40}$-alkoxy,

which is optionally substituted with one or more substituents $R^{12}$ and wherein one or more non-adjacent $CH_2$-groups are optionally substituted by $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_1$-$C_{40}$-thioalkoxy,

which is optionally substituted with one or more substituents $R^{12}$ and wherein one or more non-adjacent $CH_2$-groups are optionally substituted by $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_2$-$C_{40}$-alkenyl,

which is optionally substituted with one or more substituents $R^{12}$ and wherein one or more non-adjacent $CH_2$-groups are optionally substituted by $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_2$-$C_{40}$-alkynyl,

which is optionally substituted with one or more substituents $R^{12}$ and wherein one or more non-adjacent $CH_2$-groups are optionally substituted by $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_6$-$C_{60}$-aryl,
which is optionally substituted with one or more substituents $R^{12}$; and
$C_3$-$C_{57}$-heteroaryl,
which is optionally substituted with one or more substituents $R^{12}$;
$R^{11}$ is at each occurrence independently of each other selected from the group consisting of: hydrogen, deuterium, Me, $^iPr$, $^tBu$, CN, $CF_3$, and
phenyl, which is optionally substituted with one or more substituents independently of each other selected from the group consisting of Me, $^iPr$, $^tBu$, CN, $CF_3$ and Ph;
$R^{12}$ is at each occurrence independently of each other selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6\text{-}C_{18}\text{-aryl})_2$;
$N(C_3\text{-}C_{17}\text{-heteroaryl})_2$,

$N(C_3-C_{17}$-heteroaryl)($C_6-C_{18}$-aryl), and
an aliphatic cyclic amino group comprising 5 to 8 carbon atoms (preferably pyrrolidinyl and piperidinyl);
wherein, optionally, any adjacent substituents $R^b$, $R^c$, $R^d$, $R^9$, and $R^{10}$ independently of each other form a mono- or polycyclic, aliphatic, aromatic and/or benzo-fused ring system; wherein one or more hydrogen atoms of the optionally so formed ring system may be substituted by $R^{12}$; and
wherein at least one, but not more than three groups $Q^1$ are nitrogen (N), with the restriction that in formula $E^B$-II two adjacent groups $Q^1$ are not both N; and
at least one, but not more than three groups $Q^1$ are $CR^6$, with the restriction that in formula $E^B$-II, two adjacent groups $Q^1$ are not both $CR^6$; and
wherein in formula $E^B$-III, at least one group $Q^3$ is nitrogen (N); and
wherein preferably: $1 \leq (m + p) \leq 4$; and

$$1 \leq (n + q) \leq 4;$$

and

$$1 \leq (m + n + o) \leq 5.$$

In a preferred embodiment of the invention, at least one substituent of the group consisting of $R^b$, $R^c$, and $R^d$ is not hydrogen, whenever $Z^3$ is a direct bond, so that preferably no donor moiety within a TADF material $E^B$ is an unsubstituted carbazolyl group.

In a preferred embodiment of the invention,

$R^7$ is at each occurrence independently of each other selected from the group consisting of hydrogen, deuterium, Me, $^i$Pr, $^t$Bu, CN, $CF_3$, and
Ph, which is optionally substituted with one or more substituents independently of each other selected from the group consisting of hydrogen, deuterium, Me, $^i$Pr, $^t$Bu, CN, $CF_3$, and phenyl;
$R^8$ is at each occurrence independently of each other selected from the group consisting of hydrogen, deuterium, Me, $^i$Pr, $^t$Bu, CN, $CF_3$, and
Ph, which is optionally substituted with one or more substituents independently of each other selected from the group consisting of hydrogen, deuterium, Me, $^i$Pr, $^t$Bu, CN, $CF_3$, and phenyl;
$R^{Q2}$ is at is at each occurrence independently of each other selected from the group consisting of: hydrogen, deuterium, Ph,
carbazolyl, which is optionally substituted with one or more substituents independently from each other selected from the group consisting of Me, $^i$Pr, $^t$Bu, CN, $CF_3$, Ph, and $N(Ph)_2$;
$R^b$, $R^c$, and $R^d$ are at each occurrence independently from another selected from the group consisting of: hydrogen, Me, $^i$Pr, $^t$Bu, CN, $CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$;
$R^e$ is at each occurrence independently of each other selected from the group consisting of: hydrogen, deuterium, $C_1-C_5$-alkyl,
wherein one or more hydrogen atoms are optionally substituted by deuterium;
$C_6-C_{18}$-aryl,
which is optionally substituted with one or more $C_1-C_5$-alkyl groups and/or one or more $C_6-C_{18}$-aryl groups;
wherein, optionally, any adjacent substituents $R^b$, $R^c$, $R^d$, $R^9$, and $R^{10}$ independently of each other form a mono- or polycyclic, aliphatic, aromatic and/or benzo-fused ring system comprising 3 to 30 carbon atoms; and
wherein apart from that the above-mentioned definitions apply.

In an even more preferred embodiment of the invention, $Z^3$ is at each occurrence a direct bond; and

$R^7$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^{11}$; and
$C_3$-$C_{17}$-heteroaryl, which is optionally substituted with one or more substituents $R^{11}$;
$R^8$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^{11}$; and
$C_3$-$C_{17}$-heteroaryl,
which is optionally substituted with one or more substituents $R^{11}$.is at each occurrence independently of each other selected from the group consisting of hydrogen, deuterium, Me, $^i$Pr, $^t$Bu, CN, $CF_3$, and
Ph, which is optionally substituted with one or more substituents independently of each other selected from the group consisting of hydrogen, deuterium, Me, $^i$Pr, $^t$Bu, CN, $CF_3$, and phenyl;
$R^{Q2}$ is at is at each occurrence independently of each other selected from the group consisting of: hydrogen, deuterium, Ph, and
carbazolyl, which is optionally substituted with one or more substituents independently from each other selected from the group consisting of Ph and $N(Ph)_2$;
$R^b$, $R^c$, and $R^d$ are at each occurrence independently from another selected from the group consisting of: hydrogen, Me, $^i$Pr, $^t$Bu, CN, $CF_3$, and
Ph, which is optionally substituted with one or more substituents independently from each other selected from the group consisting of Me, $^i$Pr, $^t$Bu, CN, $CF_3$, and Ph;
carbazolyl, which is optionally substituted with one or more substituents independently from each other selected from the group consisting of Me, $^i$Pr, $^t$Bu, CN, $CF_3$, and Ph;
triazinyl, which is optionally substituted with one or more substituents independently from each other selected from the group consisting of Me, $^i$Pr, $^t$Bu, CN, $CF_3$, and Ph; and
$R^e$ is at each occurrence independently of each other selected from the group consisting of: hydrogen, deuterium, Me, $^i$Pr, $^t$Bu, and
$C_6$-$C_{18}$-aryl,
which is optionally substituted with one or more substituents selected from the group consisting of deuterium, Me, $^i$Pr, $^t$Bu;
wherein, optionally, any adjacent substituents $R^b$, $R^c$, $R^d$, $R^9$, and $R^{10}$ independently of each other form a mono- or polycyclic, aliphatic, aromatic and/or benzo-fused ring system comprising 3 to 18 carbon atoms; and
wherein apart from that the above-mentioned definitions apply.

In a still even more preferred embodiment of the invention, $Z^3$ is at each occurrence a direct bond; and

$R^7$ is at each occurrence independently of each other selected from the group consisting of hydrogen, deuterium, Me, $^i$Pr, $^t$Bu, CN, $CF_3$, and
Ph, which is optionally substituted with one or more substituents independently of each other selected from the group consisting of hydrogen, deuterium, Me, $^i$Pr, $^t$Bu, CN, $CF_3$, and phenyl;
$R^8$ is at each occurrence independently of each other selected from the group consisting of hydrogen, deuterium, Me, $^i$Pr, $^t$Bu, CN, $CF_3$, and
Ph, which is optionally substituted with one or more substituents independently of each other selected from the group consisting of hydrogen, deuterium, Me, $^i$Pr, $^t$Bu, CN, $CF_3$, and phenyl;
$R^{Q2}$ is at is at each occurrence independently of each other selected from the group consisting of: hydrogen, deuterium, and
carbazolyl, which is optionally substituted with one or more substituents independently from each other selected from the group consisting of Ph and $N(Ph)_2$;
$R^b$ is at each occurrence hydrogen;
$R^c$ and $R^d$ are at each occurrence independently of each other selected from the group consisting of: hydrogen, Me,

$^iPr$, $^tBu$, CN, $CF_3$,
Ph, which is optionally substituted with one or more substituents independently from each other selected from the group consisting of Me, $^iPr$, $^tBu$, CN, $CF_3$, and Ph,
pyridinyl, which is optionally substituted with one or more substituents independently from each other selected from the group consisting of Me, $^iPr$, $^tBu$, CN, $CF_3$, and Ph,
pyrimidinyl, which is optionally substituted with one or more substituents independently from each other selected from the group consisting of Me, $^iPr$, $^tBu$, CN, $CF_3$, and Ph,
carbazolyl, which is optionally substituted with one or more substituents independently from each other selected from the group consisting of Me, $^iPr$, $^tBu$, CN, $CF_3$, and Ph,
triazinyl, which is optionally substituted with one or more substituents independently from each other selected from the group consisting of Me, $^iPr$, $^tBu$, CN, $CF_3$, and Ph,
and $N(Ph)_2$;
$R^e$ is at each occurrence independently of each other selected from the group consisting of: hydrogen, deuterium, Me, $^iPr$, $^tBu$, and
Ph, which is optionally substituted with one or more substituents selected from the group consisting of deuterium, Me, $^iPr$, $^tBu$; and
wherein apart from that the above-mentioned definitions apply.

In a particularly preferred embodiment of the invention, $Z^3$ is at each occurrence a direct bond; and

$Ar^{EWG}$ is at each occurrence independently of each other represented by a structure according to any of formulas $Ar^{EWG}$-I, $Ar^{EWG}$-VII, $Ar^{EWG}$-VIII, $Ar^{EWG}$-IX, $Ar^{EWG}$-X $Ar^{EWG}$-XI, $Ar^{EWG}$-XII, and $Ar^{EWG}$-XIII;
$R^7$ is at each occurrence independently of each other selected from the group consisting of hydrogen, deuterium, Me, $^iPr$, $^tBu$, CN, $CF_3$, and
Ph, which is optionally substituted with one or more substituents independently of each other selected from the group consisting of hydrogen, deuterium, Me, $^iPr$, $^tBu$, CN, $CF_3$, and phenyl;
$R^8$ is at each occurrence independently of each other selected from the group consisting of hydrogen, deuterium, Me, $^iPr$, $^tBu$, CN, $CF_3$, and
Ph, which is optionally substituted with one or more substituents independently of each other selected from the group consisting of hydrogen, deuterium, Me, $^iPr$, $^tBu$, CN, $CF_3$, and phenyl;
$R^{Q2}$ is at is at each occurrence independently of each other selected from the group consisting of: hydrogen, deuterium, and
carbazolyl, which is optionally substituted with one or more substituents independently from each other selected from the group consisting of Ph and $N(Ph)_2$;
$R^b$ is at each occurrence hydrogen;
$R^c$ and $R^d$ are at each occurrence independently from another selected from the group consisting of: hydrogen, Me, $^iPr$, $^tBu$, CN, $CF_3$, and
Ph, which is optionally substituted with one or more substituents independently of each other selected from the group consisting of Me, $^iPr$, $^tBu$, CN, $CF_3$, and Ph,
carbazolyl, which is optionally substituted with one or more substituents independently from each other selected from the group consisting of Me, $^iPr$, $^tBu$, CN, $CF_3$, and Ph;
$R^e$ is at each occurrence hydrogen; and
wherein apart from that the above-mentioned definitions apply.

In one embodiment of the invention, each of the one or more TADF materials $E^B$ has a structure represented by any of the formulas $E^B$-I-1, $E^B$-I-2, $E^B$-I-3, $E^B$-I-4, $E^B$-I-5, $E^B$-I-6, $E^B$-I-7, and $E^B$-I-8:

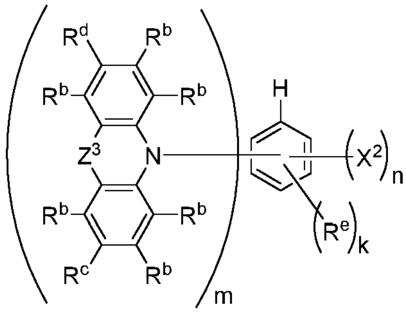

Formula $E^B$-I-1

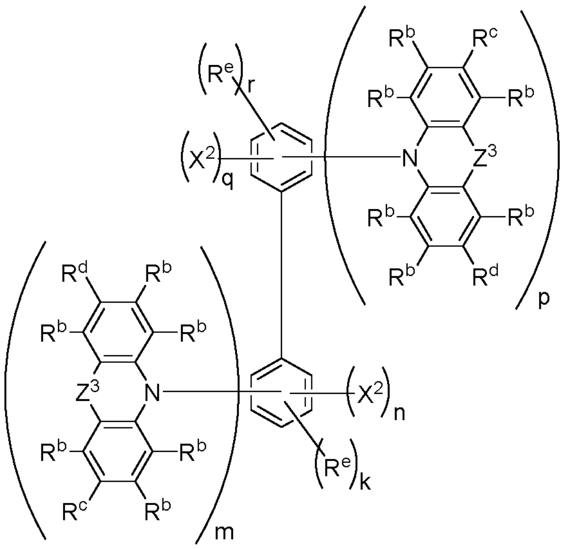

Formula $E^B$-I-2

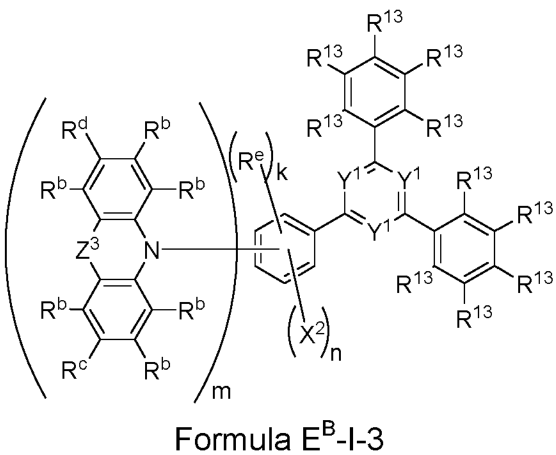

Formula $E^B$-I-3

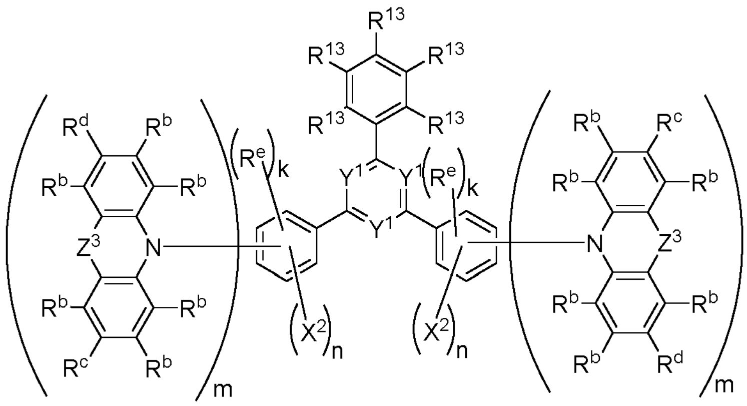

Formula $E^B$-I-4

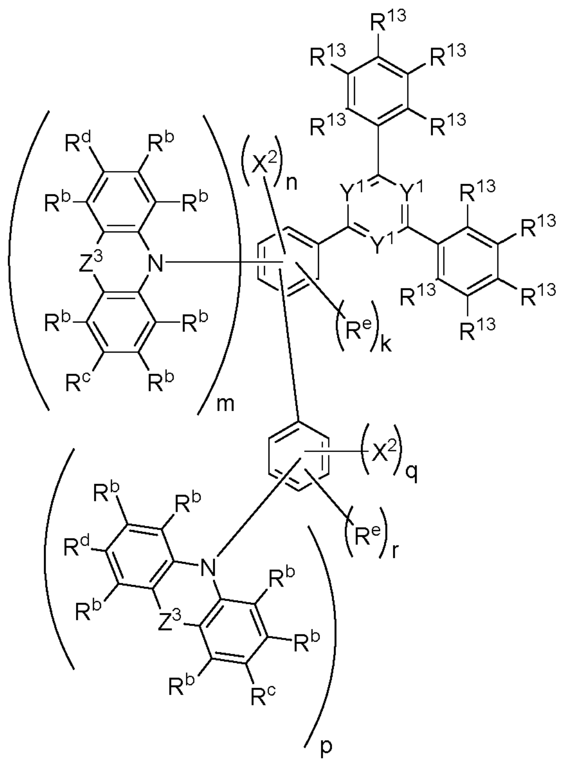

Formula $E^B$-I-5

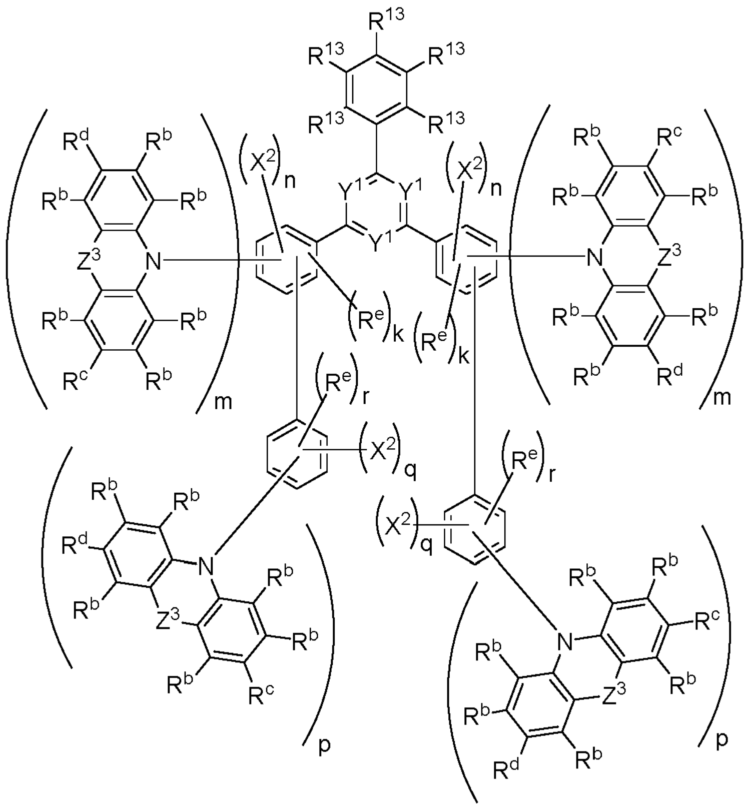

Formula $E^B$-I-6

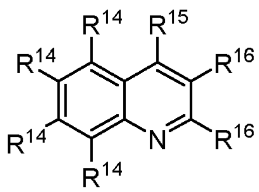

Formula $E^B$-I-7

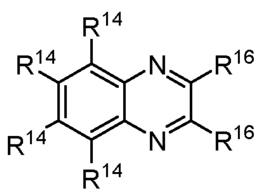

Formula $E^B$-I-8

wherein

$Y^1$ is at each occurrence nitrogen (N) or CH, and at least one $Y^1$ is N;

$R^{13}$ is at each occurrence independently of each other selected from the group consisting of: hydrogen, deuterium, CN, $CF_3$, $Ar^{EWG}$,

$C_1$-$C_5$-alkyl,

wherein one or more hydrogen atoms are optionally substituted by deuterium;

$C_6$-$C_{18}$-aryl,

which is optionally substituted with one or more substuítuents independently of each other selected from deuterium, $C_1$-$C_5$-alkyl groups, and $C_6$-$C_{18}$-aryl groups; and

$R^{14}$ and $R^{15}$ are at each occurrence independently of each other selected from the group consisting of C-$Ar^{EWG}$ and $CR^{Q2}$;

$R^{16}$ is at each occurrence independently of each other selected from the group consisting of $CR^6$, C-$Ar^{EWG}$ and $CR^{Q2}$; wherein not more than two groups $R^{13}$ are CN, $CF_3$ or $Ar^{EWG}$; and wherein apart from that the above-mentioned definitions apply.

In a preferred embodiment of the invention, each of the one or more TADF materials $E^B$ has a structure represented by any of the formulas $E^B$-I-1a, $E^B$-I-2a, $E^B$-I-3a, $E^B$-I-4a, $E^B$-I-5a, $E^B$-I-6a, $E^B$-I-7 (see above), and $E^B$-I-8 (see above),

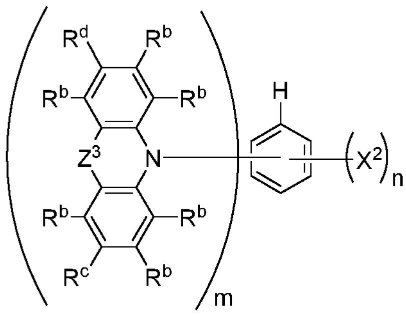

Formula $E^B$-I-1a

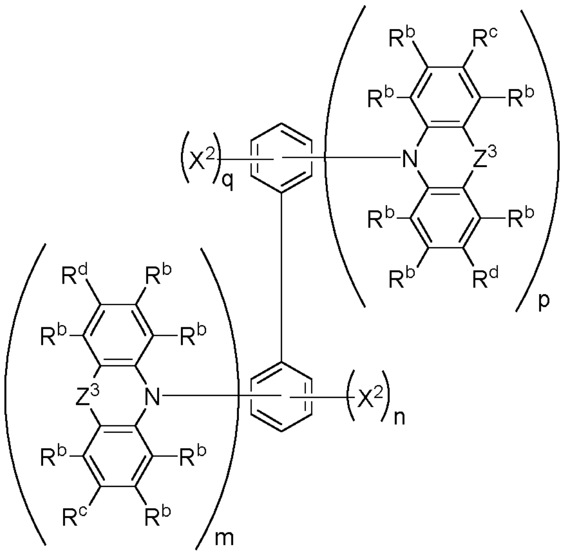

Formula $E^B$-I-2a

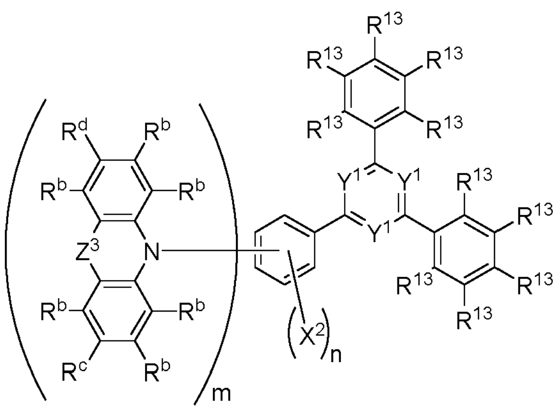

Formula $E^B$-I-3a

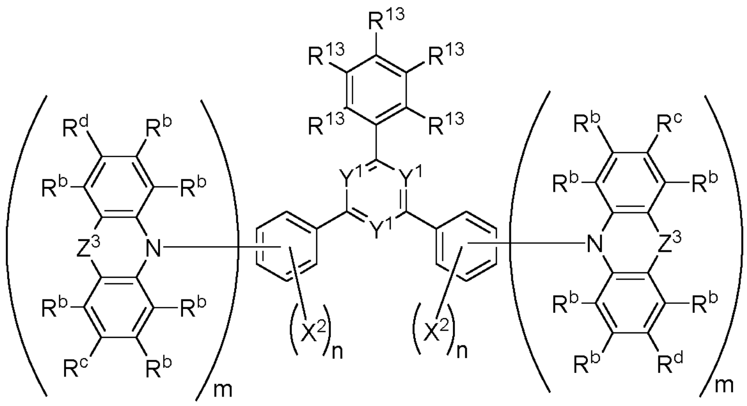

Formula $E^B$-I-4a

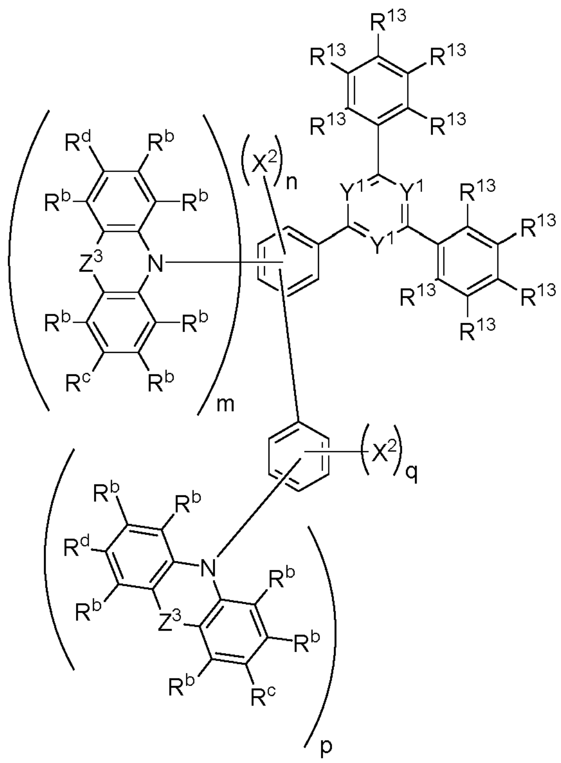

Formula $E^B$-I-5a

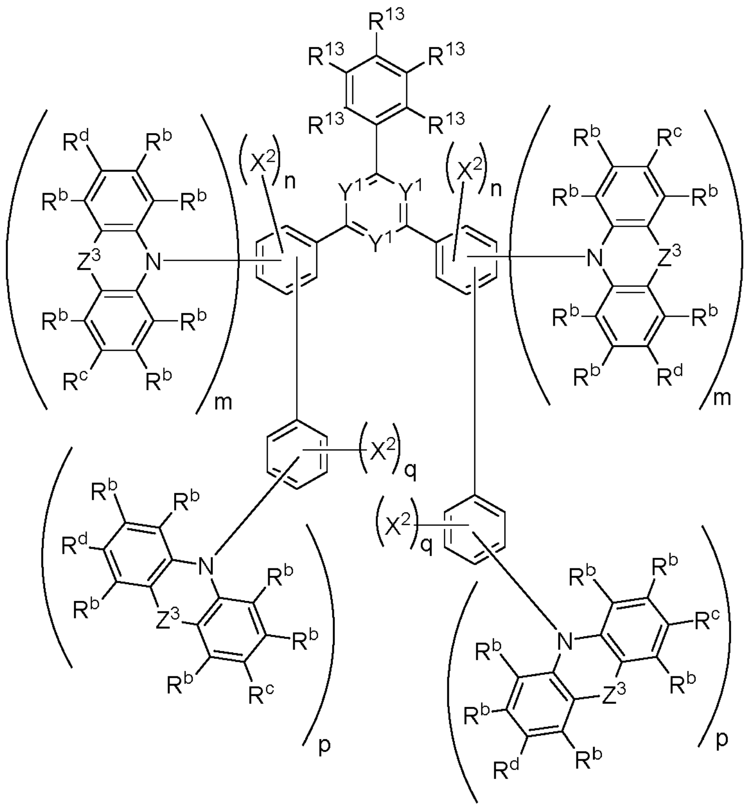

Formula $E^B$-I-6a

wherein $R^{13}$ is at each occurrence independently of each other selected from the group consisting of: hydrogen, deuterium, Me, $^iPr$, $^tBu$, CN, $CF_3$, $Ar^{EWG}$ and
Ph, which is optionally substituted with one or more substituents independently of each other selected from the group consisting of deuterium, Me, $^iPr$, $^tBu$, and Ph;
wherein not more than two groups $R^{13}$ are CN, $CF_3$ or $Ar^{EWG}$; and
wherein apart from that the above-mentioned definitions apply.

Optionally, any adjacent substituents $R^b$, $R^c$, $R^d$, $R^9$, and $R^{10}$ independently of each other may form a mono- or polycyclic, aliphatic, aromatic and/or benzo-fused ring system; wherein one or more hydrogen atoms of the optionally so formed ring system may be substituted by $R^{12}$.

In a preferred embodiment, not more than two groups $R^{13}$ are CN, $CF_3$ or $Ar^{EWG}$

In a preferred embodiment: $1 \leq (m + p)$ and/or $1 \leq (n + q)$.

In an even more preferred embodiment of the invention, each of the one or more TADF materials $E^B$ has a structure represented by any of the formulas $E^B$-I-1a-1, $E^B$-I-2a-1, $E^B$-I-3a-1, $E^B$-I-4a-1, $E^B$-I-5a-1, $E^B$-I-6a-1, $E^B$-I-7 (see above), and $E^B$-I-8 (see above),

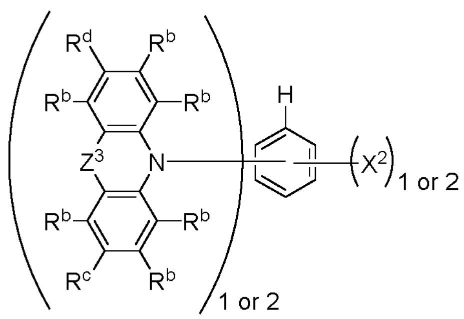

Formula $E^B$-I-1a-1

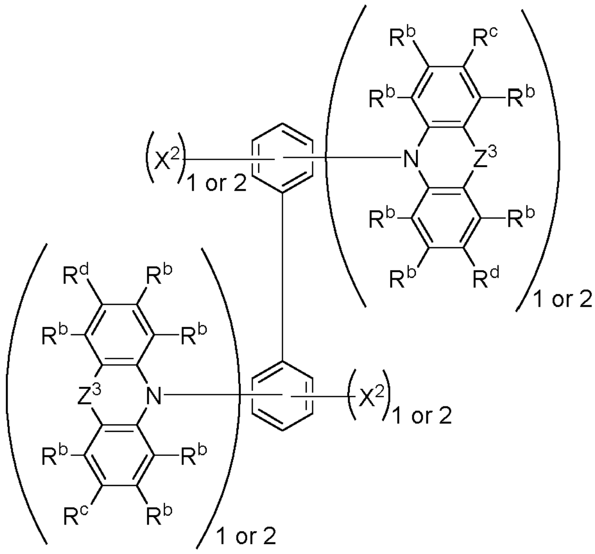

Formula $E^B$-I-2a-1

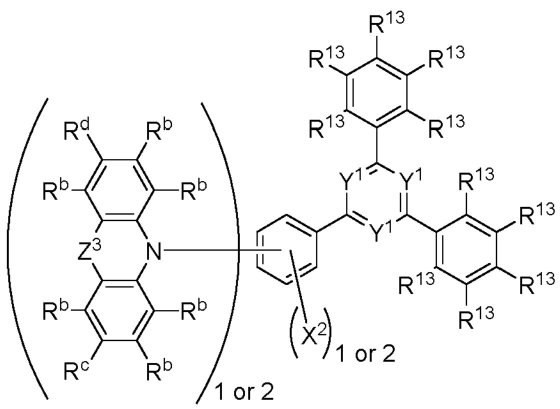

Formula $E^B$-I-3a-1

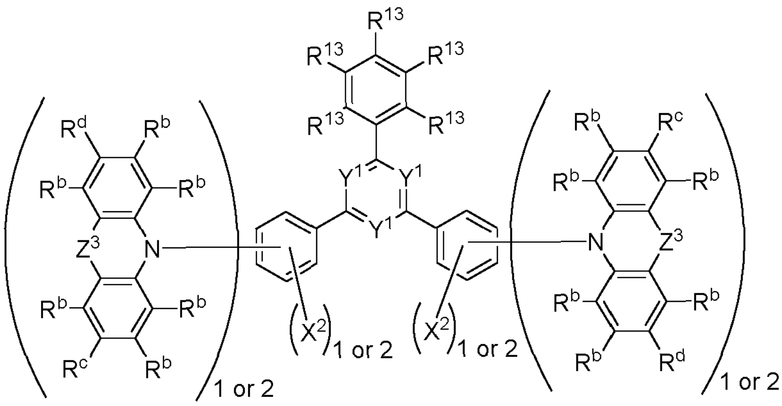

Formula $E^B$-I-4a-1

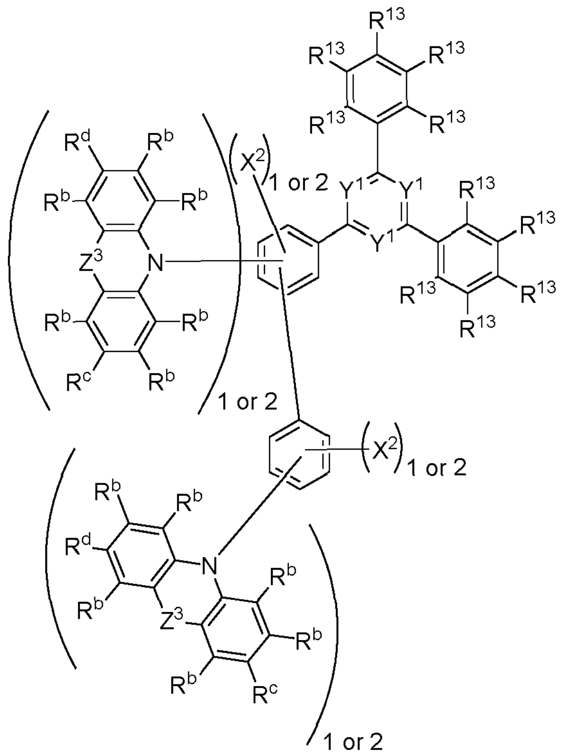

Formula $E^B$-I-5a-1

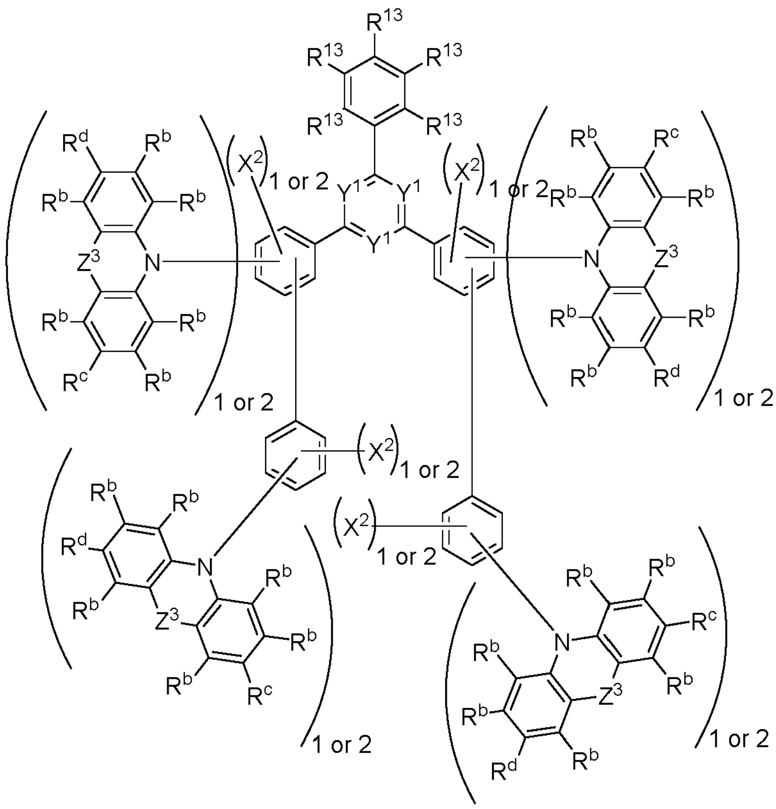

Formula $E^B$-I-6a-1

wherein $R^{13}$ is at each occurrence independently of each other selected from the group consisting of: hydrogen, deuterium, Me, $^iPr$, $^tBu$, CN, $CF_3$, $Ar^{EWG}$ and

Ph, which is optionally substituted with one or more substituents independently of each other selected from the group consisting of deuterium, Me, $^{i}$Pr, $^{t}$Bu, and Ph;
wherein not more than two groups $R^{13}$ attached to the same benzene ring are CN, CF3 or $Ar^{EWG}$ and
wherein apart from that the above-mentioned definitions apply.

In a still even more preferred embodiment of the invention, each of the one or more TADF materials $E^B$ has a structure represented by any of the formulas $E^B$-I-1a-1, $E^B$-I-2a-1, $E^B$-I-3a-1, $E^B$-I-4a-1, $E^B$-I-5a-1, $E^B$-I-6a-1, $E^B$-I-7 (see above), and $E^B$-I-8,

wherein $Z^3$ is at each occurrence a direct bond; and
wherein apart from that the above-mentioned definitions apply.

In a preferred embodiment of the invention, $X^2$ is at each occurrence independently of each other selected from the group consisting of CN,

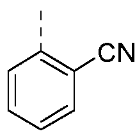 , 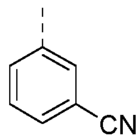 , and 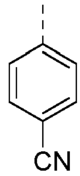 ;

which are bonded via the position marked by the dashed line;
In a preferred embodiment of the invention, $Y^1$ is at each occurrence nitrogen (N).
In a particularly preferred embodiment of the invention, each of the one or more TADF materials $E^B$ has a structure represented by any of the formulas $E^B$-I-3a-1a, $E^B$-1-3a-1b, $E^B$-I-4a-1a, and $E^B$-I-4a-1b:

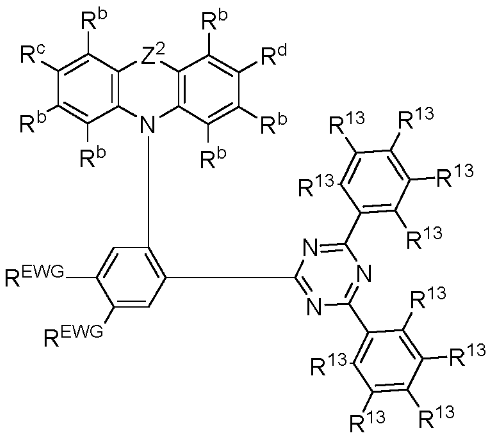

$E^B$-I-3a-1a

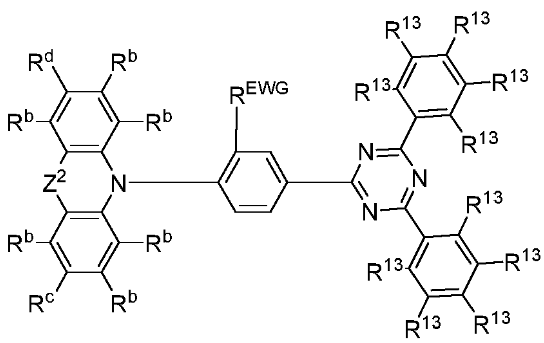

$E^B$-I-3a-1b

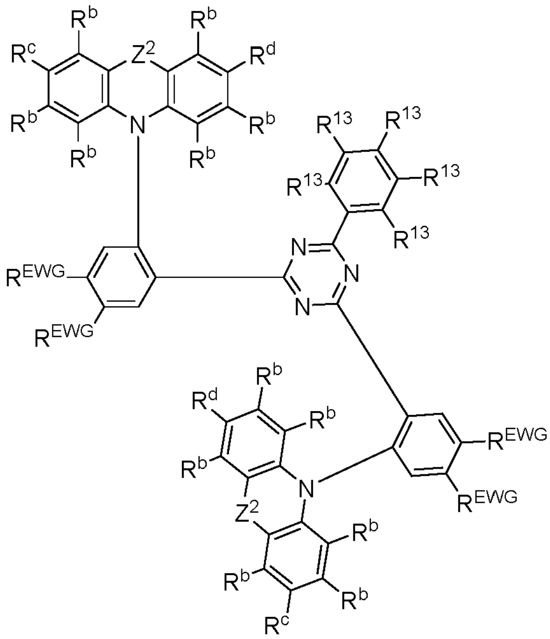

$E^B$-I-4a-1a

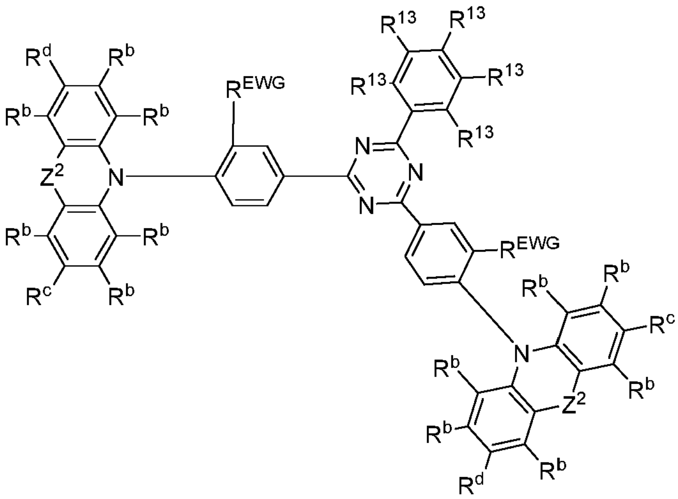

$E^B$-I-4a-1b,

wherein preferably $R^{EWG}$ is at each occurrence independently of each other selected from the group consisting of: hydrogen, CN, and

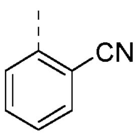
, 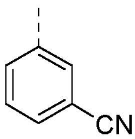
, and 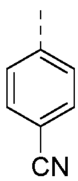
;

wherein, in formulas $E^B$-I-3a-1a and $E^B$-I-4a-1a preferably exactly one of the two $R^{EWG}$ attached to the same benzene ring is hydrogen.

Examples of TADF materials $E^B$ for use in organic electroluminescent devices according to the invention are listed below, whereat the invention is of course not limited to devices comprising one of these molecules.

Particularly preferred examples of TADF materials $E^B$ according to formula $E^B$-I-3a-1a are listed below:

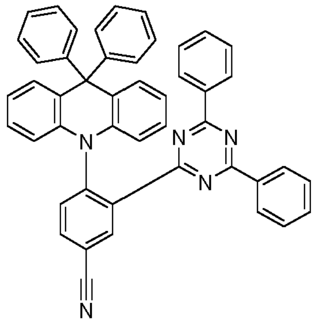

,

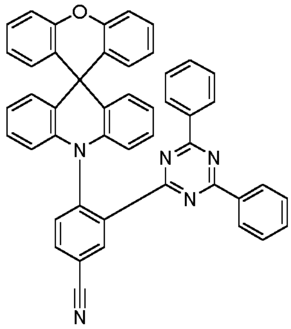

,

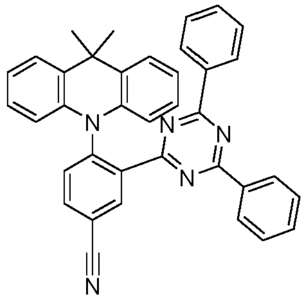

,

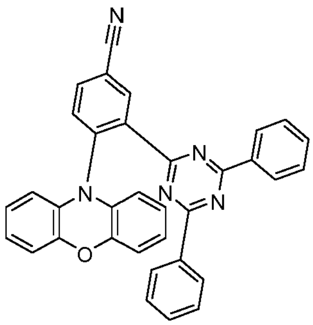

,

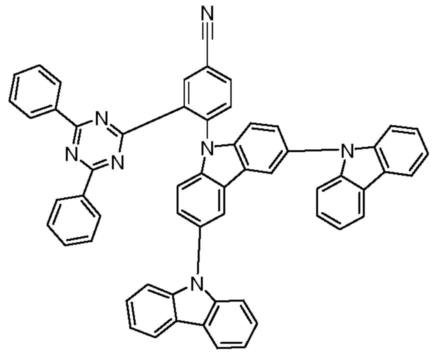

,

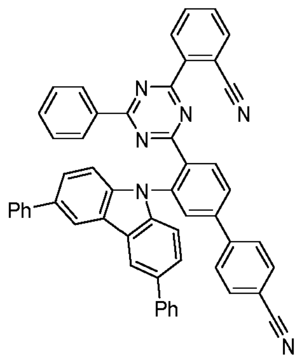

.

Particularly preferred examples of TADF materials $E^B$ according to formula $E^B$-I-3a-1b are listed below:

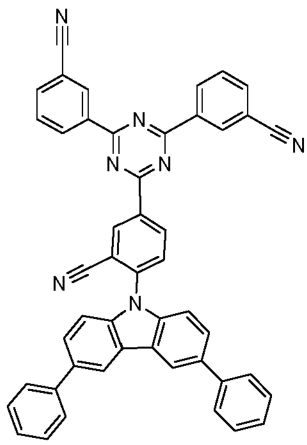

,

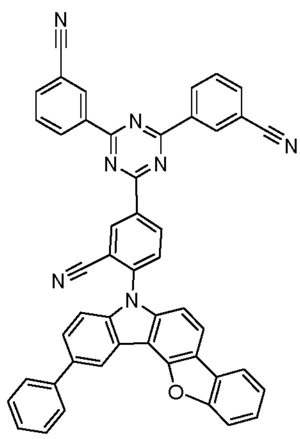

,

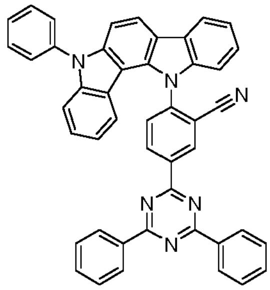

.

Particularly preferred examples of TADF materials $E^B$ according to formula $E^B$-I-4a-1a are listed below:

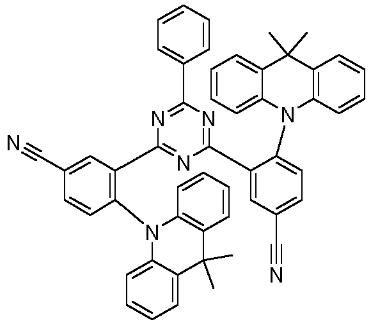 , 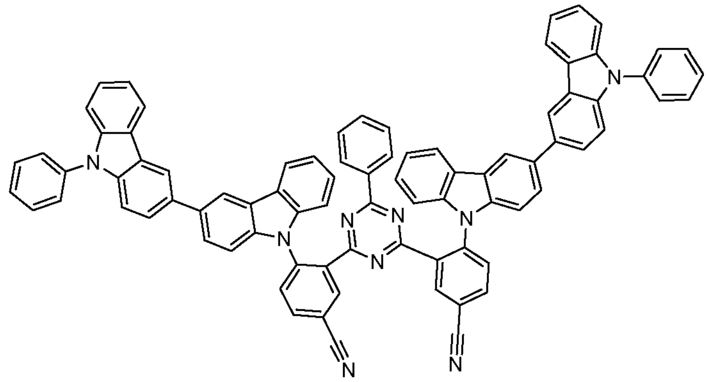 ,

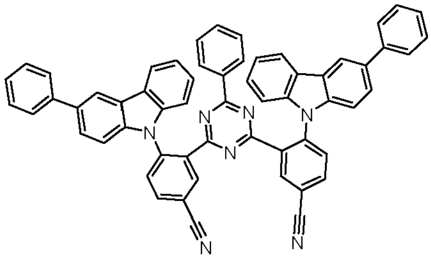 , 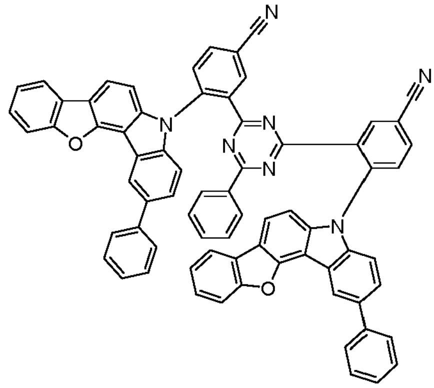 ,

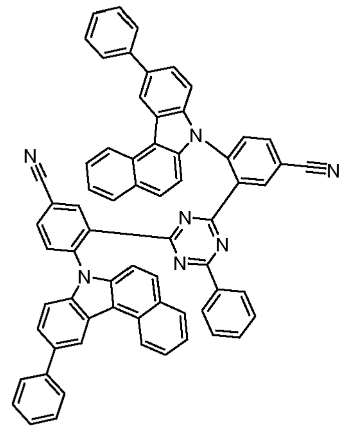 , 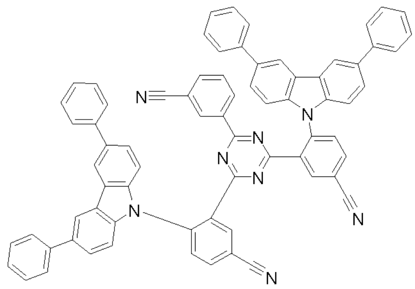 , 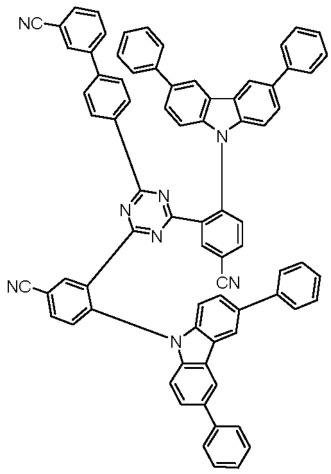 ,

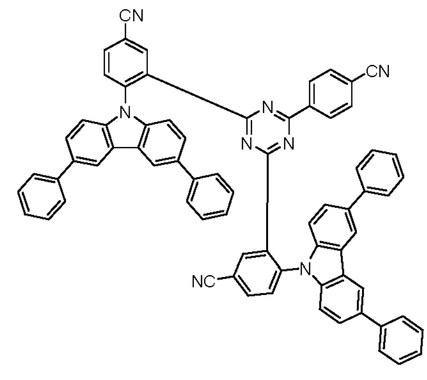 , 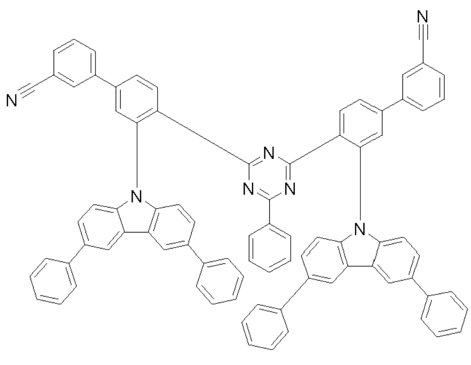 ,

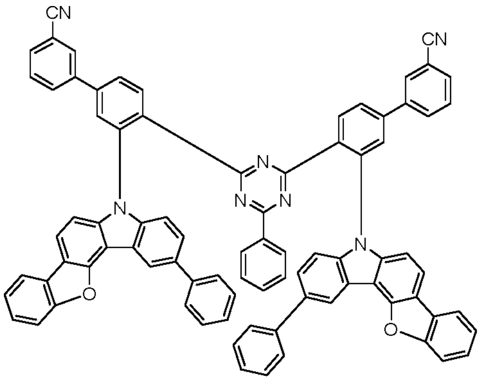,
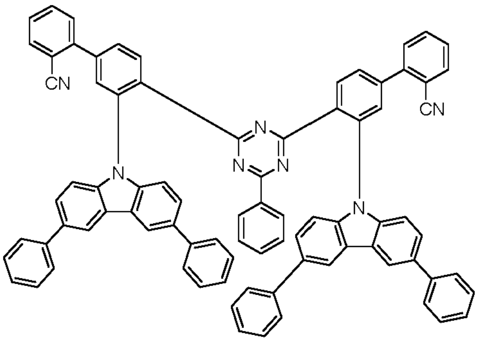,

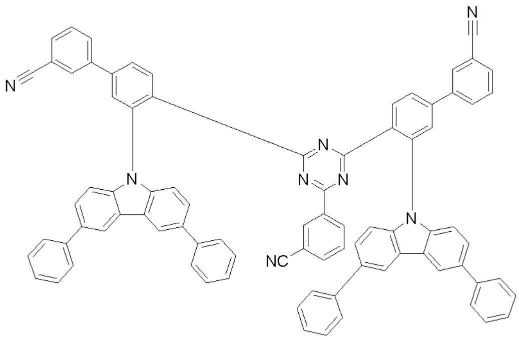,
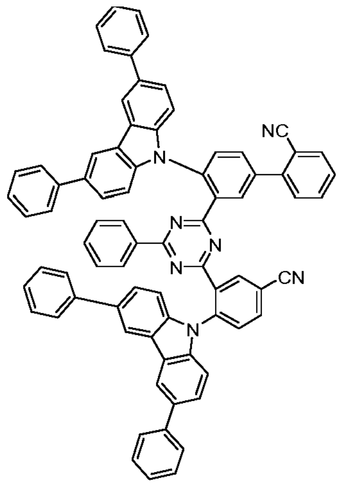,

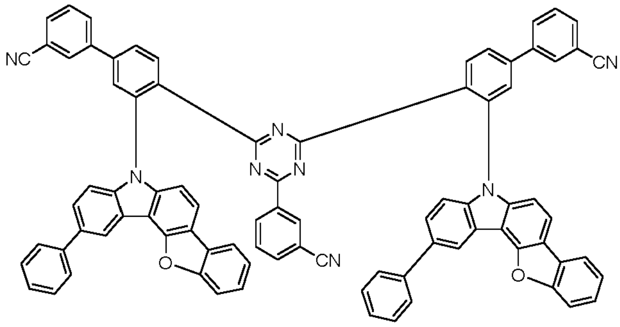,
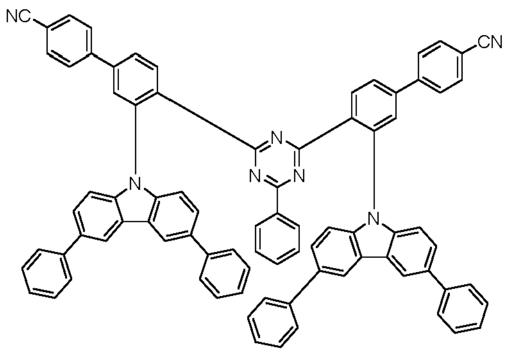,

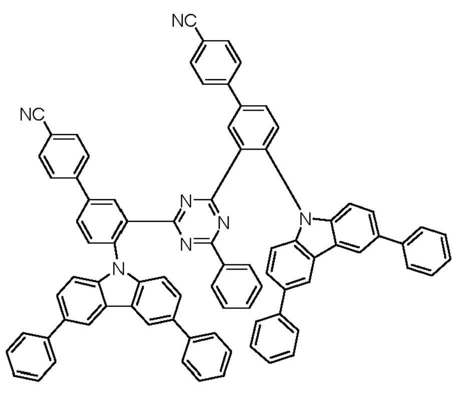,
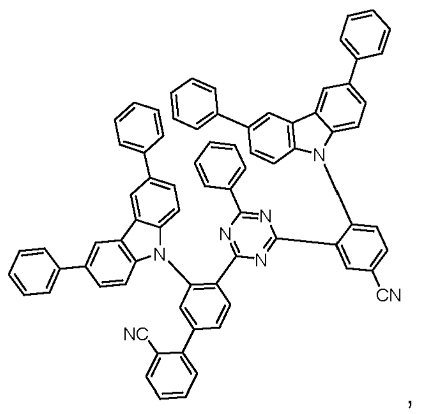,

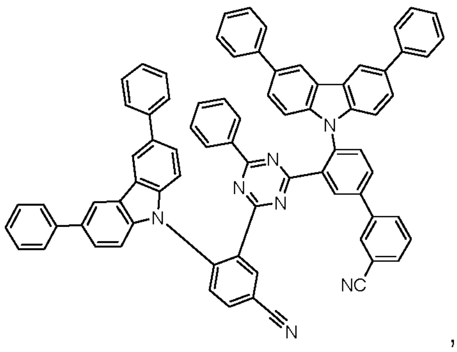
,
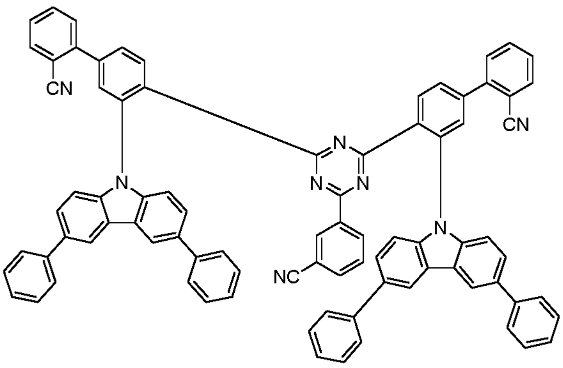
,

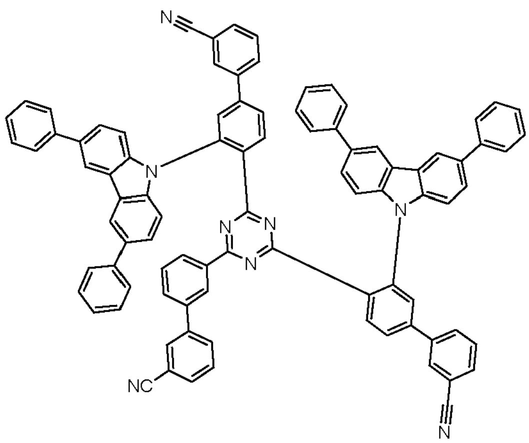
,
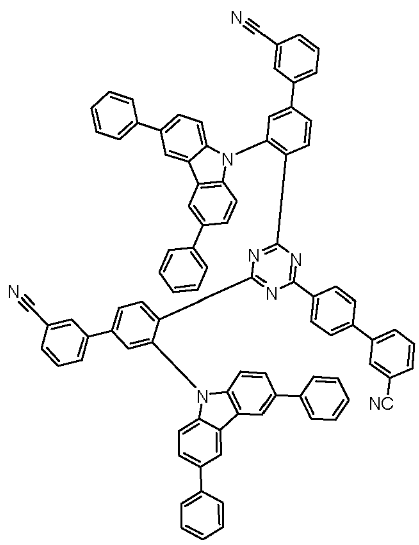
,

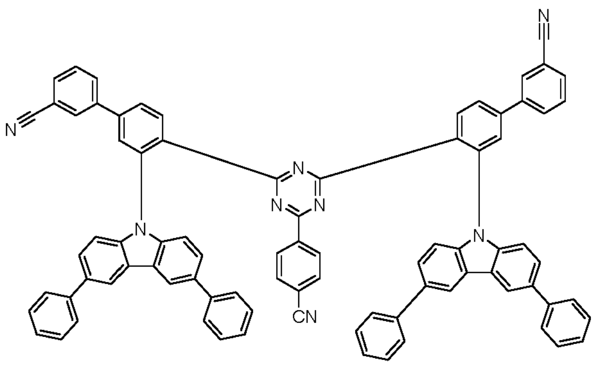
,
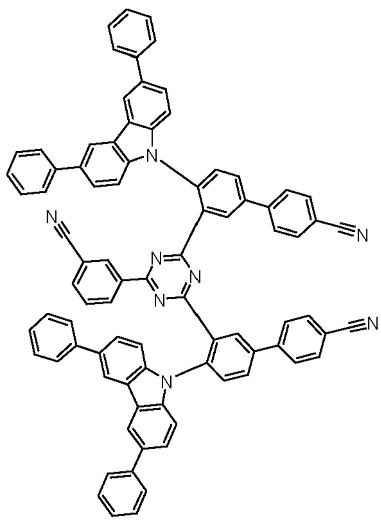
,

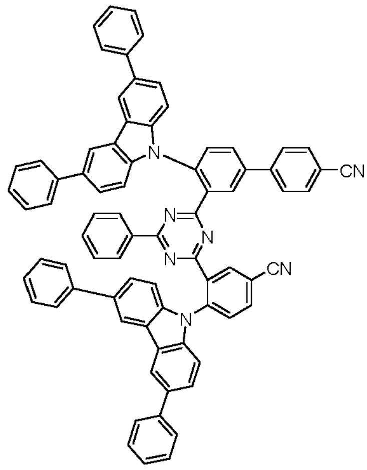

.

Particularly preferred examples of TADF materials $E^B$ according to formula $E^B$-I-4a-1b are listed below:

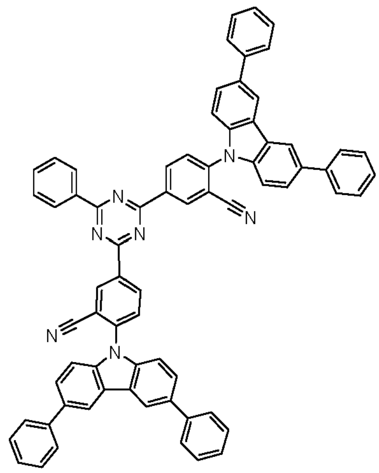

.

Additional examples of TADF materials $E^B$ for use according to the present invention are listed below:

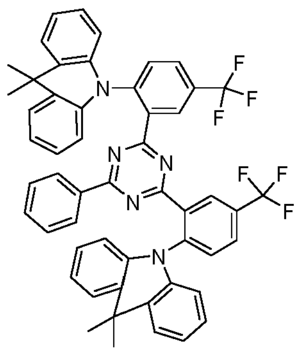

,

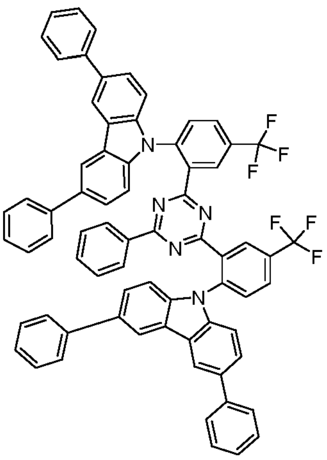

,

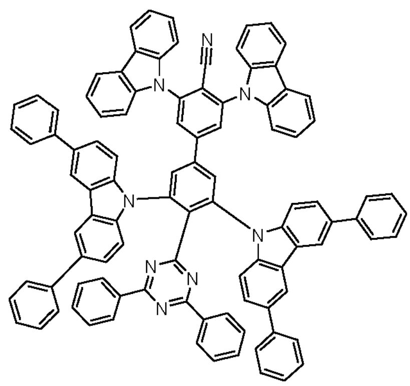

,

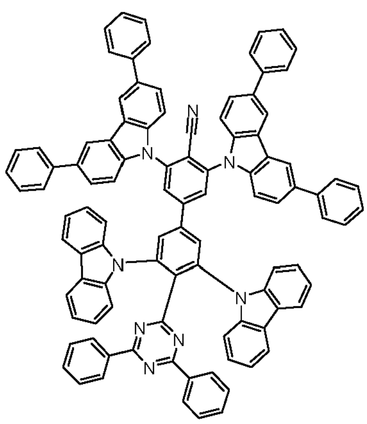,

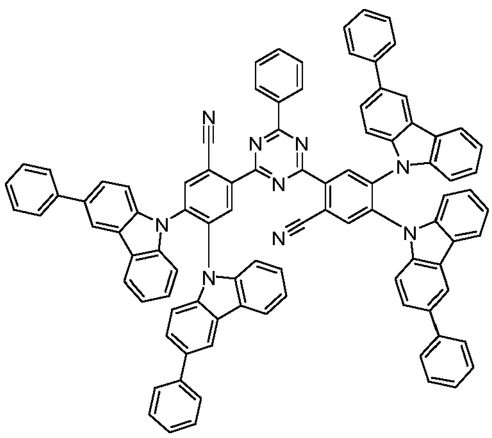,

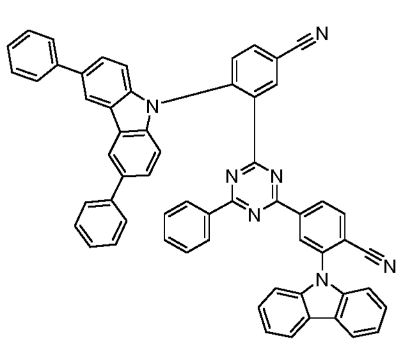,

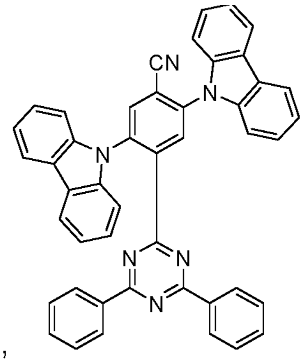,

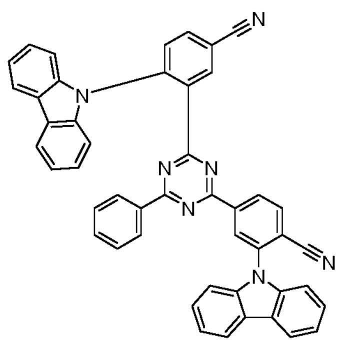,

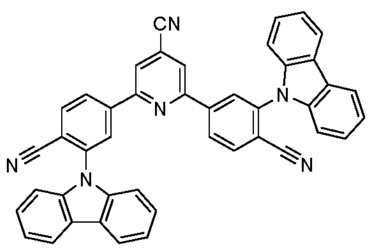,

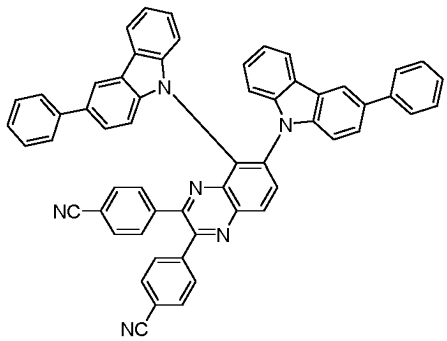,

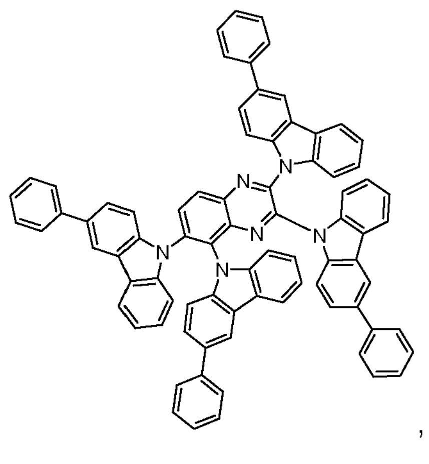,

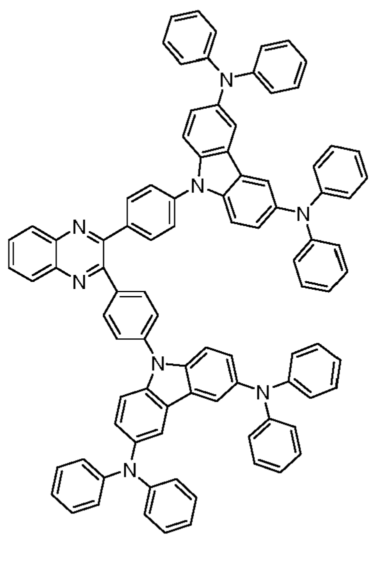,

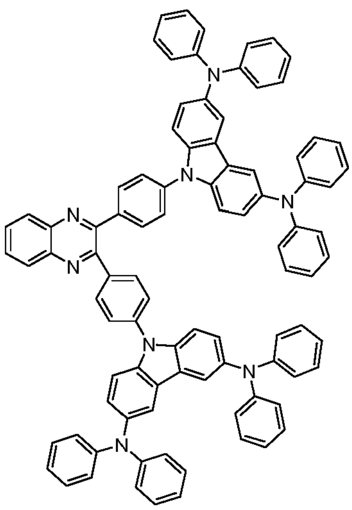 , 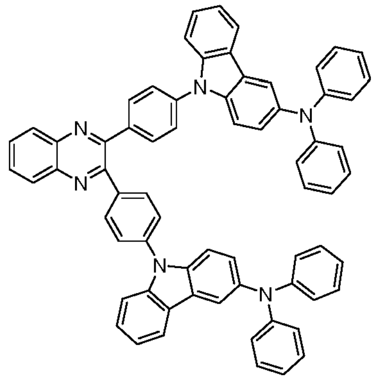 , 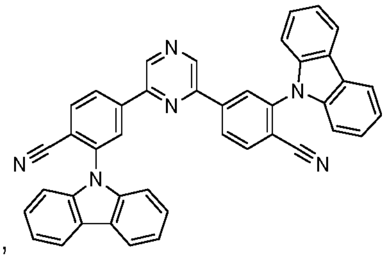 ,

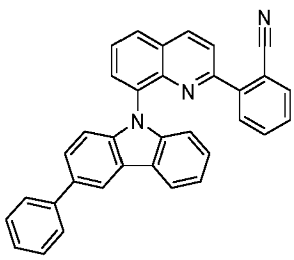 , 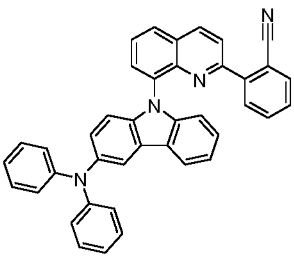 , 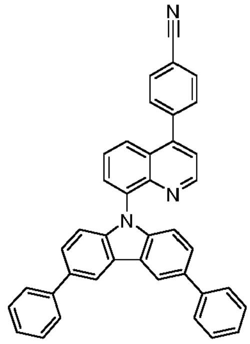 , 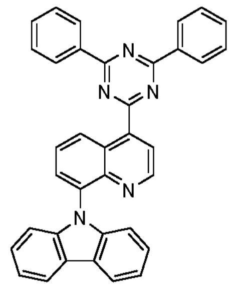 ,

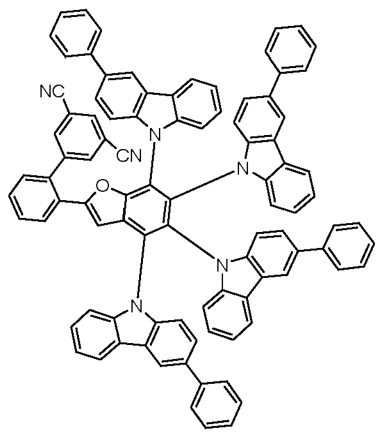 , 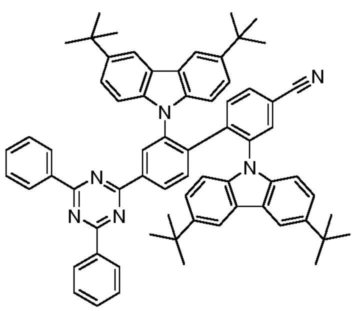 , 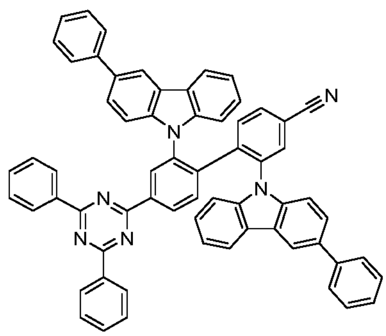 ,

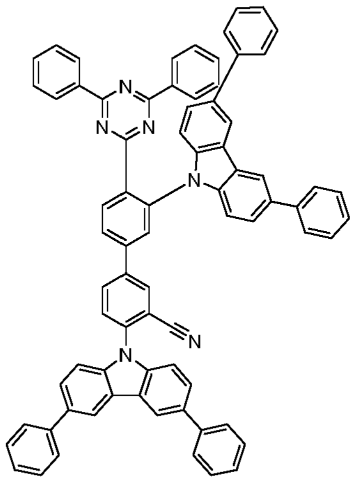
,
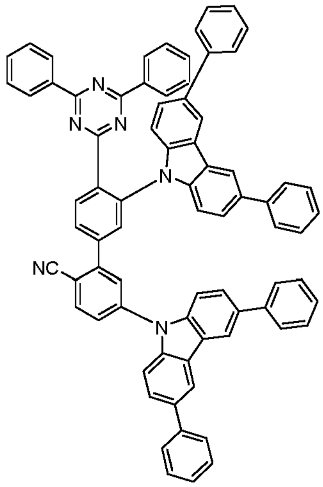
,
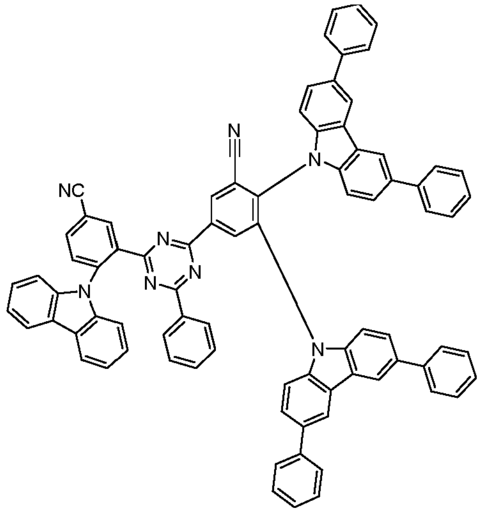

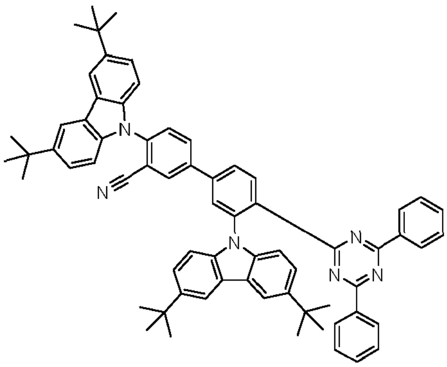
,
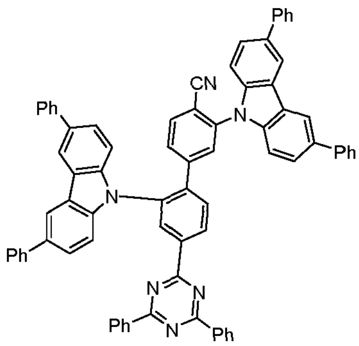
,
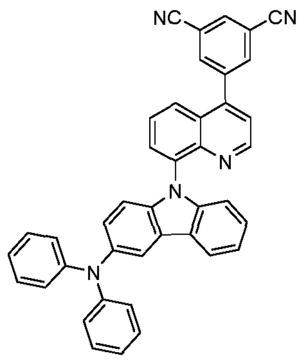
,

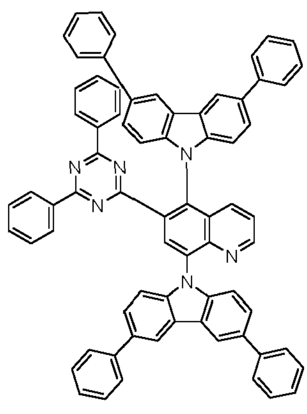
,
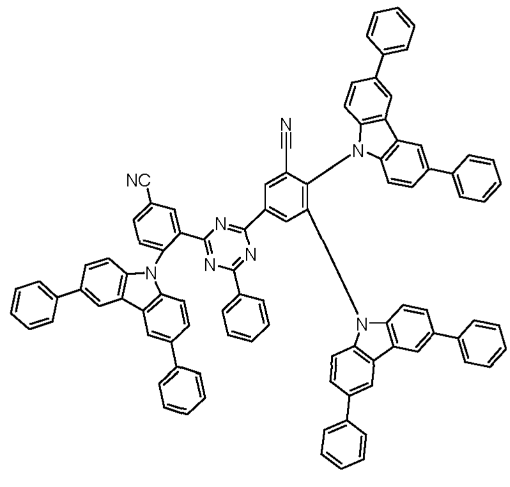
,

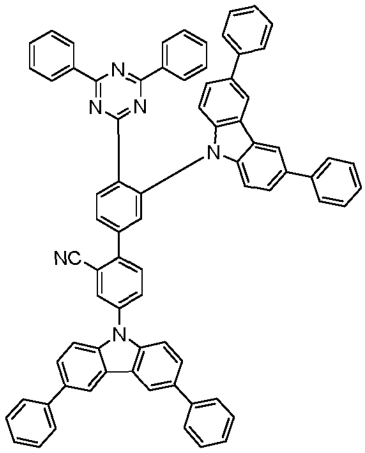
,
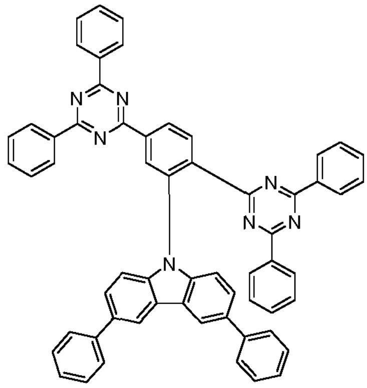
,

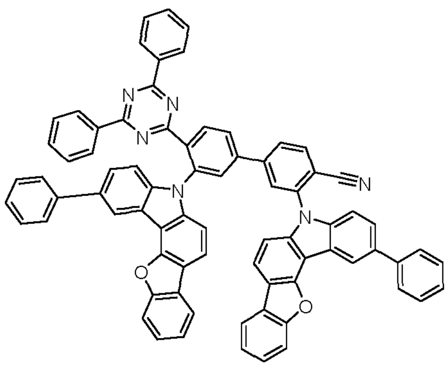
,
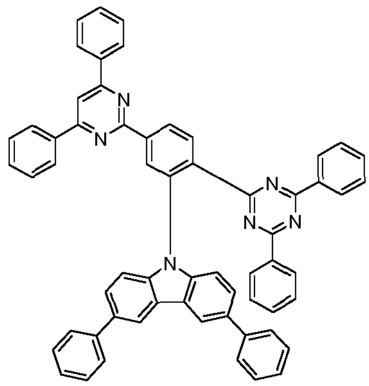
,

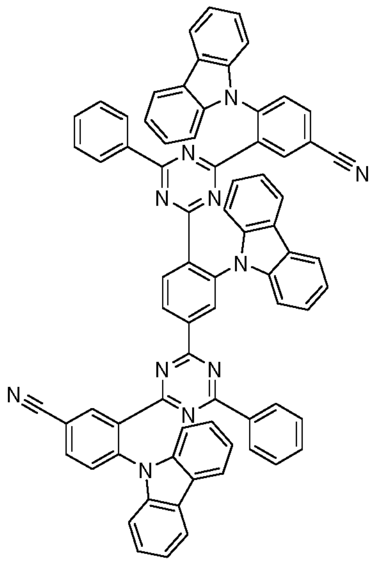
,
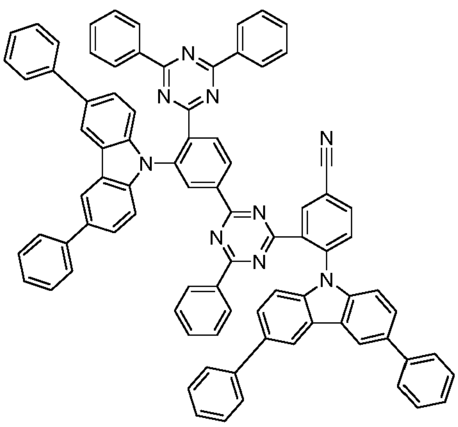
,

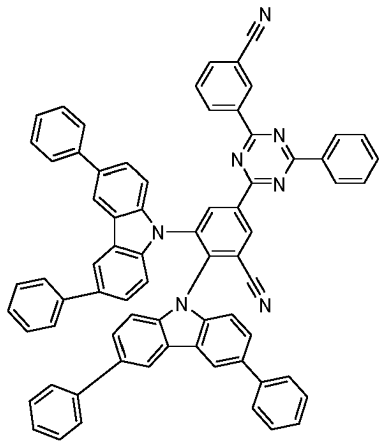

,

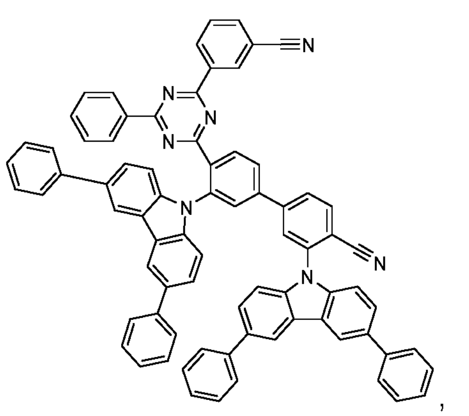

,

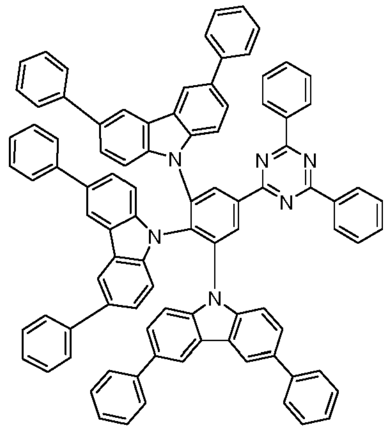

,

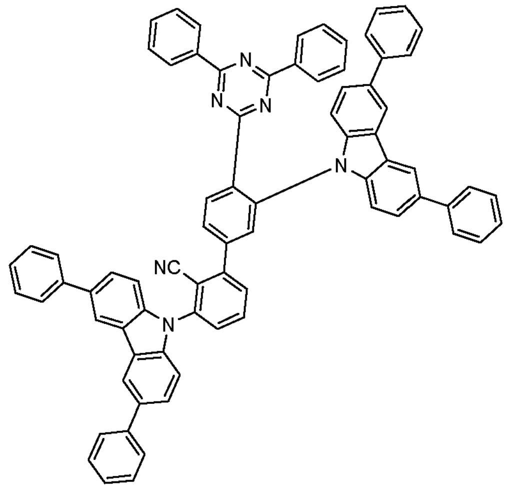

,

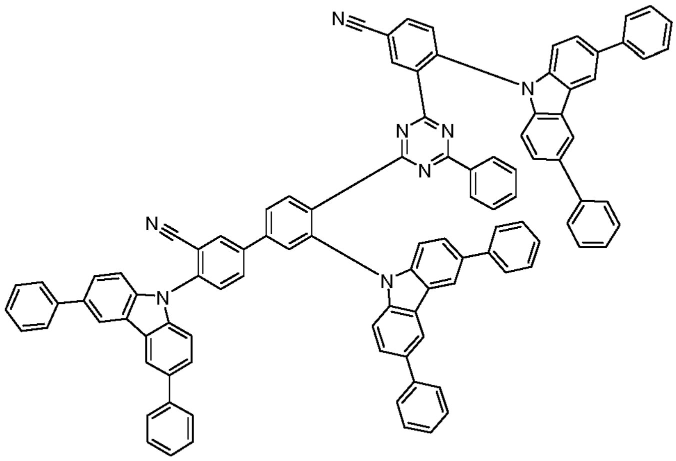

,

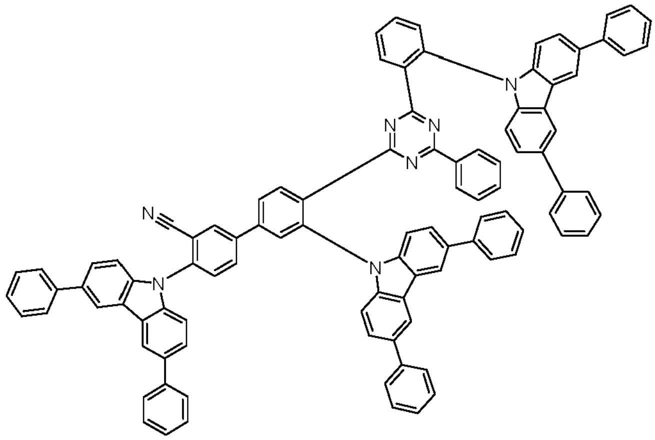,
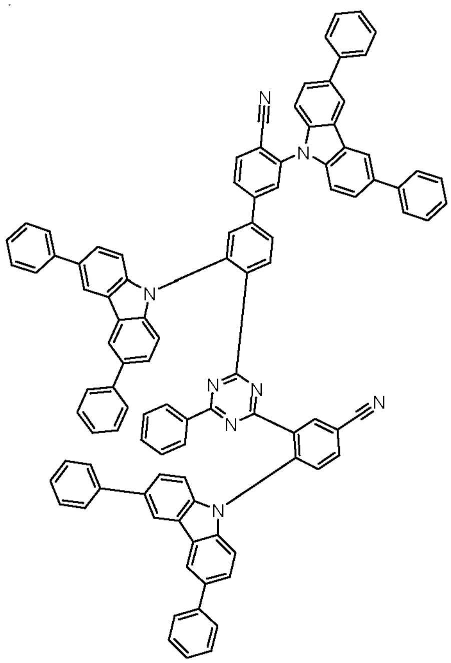,

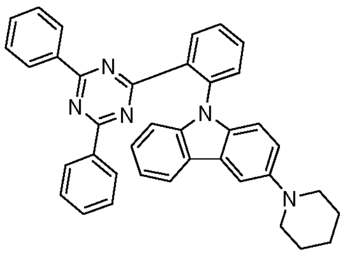,

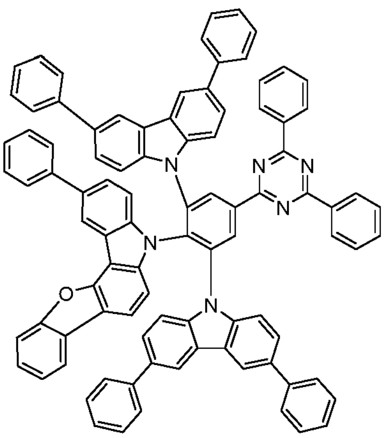,
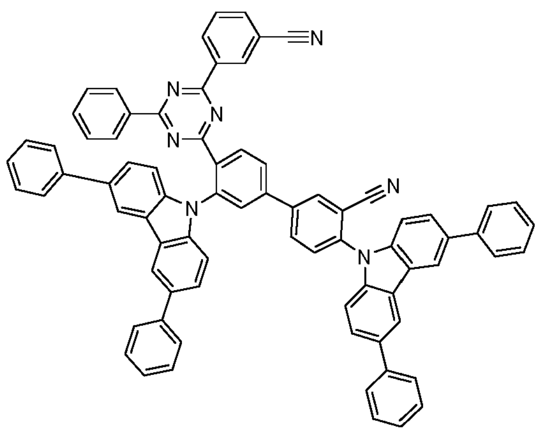,

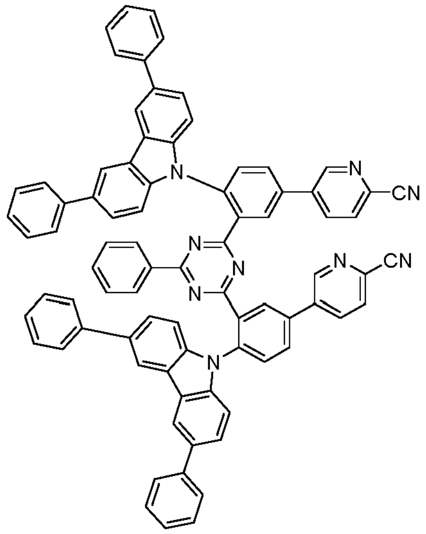
,
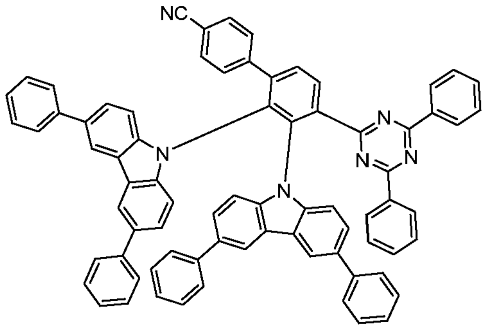
,

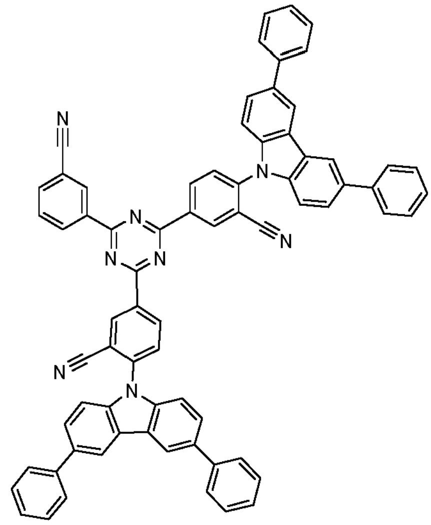
,
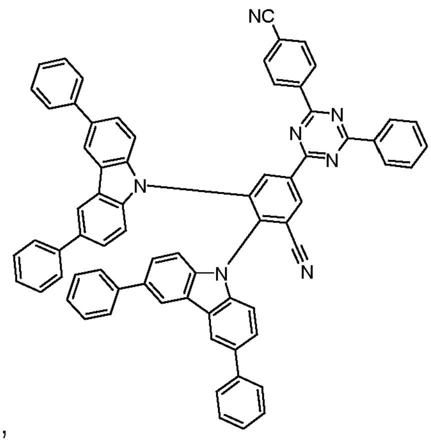
,

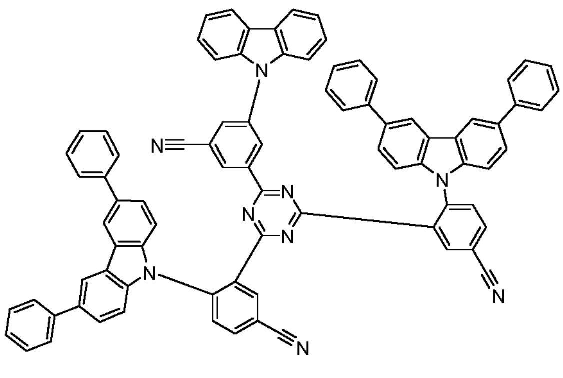
,
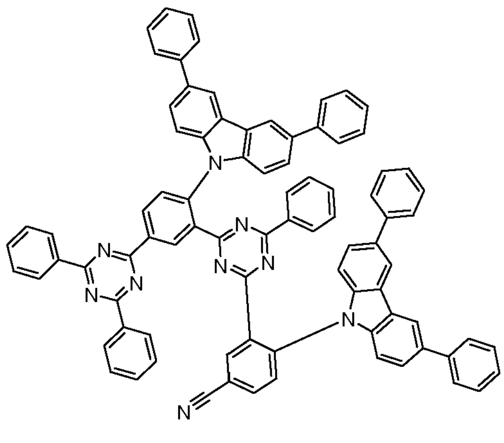
,

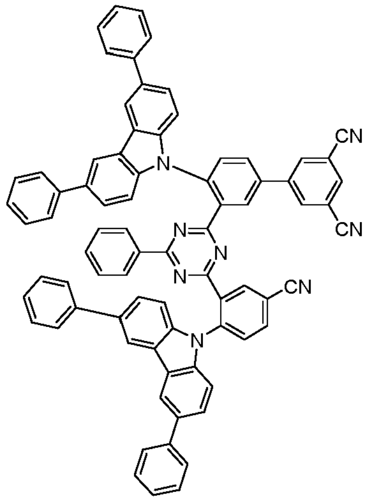
,
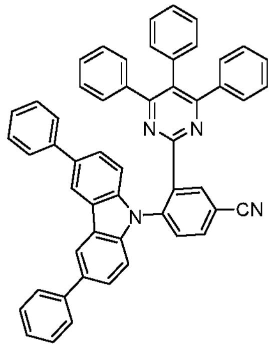
,
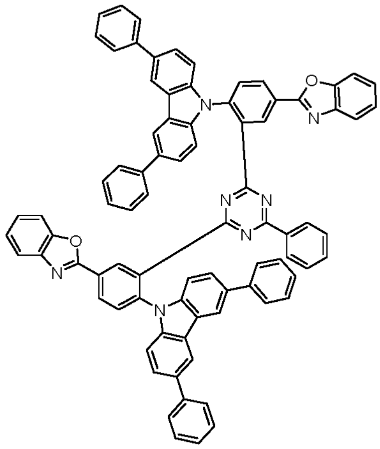
,

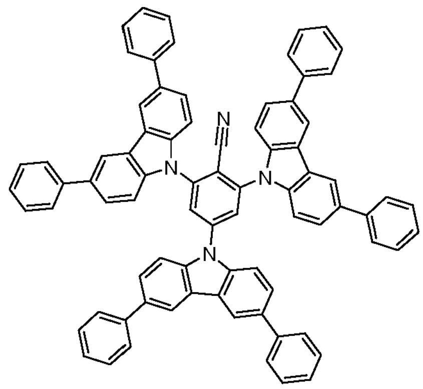
.

The synthesis of TADF materials $E^B$ can be accomplished via standard reactions and reaction conditions known to the skilled artisan. Typically, in a first step, a coupling reaction, preferably a palladium-catalyzed coupling reaction, may be performed, which is exemplarily shown below for the synthesis of a TADF material $E^B$ according to formula $E^B$-I-3a-1:

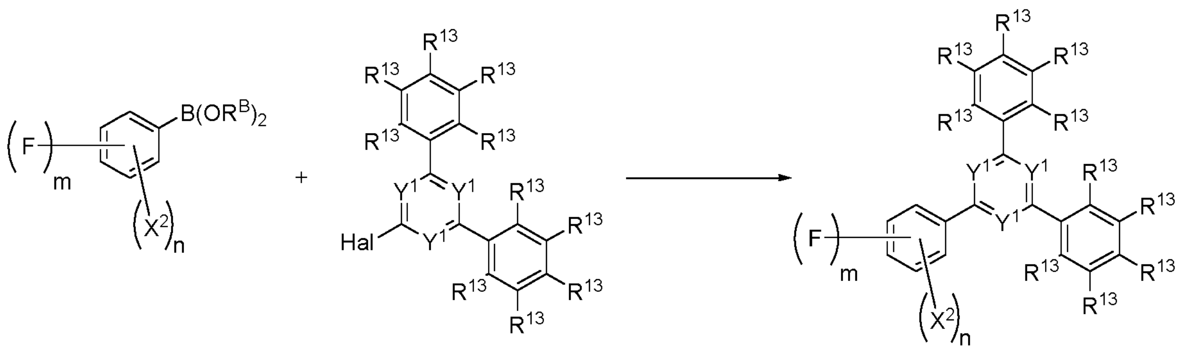

**E1** **E2** **E3**

**E1** can be any boronic acid ($R^B$=H) or an equivalent boronic acid ester ($R^B$ = alkyl or aryl), in particular two $R^B$ form a ring to give e.g. boronic acid pinacol esters, of fluoro-(trifluoromethyl)phenyl, difluoro-(trifluoromethyl)phenyl, fluoro-(cyano)phenyl or difluoro-(cyano)phenyl. As second reactant E2 is used, wherein Hal refers to halogen and may be I, Br or Cl, but preferably is Br. Reaction conditions of such palladium-catalyzed coupling reactions are known the person skilled in the art, e.g. from WO 2017/005699, and it is known that the reacting groups of E1 and E2 can be interchanged as shown below to optimize the reaction yields:

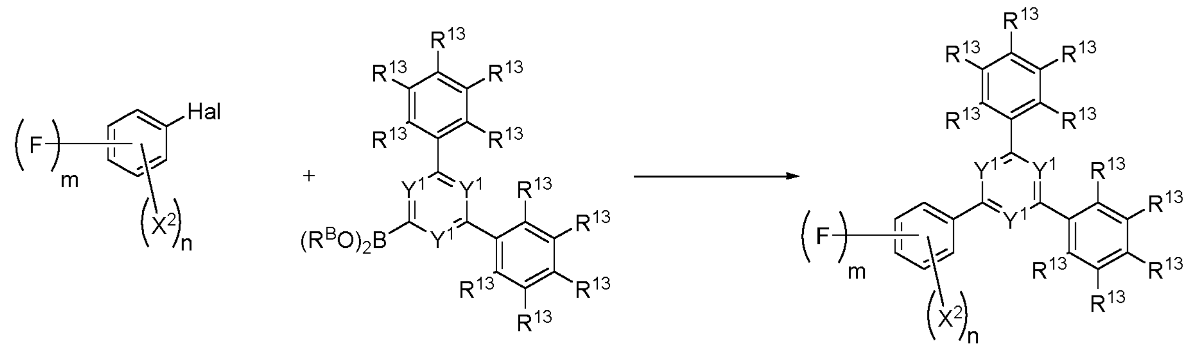

E1 E2 E3

In a second step, the molecules according to formula $E^B$-I-3a-1 are obtained via the reaction of a nitrogen heterocycle in a nucleophilic aromatic substitution with the aryl halide, preferably aryl fluoride, or aryl dihalide, preferably aryl difluoride, E3. Typical conditions include the use of a base, such as tribasic potassium phosphate or sodium hydride, for example, in an aprotic polar solvent, such as dimethyl sulfoxide (DMSO) or N,N-dimethylformamide (DMF), for example.

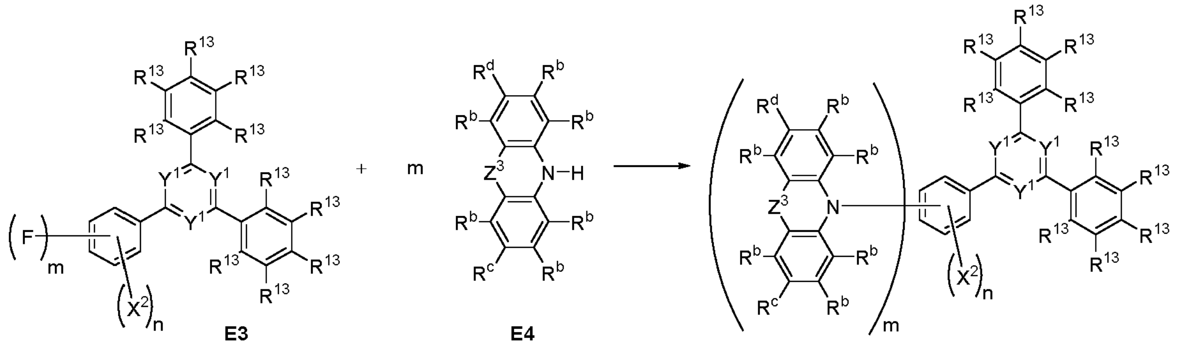

E3 E4

In particular, the donor molecule **E4** is a 3,6-substituted carbazole (e.g., 3,6-dimethylcarbazole, 3,6 diphenylcarbazole, 3,6-di-tert-butylcarbazole), a 2,7-substituted carbazole (e.g., 2,7 dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1 substituted carbazole (e.g., 1-methylcarbazole, 1-phenylcarbazole, 1-tert-butylcarbazole), a 2 substituted carbazole (e.g., 2-methylcarbazole, 2-phenylcarbazole, 2-tert-butylcarbazole), or a 3 substituted carbazole (e.g., 3-methylcarbazole, 3-phenylcarbazole, 3-tert-butylcarbazole).

Alternatively, a halogen-substituted carbazole, particularly 3-bromocarbazole, can be used as **E4.**

In a subsequent reaction, a boronic acid ester functional group or boronic acid functional group may be exemplarily introduced at the position of the one or more halogen substituents, which was introduced via **E4,** to yield the corresponding carbazol-3-ylboronic acid ester or carbazol-3-ylboronic acid, e.g., via the reaction with bis(pinacolato) diboron (CAS No. 73183-34-3). Subsequently, one or more substituents $R^b$, $R^c$ or $R^d$ may be introduced in place of the boronic acid ester group or the boronic acid group via a coupling reaction with the corresponding halogenated reactant, e.g. $R^c$-Hal, preferably $R^c$-Cl and $R^c$-Br.

Alternatively, one or more substituents $R^{b,}$ $R^c$ or $R^d$ may be introduced at the position of the one or more halogen substituents, which was introduced via D-H, via the reaction with a boronic acid of the substituent $R^b$ [$R^b$-$B(OH)_2$], $R^c$ [$R^c$-$B(OH)_2$] or $R^d$ [$R^d$-$B(OH)_2$] or a corresponding boronic acid ester.

Further TADF emitter materials $E^B$ may be obtained analogously. A TADF emitter material $E^B$ may also be obtained by any alternative synthesis route suitable for this purpose.

An alternative synthesis route may comprise the introduction of a nitrogen heterocycle via copper-or palladium-catalyzed coupling to an aryl halide or aryl pseudohalide, preferably an aryl bromide, an aryl iodide, aryl triflate or an aryl tosylate.

*Small FWHM emitter(s) $S^B$*

A small full width at half maximum (FWHM) emitter $S^B$ in the context of the present invention is any emitter that has an emission spectrum, which exhibits an FWHM of less than or equal to 0.25 eV (≤ 0.25 eV), measured with 1 to 5 % by weight, in particular with 1 % by weight of emitter in poly(methyl methacrylate) PMMA at room temperature (i.e.,

(approximately) 20°C).

In a preferred embodiment of the present invention, a small FWHM emitter $S^B$ in the context of the present invention is any emitter that has an emission spectrum, which exhibits an FWHM of ≤ 0.24 eV, more preferably of ≤ 0.23 eV, even more preferably of ≤ 0.22 eV, of ≤ 0.21 eV or of ≤ 0.20 eV, measured (with 1 to 5 % by weight, in particular with 1 % by weight of emitter $S^B$) in PMMA at room temperature. In other embodiments of the present invention, any of the at least one small FWHM emitters $S^B$ exhibits an FWHM of ≤ 0.19 eV, of ≤ 0.18 eV, of ≤ 0.17 eV, of ≤ 0.16 eV, of ≤ 0.15 eV, of ≤ 0.14 eV, of ≤ 0.13 eV, of ≤ 0.12 eV, or of ≤ 0.11 eV.

Additionally, a small FWHM emitter $S^B$ in the context of the present invention exhibits an emission maximum within the wavelength range of 500 nm to 560 nm, measured (with 1 to 5 % by weight, in particular with 1 % by weight of the emitter $S^B$) in PMMA at room temperature.

In a preferred embodiment of the invention, one or more of the at least one small FWHM emitters $S^B$ exhibit an emission maximum within the wavelength range of 520 nm to 540 nm, measured (with 1 to 5 % by weight, in particular with 1 % by weight) of the emitter $S^B$ in PMMA at room temperature.

It is understood that a TADF material $E^B$ comprised in the at least one light-emitting layer B of an organic electroluminescent device according to the invention may optionally also be an emitter with an emission spectrum which exhibits an FWHM of less than or equal to 0.25 eV (≤ 0.25 eV). Optionally, a TADF material $E^B$ comprised in the at least one light-emitting layer B of an organic electroluminescent device according to the invention may also exhibit an emission maximum within the wavelength range of 500 nm to 560 nm. A TADF material $E^B$ in the context of the present invention differs from any small FWHM emitter $S^B$ in the context of the present invention in that $E^B$ typically mainly functions as energy pump transferring energy to at least one small FWHM emitter $S^B$ while the main contribution to the emission band of the optoelectronic device according to the invention can preferably be attributed to the emission of at least one small FWHM emitter $S^B$.

In a preferred embodiment of the invention, any small FWHM emitter $S^B$ is an organic emitter, which, in the context of the invention, means that it does not contain any transition metals. Preferably, any small FWHM emitter $S^B$ according to the invention predominantly consists of the elements hydrogen (H), carbon (C), nitrogen (N), and boron (B), but may for example also comprise oxygen (O), silicon (Si), fluorine (F), and bromine (Br).

In a preferred embodiment of the invention, a small FWHM emitter $S^B$ in the context of the invention is a *near-range-charge-transfer* (NRCT) emitter.

Typical NRCT emitters are described in literature by Hatakeyama et al. (Advanced Materials, 2016, 28(14):2777-2781, DOI: 10.1002/adma.201505491) to show a delayed component in the time-resolved photoluminescence spectrum and exhibit a near-range HOMO-LUMO separation.

Typical NRCT emitters only show one emission band in the emission spectrum, wherein typical fluorescence emitters display several distinct emission bands due to vibrational progression.

According to the invention, any small FWHM emitter $S^B$ comprised in any of the at least one light-emitting layers B of an organic electroluminescent device according to the invention has a highest occupied molecular orbital HOMO($S^B$) having an energy $E^{HOMO}(S^B)$, wherein preferably: $-5.6\ eV \leq E^{HOMO}(S^B) \leq -5.4\ eV$.

According to the invention, any small FWHM emitter $S^B$ comprised in any of the at least one light-emitting layers B of an organic electroluminescent device according to the invention has a lowest unoccupied molecular orbital LUMO($S^B$) having an energy $E^{LUMO}(S^B)$, wherein preferably: $-3.1\ eV \leq E^{LUMO}(S^B) \leq -2.9\ eV$.

According to the invention, any small FWHM emitter $S^B$ comprised in any of the at least one light-emitting layers B of an organic electroluminescent device according to the invention has a lowermost excited singlet state energy level $E(S1^S)$, wherein preferably: $2.4\ eV \leq E(S1^S) \leq 2.6\ eV$.

According to the invention, any small FWHM emitter $S^B$ comprised in any of the at least one light-emitting layers B of an organic electroluminescent device according to the invention has a lowermost excited triplet state energy level $E(T1^S)$, whose preferred range may be defined by the above-mentioned preferred range for the singlet state energy level $E(S1^S)$ and the preferred feature that $\Delta E_{ST}$ of the small FWHM emitter $S^B$ is smaller than or equal to 0.5 eV.

In a preferred embodiment of the invention, each of the at least one small FWHM emitter $S^B$ is a boron(B)-containing emitter.

Known examples of boron(B)-containing small FWHM emitters comprise structures with a boron-dipyrromethene core, wherein typically, the boron atom is additionally substituted with either two fluorine substituents or two alkoxy substituents, wherein one or more hydrogen atoms may be substituted by fluorine (F), or two aryloxy substituents. Specific examples of such emitters are shown in the following:

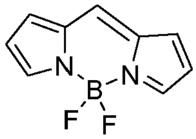 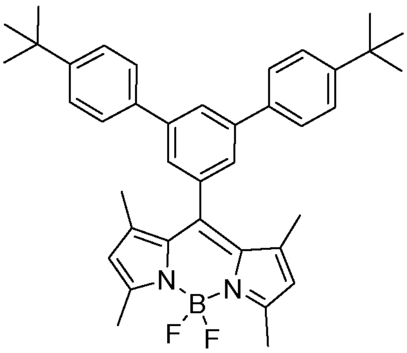 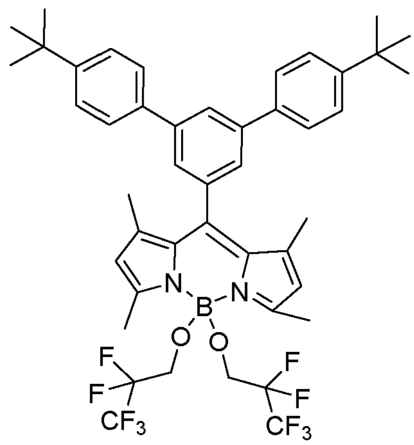 .

In one embodiment, each of the at least one small FWHM emitters $S^B$ comprises or consists of a polycyclic aromatic compound.

In one embodiment of the invention, each of the at least one small FWHM emitters $S^B$ comprises or consists of a structure according to formula B-I:

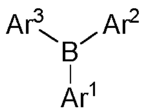

Formula $S^B$-I

wherein B is boron,

$Ar^1$, $Ar^2$, and $Ar^3$ are at each occurrence independently of each other selected from the group consisting of an aromatic ring and a heteroaromatic ring, and $Ar^1$, $Ar^2$, $Ar^3$ may optionally be linked to each other to form one or more additional rings.

The aromatic ring as $Ar^1$, $Ar^2$, $Ar^3$ of the general formula B-I is, for example, an aryl ring having 6 to 30 carbon atoms, and the aryl ring is preferably an aryl ring having 6 to 16 carbon atoms, more preferably an aryl ring having 6 to 12 carbon atoms, and particularly preferably an aryl ring having 6 to 10 carbon atoms.

Specific examples of the aromatic ring as $Ar^1$, $Ar^2$, $Ar^3$ of the general formula B-I include a benzene ring which is a monocyclic system; a biphenyl ring which is a bicyclic system; a naphthalene ring which is a fused bicyclic system; a terphenyl ring (m-terphenyl, o-terphenyl, or p-terphenyl) which is a tricyclic system; an acenaphthylene ring, a fluorene ring, a phenalene ring and a phenanthrene ring, which are fused tricyclic systems; a triphenylene ring, a pyrene ring and a naphthacene ring, which are fused tetracyclic systems; and a perylene ring and a pentacene ring, which are fused pentacyclic systems.

The heteroaromatic ring as $Ar^1$, $Ar^2$, $Ar^3$ of the general formula B-I is, for example, a heteroaryl ring having 2 to 30 carbon atoms, and the heteroaryl ring is preferably a heteroaryl ring having 2 to 25 carbon atoms, more preferably a heteroaryl ring having 2 to 20 carbon atoms, even more preferably a heteroaryl ring having 2 to 15 carbon atoms, and particularly preferably a heteroaryl ring having 2 to 10 carbon atoms. Furthermore, the heteroaromatic ring as $Ar^1$, $Ar^2$, $Ar^3$ of the general formula B-I may be, for example, a heterocyclic ring containing 1 to 5 heteroatoms selected from oxygen, sulfur, and nitrogen in addition to carbon as the ring-constituting atoms.

Specific examples of the heteroaromatic ring as $Ar^1$, $Ar^2$, $Ar^3$ of the general formula B-I include a pyrrole ring, an oxazole ring, an isoxazole ring, a thiazole ring, an isothiazole ring, an imidazole ring, an oxadiazole ring, a thiadiazole ring, a triazole ring, a tetrazole ring, a pyrazole ring, a pyridine ring, a pyrimidine ring, a pyridazine ring, a pyrazine ring, a triazine ring, an indole ring, an isoindole ring, a 1H-indazole ring, a benzimidazole ring, a benzoxazole ring, a benzothiazole ring, a 1H-benzotriazole ring, a quinoline ring, an isoquinoline ring, a cinnoline ring, a quinazoline ring, a quinoxaline ring, a phthalazine ring, a naphthyridine ring, a purine ring, a pteridine ring, a carbazole ring, an acridine ring, a phenoxathiin ring, a phenoxazine ring, a phenothiazine ring, a phenazine ring, an indolizine ring, a furan ring, a benzofuran ring, an isobenzofuran ring, a dibenzofuran ring, a thiophene ring, a benzothiophene ring, a dibenzothiophene ring, a furazane ring, an oxadiazole ring, and a thianthrene ring.

One or more hydrogen atoms in the aforementioned aromatic or heteroaromatic ring as $Ar^1$, $Ar^2$, $Ar^3$ of the general formula B-I may be substituted by a substituent $R^f$,

wherein $R^f$ is at each occurrence independently from another selected from the group consisting of: hydrogen, deuterium, $N(R^{17})_2$, $OR^{17}$, $SR^{17}$, $Si(R^{17})_3$, $B(OR^{17})_2$, $OSO_2R^{17}$, $CF_3$, CN, halogen,
$C_1$-$C_{40}$-alkyl,

which is optionally substituted with one or more substituents $R^{17}$ and wherein one or more non-adjacent $CH_2$-groups are optionally substituted by $R^{17}C=CR^{17}$, C≡C, $Si(R^{17})_2$, $Ge(R^{17})_2$, $Sn(R^{17})_2$, C=O, C=S, C=Se, $C=NR^{17}$, $P(=O)(R^{17})$, SO, $SO_2$, $NR^{17}$, O, S or $CONR^{17}$;

$C_1$-$C_{40}$-alkoxy,

which is optionally substituted with one or more substituents $R^{17}$ and wherein one or more non-adjacent $CH_2$-groups are optionally substituted by $R^{17}C=CR^{17}$, C≡C, $Si(R^{17})_2$, $Ge(R^{17})_2$, $Sn(R^{17})_2$, C=O, C=S, C=Se, $C=NR^{17}$, $P(=O)(R^{17})$, SO, $SO_2$, $NR^{17}$, O, S or $CONR^{17}$;

$C_1$-$C_{40}$-thioalkoxy,

which is optionally substituted with one or more substituents $R^{17}$ and wherein one or more non-adjacent $CH_2$-groups are optionally substituted by $R^{17}C=CR^{17}$, C≡C, $Si(R^{17})_2$, $Ge(R^{17})_2$, $Sn(R^{17})_2$, C=O, C=S, C=Se, $C=NR^{17}$, $P(=O)(R^{17})$, SO, $SO_2$, $NR^{17}$, O, S or $CONR^{17}$;

$C_2$-$C_{40}$-alkenyl,

which is optionally substituted with one or more substituents $R^{17}$ and wherein one or more non-adjacent $CH_2$-groups are optionally substituted by $R^{17}C=CR^{17}$, C≡C, $Si(R^{17})_2$, $Ge(R^{17})_2$, $Sn(R^{17})_2$, C=O, C=S, C=Se, $C=NR^{17}$, $P(=O)(R^{17})$, SO, $SO_2$, $NR^{17}$, O, S or $CONR^{17}$;

$C_2$-$C_{40}$-alkynyl,

which is optionally substituted with one or more substituents $R^{17}$ and wherein one or more non-adjacent $CH_2$-groups are optionally substituted by $R^{17}C=CR^{17}$, C≡C, $Si(R^{17})_2$, $Ge(R^{17})_2$, $Sn(R^{17})_2$, C=O, C=S, C=Se, $C=NR^{17}$, $P(=O)(R^{17})$, SO, $SO_2$, $NR^{17}$, O, S or $CONR^{17}$;

$C_6$-$C_{60}$-aryl,
which is optionally substituted with one or more substituents $R^{17}$; and
$C_3$-$C_{57}$-heteroaryl,
which is optionally substituted with one or more substituents $R^{17}$;
$R^{17}$ is at each occurrence independently from another selected from the group consisting of: hydrogen, deuterium, OPh, SPh, $CF_3$, CN, F, $Si(C_1$-$C_5$-alkyl$)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,

$N(C_3\text{-}C_{17}\text{-heteroaryl})_2$; and
$N(C_3\text{-}C_{17}\text{-heteroaryl})(C_6\text{-}C_{18}\text{-aryl})$; wherein
any of the substituents $R^f$ and $R^{17}$ independently of each other may optionally form a mono- or polycyclic, aliphatic, aromatic, heteroaromatic and/or benzo-fused ring system with one or more substituents $R^f$, $R^{17}$ and/or the aromatic or heteroaromatic rings $Ar^1$, $Ar^2$, and $Ar^3$, whereat the so formed rings may optionally be substituted with one or more substituents $R^f$.

In one embodiment of the invention, each of the at least one small FWHM emitters $S^B$ comprises or consists of a structure according to any of formulas $S^B$-II, $S^B$-III, and $S^B$-IV:

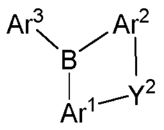
Formula $S^B$-II

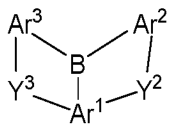
Formula $S^B$-III

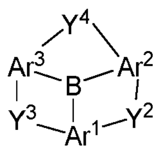
Formula $S^B$-IV

wherein

for $Ar^1$, $Ar^2$, $Ar^3$, $R^f$, and $R^{17}$ the aforementioned definitions apply;
$Y^2$, $Y^3$, and $Y^4$ are at each occurrence independently of each other selected from the group consisting of:

$NR^{18}$, O, $C(R^{18})_2$, S or $Si(R^{18})_2$; wherein
$R^{18}$ is at each occurrence independently of each other selected from the group consisting of:

$C_1$-$C_5$-alkyl, which is optionally substituted with one or more substituents $R^{19}$;
$C_6$-$C_{60}$-aryl, which is optionally substituted with one or more substituents $R^{19}$; and
$C_3$-$C_{57}$-heteroaryl, which is optionally substituted with one or more substituents $R^{19}$;
$R^{19}$ is at each occurrence independently from another selected from the group consisting of: hydrogen, deuterium, $N(R^{20})_2$, $OR^{20}$, $SR^{20}$, $Si(R^{20})_3$, $B(OR^{20})_2$, $OSO_2R^{20}$, $CF_3$, CN, halogen,
$C_1$-$C_{40}$-alkyl,

which is optionally substituted with one or more substituents $R^{20}$ and
wherein one or more non-adjacent $CH_2$-groups are optionally substituted by $R^{20}C{=}CR^{20}$, $C{\equiv}C$, $Si(R^{20})_2$, $Ge(R^{20})_2$, $Sn(R^{20})_2$, C=O, C=S, C=Se, $C{=}NR^{20}$, $P({=}O)(R^{20})$, SO, $SO_2$, $NR^{20}$, O, S or $CONR^{20}$;

$C_1$-$C_{40}$-alkoxy,

which is optionally substituted with one or more substituents $R^{20}$ and
wherein one or more non-adjacent $CH_2$-groups are optionally substituted by $R^{20}C{=}CR^{20}$, $C{\equiv}C$, $Si(R^{20})_2$, $Ge(R^{20})_2$, $Sn(R^{20})_2$, C=O, C=S, C=Se, $C{=}NR^{20}$, $P({=}O)(R^{20})$, SO, $SO_2$, $NR^{20}$, O, S or $CONR^{20}$;

$C_1$-$C_{40}$-thioalkoxy,

which is optionally substituted with one or more substituents $R^{20}$ and
wherein one or more non-adjacent $CH_2$-groups are optionally substituted by $R^{20}C{=}CR^{20}$, $C{\equiv}C$, $Si(R^{20})_2$, $Ge(R^{20})_2$, $Sn(R^{20})_2$, C=O, C=S, C=Se, $C{=}NR^{20}$, $P({=}O)(R^{20})$, SO, $SO_2$, $NR^{20}$, O, S or $CONR^{20}$;

$C_2$-$C_{40}$-alkenyl,

which is optionally substituted with one or more substituents $R^{20}$ and
wherein one or more non-adjacent $CH_2$-groups are optionally substituted by $R^{20}C{=}CR^{20}$, $C{\equiv}C$, $Si(R^{20})_2$, $Ge(R^{20})_2$, $Sn(R^{20})_2$, C=O, C=S, C=Se, $C{=}NR^{20}$, $P({=}O)(R^{20})$, SO, $SO_2$, $NR^{20}$, O, S or $CONR^{20}$;

$C_2$-$C_{40}$-alkynyl,

which is optionally substituted with one or more substituents $R^{20}$ and
wherein one or more non-adjacent $CH_2$-groups are optionally substituted by $R^{20}C{=}CR^{20}$, $C{\equiv}C$, $Si(R^{20})_2$,

$Ge(R^{20})_2$, $Sn(R^{20})_2$, C=O, C=S, C=Se, $C=NR^{20}$, $P(=O)(R^{20})$, SO, $SO_2$, $NR^{20}$, O, S or $CONR^{20}$;

$C_6$-$C_{60}$-aryl,
which is optionally substituted with one or more substituents $R^{20}$; and
$C_3$-$C_{57}$-heteroaryl,
which is optionally substituted with one or more substituents $R^{20}$;
$R^{20}$ is at each occurrence independently from another selected from the group consisting of: hydrogen, deuterium, OPh, SPh, $CF_3$, CN, F, $Si(C_1$-$C_5$-alkyl$)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
$N(C_3$-$C_{17}$-heteroaryl$)_2$- and
$N(C_3$-$C_{17}$-rheteroaryl$)(C_6$-$C_{18}$-aryl); wherein
any of the substituents $R^f$, $R^{17}$, $R^{18}$, and $R^{19}$ independently of each other may optionally form a mono- or polycyclic, aliphatic, aromatic, heteroaromatic and/or benzo-fused ring system with one or more substituents $R^f$, $R^{17}$, $R^{18}$, $R^{19}$ and/or the aromatic or heteroaromatic rings $Ar^1$, $Ar^2$, and $Ar^3$, whereat the so formed rings may optionally be substituted with one or more substituents $R^f$.

In one embodiment of the invention, each of the at least one small FWHM emitters $S^B$ comprises or consists of a structure of formula $S^B$-III-3a:

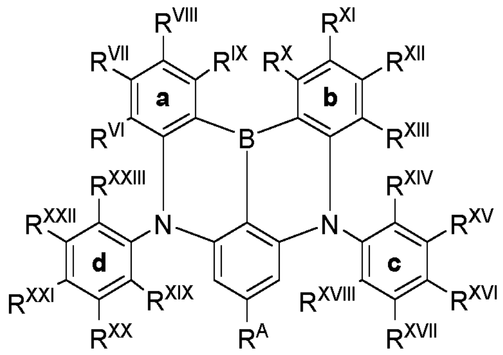

Formula $S^B$-III-3a

wherein $R^{VI}$, $R^{VII}$, $R^{VIII}$, $R^{IX}$, $R^X$, $R^{XI}$, $R^{XII}$, $R^{XIII}$, $R^{XIV}$, $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, $N(R^{21})_2$, $OR^{21}$, $SR^{21}$, $Si(R^{21})_3$, $B(OR^{21})_2$, $OSO_2R^{21}$, $CF_3$, CN, halogen,
$C_1$-$C_{40}$-alkyl,

which is optionally substituted with one or more substituents $R^{21}$ and

wherein one or more non-adjacent $CH_2$-groups are optionally substituted by $R^{21}C=CR^{21}$ , C≡C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;

$C_1$-$C_{40}$-alkoxy,

which is optionally substituted with one or more substituents $R^{21}$ and
wherein one or more non-adjacent $CH_2$-groups are optionally substituted by $R^{21}C=CR^{21}$, C≡C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;

$C_1$-$C_{40}$-thioalkoxy,

which is optionally substituted with one or more substituents $R^{21}$ and
wherein one or more non-adjacent $CH_2$-groups are optionally substituted by $R^{21}C=CR^{21}$, C≡C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;

$C_2$-$C_{40}$-alkenyl,

which is optionally substituted with one or more substituents $R^{21}$ and
wherein one or more non-adjacent $CH_2$-groups are optionally substituted by $R^{21}C=CR^{21}$, C≡C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;

$C_2$-$C_{40}$-alkynyl,

which is optionally substituted with one or more substituents $R^{21}$ and
wherein one or more non-adjacent $CH_2$-groups are optionally substituted by $R^{21}C=CR^{21}$, C≡C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;

$C_6$-$C_{60}$-aryl,
which is optionally substituted with one or more substituents $R^{21}$; and
$C_3$-$C_{57}$-heteroaryl,
which is optionally substituted with one or more substituents $R^{21}$;
wherein optionally one or more pair of adjacent groups $R^{VI}$ and $R^{VII}$, $R^{VII}$ and $R^{VIII}$, $R^{VIII}$ and $R^{IX}$, $R^{IX}$, and $R^{IX}$, $R^{XI}$ and $R^{XII}$, $R^{XII}$ and $R^{XIII}$, $R^{XIV}$ and $R^{XV}$, $R^{XV}$ and $R^{XVI}$, $R^{XVI}$ and $R^{XVII}$, $R^{XVII}$ and $R^{XVIII}$, $R^{XIX}$ and $R^{XX}$, $R^{XX}$, and $R^{IX}$, $R^{XXI}$ and $R^{XXII}$, $R^{XXII}$ and $R^{XXIII}$ forms an aromatic ring system which is fused to the adjacent benzene ring a, b, c or d of general formula $S^B$-III-3a and which is optionally substituted with one or more substituents $R^{21}$;
wherein optionally one or both pairs $R^{VI}$ and $R^{XXIII}$, $R^{XIII}$ and $R^{XIV}$ are joint to form a group $Z^4$ which is at each occurrence independently of each other selected from the group consisting of: a direct bond, $CR^{22}R^{23}$, $C=CR^{22}R^{23}$, C=O, $C=NR^{22}$, $NR^{22}$, O, $SiR^{22}R^{23}$, S, S(O), and $S(O)_2$;
$R^{21}$ is at each occurrence independently from another selected from the group consisting of: hydrogen, deuterium, OPh, SPh, $CF_3$, CN, F, $Si(C_1$-$C_5$-$alkyl)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,

which is optionally substituted with one or more $C_1$-$C_5$-alkyl substituents;
$N(C_6$-$C_{18}$-aryl$)_2$,
$N(C_3$-$C_{17}$-heteroaryl$)_2$- and
$N(C_3$-$C_{17}$-rheteroaryl$)(C_6$-$C_{18}$-aryl);
$R^{22}$ and $R^{23}$ are at each occurrence independently of each other selected from the group consisting of: hydrogen, deuterium, $N(R^{24})_2$, $OR^{24}$, $Si(R^{24})_3$, $B(OR^{24})_2$, $OSO_2R^{24}$, $CF_3$, CN, F, Br, I,
$C_1$-$C_{40}$-alkyl,

which is optionally substituted with one or more substituents $R^{24}$ and
wherein one or more non-adjacent $CH_2$-groups are optionally substituted by $R^{24}C=CR^{24}$, C≡C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;

$C_1$-$C_{40}$-alkoxy,

which is optionally substituted with one or more substituents $R^{24}$ and
wherein one or more non-adjacent $CH_2$-groups are optionally substituted by $R^{24}C=CR^{24}$, C≡C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;

$C_1$-$C_{40}$-thioalkoxy,

which is optionally substituted with one or more substituents $R^{24}$ and
wherein one or more non-adjacent $CH_2$-groups are optionally substituted by $R^{24}C=CR^{24}$, C≡C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;

$C_2$-$C_{40}$-alkenyl,

which is optionally substituted with one or more substituents $R^{24}$ and
wherein one or more non-adjacent $CH_2$-groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;

$C_2$-$C_{40}$-alkynyl,

which is optionally substituted with one or more substituents $R^{24}$ and
wherein one or more non-adjacent $CH_2$-groups are optionally substituted by $R^{24}C=CR^{24}$, C≡C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;

$C_6$-$C_{60}$-aryl,
which is optionally substituted with one or more substituents $R^{24}$; and
$C_3$-$C_{57}$-heteroaryl,
which is optionally substituted with one or more substituents $R^{24}$;
$R^{24}$ is at each occurrence independently from another selected from the group consisting of: hydrogen, deuterium, OPh, SPh, $CF_3$, CN, F, $Si(C_1$-$C_5$-alkyl$)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,

which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
$N(C_3$-$C_{17}$-heteroaryl$)_2$; and
$N(C_3$-$C_{17}$-heteroaryl$)(C_6$-$C_{18}$-aryl$)$;
$R^A$ is selected from the group consisting of: hydrogen,
$C_3$-$C_{15}$-heteroaryl, wherein optionally one or more hydrogen atoms are independently of each other substituted by deuterium, halogen, $C_1$-$C_5$-alkyl, CN, $CF_3$, $SiMe_3$, $SiPh_3$ (Ph = phenyl), $C_3$-$C_{15}$-heteroaryl, and $C_6$-$C_{18}$-aryl, in which optionally one or more hydrogen atoms are independently from each other substituted by $C_1$-$C_5$-alkyl, CN, $CF_3$ and Ph;
and
$C_6$-$C_{18}$-aryl, wherein optionally one or more hydrogen atoms are independently of each other substituted by a substituent independently of each other selected from the group consisting of: by $C_1$-$C_5$-alkyl, CN, $CF_3$, and

Ph, which is optionally substituted with one or more substituents independently of each other selected from the group consisting of Me, $^i$Pr, $^t$Bu, CN, $CF_3$, and Ph,
pyridinyl, which is optionally substituted with one or more substituents independently of each other selected from the group consisting of Me, $^i$Pr, $^t$Bu, CN, $CF_3$, and Ph,
pyrimidinyl, which is optionally substituted with one or more substituents independently of each other selected from the group consisting of Me, $^i$Pr, $^t$Bu, CN, $CF_3$, and Ph, and
triazinyl, which is optionally substituted with one or more substituents independently of each other selected from the group consisting of Me, $^i$Pr, $^t$Bu, CN, $CF_3$, and Ph.

In a highly preferred embodiment of the invention, $R^A$ is selected from the group consisting of:

$C_3$-$C_{15}$-heteroaryl, wherein optionally one or more hydrogen atoms are independently of each other substituted by deuterium, halogen, $C_1$-$C_5$-alkyl, CN, $CF_3$, $SiMe_3$, $SiPh_3$ (Ph = phenyl), $C_3$-$C_{15}$-heteroaryl, and $C_6$-$C_{18}$-aryl, in which optionally one or more hydrogen atoms are independently from each other substituted by $C_1$-$C_5$-alkyl, CN, $CF_3$ and Ph;

In a particularly preferred embodiment of the invention, $R^A$ is selected from the group consisting of:

$C_3$-$C_{15}$-heteroaryl, wherein optionally one or more hydrogen atoms are independently of each other substituted by deuterium, halogen, $C_1$-$C_5$-alkyl, CN, $CF_3$, $SiMe_3$, $SiPh_3$ (Ph = phenyl), $C_3$-$C_{15}$-heteroaryl, and $C_6$-$C_{18}$-aryl, in which optionally one or more hydrogen atoms are independently from each other substituted by $C_1$-$C_5$-alkyl, CN, $CF_3$ and Ph,
wherein the binding site of $R^A$ according to formula $S^B$-III-3a is one of the $C_3$-$C_{15}$-carbon atoms of the $C_3$-$C_{15}$-heteroaryl.

In one embodiment of the invention, each of the at least one small FWHM emitters $S^B$ comprises or consists of a structure according to any of of formulas $S^B$-III-3a-1, $S^B$-III-3a-2, $S^B$-III-3a-3, $S^B$-III-3a-4, $S^B$-III-3a-5, $S^B$-III-3a-6, $S^B$-III-3a-7, $S^B$-III-3a-8, $S^B$-III-3a-9, $S^B$-III-3a-10, $S^B$-III-3a-11, $S^B$-III-3a-12, $S^B$-III-3a-13, $S^B$-III-3a-14, $S^B$-III-3a-15, $S^B$-III-3a-16, $S^B$-III-3a-17, $S^B$-III-3a-18, $S^B$-III-3a-19, $S^B$-III-3a-20, $S^B$-III-3a-21, $S^B$-III-3a-22, $S^B$-III-3a-23, $S^B$-III-3a-24, $S^B$-III-3a-25, $S^B$-III-3a-26, $S^B$-III-3a-27, $S^B$-III-3a-28, $S^B$-III-3a-29, $S^B$-III-3a-30:

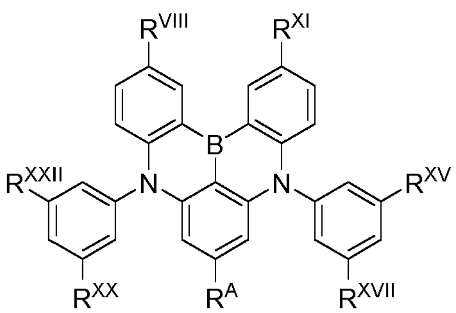

Formula $S^B$-III-3a-1

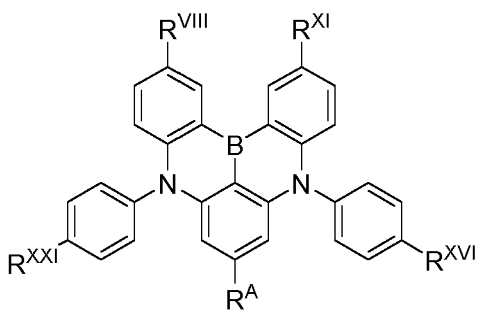

Formula $S^B$-III-3a-2

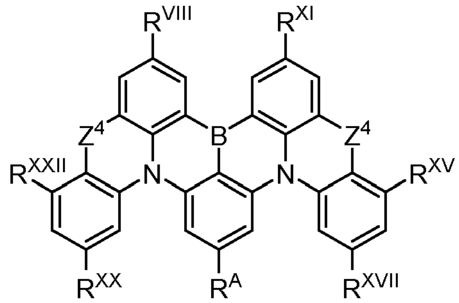

Formula $S^B$-III-3a-3

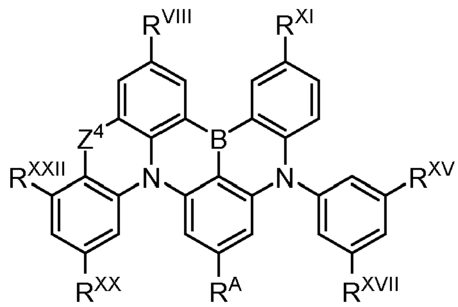

Formula $S^B$-III-3a-4

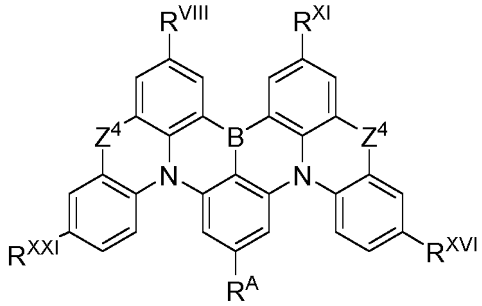

Formula $S^B$-III-3a-5

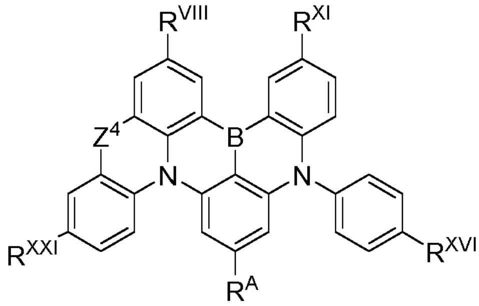

Formula $S^B$-III-3a-6

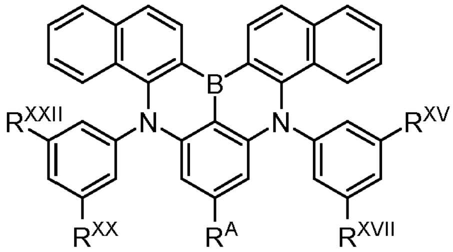

Formula $S^B$-III-3a-7

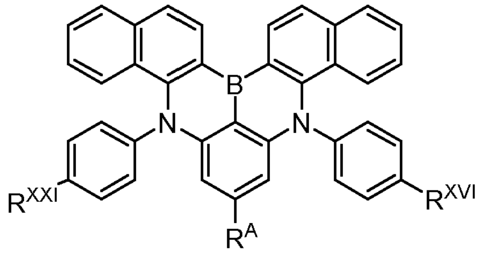

Formula $S^B$-III-3a-8

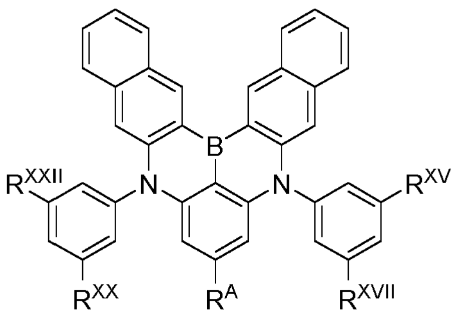

Formula $S^B$-III-3a-9

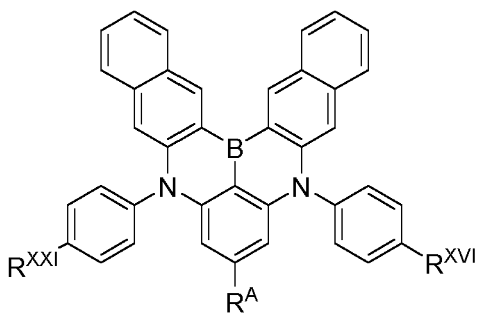

Formula $S^B$-III-3a-10

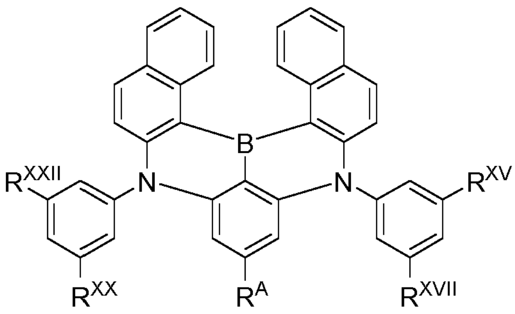

Formula S^B-III-3a-11

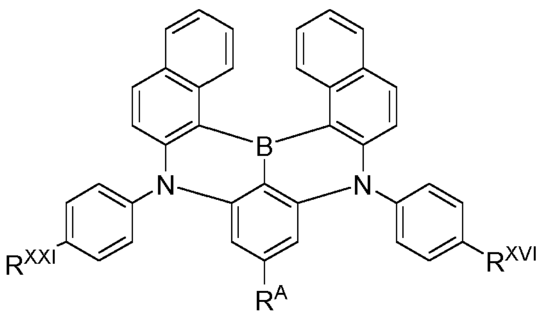

Formula S^B-III-3a-12

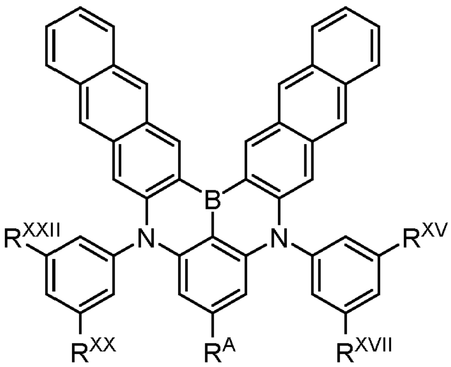

Formula S^B-III-3a-13

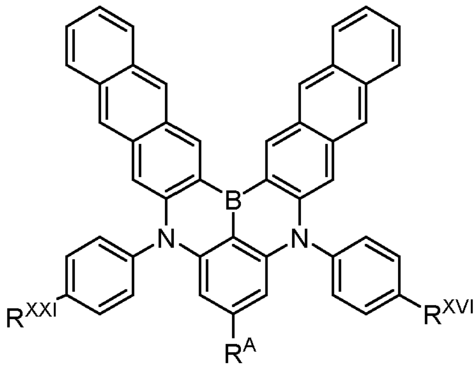

Formula S^B-III-3a-14

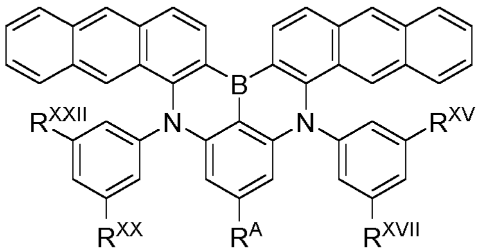

Formula S^B-III-3a-15

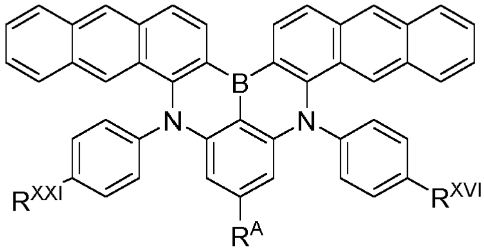

Formula S^B-III-3a-16

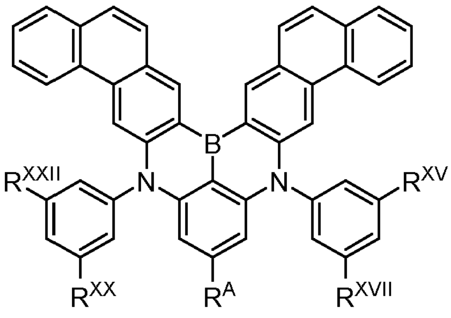

Formula S^B-III-3a-17

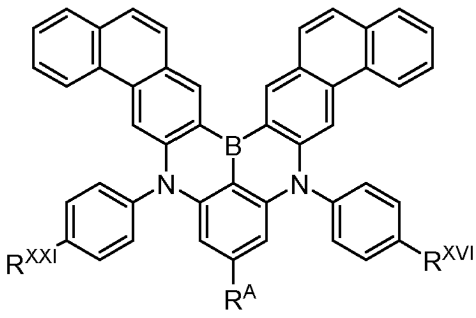

Formula S^B-III-3a-18

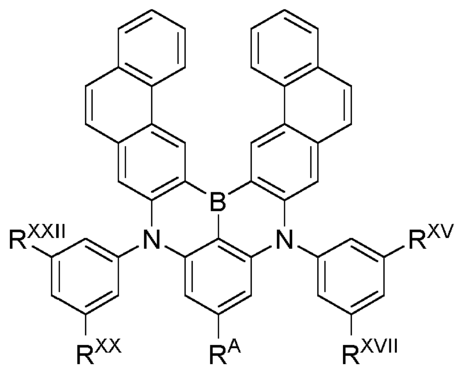

Formula $S^B$-III-3a-19

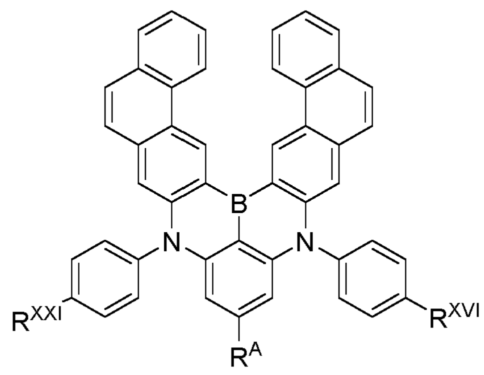

Formula $S^B$-III-3a-20

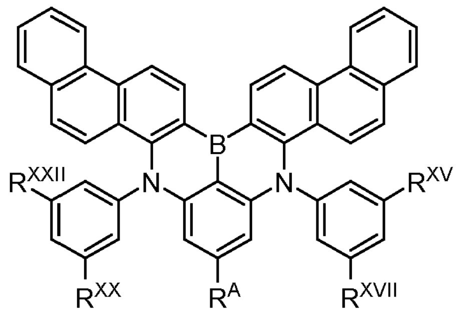

Formula $S^B$-III-3a-21

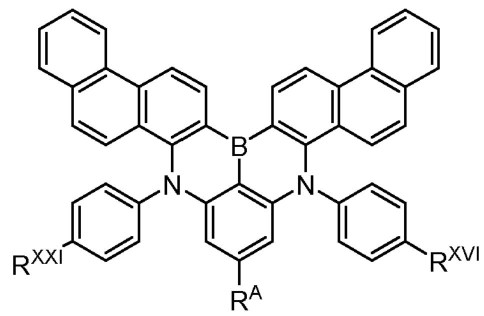

Formula $S^B$-III-3a-22

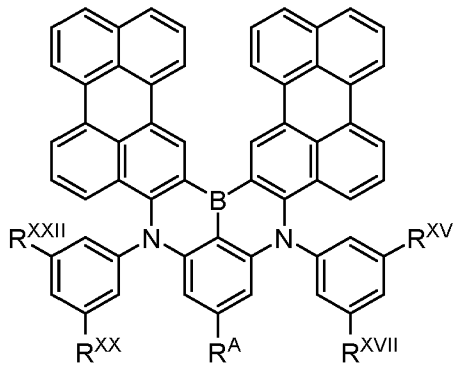

Formula $S^B$-III-3a-23

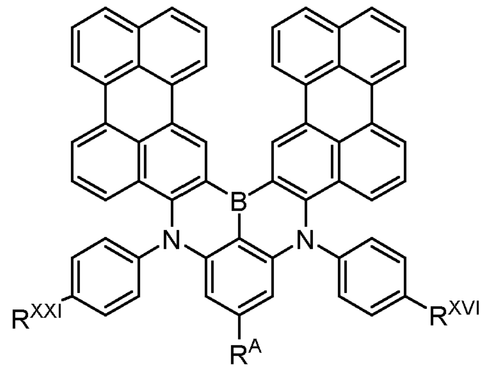

Formula $S^B$-III-3a-24

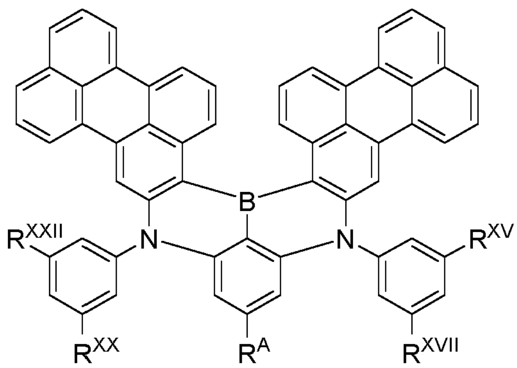

Formula $S^B$-III-3a-25

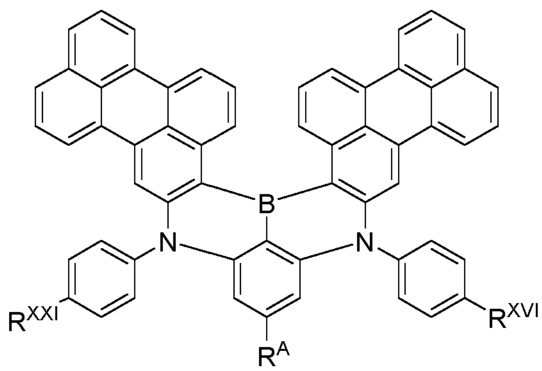

Formula $S^B$-III-3a-26

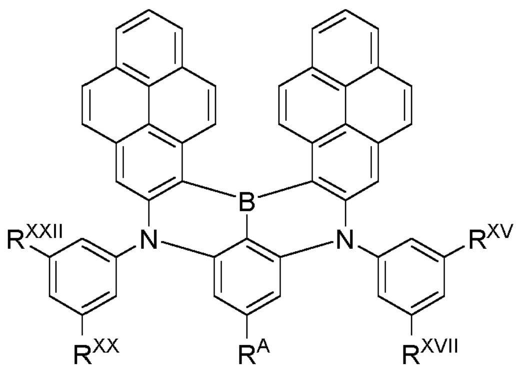

Formula $S^B$-III-3a-27

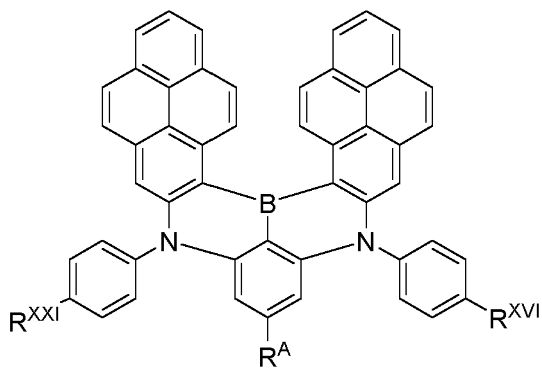

Formula $S^B$-III-3a-28

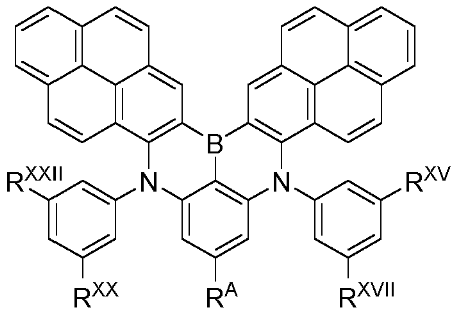

Formula $S^B$-III-3a-29

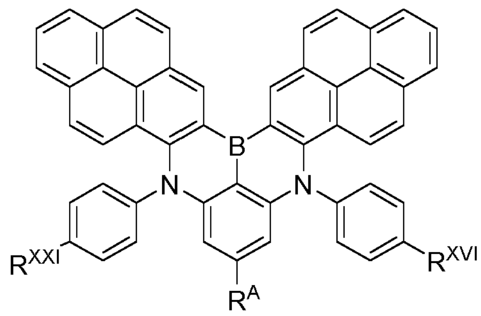

Formula $S^B$-III-3a-30.

In a preferred embodiment of the invention, each of the at least one small FWHM emitters $S^B$ comprises or consists of a structure according to any of formulas $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, and $S^B$-III-3a-10:

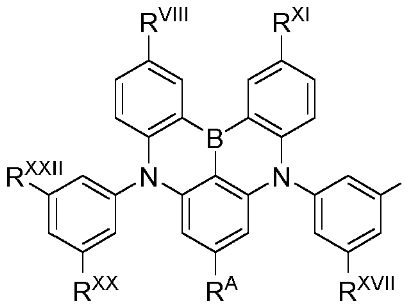

Formula $S^B$-III-3a-1

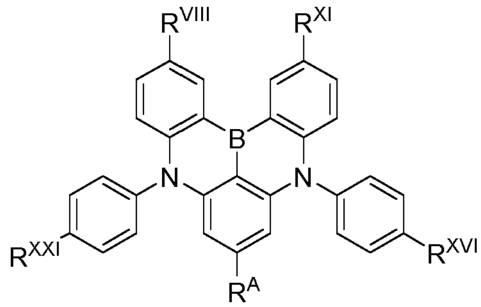

Formula $S^B$-III-3a-2

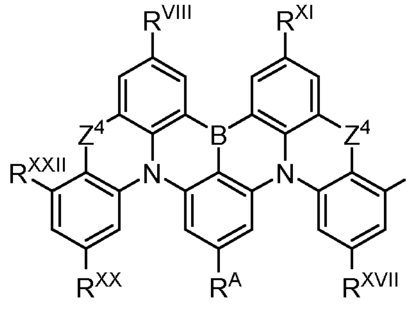

Formula $S^B$-III-3a-3

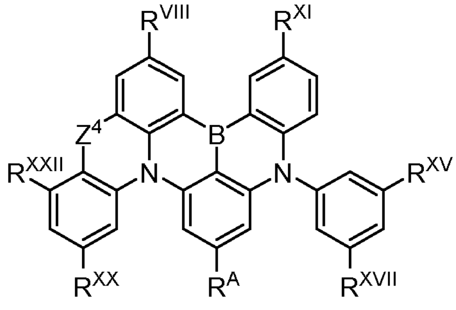

Formula $S^B$-III-3a-4

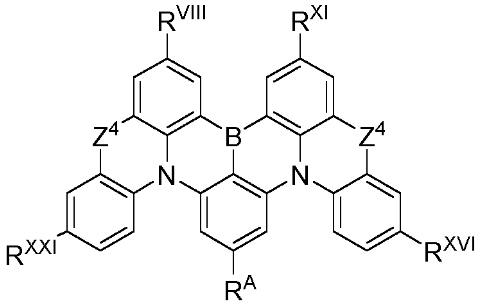

Formula $S^B$-III-3a-5

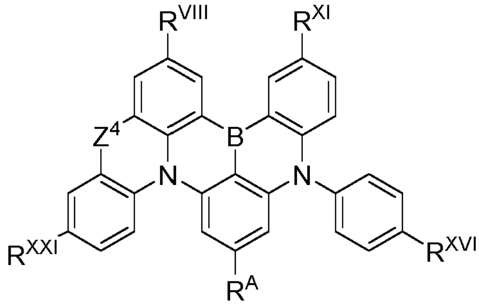

Formula $S^B$-III-3a-6

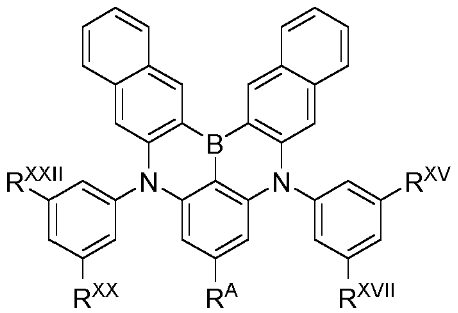

Formula $S^B$-III-3a-9

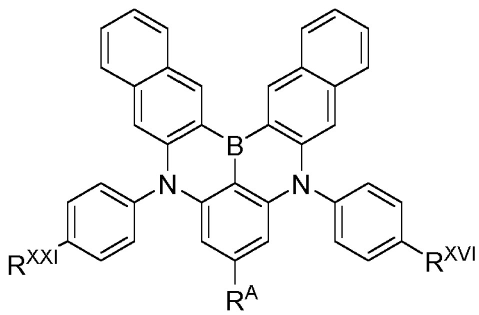

Formula $S^B$-III-3a-10.

In a preferred embodiment of the invention, each of the at least one small FWHM emitters $S^B$ comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-7, $S^B$-III-3a-8, $S^B$-III-3a-9, $S^B$-III-3a-10, $S^B$-III-3a-11, $S^B$-III-3a-12, $S^B$-III-3a-13, $S^B$-III-3a-14, $S^B$-III-3a-15, $S^B$-III-3a-16, $S^B$-III-3a-17, $S^B$-III-3a-18, $S^B$-III-3a-19, $S^B$-III-3a-20, $S^B$-III-3a-21, $S^B$-III-3a-22, $S^B$-III-3a-23, $S^B$-III-3a-24, $S^B$-III-3a-25, $S^B$-III-3a-26, $S^B$-III-3a-27, $S^B$-III-3a-28, $S^B$-III-3a-29, $S^B$-III-3a-30, wherein $R^A$ is hydrogen or has a structure represented by any of the formulas $R^A$-I, $R^A$-II, $R^A$-III, $R^A$-IV , $R^A$-V, $R^A$-VI, $R^A$-VII, $R^A$-VIII, $R^A$-IX, $R^A$-X, $R^A$-XI, $R^A$-XII, and $R^A$-XIII,

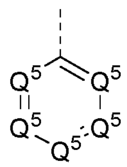

Formula $R^A$-I

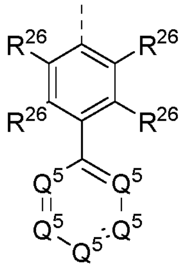

Formula $R^A$-II

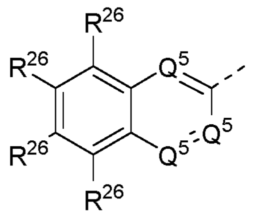

Formula $R^A$-III

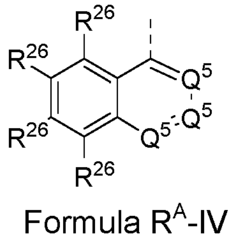

Formula $R^A$-IV

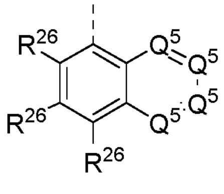

Formula $R^A$-V

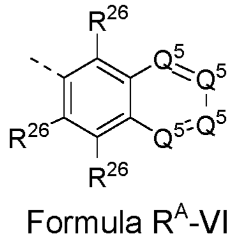

Formula $R^A$-VI

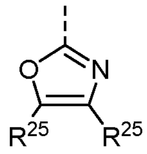

Formula $R^A$-VII

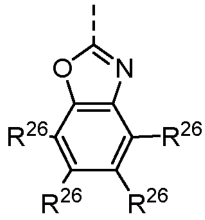

Formula $R^A$-VIII

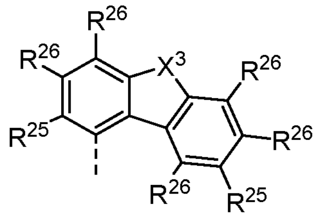

Formula $R^A$-IX

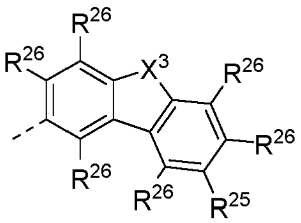

Formula $R^A$-X

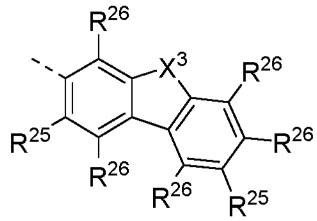

Formula $R^A$-XI

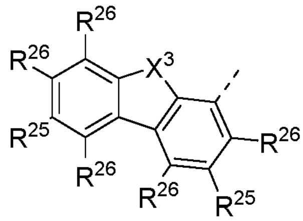

Formula $R^A$-XII

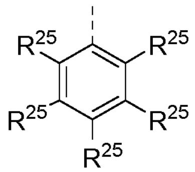

Formula $R^A$-XIII

wherein

the dashed line indicates the binding site to the core structure;
$Q^5$ is at each occurrence independently of each other selected from the group consisting of nitrogen (N) and $R^{25}$;
$X^3$ is at each occurrence independently of each other selected from the group consisting of oxygen (O), sulfur (S), $C(R^{25})_2$, and $NR^{25}$;
$R^{25}$ is at each occurrence independently of each other selected from the group consisting of: hydrogen, deuterium, halogen, $C_1$-$C_5$-alkyl, CN, $CF_3$, $SiMe_3$, $SiPh_3$ (Ph = phenyl), and
$C_6$-$C_{18}$-aryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl;
$C_3$-$C_{15}$-heteroaryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl;
$R^{26}$ is at each occurrence independently of each other selected from the group consisting of: hydrogen, deuterium, halogen, $C_1$-$C_5$-alkyl, CN, $CF_3$, $SiMe_3$, $SiPh_3$ (Ph = phenyl), and
$C_6$-$C_{18}$-aryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl;
$C_3$-$C_{15}$-heteroaryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl;
wherein two or more adjacent substituents $R^{25}$ and/or $R^{26}$ may optionally form an $C_3$-$C_{30}$-aromatic or a $C_3$-$C_{15}$-heteroaromatic ring system; and
wherein in formulas $R^A$-III, $R^A$-IV, $R^A$-V, and $R^A$-VI, at least one $Q^5$ is N; and
wherein in formulas $R^A$-III, $R^A$-IV, $R^A$-V, and $R^A$-VI, two adjacent groups $Q^5$ are not both N.

In an even more preferred embodiment of the invention, each of the at least one small FWHM emitters $S^B$ comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-7, $S^B$-III-3a-8, $S^B$-III-3a-9, $S^B$-III-3a-10, $S^B$-III-3a-11, $S^B$-III-3a-12, $S^B$-III-3a-13, $S^B$-III-3a-14, $S^B$-III-3a-15, $S^B$-III-3a-16, $S^B$-III-3a-17, $S^B$-III-3a-18, $S^B$-III-3a-19, $S^B$-III-3a-20, $S^B$-III-3a-21, $S^B$-III-3a-22, $S^B$-III-3a-23, $S^B$-III-3a-24, $S^B$-III-3a-25, $S^B$-III-3a-26, $S^B$-III-3a-27, $S^B$-III-3a-28, $S^B$-III-3a-29, $S^B$-III-3a-30,

wherein $R^A$ is hydrogen or has a structure represented by any of the formulas $R^A$-I, $R^A$-II, $R^A$-III, $R^A$-IV, $R^A$-V , $R^A$-VI,

$R^A$-VII, $R^A$-VIII, $R^A$-IX, $R^A$-X , $R^A$-XI, $R^A$-XII, and $R^A$-XIII, and wherein $R^{26}$ is at each occurrence hydrogen.

In a still even more preferred embodiment of the invention, each of the at least one small FWHM emitters $S^B$ comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-7, $S^B$-III-3a-8, $S^B$-III-3a-9, $S^B$-III-3a-10, $S^B$-III-3a-11, $S^B$-III-3a-12, $S^B$-III-3a-13, $S^B$-III-3a-14, $S^B$-III-3a-15, $S^B$-III-3a-16, $S^B$-III-3a-17, $S^B$-III-3a-18, $S^B$-III-3a-19, $S^B$-III-3a-20, $S^B$-III-3a-21, $S^B$-III-3a-22, $S^B$-III-3a-23, $S^B$-III-3a-24, $S^B$-III-3a-25, $S^B$-III-3a-26, $S^B$-III-3a-27, $S^B$-III-3a-28, $S^B$-III-3a-29, $S^B$-III-3a-30, wherein $R^A$ is hydrogen or has a structure represented by any of the formulas $R^A$-Ia, $R^A$-Ib, $R^A$-Ic, $R^A$-Id, $R^A$- Ie, $R^A$-If, $R^A$-Ig, $R^A$-IIIa, $R^A$-IIIb, $R^A$-IVa, $R^A$-IVb, $R^A$-Va, $R^A$-Vb, $R^A$-Vc, $R^A$-Vd, $R^A$-Ve, $R^A$-Vf, $R^A$-VIa, $R^A$-VIb, $R^A$-VIc, $R^A$-VId, $R^A$-VIe, $R^A$-VIf, $R^A$-VII, $R^A$-VIIIa, $R^A$-VIIIb, $R^A$-VIIIc, $R^A$-IXa, $R^A$-IXb, and $R^A$-IXc:

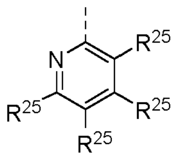
Formula $R^A$-Ia

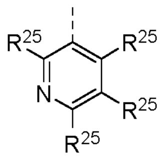
Formula $R^A$-Ib

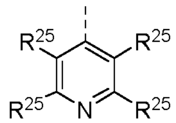
Formula $R^A$-Ic

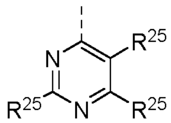
Formula $R^A$-Id

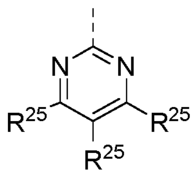
Formula $R^A$-Ie

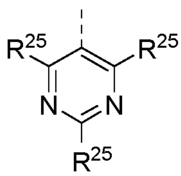
Formula $R^A$-If

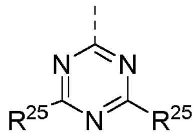
Formula $R^A$-Ig

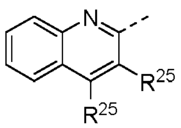
Formula $R^A$-IIIa

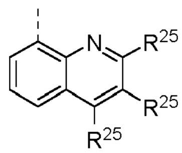
Formula $R^A$-Va

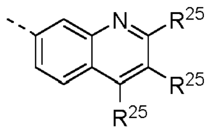
Formula $R^A$-VIa

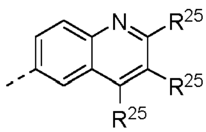
Formula $R^A$-VIb

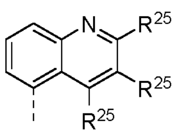
Formula $R^A$-Vb

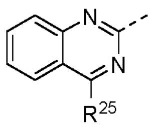
Formula $R^A$-IIIb

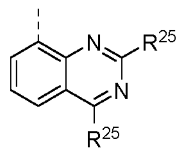
Formula $R^A$-Vc

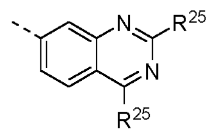
Formula $R^A$-VIc

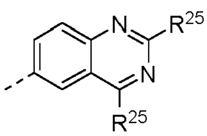
Formula $R^A$-VId

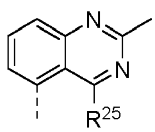
Formula $R^A$-Vd

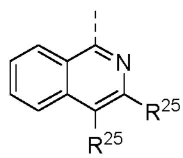
Formula $R^A$-IVa

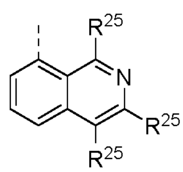
Formula $R^A$-Ve

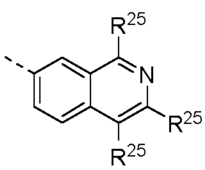
Formula $R^A$-VIe

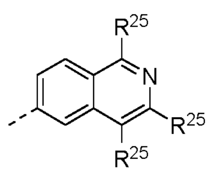
Formula $R^A$-VIf

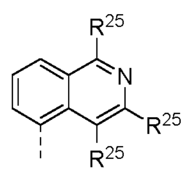
Formula $R^A$-Vf

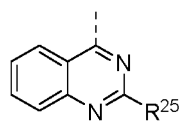
Formula $R^A$-IVb

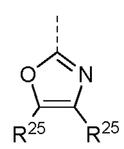
Formula $R^A$-VII

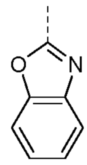
Formula $R^A$-VIIIa

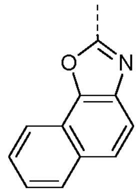
Formula $R^A$-VIIIb

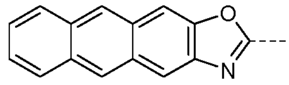
Formula $R^A$-VIIIc

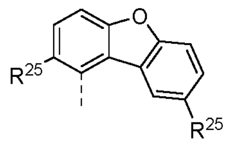
Formula $R^A$-IXa

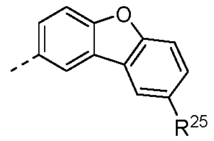
Formula $R^A$-Xa

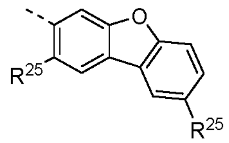
Formula $R^A$-XIa

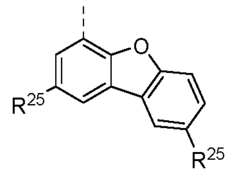
Formula $R^A$-XIIa

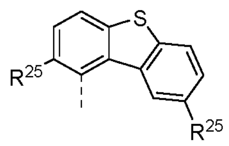
Formula $R^A$-IXb

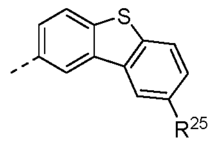
Formula $R^A$-Xb

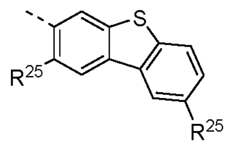
Formula $R^A$-XIb

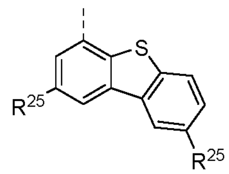
Formula $R^A$-XIIb

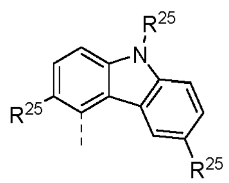
Formula $R^A$-IXc

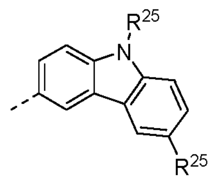
Formula $R^A$-Xc

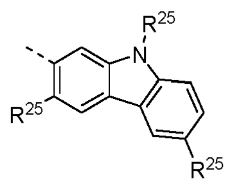
Formula $R^A$-XIc

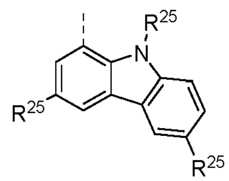
Formula $R^A$-XIIc

wherein

the dashed line indicates the binding site to the core structure;
$R^{25}$ is at each occurrence independently of each other selected from the group consisting of: hydrogen, deuterium, $C_1$-$C_5$-alkyl, CN, $CF_3$, $SiMe_3$, $SiPh_3$ (Ph = phenyl), and
$C_6$-$C_{18}$-aryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl;
$C_3$-$C_{15}$-heteroaryl, which is optionally substituted with one or more substituents independently of each other selected from the group consisting of $C_1$-$C_5$-alkyl, CN, $CF_3$, $C_6$-$C_{18}$-aryl, and $C_3$-$C_{15}$-heteroaryl; and wherein
adjacent groups $R^{25}$ do not combine to form any kind of additional ring system.

In a still even more preferred embodiment of the invention, $R^{25}$ is at each occurrence independently of each other selected from the group consisting of: hydrogen, deuterium, Me, $^{i}$Pr, $^{t}$Bu, CN, $CF_3$, $SiMe_3$, $SiPh_3$ (Ph = phenyl), and

$C_6$-$C_{18}$-aryl, which is optionally substituted with one or more substituents independently of each other selected from the group consisting of Me, $^{i}$Pr, $^{t}$Bu, CN, $CF_3$, and Ph;
$C_3$-$C_{15}$-heteroaryl, which is optionally substituted with one or more substituents independently of each other selected from the group consisting of , Me, $^{i}$Pr, $^{t}$Bu, CN, $CF_3$, $C_6$-$C_{18}$-aryl, and Ph; and wherein
adjacent groups $R^{25}$ do not combine to form any kind of additional ring system.

In a still even more preferred embodiment of the invention, $R^{25}$ is at each occurrence independently of each other selected from the group consisting of: hydrogen, deuterium, Me, $^{i}$Pr, $^{t}$Bu, CN, $CF_3$, $SiMe_3$, $SiPh_3$ (Ph = phenyl), and

Ph, which is optionally substituted with one or more substituents independently of each other selected from the group

consisting of: Me, $^{i}Pr$, $^{t}Bu$, CN, $CF_3$, and Ph; and wherein
adjacent groups $R^{25}$ do not combine to form any kind of additional ring system.

In one embodiment , $R^{VI}$, $R^{VII}$, $R^{VIII}$, $R^{IX}$, $R^{X}$, $R^{XI}$, $R^{XII}$, $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, halogen, CN, $CF_3$, $SiMe_3$, $SiPh_3$,

$C_1$-$C_5$-alkyl,
wherein one or more hydrogen atoms are optionally substituted by deuterium;
$C_6$-$C_{18}$-aryl,
wherein optionally one or more hydrogen atoms are independently substituted $C_1$-$C_5$-alkyl, $C_6$-$C_{18}$-aryl, $C_3$-$C_{17}$-heteroaryl, CN or $CF_3$;
$C_3$-$C_{15}$-heteroaryl,
wherein optionally one or more hydrogen atoms are independently substituted by $C_1$-$C_5$-alkyl, $C_6$-$C_{18}$-aryl, $C_3$-$C_{17}$-heteroaryl, CN or $CF_3$; and
$N(Ph)_2$;
wherein, optionally, at least one pair of adjacent groups $R^{VI}$ and $R^{VII}$, $R^{VII}$and $R^{VIII}$, $R^{VIII}$and $R^{IX}$ forms an aromatic ring system which is fused to the adjacent benzene ring a of formula $S^B$-III-3a and/or, optionally, at least one pair of adjacent groups $R^{X}$ and $R^{XI}$, $R^{XI}$ and $R^{XII}$, $R^{XII}$ and $R^{XIII}$ forms an aromatic ring system which is fused to the adjacent benzene ring b of general formula $S^B$-III-3a;
wherein each of the optionally so formed aromatic ring systems comprises 3 to 30 carbon atoms and is optionally substituted with one or more substituents $R^{21}$; and wherein it is particularly preferred that the optionally so formed two aromatic ring systems are identical; and
wherein optionally one or both pairs $R^{VI}$ and $R^{XXIII}$, $R^{XIII}$ and $R^{XIV}$ are joint to form a group $Z^4$ which is at each occurrence independently of each other selected from the group consisting of: a direct bond, $CR^{22}R^{23}$, $C=CR^{22}R^{23}$, C=O, $C=NR^{22}$, $NR^{22}$, O, $SiR^{22}R^{23}$, S, S(O), and $S(O)_2$;
wherein $R^{21}$, $R^{22}$, and $R^{23}$ are at each occurrence independently of each other selected from the group consisting of: hydrogen, deuterium, halogen, CN, $CF_3$, $SiMe_3$, $SiPh_3$,
$C_1$-$C_5$-alkyl,
wherein one or more hydrogen atoms are optionally substituted by deuterium;
$C_6$-$C_{18}$-aryl,
wherein optionally one or more hydrogen atoms are independently substituted $C_1$-$C_5$-alkyl, $C_6$-$C_{18}$-aryl, $C_3$-$C_{17}$-heteroaryl, CN or $CF_3$;
$C_3$-$C_{15}$-heteroaryl,
wherein optionally one or more hydrogen atoms are independently substituted by $C_1$-$C_5$-alkyl, $C_6$-$C_{18}$-aryl, $C_3$-$C_{17}$-heteroaryl, CN or $CF_3$; and
$N(Ph)_2$.

In a still even more preferred embodiment of the invention, each of the at least one small FWHM emitters $S^B$ comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, $S^B$-III-3a-10;

wherein $R^{VI}$, $R^{VII}$, $R^{VIII}$, $R^{IX}$, $R^{X}$, $R^{XI}$, $R^{XII}$ , $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, halogen, CN, $CF_3$, $SiMe_3$, $SiPh_3$,
$C_1$-$C_5$-alkyl,
wherein one or more hydrogen atoms are optionally substituted by deuterium;
$C_6$-$C_{18}$-aryl,
wherein optionally one or more hydrogen atoms are independently substituted $C_1$-$C_5$-alkyl, $C_6$-$C_{18}$-aryl, $C_3$-$C_{17}$-heteroaryl, CN or $CF_3$;
$C_3$-$C_{15}$-heteroaryl,
wherein optionally one or more hydrogen atoms are independently substituted by $C_1$-$C_5$-alkyl, $C_6$-$C_{18}$-aryl, $C_3$-$C_{17}$-heteroaryl, CN or $CF_3$; and
$N(Ph)_2$;
wherein, optionally, at least one pair of adjacent groups $R^{VI}$ and $R^{VII}$, $R^{VII}$and $R^{VIII}$, $R^{VIII}$and $R^{IX}$ forms an aromatic ring system which is fused to the adjacent benzene ring a of formula $S^B$-III-3a and/or, optionally, at least one pair of adjacent groups $R^{X}$ and $R^{XI}$, $R^{XI}$ and $R^{XII}$, $R^{XII}$ and $R^{XIII}$ forms an aromatic ring system which is fused to the adjacent benzene ring b of general formula $S^B$-III-3a;

wherein each of the optionally so formed aromatic ring systems comprises 3 to 30 carbon atoms and is optionally substituted with one or more substituents $R^{21}$; and wherein it is particularly preferred that the optionally so formed two aromatic ring systems are identical; and

wherein optionally one or both pairs $R^{VI}$ and $R^{XXIII}$, $R^{XIII}$ and $R^{XIV}$ are joint to form a group $Z^4$ which is at each occurrence independently of each other selected from the group consisting of: a direct bond, $CR^{22}R^{23}$, $C=CR^{22}R^{23}$, C=O, $C=NR^{22}$, $NR^{22}$, O, $SiR^{22}R^{23}$, S, S(O), and $S(O)_2$;

wherein $R^{21}$, $R^{22}$, and $R^{23}$ are at each occurrence independently of each other selected from the group consisting of: hydrogen, deuterium, halogen, CN, $CF_3$, $SiMe_3$, $SiPh_3$,

$C_1$-$C_5$-alkyl,

wherein one or more hydrogen atoms are optionally substituted by deuterium;

$C_6$-$C_{18}$-aryl,

wherein optionally one or more hydrogen atoms are independently substituted $C_1$-$C_5$-alkyl, $C_6$-$C_{18}$-aryl, $C_3$-$C_{17}$-heteroaryl, CN or $CF_3$;

$C_3$-$C_{15}$-heteroaryl,

wherein optionally one or more hydrogen atoms are independently substituted by $C_3$-$C_{17}$-alkyl, $C_3$-$C_{17}$-heteroaryl, CN or $CF_3$; and

$N(Ph)_2$.

In an even more preferred embodiment of the invention, each of the at least one small FWHM emitters $S^B$ comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, $S^B$-III-3a-10;

wherein $R^{VI}$, $R^{VII}$, $R^{VIII}$, $R^{IX}$, $R^X$, $R^{XI}$, $R^{XII}$ , $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, CN, $CF_3$, $SiMe_3$, $SiPh_3$, $N(Ph)_2$,

$C_1$-$C_5$-alkyl,

wherein one or more hydrogen atoms are optionally substituted by deuterium;

$C_6$-$C_{18}$-aryl,

wherein optionally one or more hydrogen atoms are independently substituted by Me, $^i$Pr, $^t$Bu, CN, $CF_3$ or Ph;

$C_3$-$C_{15}$-heteroaryl,

wherein optionally one or more hydrogen atoms are independently substituted by Me, $^i$Pr, $^t$Bu, CN, $CF_3$ or Ph; and wherein, optionally, at least one pair of adjacent groups $R^{VI}$ and $R^{VII}$, $R^{VII}$and $R^{VIII}$, $R^{VIII}$and $R^{IX}$ forms an aromatic ring system which is fused to the adjacent benzene ring a of formula $S^B$-III-3a and/or, optionally, at least one pair of adjacent groups $R^X$ and $R^{XI}$, $R^{XI}$ and $R^{XII}$, $R^{XII}$ and $R^{XIII}$ forms an aromatic ring system which is fused to the adjacent benzene ring b of general formula $S^B$-III-3a;

wherein each of the optionally so formed aromatic ring systems comprises 3 to 30 carbon atoms; and

wherein it is particularly preferred that the optionally so formed two aromatic ring systems are identical; and

wherein optionally one or both pairs $R^{VI}$ and $R^{XXIII}$, $R^{XIII}$ and $R^{XIV}$ are joint to form a group $Z^4$ which is at each occurrence independently of each other selected from the group consisting of: a direct bond, $CR^{22}R^{23}$, $C=CR^{22}R^{23}$, C=O, $C=NR^{22}$, $NR^{22}$, O, $SiR^{22}R^{23}$, S, S(O), and $S(O)_2$;

wherein $R^{22}$, and $R^{23}$ are at each occurrence independently of each other selected from the group consisting of: hydrogen, deuterium, CN, $CF_3$, $SiMe_3$, $SiPh_3$,

$C_1$-$C_5$-alkyl,

wherein one or more hydrogen atoms are optionally substituted by deuterium;

$C_6$-$C_{18}$-aryl,

wherein optionally one or more hydrogen atoms are independently substituted by Me, $^i$Pr, $^t$Bu, CN, $CF_3$ or Ph;

$C_3$-$C_{15}$-heteroaryl,

wherein optionally one or more hydrogen atoms are independently substituted by Me, $^i$Pr, $^t$Bu, CN, $CF_3$ or Ph.

In a preferred embodiment of the invention, $R^{VI}$, $R^{VII}$, $R^{VIII}$, $R^{IX}$, $R^X$, $R^{XI}$, $R^{XII}$, $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, Me, $^i$Pr, $^t$Bu, CN, $CF_3$, $N(Ph)_2$, and

Ph, wherein one or more hydrogen atoms are optionally substituted by deuterium, Me, $^i$Pr, $^t$Bu, CN, $CF_3$; and wherein optionally one or both pairs $R^{VI}$ and $R^{XXIII}$, $R^{XIII}$ and $R^{XIV}$ are joint to form a group $Z^4$ which is at each occurrence a direct bond.

In a particularly preferred embodiment of the invention, each of the at least one small FWHM emitters $S^B$

comprises or consists of a structure according to any of the formulas $S^B$-III-3a, $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, $S^B$-III-3a-10;

wherein $R^{VI}$, $R^{VII}$, $R^{VIII}$, $R^{IX}$, $R^{X}$, $R^{XI}$, $R^{XII}$ , $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, Me, $^i$Pr, $^t$Bu, CN, $CF_3$, $N(Ph)_2$, and
Ph, wherein one or more hydrogen atoms are optionally substituted by deuterium, Me, $^i$Pr, $^t$Bu, CN, $CF_3$; and wherein optionally one or both pairs $R^{VI}$ and $R^{XXIII}$, $R^{XIII}$ and $R^{XIV}$ are joint to form a group $Z^4$ which is at each occurrence a direct bond.

Examples of small FWHM emitters $S^B$ for use in organic electroluminescent devices according to the invention are listed below, whereat the invention is of course not limited to devices comprising one of these molecules.

Examples of small FWHM emitters $S^B$ according to formula $S^B$-III-3a-1 are listed below:

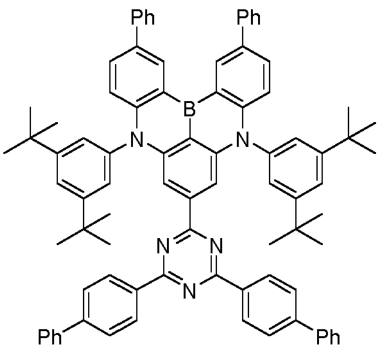
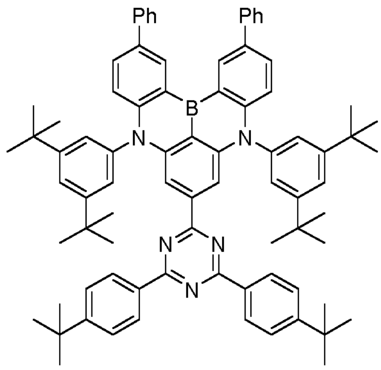
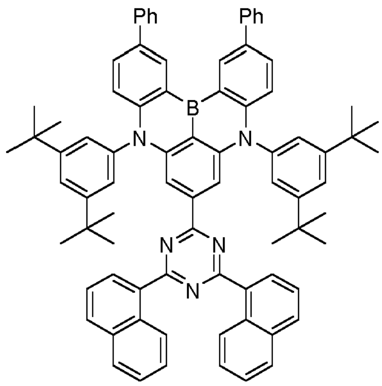

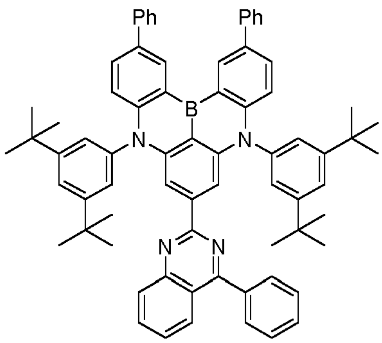
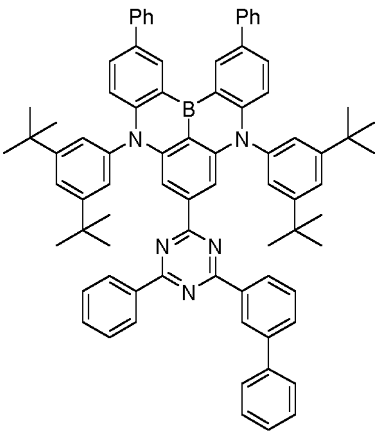
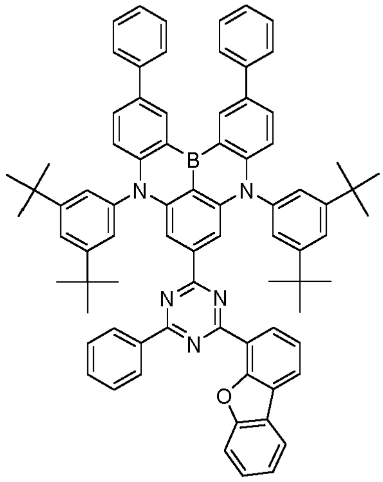

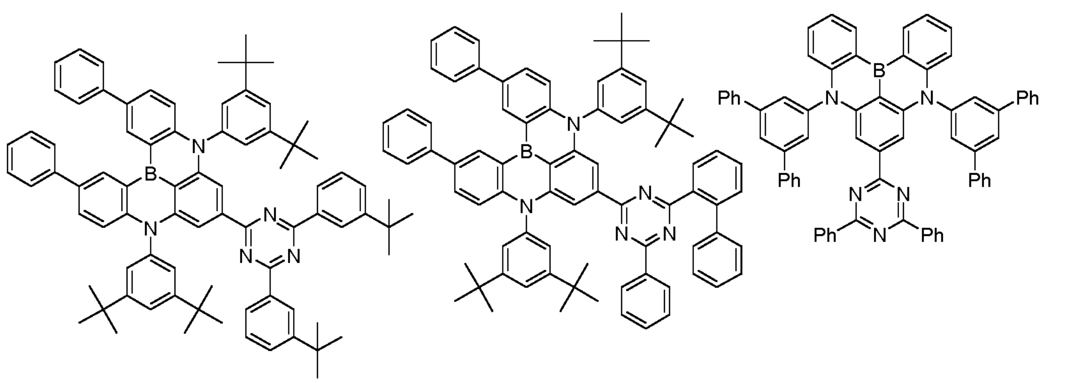

Examples of small FWHM emitters $S^B$ according to formula $S^B$-III-3a-2 are listed below:

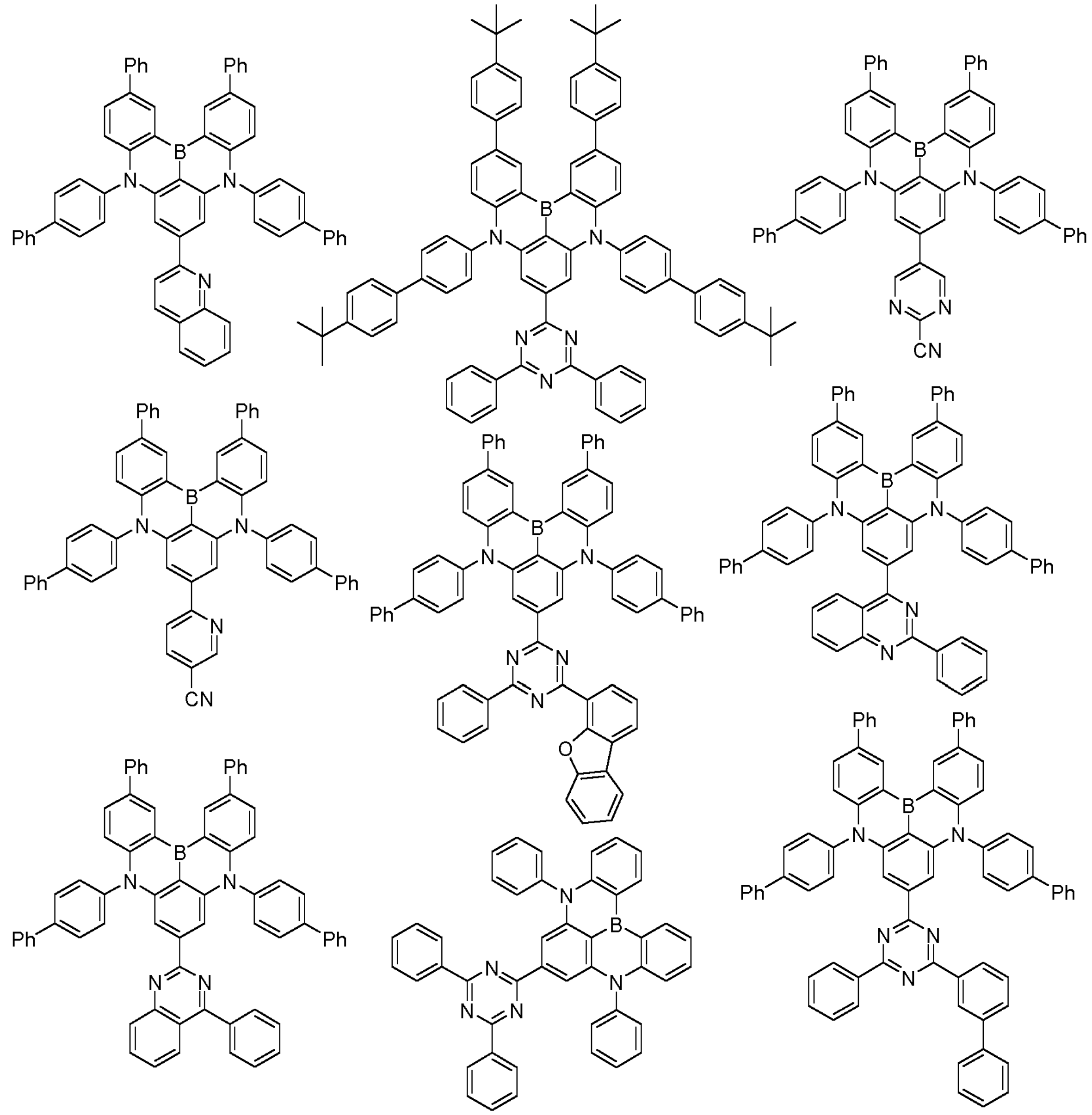

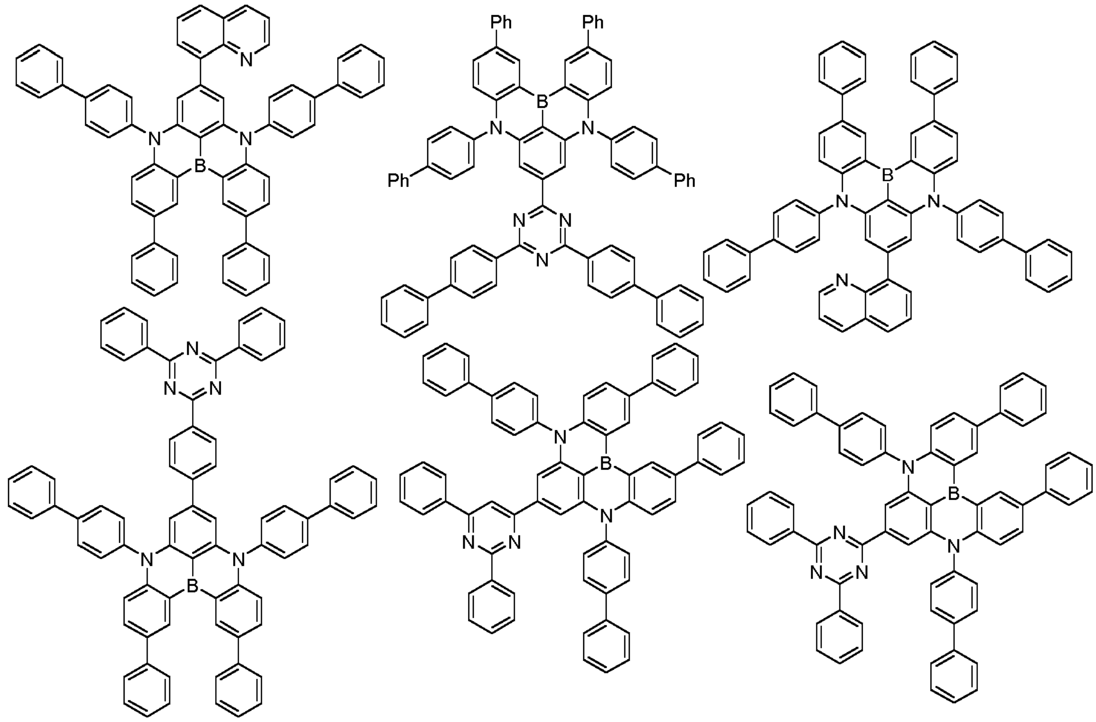

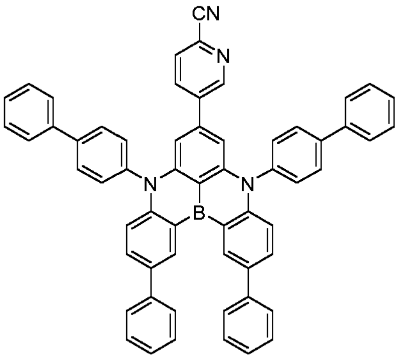

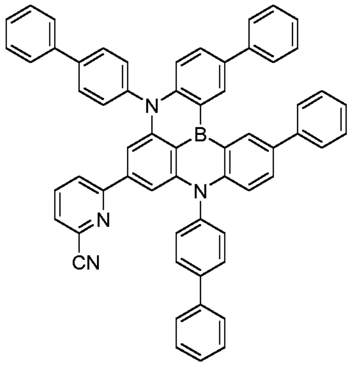

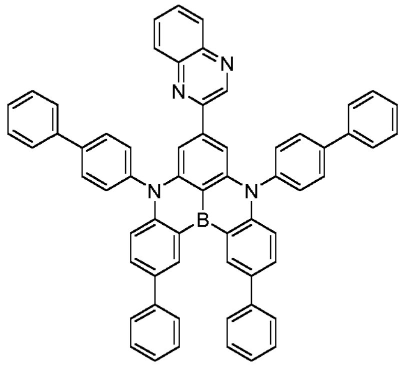

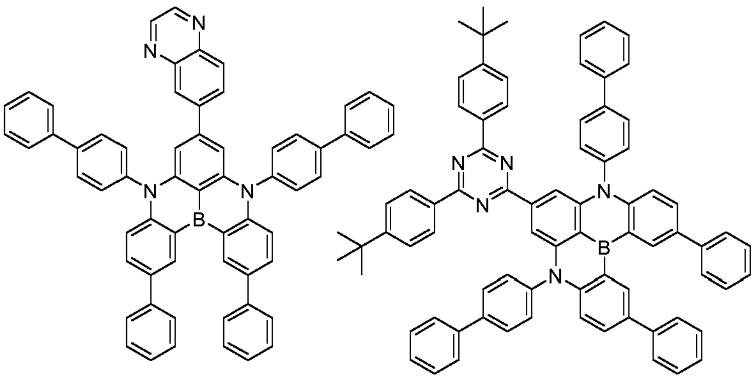

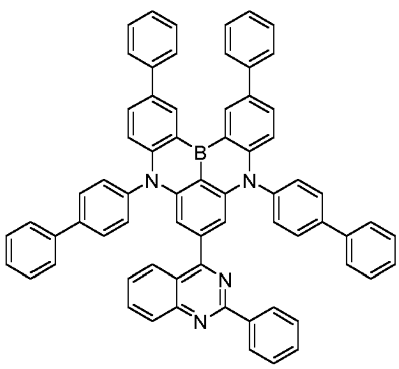

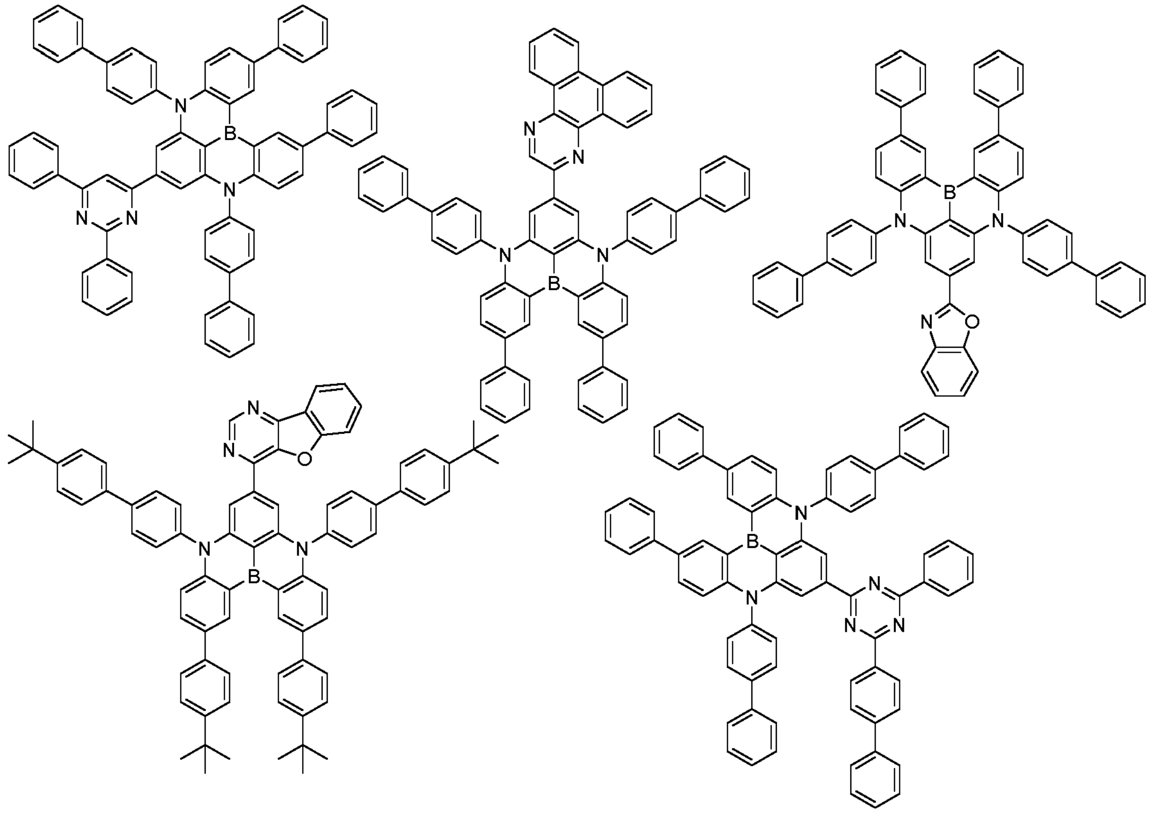

Examples of small FWHM emitters $S^B$ according to formulas $S^B$-III-3a-3, $S^B$-III-3a-4, $S^B$-III-3a-5, and $S^B$-III-3a-6 are listed below:

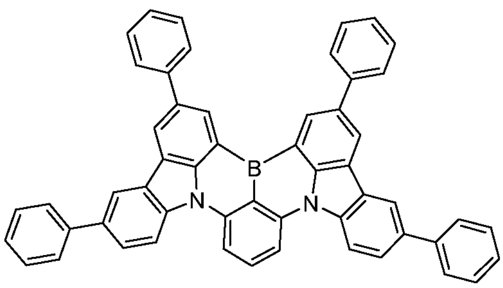

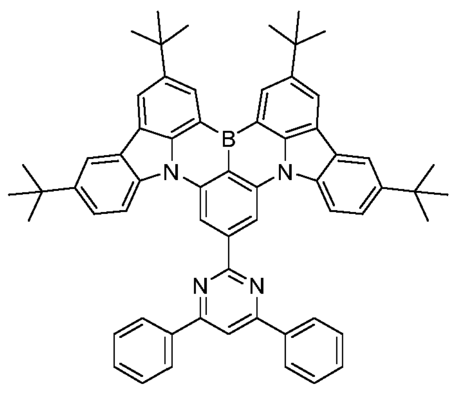

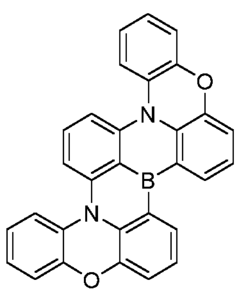

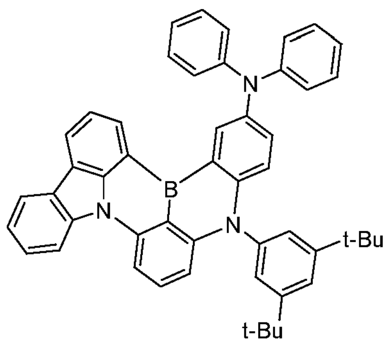

Examples of small FWHM emitters $S^B$ according to formulas $S^B$-III-3a-9, are listed below:

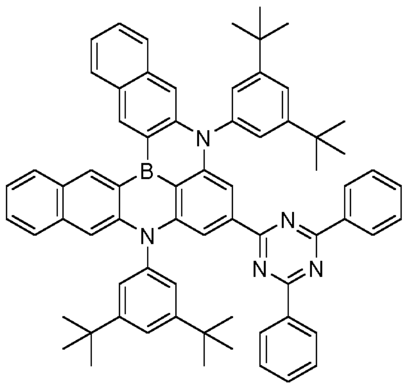

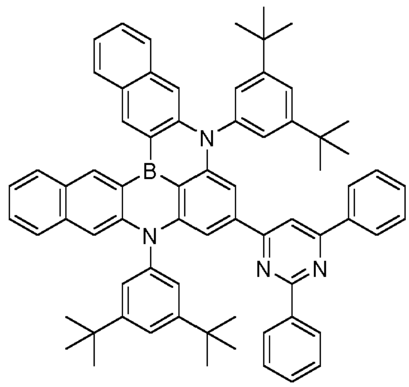

The synthesis of small FWHM emitters $S^B$ may be accomplished via standard reactions and reaction conditions known to the skilled artisan. An example of a typical reaction scheme for small FWHM emitters $S^B$ comprising or consisting of a structure of formula $S^B$-III-3a is described in the following, wherein $R^{XVIII} = R^{XIX}$, $R^{XVII} = R^{XX}$, $R^{XVI} = R^{XXI}$, $R^{XV} = R^{XXII}$, $R^{XIV} = R^{XXIII}$, $R^{XIII} = R^{VI}$, $R^{XII} = R^{VII}$, $R^{XI} = R^{VIII}$, and $R^{X} = R^{IX}$:

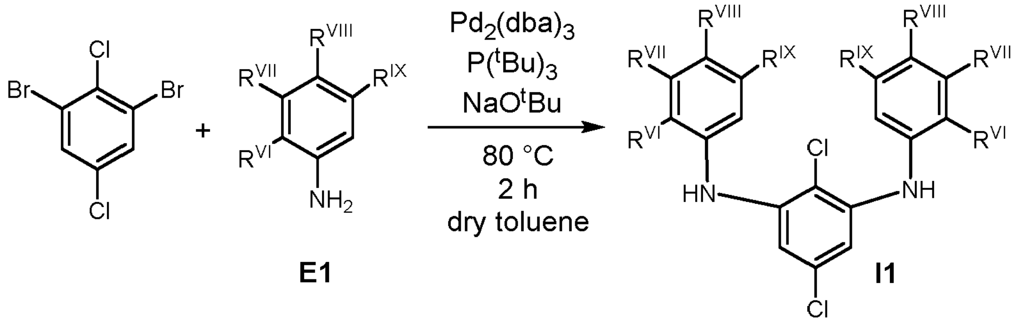


E1 I1

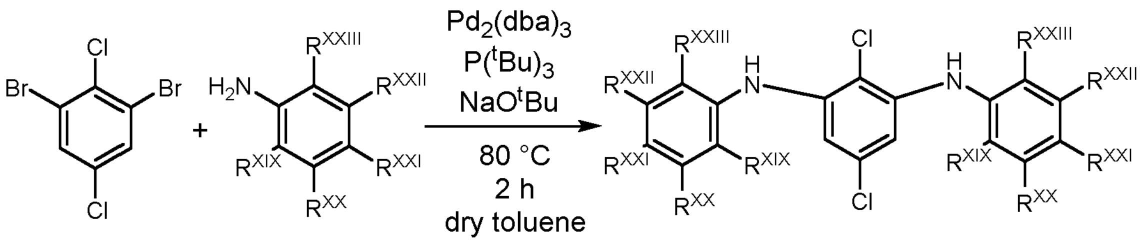


E1 I1

1,3-Dibromo-2,5-dichlorbenzene (CAS: 81067-41-6, 1.00 equivalents), E1 (2.20 equivalents), tris(dibenzylideneacetone)dipalladium $Pd_2(dba)_3$ (0.02 equivalents; CAS: 51364-51-3), tri-tert-butyl-phosphine ($P(^tBu)_3$, CAS: 13716-12-6 , 0.08 equivalents) and sodium tert-butoxide ($NaO^tBu$; 6.00 equivalents) are stirred under nitrogen atmosphere in toluene at 80 °C for 2 h. After cooling down to room temperature (rt) the reaction mixture is extracted with toluene and brine and the phases are separated. The combined organic layers are dried over $MgSO_4$ and then the solvent is removed under reduced pressure. The crude product obtained is purified by recrystallization or column chromatography and 11 is obtained as solid.

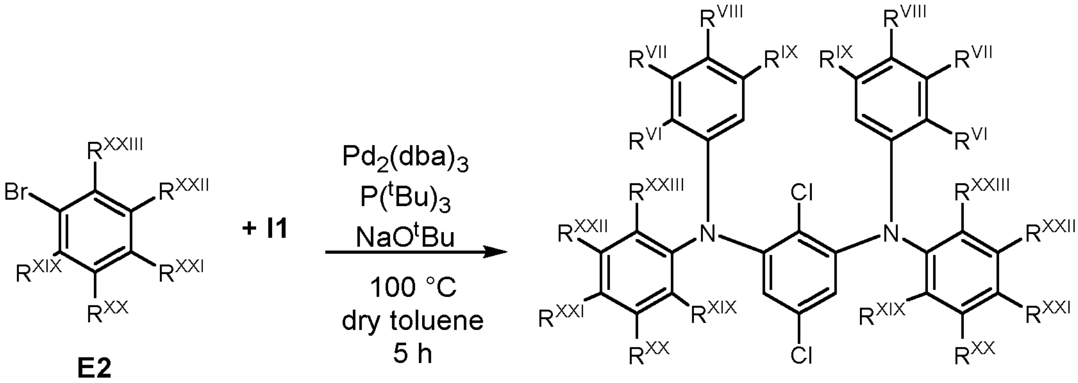


E2

I2

**11** (1.00 equivalents), **E2** (2.20 equivalents, tris(dibenzylideneacetone)dipalladium $Pd_2(dba)_3$ (0.02 equivalents; CAS: 51364-51-3), tri-tert-butyl-phosphine (0.08 equivalents, $P(^tBu)_3$, CAS: 13716-12-6) and sodium tert-butoxide ($NaO^tBu$; 5.00 equivalents) are stirred under nitrogen atmosphere in toluene at 100 °C for 5 h. After cooling down to room temperature (rt) the reaction mixture is extracted with toluene and brine and the phases are separated. The combined organic layers are dried over $MgSO_4$ and then the solvent is removed under reduced pressure. The crude product obtained is purified by recrystallization or column chromatography and 12 is obtained as solid.

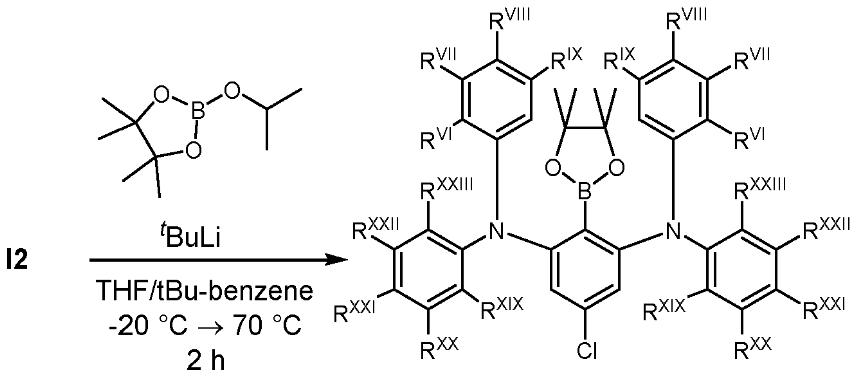


I3

After dissolving 12 (1 equivalent) under nitrogen atmosphere in THF and cooling to -20 °C or in tert-butylbenzene and cooling to -10 °C, $^tBuLi$ (2 equivalents, CAS: 594-19-4) is added and the reaction mixture is stirred at 0 °C. After complete lithiation, the reaction is quenched and 1,3,2-dioxaborolane (2 equivalents, CAS: 61676-62-8) is added and the reaction mixture is stirred under reflux at 70 °C for 2 h. After cooling down to room temperature (rt), the reaction mixture is extracted between toluene and brine and the phases are separated. The combined organic layers are dried over $MgSO_4$ and then the solvent is removed under reduced pressure. The crude product obtained is purified by recrystallization or column chromatography and 13 is obtained as solid.

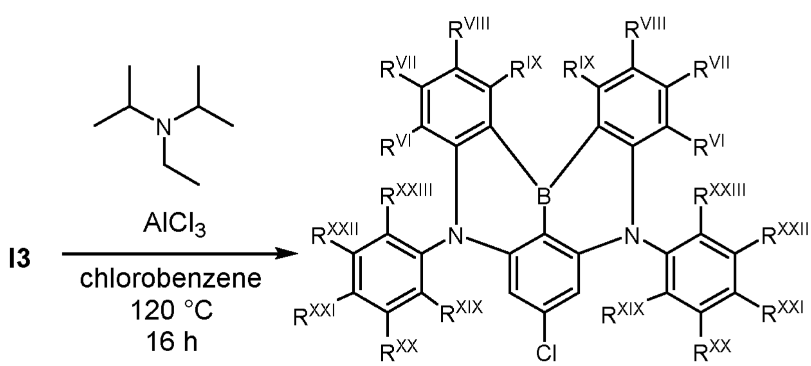


I4

**13** (1 equivalent), N,N-diisopropylethylamine (10 equivalents, CAS: 7087-68-5) and $AlCl_3$ (10 equivalents, CAS: 7446-70-0) are stirred under nitrogen atmosphere in chlorobenzene at 120°C for 16 h. After cooling down to room temperature (rt) the reaction mixture is extracted between toluene and brine and the phases are separated. The combined organic layers are dried over $MgSO_4$ and then the solvent is removed under reduced pressure. The crude product obtained is purified by recrystallization or column chromatography and **I4** is obtained as solid.

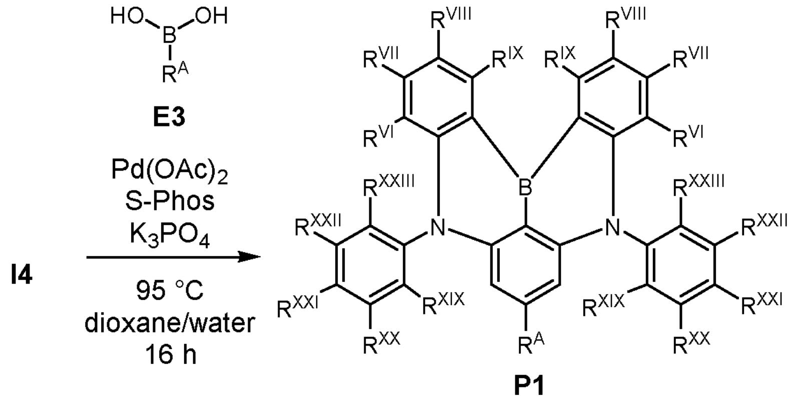


**I4** (1 equivalent), **E3** (1.1 equivalents), palladium(II) acetate (CAS: 3375-31-3, 0.1 equivalents), S-Phos (CAS: 657408-07-6, 0.24 equivalents) and potassium phosphate tribasic (5 equivalents) are stirred under nitrogen atmosphere in dioxane/water 5:1 at 100°C for 16 h. After cooling down to room temperature (rt) the reaction mixture is extracted between toluene and brine and the phases are separated. The combined organic layers are dried over $MgSO_4$ and then the solvent is removed under reduced pressure. The crude product obtained is purified by recrystallization or column chromatography and **P1** is obtained as solid.

Further FWHM emitters $S^B$ may be obtained analogously. An FWHM emitter $S^B$ may also be obtained by any alternative synthesis route suitable for this purpose.

In a preferred embodiment of the invention, each TADF material $E^B$ comprised in the at least one light-emitting layer B has a structure according to formula $E^B$-I and each FWHM emitter $S^B$ comprised in the at least one light-emitting layer B has a structure according to formula $S^B$-I.

In an even more preferred embodiment of the invention, each TADF material $E^B$ comprised in the at least one light-emitting layer B has a structure according to any of the formulas $E^B$-I-1, $E^B$-I-2, $E^B$-I-3, $E^B$-I-4, $E^B$-I-5, $E^B$-I-6, $E^B$-I-7, and $E^B$-I-8 and each FWHM emitter $S^B$ comprised in the at least one light-emitting layer B has a structure according to any of formulas $S^B$-II, $S^B$-III, and $S^B$-IV.

In a still even more preferred embodiment of the invention, each TADF material $E^B$ comprised in the at least one light-emitting layer B has a structure according to any of the formulas $E^B$-I-1a, $E^B$-I-2a, $E^B$-I-3a, $E^B$-I-4a, $E^B$-I-5a, $E^B$-I-6a, $E^B$-I-7, and $E^B$-I-8, and each FWHM emitter $S^B$ comprised in the at least one light-emitting layer B has a structure according to any of formula $S^B$-III-3a.

In a still even more preferred embodiment of the invention, each TADF material $E^B$ comprised in the at least one light-emitting layer B has a structure according to any of the formulas $E^B$-I-1a-1, $E^B$-I-2a-1, $E^B$-I-3a-1, $E^B$-I-4a-1, $E^B$-I-5a-1, $E^B$-I-6a-1, $E^B$-I-7, and $E^B$-I-8, and each FWHM emitter $S^B$ comprised in the at least one light-emitting layer B has a structure according to any of formula $S^B$-III-3a.

In a still even more preferred embodiment of the invention, each TADF material $E^B$ comprised in the at least one light-emitting layer B has a structure according to any of the formulas $E^B$-I-3a-1a, $E^B$-I-3a-1b, $E^B$-I-5a-1a, and $E^B$-I-6a-1a, and each FWHM emitter $S^B$ comprised in the at least one light-emitting layer B has a structure according to any of formulas $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, and $S^B$-III-3a-10.

In a particularly preferred embodiment of the invention, each TADF material $E^B$ comprised in the at least one light-emitting layer B has a structure according to any of the formulas $E^B$-I-3a-1a, $E^B$-I-3a-1b, $E^B$-I-5a-1a, and $E^B$-I-6a-1a, and each FWHM emitter $S^B$ comprised in the at least one light-emitting layer B has a structure according to any of formulas $S^B$-III-3a-1, $S^B$-III-3a-2, $S^B$-III-3a-3, $S^B$-III-3a-4, $S^B$-III-3a-5, $S^B$-III-3a-6, $S^B$-III-3a-9, and $S^B$-III-3a-10.

In a particularly preferred embodiment of the invention, each TADF material $E^B$ comprised in the at least one light-emitting layer B has a structure according to any of the particularly preferred examples shown herein and each FWHM emitter $S^B$ comprised in the at least one light-emitting layer B has a structure according to any of the particularly preferred examples shown herein.

Relations of HOMO- and LUMO-energy levels of components within the light-emitting layer(s) B

In a preferred embodiment of the invention, as far as at least one p-host $H^P$ is present in a light -emitting layer B, one or more or all of the relations expressed by the following formulas (20) to (22) preferably apply:

$$E^{LUMO}(H^P) > E^{LUMO}(E^B) \quad (20)$$

$$E^{HOMO}(H^P) \leq E^{HOMO}(S^B) \quad (21)$$

$$E^{LUMO}(H^P) > E^{LUMO}(S^B) \quad (22).$$

In a preferred embodiment of the invention, as far as at least one p-host $H^P$ and at least one n-host $H^N$ are present in a light -emitting layer B, one or more or all of the relations expressed by the following formulas (18) to (22) preferably apply:

$$E^{HOMO}(H^P) > E^{HOMO}(H^N) \quad (18)$$

$$E^{LUMO}(H^P) > E^{LUMO}(H^N) \quad (19)$$

$$E^{LUMO}(H^P) > E^{LUMO}(E^B) \quad (20)$$

$$E^{HOMO}(H^P) \leq E^{HOMO}(S^B) \quad (21)$$

$$E^{LUMO}(H^P) > E^{LUMO}(S^B) \quad (22).$$

In a preferred embodiment of the invention, as far as at least one p-host $H^P$ and at least one n-host $H^N$ and at least one bipolar host $H^{BP}$ are present in a light - emitting layer B, one or more or all of the relations expressed by the following formulas (18) to (23) preferably apply:

$$E^{HOMO}(H^P) > E^{HOMO}(H^N) \quad (18)$$

$$E^{LUMO}(H^P) > E^{LUMO}(H^N) \quad (19)$$

$$E^{LUMO}(H^P) > E^{LUMO}(E^B) \quad (20)$$

$$E^{HOMO}(H^P) \leq E^{HOMO}(S^B) \quad (21)$$

$$E^{LUMO}(H^P) > E^{LUMO}(S^B) \quad (22)$$

$$E^{LUMO}(H^P) > E^{LUMO}(H^{BP}) \quad (23).$$

Accordingly, a p-host $H^P$ optionally comprised in the at least one light-emitting layer B of an organic electroluminescent device according to the invention has a highest occupied molecular orbital HOMO($H^P$) having an energy $E^{HOMO}(H^P)$ which preferably is higher than the energy $E^{HOMO}(H^N)$ of the highest occupied molecular orbital HOMO($H^N$) of an n-host $H^N$ optionally comprised in the at least one light-emitting layer B of an organic electroluminescent device according to the invention.

Furthermore, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ which preferably is higher than the energy $E^{LUMO}(H^N)$ of the lowest unoccupied molecular orbital LUMO($H^N$) of an n-host $H^N$ optionally comprised in the at least one light-emitting layer B of an organic electroluminescent device

according to the invention.

Additionally, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ which preferably is higher than the energy $E^{LUMO}(E^B)$ of the lowest unoccupied molecular orbital LUMO($E^B$) of a TADF material $E^B$ comprised in the at least one light-emitting layer B of an organic electroluminescent device according to the invention.

A p-host $H^P$ optionally comprised in the at least one light-emitting layer B of an organic electroluminescent device according to the invention has a highest occupied molecular orbital HOMO($H^P$) having an energy $E^{HOMO}(H^P)$ which preferably is lower than or equal to the energy $E^{HOMO}(S^B)$ of the highest occupied molecular orbital HOMO($S^B$) of a small FWHM emitter $S^B$ comprised in the at least one light-emitting layer B of an organic electroluminescent device according to the invention.

Furthermore, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ which preferably is higher than the energy $E^{LUMO}(S^B)$ of the lowest unoccupied molecular orbital LUMO($S^B$) of a small FWHM emitter $S^B$ comprised in the at least one light-emitting layer B of an organic electroluminescent device according to the invention.

Additionally, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ which preferably is higher than the energy $E^{LUMO}(H^{BP})$ of the lowest unoccupied molecular orbital LUMO($H^{BP}$) of a bipolar host $H^{BP}$ optionally comprised in the at least one light-emitting layer B of an organic electroluminescent device according to the invention.

In a preferred embodiment of the invention, as far as a p-host $H^P$ is present in a light-emitting layer $E^B$, one or both of the relations selected from the group consisting of those expressed by the following formulas (26) and/or (27) preferably apply:

$$-0.3\ eV \leq E^{HOMO}(H^P) - E^{HOMO}(E^B) \leq 0.3\ eV \qquad (26)$$

$$E^{LUMO}(H^P) - E^{LUMO}(E^B) \geq 0.3\ eV \qquad (27).$$

In a preferred embodiment of the invention, as far as a p-host $H^P$ and n-host $H^N$ are present in a light-emitting layer $E^B$, one or more or all of the relations selected from the group consisting of those expressed by the following formulas (24) to (27) preferably apply:

$$E^{HOMO}(H^P) - E^{HOMO}(H^N) \geq 0.3\ eV \qquad (24)$$

$$E^{LUMO}(H^P) - E^{LUMO}(H^N) \geq 0.3\ eV \qquad (25)$$

$$-0.3\ eV \leq E^{HOMO}(H^P) - E^{HOMO}(E^B) \leq 0.3\ eV \qquad (26)$$

$$E^{LUMO}(H^P) - E^{LUMO}(E^B) \geq 0.3\ eV \qquad (27).$$

In a preferred embodiment of the invention, as far as a p-host $H^P$ and n-host $H^N$ and a bipolar host $H^{BP}$ are present in a light-emitting layer $E^B$, one or more or all of the relations selected from the group consisting of those expressed by the following formulas (24) to (28) preferably apply:

$$E^{HOMO}(H^P) - E^{HOMO}(H^N) \geq 0.3\ eV \qquad (24)$$

$$E^{LUMO}(H^P) - E^{LUMO}(H^N) \geq 0.3\ eV \qquad (25)$$

$$-0.3\ eV \leq E^{HOMO}(H^P) - E^{HOMO}(E^B) \leq 0.3\ eV \qquad (26)$$

$$E^{LUMO}(H^P) - E^{LUMO}(E^B) \geq 0.3\ eV \qquad (27)$$

$$E^{LUMO}(H^P) - E^{LUMO}(H^{BP}) \geq 0.3\ eV \qquad (28),$$

Accordingly, in a preferred embodiment of the invention, a p-host $H^P$ optionally comprised in the at least one light-emitting layer B of an organic electroluminescent device according to the invention has a highest occupied molecular orbital HOMO($H^P$) having an energy $E^{HOMO}(H^P)$ and an n-host $H^N$ optionally comprised in the at least one light-emitting layer B of an organic electroluminescent device according to the invention has a highest occupied molecular orbital HOMO($H^N$) having an energy $E^{HOMO}(H^N)$, wherein preferably: $E^{HOMO}(H^P) - E^{HOMO}(H^N) \geq 0.3$ eV. In other words, the energy difference between $E^{HOMO}(H^P)$ and $E^{HOMO}(H^N)$ preferably is equal to or larger than 0.3 eV.

In a preferred embodiment of the invention, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ and an n-host $H^N$ optionally comprised in the at least one light-emitting layer B of an organic electroluminescent device according to the invention has a lowest unoccupied molecular orbital LUMO($H^N$) having an energy $E^{LUMO}(H^N)$, wherein preferably: $E^{LUMO}(H^P) - E^{LUMO}(H^N) \geq 0.3$ eV. In other words, the energy difference between $E^{LUMO}(H^P)$ and $E^{LUMO}(H^N)$ preferably is equal to or larger than 0.3 eV.

In a preferred embodiment of the invention, a p-host $H^P$ optionally comprised in the at least one light-emitting layer B of an organic electroluminescent device according to the invention has a highest occupied molecular orbital HOMO($H^P$) having an energy $E^{HOMO}(H^P)$ and a TADF material $E^B$ comprised in the at least one light-emitting layer B of an organic electroluminescent device according to the invention has a highest occupied molecular orbital HOMO($E^B$) having an energy $E^{HOMO}(E^B)$, wherein preferably: $-0.3\ eV \leq E^{HOMO}(H^P) - E^{HOMO}(E^B) \leq 0.3$ eV. In other words, the HOMO($H^P$) of a p-host $H^P$ comprised in the at least one light-emitting layer B of an organic electroluminescent device according to the invention may be higher or lower in energy than the HOMO($E^B$) of a TADF emitter $E^B$ comprised in the at least one light-emitting layer B of an organic electroluminescent device according to the invention, but the energy difference does preferably not exceed 0.3 eV.

In a preferred embodiment of the invention, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ and a TADF material $E^B$ comprised in the at least one light-emitting layer B of an organic electroluminescent device according to the invention has a lowest unoccupied molecular orbital LUMO($E^B$) having an energy $E^{LUMO}(E^B)$, wherein preferably: $E^{LUMO}(H^P) - E^{LUMO}(E^B) \geq 0.3$ eV. In other words, the energy difference between $E^{LUMO}(H^P)$ and $E^{LUMO}(E^B)$ preferably is equal to or larger than 0.3 eV.

In a preferred embodiment of the invention, a p-host $H^P$ optionally comprised in the at least one light-emitting layer B of an organic electroluminescent device according to the invention has a lowest unoccupied molecular orbital LUMO($H^P$) having an energy $E^{LUMO}(H^P)$ and a bipolar host $H^{BP}$ optionally comprised in the at least one light-emitting layer B of an organic electroluminescent device according to the invention has a lowest unoccupied molecular orbital LUMO($H^{BP}$) having an energy $E^{LUMO}(H^{BP})$, wherein preferably: $E^{LUMO}(H^P) - E^{LUMO}(H^{BP}) \geq 0.3$ eV. In other words, the energy difference between $E^{LUMO}(H^P)$ and $E^{LUMO}(H^{BP})$ preferably is equal to or larger than 0.3 eV.

In a preferred embodiment of the invention, as far as an n-host $H^N$ is present in a light-emitting layer $E^B$, one or more or all of the relations expressed by the following formulas (29) to (32) preferably apply:

$$E^{HOMO}(H^N) \leq E^{HOMO}(E^B) \qquad (29)$$

$$E^{LUMO}(H^N) \leq E^{LUMO}(E^B) \qquad (30)$$

$$E^{HOMO}(H^N) < E^{HOMO}(S^B) \qquad (31)$$

$$E^{LUMO}(H^N) < E^{LUMO}(S^B) \qquad (32).$$

In a preferred embodiment of the invention, as far as an n-host $H^N$ and a bipolar host $H^{BP}$ are present in a light-emitting layer $E^B$, one or more or all of the relations expressed by the following formulas (29) to (33) preferably apply:

$$E^{HOMO}(H^N) \leq E^{HOMO}(E^B) \qquad (29)$$

$$E^{LUMO}(H^N) \leq E^{LUMO}(E^B) \quad (30)$$

$$E^{HOMO}(H^N) < E^{HOMO}(S^B) \quad (31)$$

$$E^{LUMO}(H^N) < E^{LUMO}(S^B) \quad (32)$$

$$E^{HOMO}(H^N) < E^{HOMO}(H^{BP}) \quad (33).$$

Accordingly, an n-host $H^N$ optionally comprised in the at least one light-emitting layer B of an organic electroluminescent device according to the invention has a highest occupied molecular orbital HOMO($H^N$) having an energy $E^{HOMO}(H^N)$ which preferably is lower than or equal to the energy $E^{HOMO}(E^B)$ of the highest occupied molecular orbital HOMO($E^B$) of a TADF material $E^B$ comprised in the at least one light-emitting layer B of an organic electroluminescent device according to the invention.

Furthermore, an n-host $H^N$ optionally comprised in the at least one light-emitting layer B of an organic electroluminescent device according to the invention has a lowest unoccupied molecular orbital LUMO($H^N$) having an energy $E^{LUMO}(H^N)$ which preferably is equal to or lower than the energy $E^{LUMO}(E^B)$ of the lowest unoccupied molecular orbital LUMO($E^B$) of a TADF material $E^B$ comprised in the at least one light-emitting layer B of an organic electroluminescent device according to the invention.

Additionally, an n-host $H^N$ optionally comprised in the at least one light-emitting layer B of an organic electroluminescent device according to the invention has a highest occupied molecular orbital HOMO($H^N$) having an energy $E^{HOMO}(H^N)$ which preferably is lower than the energy $E^{HOMO}(S^B)$ of the highest occupied molecular orbital HOMO($S^B$) of a small FWHM emitter $S^B$ comprised in the at least one light-emitting layer B of an organic electroluminescent device according to the invention.

An n-host $H^N$ optionally comprised in the at least one light-emitting layer B of an organic electroluminescent device according to the invention has a lowest unoccupied molecular orbital LUMO($H^N$) having an energy $E^{LUMO}(H^N)$ which preferably is lower than the energy $E^{LUMO}(S^B)$ of the lowest unoccupied molecular orbital LUMO($S^B$) of a small FWHM emitter $S^B$ comprised in the at least one light-emitting layer B of an organic electroluminescent device according to the invention.

Furthermore, an n-host $H^N$ optionally comprised in the at least one light-emitting layer B of an organic electroluminescent device according to the invention has a highest occupied molecular orbital HOMO($H^N$) having an energy $E^{HOMO}(H^N)$ which preferably is lower than the energy $E^{HOMO}(H^{BP})$ of the highest occupied molecular orbital HOMO($H^{BP}$) of a bipolar host $H^{BP}$ optionally comprised in the at least one light-emitting layer B of an organic electroluminescent device according to the invention.

In a preferred embodiment of the invention, as far as an n-host $H^N$ is present in a light-emitting layer $E^B$, one or more or all of the relations expressed by the following formulas (34) to (36) preferably apply:

$$E^{HOMO}(E^B) - E^{HOMO}(H^N) \geq 0.3 \text{ eV} \quad (34)$$

$$E^{LUMO}(E^B) - E^{LUMO}(H^N) \geq 0.2 \text{ eV} \quad (35)$$

$$E^{LUMO}(S^B) - E^{LUMO}(H^N) \geq 0.2 \text{ eV} \quad (36)$$

In a preferred embodiment of the invention, as far as an n-host $H^N$ and a bipolar host $H^{BP}$ are present in a light-emitting layer $E^B$, one or more or all of the relations expressed by the following formulas (34) to (37) apply:

$$E^{HOMO}(E^B) - E^{HOMO}(H^N) \geq 0.3 \text{ eV} \quad (34)$$

$$E^{LUMO}(E^B) - E^{LUMO}(H^N) \geq 0.2 \text{ eV} \quad (35)$$

$$E^{LUMO}(S^B) - E^{LUMO}(H^N) \geq 0.2\ eV \qquad (36)$$

$$E^{HOMO}(H^{BP}) - E^{HOMO}(H^N) \geq 0.3\ eV \qquad (37).$$

Accordingly, in a preferred embodiment of the invention, a TADF material $E^B$ comprised in the at least one light-emitting layer B of an organic electroluminescent device according to the invention has a highest occupied molecular orbital HOMO($E^B$) having an energy $E^{HOMO}(E^B)$ and an n-host $H^N$ optionally comprised in the at least one light-emitting layer B of an organic electroluminescent device according to the invention has a highest occupied molecular orbital HOMO($H^N$) having an energy $E^{HOMO}(H^N)$, wherein preferably: $E^{HOMO}(E^B) - E^{HOMO}(H^N) \geq 0.3$ eV. In other words, the energy difference between $E^{HOMO}(E^B)$ and $E^{HOMO}(H^N)$ preferably is equal to or larger than 0.3 eV.

In a preferred embodiment of the invention, a TADF material $E^B$ comprised in the at least one light-emitting layer B of an organic electroluminescent device according to the invention has a lowest unoccupied molecular orbital LUMO($E^B$) having an energy $E^{LUMO}(E^B)$ and an n-host $H^N$ optionally comprised in the at least one light-emitting layer B of an organic electroluminescent device according to the invention has a lowest unoccupied molecular orbital LUMO($H^N$) having an energy $E^{LUMO}(H^N)$, wherein preferably: $E^{LUMO}(E^B) - E^{LUMO}(H^N) \geq 0.2$ eV. In other words, the energy difference between $E^{LUMO}(E^B)$ and $E^{LUMO}(H^N)$ preferably is equal to or larger than 0.2 eV.

In a preferred embodiment of the invention, a small FWHM emitter $S^B$ comprised in the at least one light-emitting layer B of an organic electroluminescent device according to the invention has a lowest unoccupied molecular orbital LUMO($S^B$) having an energy $E^{LUMO}(S^B)$ and an n-host $H^N$ optionally comprised in the at least one light-emitting layer B of an organic electroluminescent device according to the invention has a lowest unoccupied molecular orbital LUMO($H^N$) having an energy $E^{LUMO}(H^N)$, wherein preferably: $E^{LUMO}(S^B) - E^{LUMO}(H^N) \geq 0.2$ eV. In other words, the energy difference between $E^{LUMO}(S^B)$ and $E^{LUMO}(H^N)$ preferably is equal to or larger than 0.2 eV.

In a preferred embodiment of the invention, a bipolar host $H^{BP}$ optionally comprised in the at least one light-emitting layer B of an organic electroluminescent device according to the invention has a highest occupied molecular orbital HOMO($H^{BP}$) having an energy $E^{HOMO}(H^{BP})$ and an n-host $H^N$ optionally comprised in the at least one light-emitting layer B of an organic electroluminescent device according to the invention has a highest occupied molecular orbital HOMO($H^N$) having an energy $E^{HOMO}(H^N)$, wherein preferably: $E^{HOMO}(H^{BP}) - E^{HOMO}(H^N) \geq 0.3$ eV. In other words, the energy difference between $E^{HOMO}(H^{BP})$ and $E^{HOMO}(H^N)$ preferably is equal to or larger than 0.3 eV.

In a preferred embodiment of the invention, as far as a bipolar host $H^{BP}$ is present in a light-emitting layer B, one, two or all of the relations expressed by the following formulas (38) to (40) preferably apply:

$$E^{HOMO}(H^{BP}) \leq E^{HOMO}(S^B) \qquad (38)$$

$$E^{LUMO}(H^{BP}) \leq E^{LUMO}(E^B) \qquad (39)$$

$$E^{LUMO}(H^{BP}) < E^{LUMO}(S^B) \qquad (40).$$

Accordingly, a bipolar host $H^{BP}$ optionally comprised in the at least one light-emitting layer B of an organic electroluminescent device according to the invention has a highest occupied molecular orbital HOMO($H^{BP}$) having an energy $E^{HOMO}(H^{BP})$ which preferably is lower than or equal to the energy $E^{HOMO}(S^B)$ of the highest occupied molecular orbital HOMO($S^B$) of a small FWHM emitter $S^B$ comprised in the at least one light-emitting layer B of an organic electroluminescent device according to the invention.

Furthermore, a bipolar host $H^{BP}$ optionally comprised in the at least one light-emitting layer B of an organic electroluminescent device according to the invention has a lowest unoccupied molecular orbital LUMO($H^{BP}$) having an energy $E^{LUMO}(H^{BP})$ which preferably is lower than or equal to the energy $E^{LUMO}(E^B)$ of the lowest unoccupied molecular orbital LUMO($E^B$) of a TADF material $E^B$ comprised in the at least one light-emitting layer B of an organic electroluminescent device according to the invention.

Additionally, a bipolar host $H^{BP}$ optionally comprised in the at least one light-emitting layer B of an organic electroluminescent device according to the invention has a lowest unoccupied molecular orbital LUMO($H^{BP}$) having an energy $E^{LUMO}(H^{BP})$ which preferably is lower than the energy $E^{LUMO}(S^B)$ of the lowest unoccupied molecular orbital LUMO($S^B$) of a small FWHM emitter $S^B$ comprised in the at least one light-emitting layer B of an organic electroluminescent device according to the invention.

In a preferred embodiment of the invention, as far as a bipolar host $H^{BP}$ is present in a light-emitting layer B, one, two or all of the relations expressed by the following formulas (41) to (43) preferably apply:

$$-0.3\ eV \leq E^{HOMO}(H^{BP}) - E^{HOMO}(E^{B}) \leq 0.3\ eV \quad (41)$$

$$E^{LUMO}(E^{B}) - E^{LUMO}(H^{BP}) \geq 0.2\ eV \quad (42)$$

$$E^{LUMO}(H^{BP}) - E^{LUMO}(S^{B}) \geq 0.2\ eV \quad (43).$$

Accordingly, in a preferred embodiment of the invention, a bipolar host $H^{BP}$ optionally comprised in the at least one light-emitting layer B of an organic electroluminescent device according to the invention has a highest occupied molecular orbital HOMO($H^{BP}$) having an energy $E^{HOMO}(H^{BP})$ and a **TADF** material $E^B$ comprised in the at least one light-emitting layer B of an organic electroluminescent device according to the invention has a highest occupied molecular orbital HOMO($E^B$) having an energy $E^{HOMO}(E^B)$, wherein preferably: $-0.3\ eV \leq E^{HOMO}(H^{BP}) - E^{HOMO}(E^B) \leq 0.3\ eV$. **In** other words, the HOMO($H^{BP}$) of a bipolar host $H^{BP}$ optionally comprised in the at least one light-emitting layer B of an organic electroluminescent device according to the invention may be higher or lower in energy than the HOMO($E^B$) of a TADF emitter $E^B$ comprised in the at least one light-emitting layer B of an organic electroluminescent device according to the invention, but the energy difference does preferably not exceed 0.3 eV.

Furthermore, a TADF material $E^B$ comprised in the at least one light-emitting layer B of an organic electroluminescent device according to the invention has a lowest unoccupied molecular orbital LUMO($E^B$) having an energy $E^{LUMO}(E^B)$ and a bipolar host $H^{BP}$ optionally comprised in the at least one light-emitting layer B of an organic electroluminescent device according to the invention has a lowest unoccupied molecular orbital LUMO($H^{BP}$) having an energy $E^{LUMO}(H^{BP})$, wherein preferably: $E^{LUMO}(E^B) - E^{LUMO}(H^{BP}) \geq 0.2\ eV$. In other words, the energy difference between $E^{LUMO}(E^B)$ and $E^{LUMO}(H^{BP})$ preferably is equal to or larger than 0.2 eV. Additionally, a bipolar host $H^{BP}$ optionally comprised in the at least one light-emitting layer B of an organic electroluminescent device according to the invention has a lowest unoccupied molecular orbital LUMO($H^{BP}$) having an energy $E^{LUMO}(H^{BP})$ and a TADF material $E^B$ comprised in the at least one light-emitting layer B of an organic electroluminescent device according to the invention has a lowest unoccupied molecular orbital LUMO($E^B$) having an energy $E^{LUMO}(E^B)$, wherein preferably: $E^{LUMO}(H^{BP}) - E^{LUMO}(S^B) \geq 0.2\ eV$. In other words, the energy difference between $E^{LUMO}(H^{BP})$ and $E^{LUMO}(E^B)$ preferably is equal to or larger than 0.2 eV.

In a preferred embodiment of the invention, the relations expressed by the following formulas (44) and (45) apply:

$$E^{HOMO}(E^{B}) \leq E^{HOMO}(S^{B}) \quad (44)$$

$$E^{LUMO}(E^{B}) < E^{LUMO}(S^{B}) \quad (45).$$

Accordingly, in a preferred embodiment of the invention, a TADF material $E^B$ comprised in the at least one light-emitting layer B of an organic electroluminescent device according to the invention has a highest occupied molecular orbital HOMO($E^B$) having an energy $E^{HOMO}(E^B)$ which preferably is equal to or lower than the energy $E^{HOMO}(S^B)$ of the highest occupied molecular orbital HOMO($S^B$) of a small FWHM emitter $S^B$ comprised in the at least one light-emitting layer B of an organic electroluminescent device according to the invention.

In a preferred embodiment of the invention, a TADF material $E^B$ comprised in the at least one light-emitting layer B of an organic electroluminescent device according to the invention has a lowest unoccupied molecular orbital LUMO($E^B$) having an energy $E^{LUMO}(E^B)$ which is lower than the energy $E^{LUMO}(S^B)$ of the lowest unoccupied molecular orbital LUMO($S^B$) of a small FWHM emitter $S^B$ comprised in the at least one light-emitting layer B of an organic electroluminescent device according to the invention.

In a preferred embodiment of the invention, the relation expressed by the following formula (46) applies:

$$E^{LUMO}(E^{B}) - E^{LUMO}(S^{B}) \geq 0.2\ eV \quad (46).$$

Accordingly, in a preferred embodiment of the invention, a TADF material $E^B$ comprised in the at least one light-emitting layer B of an organic electroluminescent device according to the invention has a lowest unoccupied molecular orbital LUMO($E^B$) having an energy $E^{LUMO}(E^B)$ and a small FWHM emitter $S^B$ comprised in the at least one light-emitting layer B of an organic electroluminescent device according to the invention has a lowest unoccupied molecular orbital LUMO($S^B$) having an energy $E^{LUMO}(S^B)$, wherein preferably: $E^{LUMO}(E^B) - E^{LUMO}(S^B) \geq 0.2\ eV$. In other words, the energy difference between $E^{LUMO}(E^B)$ and $E^{LUMO}(S^B)$ preferably is equal to or larger than 0.2 eV.

In an even more preferred embodiment of the invention, two, three, more than three or all relations expressed by

the above-mentioned formulas (1) to (23), (29) to (33), (38) to (40), (44), and (45) apply, wherein this does not imply that all species referred to in these relations are preferably comprised in the at least one light-emitting layer B of an organic electroluminescent device according to the invention, but instead that it is particularly preferred that all relations selected from the group of the listed relations which refer to any comprised species apply. For example, if no n-host $H^N$ is comprised in any of the at least one light-emitting layers B of an organic electroluminescent device according to the invention, it is understood, that the relations referring to an n-host $H^N$ do not apply in this particular case while they preferably apply whenever an n-host $H^N$ is comprised.

In a particularly preferred embodiment of the invention, two, three, more than three or all relations expressed by the above-mentioned formulas (1) to (46) apply, wherein this does not imply that all species referred to in these relations are preferably comprised in the at least one light-emitting layer B of an organic electroluminescent device according to the invention, but instead that it is particularly preferred that all relations selected from the group of relations which refer to any comprised species apply.

*Composition of the at least one light-emitting layer B*

The one or more hosts $H^B$ (e.g., one or more p-host $H^P$ and/or one or more n-hosts $H^N$ and/or one or more bipolar host(s) $H^{BP}$), one or more TADF emitters $E^B$, and one or more FWHM emitters $S^B$ may be comprised in the organic electroluminescent device in any amount and any ratio.

In a preferred embodiment of the invention, each of the at least one light-emitting layers B in an organic electroluminescent device according to the present invention comprises more of the at least one host material $H^B$ (more specific: $H^P$ and/or $H^N$ and/or $H^{BP}$), than of the at least one TADF material $E^B$, according to the weight.

In a preferred embodiment of the invention, each of the at least one light-emitting layers B in an organic electroluminescent device according to the present invention comprises more of the at least one TADF material $E^B$ than of the at least one small FWHM emitter $S^B$, according to the weight.

In a preferred embodiment, in an organic electroluminescent device according to the present invention, any of the at least one light-emitting layers B comprises (or consists of):

(i) 30-89.9 % by weight of one or more host compound $H^B$;
(ii) 10-60 % by weight of one or more TADF material $E^B$; and
(iii) 0.1-10 % by weight of one or more small FWHM emitter $S^B$; and optionally
(iv) 0-72 % by weight of one or more solvents.

In a preferred embodiment, wherein $H^N$ is optional, in an organic electroluminescent device according to the present invention, any of the at least one light-emitting layers B comprises (or consists of):

(i) 10-89.9 % by weight of one or more p-host compound $H^P$; optionally
(ii) 0-79.9 % by weight of one or more n-host compound $H^N$;
(iii) 10-50 % by weight of one or more TADF material $E^B$; and
(iv) 0.1-10 % by weight of one or more small FWHM emitter $S^B$; and optionally
(v) 0-72 % by weight of one or more solvents.

In an even more preferred embodiment, wherein $H^N$ is optional, in an organic electroluminescent device according to the present invention, any of the at least one light-emitting layer B comprises (or consists of):

(i) 22-87.5 % by weight of one or more p-host compound $H^P$; optionally
(ii) 0-65.5 % by weight of one or more n-host compound $H^N$;
(iii) 12-40 % by weight of one or more TADF material $E^B$; and
(iv) 0.5-5 % by weight of one or more small FWHM emitter $S^B$; and optionally
(v) 0-65.5 % by weight of one or more solvents.

In another preferred embodiment, wherein $H^N$ is necessary, in an organic electroluminescent device according to the present invention, the light-emitting layer B comprises (or consists of):

(i) 10-30 % by weight of one or more p-host compound $H^P$;
(ii) 40-79.9 % by weight of one or more n-host compound $H^N$;
(iii) 10-49 % by weight of one or more TADF material $E^B$; and
(iv) 0.1-10 % by weight of one or more small FWHM emitter $S^B$; and optionally
(v) 0-34 % by weight of one or more solvents.

In another preferred embodiment, wherein $H^N$ is necessary, in an organic electroluminescent device according to the present invention, the light-emitting layer B comprises (or consists of):

(i) 40-74 % by weight of one or more p-host compound $H^P$;
(ii) 10-30 % by weight of one or more n-host compound $H^N$;
(iii) 10-49 % by weight of one or more TADF material $E^B$; and
(iv) 0.1-10 % by weight of one or more small FWHM emitter $S^B$; and optionally
(v) 0-34 % by weight of one or more solvents.

As stated previously, it is understood that different light-emitting layers B optionally comprised in the same organic electroluminescent device according to the invention do not necessarily all comprise the same materials or even the same materials in the same ratios.

In one embodiment, the light-emitting layer comprises not only the organic molecules according to the invention, but also a host material whose triplet (T1) and singlet (S1) energy levels are energetically higher than the triplet (T1) and singlet (S1) energy levels of one or more other organic molecules, in particular the at least one TADF material $E^B$ and/or the at least one FWHM emitter $S^B$.

A further aspect of the invention relates to a composition comprising or consisting of:

(a) at least one FWHM emitter $S^B$ according to the invention, in particular in the form of an emitter and/or a host, and
(b) one or more emitter (including TADF material $E^B$) and/or host materials $H^B$, which differ from the organic molecule according to the invention and
(c) optional one or more dyes and/or one or more solvents.

In one embodiment, the light-emitting layer comprises (or essentially consists of) a composition comprising or consisting of:

(a) at least one FWHM emitter $S^B$ according to the invention, in particular in the form of an emitter and/or a host, and
(b) one or more emitter (including TADF material $E^B$) and/or host materials $H^B$, which differ from the organic molecule according to the invention and
(c) optional one or more dyes and/or one or more solvents.

In an embodiment, the light-emitting layer EML comprises (or essentially consists of) a composition comprising or consisting of:

(i) 0.1-10 % by weight, preferably 0.5-5 % by weight, in particular 1-3 % by weight, of one or more FWHM emitters $S^B$ according to the invention;
(ii) 5-99 % by weight, preferably 15-85 % by weight, in particular 20-75% by weight, of at least one host compound $H^B$; and
(iii) 0.9-94.9 % by weight, preferably 14.5-80 % by weight, in particular 24-77 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) up to 30 % by weight, in particular up to 20 % by weight, preferably up to 5 % by weight, of at least one further emitter molecule F (including TADF material $E^B$) with a structure differing from the structure of the molecules according to the invention.

Preferably, energy can be transferred from the host compound $H^B$ to the one or more FWHM emitters $S^B$ according to the invention, in particular transferred from the first excited triplet state $T1(H^B)$ of the host compound $H^B$ to the first excited triplet state $T1(S^B)$ of the one or more organic molecules according to the invention $S^B$ and/ or from the first excited singlet state $S1(H^B)$ of the host compound $H^B$ to the first excited singlet state $S1(S^B)$ of the one or more organic molecules according to the invention $S^B$.

*Device architecture*

The person skilled in the art will notice that the at least one light-emitting layer B will typically be incorporated in an organic electroluminescent device of the present invention. Preferably, such an organic electroluminescent device comprises at least the following layers: at least one light-emitting layer B, at least one anode layer A and at least one cathode layer C.

Preferably, at least one light-emitting layer B is located between an anode layer A and a cathode layer C.

Accordingly, the general set-up is preferably A - B - C. This does of course not exclude the presence of one or more optional further layers. These can be present at each side of A, of B and/or of C.

Preferably, the anode layer A is located on the surface of a substrate. The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. As at least one of both electrodes should be (essentially) transparent in order to allow light emission from the electroluminescent device (e.g., OLED). Usually, the anode layer A is mostly composed of materials allowing to obtain an (essentially) transparent film. Preferably, the anode layer A comprises a large content or even consists of transparent conductive oxides (TCOs).

Such an anode layer A may exemplarily comprise indium tin oxide, aluminum zinc oxide, fluor tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene and mixtures of two or more thereof.

Particularly preferably, the anode layer A (essentially) consists of indium tin oxide (ITO) (e.g., $(InO_3)_{0.9}(SnO_2)_{0.1}$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl) amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bis-phenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)-benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer B (serving as emitting layer (EML)). The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)-amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane (F4-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, tris-Pcz, CzSi (9-(4-tert-butylphenyl)-3,6-bis(triphenylsilyl)-9H-carbazole), and/or DCB (N,N'-dicarbazolyl-1,4-dimethylbenzene).

The composition of the at least one light-emitting layer B has been described above. Any of the one or more light-emitting layers B according to the invention preferably bears a thickness of not more than 1 mm, more preferably of not more than 0.1 mm, even more preferably of not more than 10 μm, even more preferably of not more than 1 μm, and particularly preferably of not more than 0.1 μm.

In the electron transport layer (ETL), any electron transporter may be used. Exemplarily, compounds poor of electrons such as, e.g., benzimidazoles, pyridines, triazoles, oxadiazoles (e.g., 1,3,4-oxadiazole), phosphinoxides and sulfone, may be used. Exemplarily, an electron transporter ETM may also be a star-shaped heterocycle such as 1,3,5-tri(1-phenyl-1H-benzo[d]imidazol-2-yl)phenyl (TPBi). The ETM may exemplarily be NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the electron transport layer may be doped with materials such as Liq (8-hydroxyquinolinolatolithium). Optionally, a second electron transport layer may be located between electron transport layer and the cathode layer C. The electron transport layer (ETL) may also block holes or a hole-blocking layer (HBL) is introduced.

The HBL may, for example, comprise HBM1:

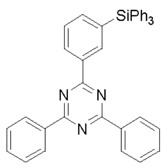,

BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), DTST (2,4-diphenyl-6-(3'-triphenylsilylphenyl)-1,3,5-triazine), DTDBF (2,8-bis(4,6-diphenyl-1,3,5-triazinyl)dibenzofurane) and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer C may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscale silver wires.

In a preferred embodiment, the organic electroluminescent device comprises at least the following layers:

A) an anode layer A containing at least one component selected from the group consisting of indium tin oxide, indium zinc oxide, PbO, SnO, graphite, doped silicium, doped germanium, doped GaAs, doped polyaniline, doped polypyrrole, doped polythiophene, and mixtures of two or more thereof;
B) a light-emitting layer B according to present invention as described herein; and
C) a cathode layer C containing at least one component selected from the group consisting of Al, Au, Ag, Pt, Cu, Zn, Ni, Fe, Pb, In, W, Pd, LiF, Ca, Ba, Mg, and mixtures or alloys of two or more thereof,

wherein the light-emitting layer B is located between the anode layer A and the a cathode layer C.

In one embodiment, when the organic electroluminescent device is an OLED, it may optionally comprise the following layer structure:

A) an anode layer A, exemplarily comprising indium tin oxide (ITO);
HTL) a hole transport layer HTL;
B) a light-emitting layer B according to present invention as described herein; ETL) an electron transport layer ETL; and
C) a cathode layer, exemplarily comprising Al, Ca and/or Mg.

Preferably, the order of the layers herein is A - HTL - B - ETL - C.

Furthermore, the organic electroluminescent device may optionally comprise one or more protective layers protecting the device from damaging exposure to harmful species in the environment including, exemplarily moisture, vapor and/or gases.

An electroluminescent device (e.g., an OLED) may further, optionally, comprise a protection layer between the electron transport layer (ETL) D and the cathode layer C (which may be designated as electron injection layer (EIL)). This layer may comprise lithium fluoride, caesium fluoride, silver, Liq (8-hydroxyquinolinolatolithium), $Li_2O$, $BaF_2$, MgO and/or NaF.

Unless otherwise specified, any of the layers of the various embodiments may be deposited by any suitable method. The layers in the context of the present invention, including the light-emitting layer B, may optionally be prepared by means of liquid processing (also designated as "film processing", "fluid processing", "solution processing" or "solvent processing"). This means that the components comprised in the respective layer are applied to the surface of a part of a device in liquid state. Preferably, the layers in the context of the present invention, including the light-emitting layer B, may be prepared by means of spin-coating. This method well-known to those skilled in the art allows obtaining thin and (essentially) homogeneous layers.

Alternatively, the layers in the context of the present invention, including the at least one light-emitting layer B, may be prepared by other methods based on liquid processing such as, e.g., casting (e.g., drop-casting) and rolling methods, and printing methods (e.g., inkjet printing, gravure printing, blade coating). This may optionally be carried out in

an inert atmosphere (e.g., in a nitrogen atmosphere).

In another preferred embodiment, the layers in the context of the present invention, including the at least one light-emitting layer B, may be prepared by any other method known in the art, including but not limited to vacuum processing methods well-known to those skilled in the art such as, e.g., thermal (co-)evaporation, organic vapor phase deposition (OVPD), and deposition by organic vapor jet printing (OVJP).

When preparing layers by means of liquid processing, the solutions including the components of the layers (i.e., with respect to the light-emitting layer B of the present invention, at least one host compound $H^B$, at least one TADF material $E^B$, and at least one small FWHM emitter $S^B$) may further comprise a volatile organic solvent. Such volatile organic solvent may optionally be one selected from the group consisting of tetrahydrofuran, dioxane, chlorobenzene, diethylene glycol diethyl ether, 2-(2-ethoxyethoxy)ethanol, gamma-butyrolactone, N-methyl pyrrolidinon, ethoxyethanol, xylene, toluene, anisole, phenetol, acetonitrile, tetrahydrothiophene, benzonitrile, pyridine, trihydrofuran, triarylamine, cyclohexanone, acetone, propylene carbonate, ethyl acetate, benzene and PGMEA (propylen glycol monoethyl ether acetate). Also a combination of two or more solvents may be used. After applied in liquid state, the layer may subsequently be dried and/or hardened by any means of the art, exemplarily at ambient conditions, at increased temperature (e.g., about 50 °C or about 60 °C) or at diminished pressure.

The organic electroluminescent device as a whole may also form a thin layer of a thickness of not more than 5 mm, more than 2 mm, more than 1 mm, more than 0.5 mm, more than 0.25 mm, more than 100 $\mu$m, or more than 10 $\mu$m.

An organic electroluminescent device (e.g., an OLED) may be a small-sized (e.g., having a surface not larger than 5 $mm^2$, or even not larger than 1 $mm^2$), medium-sized (e.g., having a surface in the range of 0.5 to 20 $cm^2$), or a large-sized (e.g., having a surface larger than 20 $cm^2$). An organic electroluminescent device (e.g., an OLED) according to the present invention may optionally be used for generating screens, as large-area illuminating device, as luminescent wallpaper, luminescent window frame or glass, luminescent label, luminescent poser or flexible screen or display. Next to the common uses, an organic electroluminescent device (e.g., an OLED) may exemplarily also be used as luminescent films, "smart packaging" labels, or innovative design elements. Further they are usable for cell detection and examination (e.g., as bio labelling).

*Further definitions and information*

As used throughout, the term "layer" in the context of the present invention preferably refers to a body that bears an extensively planar geometry.

As used herein, the terms organic electroluminescent device and optoelectronic light-emitting device may be understood in the broadest sense as any device comprising one or more light-emitting layers B, each comprising at least one host material $H^B$, at least one TADF material $E^B$, and at least one small FWHM emitter $S^B$, for all of which the above-mentioned definitions apply.

The organic electroluminescent device may be understood in the broadest sense as any device based on organic materials that is suitable for emitting light in the visible or nearest ultraviolet (UV) range, i.e., in the wavelength range from 380 to 800 nm. More preferably, an organic electroluminescent device may be able to emit light in the visible range, i.e., from 400 to 800 nm.

In a preferred embodiment of the invention, the organic electroluminescent device is a device selected from the group consisting of an organic light emitting diode (OLED), a light emitting electrochemical cell (LEC), and a light-emitting transistor.

Particularly preferably, the organic electroluminescent device is an organic light emitting diode (OLED). Optionally, the organic electroluminescent device as a whole may be intransparent, semi-transparent or (essentially) transparent.

As used throughout the present application, the term "aromatic ring system" may be understood in the broadest sense as any bi- or polycyclic aromatic moiety, for which the following definitions apply.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single

aromatic or heteroaromatic cycles, which formed the polycycle via a condensation reaction.

In particular, as used throughout the present application the term "aryl group" or "heteroaryl group" comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout the present application, the term "cyclic group" may be understood in the broadest sense as any mono-, bi- or polycyclic moieties.

As used above and herein, the term "alkyl group" may be understood in the broadest sense as any linear, branched, or cyclic alkyl substituent. In particular, the term alkyl comprises the substituents methyl (Me), ethyl (Et), n-propyl (nPr), i-propyl (iPr), cyclopropyl, n-butyl (nBu), i-butyl (iBu), s-butyl (sBu), t-butyl (tBu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used above and herein, the term "alkenyl" comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used above and herein, the term "alkynyl" comprises linear, branched, and cyclic alkynyl substituents. The term alkynyl group exemplarily comprises ethynyl, propynyl, butynyl, pentynyl, hexynyl, heptynyl or octynyl.

As used above and herein, the term "alkoxy" comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used above and herein, the term "thioalkoxy" comprises linear, branched, and cyclic thioalkoxy substituents, in which the O of the exemplarily alkoxy groups is replaced by S.

As used above and herein, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphthyl, dibenzofuryl) or as if it were the whole molecule (e.g. naphthalene, dibenzofuran). As used herein, these different ways of designating a substituent or attached fragment are considered to be equivalent.

If not stated otherwise, percentages refer to weight percentages ((weight/weight), (w/w), wt. %).

For host compounds $H^B$ (more specific: $H^P$ and $H^N$), the energy of the first excited triplet state T1 is determined from the onset of the time-gated emission spectrum at 77 K, typically with a delay time of 1 ms and an integration time of 1 ms, if not otherwise stated measured in a neat film of the host material $H^B$.

For TADF materials $E^B$, the energy of the first excited triplet state T1 is determined from the onset of the time-gated emission spectrum at 77 K, typically with a delay time of 1 ms and an integration time of 1 ms, if not otherwise stated measured in a film of poly(methyl methacrylate) (PMMA) with 10 % by weight of emitter.

For small full width at half maximum (FWHM) emitters $S^B$, the energy of the first excited triplet state T1 is determined from the onset of the time-gated emission spectrum at 77 K, typically with a delay time of 1 ms and an integration time of 1 ms, if not otherwise stated measured in a film of poly(methyl methacrylate) (PMMA) with 1 to 5 % by weight, in particular 1 % by weight of emitter.

Orbital and excited state energies can be determined by means of experimental methods known to the person skilled in the art. Experimentally, the energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$ , where $E^{gap}$ is determined as follows:

For host compounds $H^B$ (more specific: $H^P$ and $H^N$), the onset of emission of a neat film of the host material, which corresponds to the energy of the first excited singlet state S1, is used as $E^{gap}$, unless stated otherwise.

For TADF materials $E^B$, the onset of emission of a film with 10 % by weight of TADF material in poly(methyl methacrylate) (PMMA), which corresponds to the energy of the first excited singlet state S1, is used as $E^{gap}$, unless stated otherwise.

For small full width at half maximum (FWHM) emitters $S^B$, the onset of emission of a film with 1 to 5 % by weight, in particular 1 % by weight of small full width at half maximum (FWHM) emitter $S^B$ in poly(methyl methacrylate) (PMMA), which corresponds to the energy of the first excited singlet state S1, is used as $E^{gap}$, unless stated otherwise.

As used herein, if not defined more specifically in a particular context, the designation of the colors of emitted and/or absorbed light is as follows:

| | |
|---|---|
| violet: | wavelength range of >380-420 nm; |
| deep blue: | wavelength range of >420-475 nm; |
| sky blue: | wavelength range of >475-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

If not stated otherwise, with respect to small FWHM emitters $S^B$, such colors refer to the emission maximum $\lambda_{max}^{PMMA}$ of a poly(methyl methacrylate) (PMMA) film with 2% by weight of the emitter $S^B$. For TADF materials $E^B$, such colors refer to the emission maximum $\lambda_{max}^{PMMA}$ of a poly(methyl methacrylate) (PMMA) film with 10%, unless stated otherwise.

Examples

*Cyclic voltammetry*

Cyclic voltammograms of solutions having concentration of $10^{-3}$ mol/l of the organic molecules in dichloromethane or a suitable solvent and a suitable supporting electrolyte (e.g. 0.1 mol/l of tetrabutylammonium hexafluorophosphate) are measured. The measurements are conducted at room temperature and under nitrogen atmosphere with a three-electrode assembly (Working and counter electrodes: Pt wire, reference electrode: Pt wire) and calibrated using $FeCp_2/FeCp_2^+$ as internal standard. HOMO data was corrected using ferrocene as internal standard against SCE.

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration were used. The Turbomole program package was used for all calculations.

*Photophysical measurements*

Sample pretreatment: Spin-coating
Apparatus: Spin150, SPS euro.

The sample concentration is 10 mg/ml, dissolved in a suitable solvent.

Program: 1) 3 s at 400 U/min; 20 s at 1000 U/min at 1000 Upm/s. 3) 10 s at 4000 U/min at 1000 Upm/s. After coating, the films are tried at 70 °C for 1 min.

Photoluminescence spectroscopy and TCSPC *(Time-correlated single-photon*

*counting)*

Steady-state emission spectroscopy is recorded using a Horiba Scientific, Modell FluoroMax-4 equipped with a

150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.

Excitation sources:

NanoLED 370 (wavelength: 371 nm, puls duration: 1.1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) was done using the software suite DataStation and DAS6 analysis software. The fit is specified using the chi-squared-test.

*Photoluminescence quantum yield measurements*

For photoluminescence quantum yield (PLQY) measurements an *Absolute PL Quantum Yield Measurement C9920-03G* system *(Hamamatsu Photonics)* is used. Quantum yields and CIE coordinates were determined using the software U6039-05 version 3.6.0.

Emission maxima are given in nm, quantum yields Φ in % and CIE coordinates as x,y values.

PLQY was determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement

Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right] d\lambda}{\int \frac{\lambda}{hc}\left[Int_{emitted}^{reference}(\lambda) - Int_{absorbed}^{reference}(\lambda)\right] d\lambda}$$

wherein $n_{photon}$ denotes the photon count and Int. is the intensity.

*Production and characterization of organic electroluminescence devices*

Via vacuum-deposition methods OLED devices comprising organic molecules according to the invention can be produced. If a layer contains more than one compound, the weight-percentage of one or more compounds is given in %. The total weight-percentage values amount to 100 %, thus if a value is not given, the fraction of this compound equals to the difference between the given values and 100 %.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT97 to the time point, at which the measured luminance decreased to 97 % of the initial luminance etc.

Accelerated lifetime measurements are performed (e.g. applying increased current densities). Exemplarily LT80 values at 500 $cd/m^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given. Figures show the data series for one OLED pixel.

## Experimental results

### Stack materials

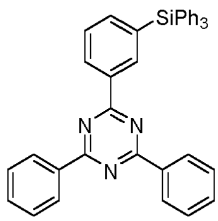

**HBM1 (Hole-blocking material)**

**Host materials $H^B$** (here for example p-hosts $H^P$)

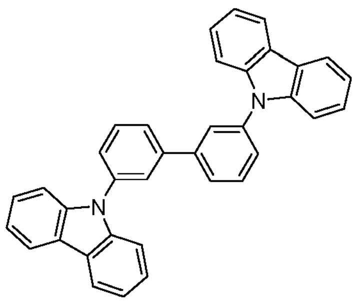

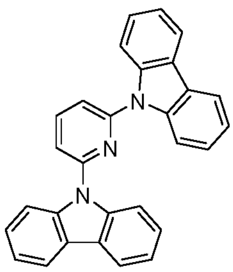

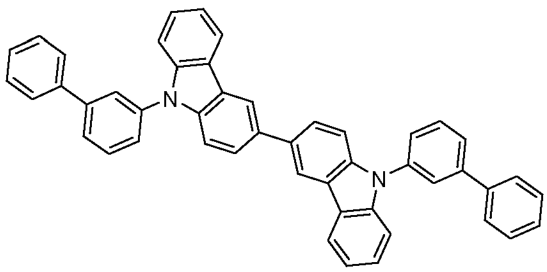

**mCBP = $H^P$-1** **PYD2 = $H^P$-2** **$H^P$-3**

**Table 1H.** Properties of the host materials.

| | Example compound | $E^{HOMO}$ [eV] | $E^{LUMO}$ [eV] | S1 [eV] | T1 [eV] | $\lambda_{max}^{PMMA}$ [nm] | FWHM [eV] |
|---|---|---|---|---|---|---|---|
| $H^P$ | **mCBP** | -6.02 | -2.42 | 3.6 | 2.82 | | |
| | **PYD2** | -6.08 | -2.55 | 3.53 | 2.81 | | |
| | **$H^P$-3** | -5.66 | -2.35 | 3.31 | 2.71 | | |

wherein $LUMO_{CV}$ is the energy of the lowest unoccupied molecular orbital, which is determined by cyclic voltammetry.

### TADF materials $E^B$

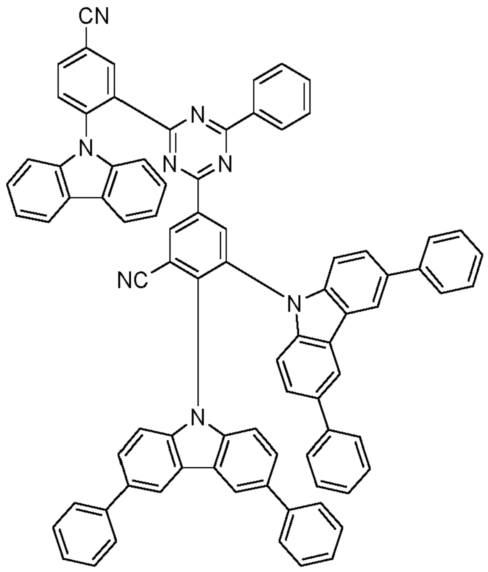

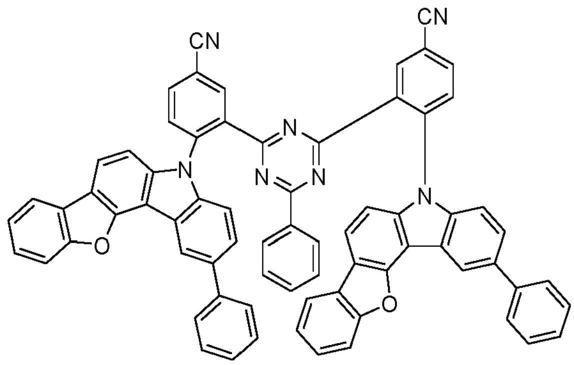

**E$^B$-1** **E$^B$-2**

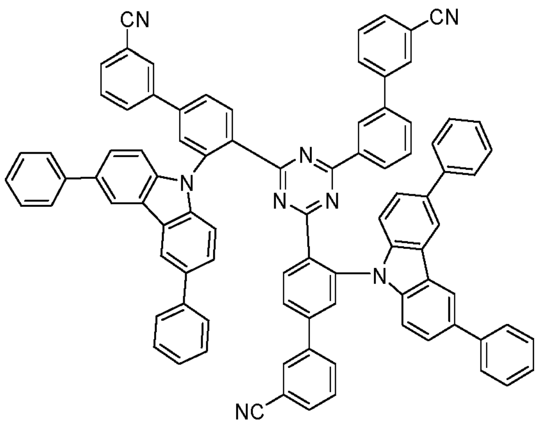

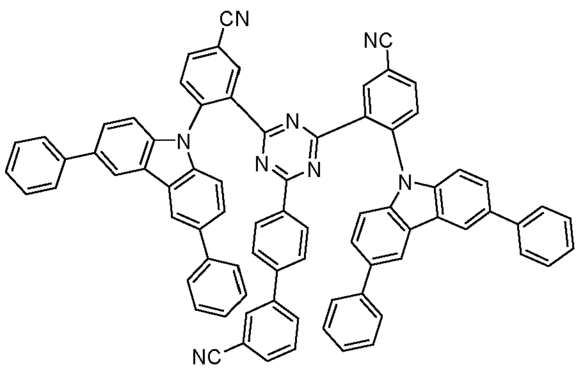

**E$^B$-3** **E$^B$-4**

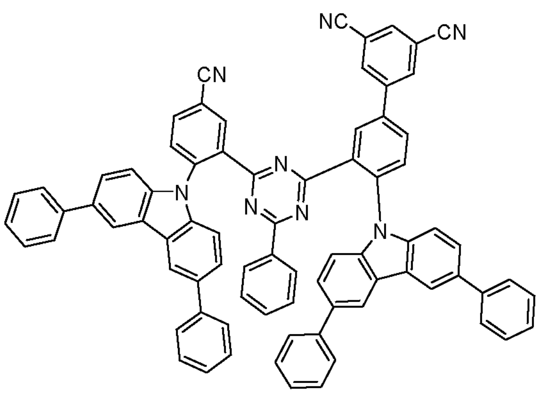

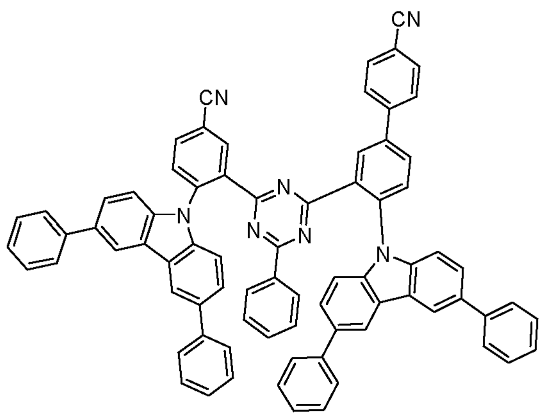

**E$^B$-5** **E$^B$-6**

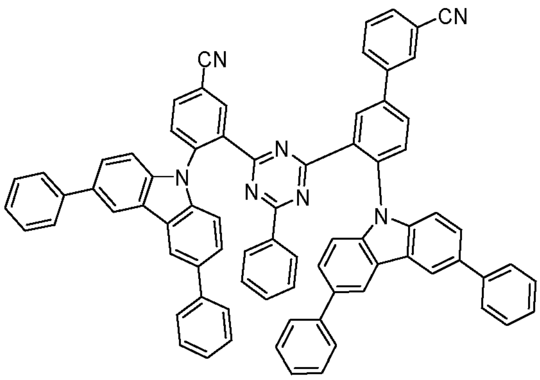

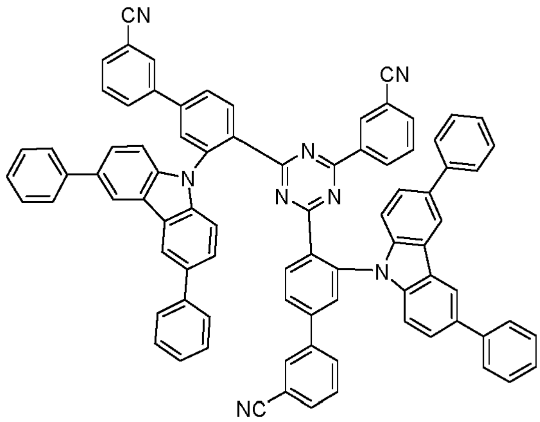

**E^B-7**

**E^B-8**

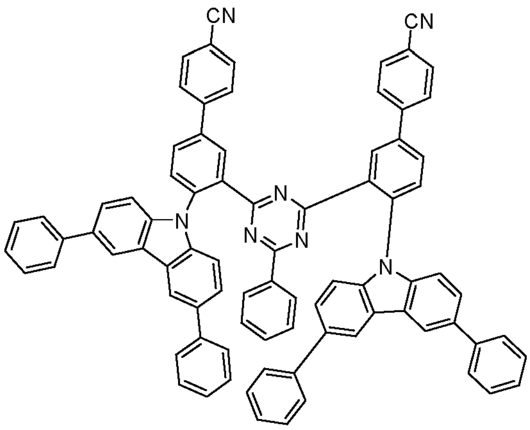

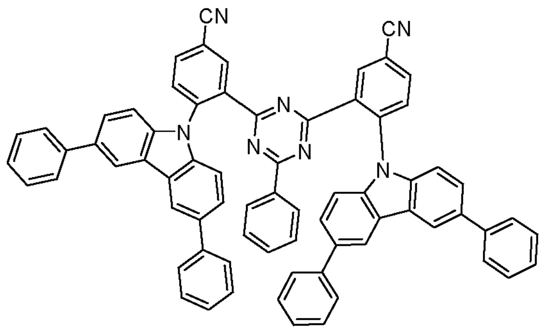

**E^B-9**

**E^B-10**

**Table 1E.** Properties of the TADF materials $E^B$.

| | Example compound | $E^{HOMO}$ [eV] | $E^{LUMO}$ [eV] | S1 [eV] | T1 [eV] | $\lambda_{max}^{PMMA}$ [nm] | FWHM [eV] |
|---|---|---|---|---|---|---|---|
| **$E^B$** | **$E^B$-1** | -5.97 | -3.28 | 2.69 | 2.63 | 518 | 0.43 |
| | **$E^B$-2** | -5.97 | -3.31 | 2.66 | 2.72 | 526 | 0.43 |
| | **$E^B$-3** | -5.92 | -3.25 | 2.67 | 2.65 | 517 | 0.40 |
| | **$E^B$-4** | -6.00 | -3.37 | 2.63 | 2.65 | 525 | 0.40 |
| | **$E^B$-5** | -5.95 | -3.27 | 2.68 | 2.64 | 508 | 0.41 |
| | **$E^B$-6** | -5.94 | -3.24 | 2.70 | 2.64 | 509 | 0.41 |
| | **$E^B$-7** | -5.94 | -3.24 | 2.70 | 2.66 | 509 | 0.41 |
| | **$E^B$-8** | -5.93 | -3.33 | 2.60 | 2.59 | 525 | 0.39 |
| | **$E^B$-9** | -5.89 | -3.15 | 2.74 | 2.64 | 498 | 0.40 |
| | **$E^B$-10** | -5.99 | -3.34 | 2.65 | 2.65 | 520 | 0.42 |

wherein $LUMO_{CV}$ is the energy of the lowest unoccupied molecular orbital, which is determined by Cyclic voltammetry.

**Small FWHM emitters $S^B$**

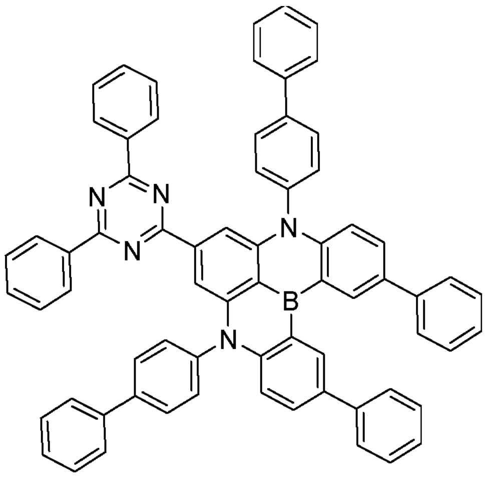

**$S^B$-1**

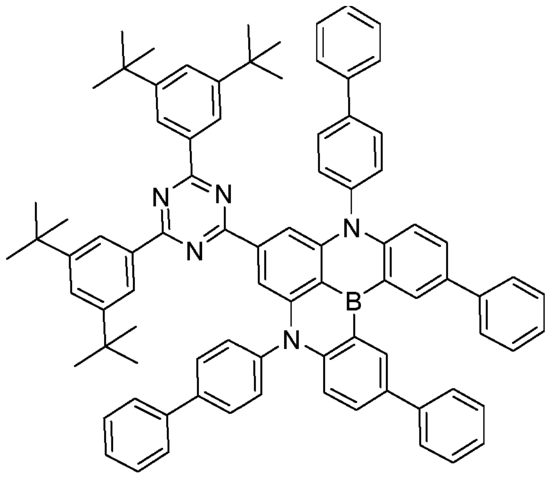

**$S^B$-2**

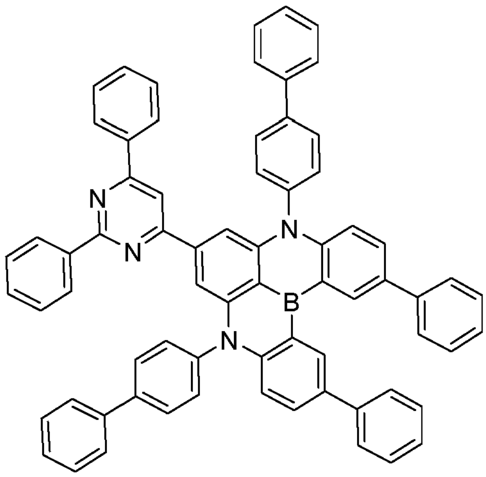

**$S^B$-3**

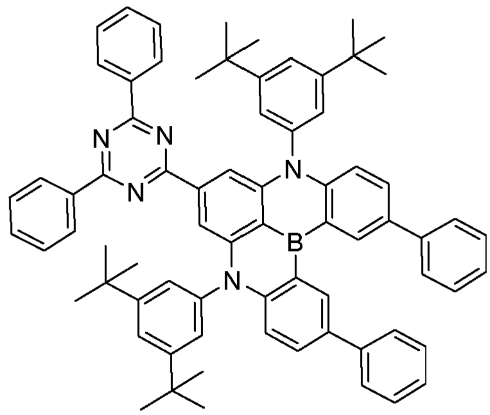

**$S^B$-4**

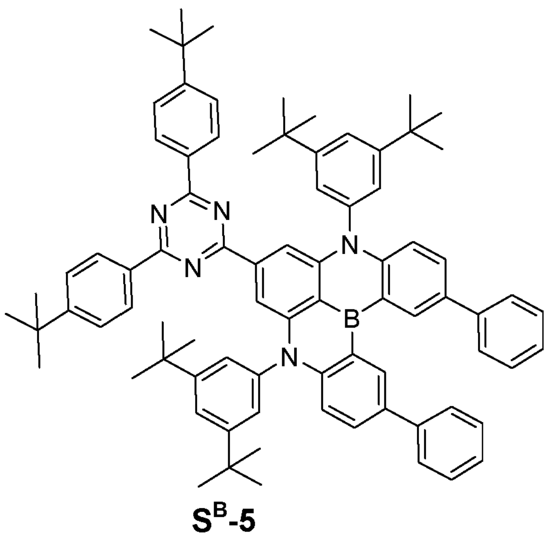

**$S^B$-5**

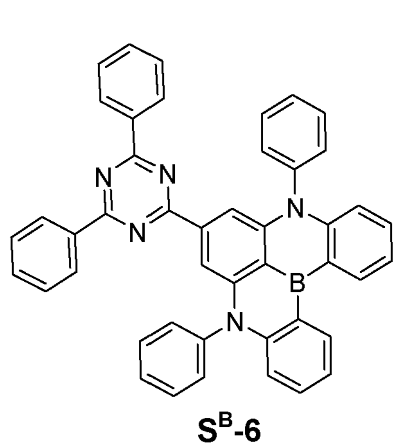

**$S^B$-6**

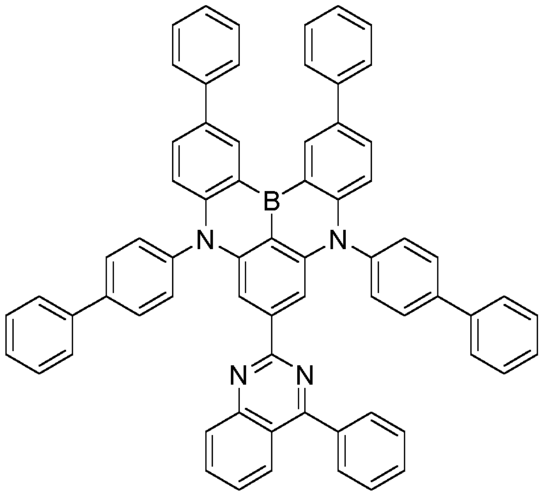

**$S^B$-7**

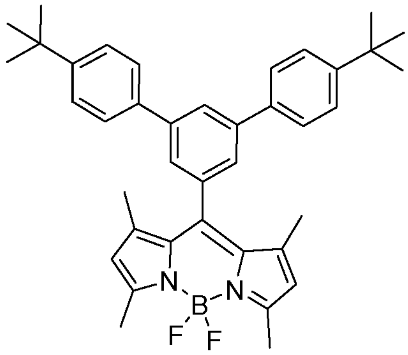

**$S^B$-8**

**Table 1S.** Properties of the Small FWHM emitters $S^B$.

| | Example compound | $E^{HOMO}$ [eV] | $E^{LUMO}$ [eV] | S1 [eV] | T1 [eV] | $\lambda_{max}^{PMMA}$ [nm] | FWHM [eV] |
|---|---|---|---|---|---|---|---|
| $S^B$ | **$S^B$-1** | -5.54 | -3.10 | 2.44 | 2.12 | 538 | 0.21 |
| | **$S^B$-2** | -5.53 | -3.04 | 2.49 | 2.26 | 525 | 0.18 |
| | **$S^B$-3** | -5.55 | -3.05 | 2.50 | 2.22 | 520 | 0.18 |
| | **$S^B$-4** | -5.48 | -3.05 | 2.43 | 2.25 | 537 | 0.17 |
| | **$S^B$-5** | -5.47 | -3.01 | 2.46 | 2.58 | 527 | 0.15 |
| | **$S^B$-6** | -5.56 | -3.03 | 2.53 | 2.19 | 518 | 0.22 |
| | **$S^B$-7** | -5.48 | -2.97 | 2.53 | 2.23 | 521 | 0.25 |
| | **$S^B$-8*** | -5.86 | -3.40 | 2.46 | | 517 | 0.10 |
| * measured in DCM (0.01 mg/mL). | | | | | | | |

**Table 2.** Setup of an example organic electroluminescent device (OLED) **H** and comparison example OLED **T.**

| **Layer** | **Thickness** | **T** | **H** |
|---|---|---|---|
| **10** | 100 nm | Al | Al |
| **9** | 2 nm | Liq | Liq |
| **8** | 20 nm | NBPhen | NBPhen |
| 7 | 10 nm | HBM1 | HBM1 |
| **6** | 50 nm | $H^P$ : $E^B$ | $H^P$ : $E^B$ : $S^B$ |
| **5** | 10 nm | $H^P$ | $H^P$ |
| **4** | 10 nm | TCTA | TCTA |
| **3** | 50 nm | NPB | NPB |
| **2** | 5 nm | HAT-CN | HAT-CN |
| **1** | 50 nm | ITO | ITO |
| **substrate** | | glass | glass |

In order to evaluate the results of the invention, comparison experiments were performed, wherein solely the composition of the emission layer (**6**) was varied.

**Results I: Variation of Host $H^B$ (exemplified as p-host $H^P$) and Emitter $S^B$**

Composition of the light-emitting layer B the percentages refer to weight percent):

| **Layer** | **T** | **H** |
|---|---|---|
| **Emission layer (6A)** | $H^P$ (85%):<br>$E^B$ (15%) | $H^P$ (84%):<br>$E^B$ (15%):<br>$S^B$ (1%) |

wherein $E^B$-10 was used as TADF material $E^B$ and the small FWHM emitter $S^B$ was varied using different p-hosts $H^P$.

Device results for $E^B$ = $E^B$-10 and $H^P$ = mCBP:

| Device type | $S^B$ | FWHM [nm] | $\lambda_{max}$ [nm] | CIEx | CIEy | Voltage at 10 mA/cm$^2$ | EQE at 1000 nits | Relative lifetime LT95 at 1200 nits |
|---|---|---|---|---|---|---|---|---|
| **T** | | 76 | 518 | 0.30 | 0.60 | 5.7 | 15.0% | 1.00 |
| **H** | **$S^B$-1** | 36 | 532 | 0.31 | 0.65 | 7.5 | 17.7% | 4.74 |
| **H** | **$S^B$-2** | 36 | 524 | 0.28 | 0.66 | 7.8 | 18.5% | 2.88 |
| **H** | **$S^B$-3** | 40 | 516 | 0.27 | 0.65 | 6.9 | 16.3% | 2.93 |

Device results for $E^B$ = $E^B$-10 and $H^P$ = PYD2:

| Device type | $S^B$ | FWHM [nm] | $\lambda_{max}$ [nm] | CIEx | CIEy | Voltage at 10 mA/cm$^2$ | EQE at 1000 nits | Relative lifetime LT95 at 1200 nits |
|---|---|---|---|---|---|---|---|---|
| **T** | | 78 | 522 | 0.31 | 0.60 | 6.2 | 11.6% | 1.00 |
| **H** | **$S^B$-1** | 42 | 534 | 0.32 | 0.64 | 7.5 | 13.6% | 7.03 |
| **H** | **$S^B$-2** | 42 | 526 | 0.29 | 0.65 | 7.9 | 13.6% | 4.03 |
| **H** | **$S^B$-3** | 42 | 518 | 0.27 | 0.65 | 9.1 | 12.7% | 1.58 |

**Results II: Variation of Host $H^B$ and TADF $E^B$**

Setup of the light-emitting layer B (the percentages refer to weight percent):

| **Layer** | **T** | **H** |
|---|---|---|
| **Emission layer (6A)** | $H^P$ (85%):<br>$E^B$ (15%) | $H^P$ (84%):<br>$E^B$ (15%):<br>$S^B$ (1%) |

wherein $S^B$-1 was used as small FWHM emitter $S^B$ and the TADF material $E^B$ was varied using different p-hosts $H^P$.

Device results for $S^B$ = $S^B$-1 and $H^P$ = mCBP:

| Device type | $E^B$ | FWHM [nm] | $\lambda_{max}$ [nm] | CIEx | CIEy | Voltage at 10 mA/cm$^2$ | EQE at 1000 nits | Relative lifetime LT95 at 1200 nits |
|---|---|---|---|---|---|---|---|---|
| **T** | **$E^B$-1** | 86 | 524 | 0.32 | 0.59 | 6.4 | 20.1% | 1.00 |
| **H** | **$E^B$-1** | 68 | 528 | 0.32 | 0.61 | 7.2 | 19.5% | 1.89 |

(continued)

| Device type | $E^B$ | FWHM [nm] | $\lambda_{max}$ [nm] | CIEx | CIEy | Voltage at 10 $mA/cm^2$ | EQE at 1000 nits | Relative lifetime LT95 at 1200 nits |
|---|---|---|---|---|---|---|---|---|
| **T** | **$E^B$-3** | 76 | 512 | 0.28 | 0.59 | 6.8 | 18.1% | 0.59 |
| **H** | **$E^B$-3** | 36 | 534 | 0.32 | 0.64 | 7.9 | 18.1% | 1.79 |
| **T** | **$E^B$-4** | 80 | 526 | 0.33 | 0.60 | 6.8 | 16.8% | 1.09 |
| **H** | **$E^B$-4** | 40 | 534 | 0.34 | 0.64 | 7.6 | 16.6% | 2.29 |
| **T** | **$E^B$-8** | 78 | 524 | 0.32 | 0.61 | 6.6 | 22.0% | 2.20 |
| **H** | **$E^B$-8** | 42 | 536 | 0.33 | 0.64 | 7.5 | 20.8% | 9.20 |
| **T** | **$E^B$-10** | 76 | 518 | 0.30 | 0.60 | 5.7 | 15.0% | 2.29 |
| **H** | **$E^B$-10** | 36 | 532 | 0.31 | 0.65 | 7.5 | 17.7% | 10.88 |

Device results for $S^B$ = $S^B$-1 and $H^P$ = PYD2:

| Device type | $E^B$ | FWHM [nm] | $\lambda_{max}$ [nm] | CIEx | CIEy | Voltage at 10 $mA/cm^2$ | EQE at 1000 nits | Relative lifetime LT95 at 1200 nits |
|---|---|---|---|---|---|---|---|---|
| **T** | **$E^B$-3** | 76 | 514 | 0.27 | 0.59 | 6.5 | 18.4% | 1.00 |
| **H** | **$E^B$-3** | 40 | 536 | 0.33 | 0.63 | 7.4 | 17.5% | 4.99 |
| **T** | **$E^B$-4** | 80 | 526 | 0.33 | 0.60 | 6.0 | 12.9% | 1.86 |
| **H** | **$E^B$-4** | 42 | 536 | 0.34 | 0.63 | 7.3 | 13.0% | 7.76 |
| **T** | **$E^B$-5** | 78 | 514 | 0.28 | 0.59 | 6.7 | 14.1% | 0.44 |
| **H** | **$E^B$-5** | 42 | 534 | 0.32 | 0.64 | 8.2 | 14.9% | 2.37 |
| **T** | **$E^B$-6** | 75 | 514 | 0.28 | 0.59 | 6.0 | 13.3% | 1.08 |
| **H** | **$E^B$-6** | 40 | 534 | 0.31 | 0.64 | 7.5 | 13.2% | 2.44 |
| **T** | **$E^B$-8** | 76 | 520 | 0.30 | 0.60 | 6.4 | 18.8% | 2.56 |
| **H** | **$E^B$-8** | 42 | 536 | 0.33 | 0.63 | 7.4 | 17.1% | 10.37 |
| **T** | **$E^B$-10** | 78 | 522 | 0.31 | 0.60 | 6.2 | 11.6% | 1.63 |
| **H** | **$E^B$-10** | 42 | 534 | 0.32 | 0.64 | 7.5 | 13.6% | 11.44 |

For all electroluminescent devices using a combination of a host, a TADF emitter and an FWHM emitter (H-type device), comprising mCBP as $H^P$ and $E^B$-10 as $E^B$, an extension of the relative lifetime of 374% (from 1.00 to 4.74) could be observed for emitter $S^B$-1, and an extension of the relative lifetime of 188% (from 1.00 to 2.88) could be observed for Emitter $S^B$-2, and an extension of the relative lifetime of 193% (from 1.00 to 2.93) could be observed for Emitter $S^B$-3 as compared to the comparative T-type device, whereas the efficiencies (EQE) stayed almost constant.

Furthermore, for all H-type devices using mCBP as $H^P$ and $E^B$-10 as $E^B$, a decrease of the full width at half maximum (FWHM) of 47% (from 76 nm to 36 nm) could be observed for emitter $S^B$-1, and a decrease of the FWHM of 47% (from 76 nm to 36 nm) could be observed for Emitter $S^B$-2, and a decrease of the FWHM of 47% (from 76 nm to 40 nm) could be observed for Emitter $S^B$-3 as compared to the comparative T-type device. All H-type and T-type devices using mCBP as $H^P$, $E^B$-10 as $E^B$, and $S^B$-1, $S^B$-2 or $S^B$-3 as $S^B$ exhibit emission maxima in the desired green wavelength range of 500 nm to 560 nm, even in the more preferred range of 510 nm to 550 nm.

For all H-type devices using PYD2 as $H^P$ and $E^B$-10 as $E^B$, an extension of the relative lifetime of 603% (from 1.00 to 7.03) could be observed for emitter $S^B$-1, and an extension of the relative lifetime of 303% (from 1.00 to 4.03) could be observed for Emitter $S^B$-2, and an extension of the relative lifetime of 58% (from 1.00 to 1.58) could be observed for Emitter $S^B$-3 as compared to the comparative T-type device, whereas the efficiencies (EQE) stayed almost constant. Furthermore, for all H-type devices using PYD2 as $H^P$ and $E^B$-10 as $E^B$, a decrease of the full width at half maximum (FWHM) of 46% (from 78 nm to 42 nm) could be observed for emitter $S^B$-1, and a decrease of the FWHM of 46% (from 78 nm to 42 nm) could

be observed for Emitter $S^B$-2, and a decrease of the FWHM of 46% (from 78 nm to 42 nm) could be observed for Emitter $S^B$-3 as compared to the comparative T-type device. All H-type and T-type devices using PYD2 as $H^P$, $E^B$-10 as $E^B$, and $S^B$-1, $S^B$-2 or $S^B$-3 as $S^B$ exhibit emission maxima in the desired green wavelength range of 500 nm to 560 nm, even in the more preferred range of 510 nm to 550 nm.

For all H-type devices using mCBP as $H^P$ and $S^B$-1 as $S^B$, an extension of the relative lifetime of 89% (from 1.00 to 1.89) could be observed for using $E^B$-1, and an extension of the relative lifetime of 120% (from 0.59 to 1.79) could be observed for $E^B$-3, and an extension of the relative lifetime of 120% (from 1.09 to 2.29) could be observed for $E^B$-4, and an extension of the relative lifetime of 700% (from 2.20 to 9.20) could be observed for $E^B$-8, and an extension of the relative lifetime of 859% (from 2.29 to 10.88) could be observed for $E^B$-8, as compared to the respective comparative T-type device, whereas the efficiency (EQE) stayed almost constant. Furthermore, for all H-type devices using mCBP as $H^P$ and $S^B$-1 as $S^B$, a decrease of the full width at half maximum (FWHM) of 23% (from 86 nm to 68 nm) could be observed for $E^B$-1, and a decrease of the FWHM of 53% (from 76 nm to 36 nm) could be observed for $E^B$-3, and a decrease of the FWHM of 50% (from 80 nm to 40 nm) could be observed for $E^B$-4, and a decrease of the FWHM of 46% (from 78 nm to 42 nm) could be observed for $E^B$-8, and a decrease of the FWHM of 53% (from 76 nm to 36 nm) could be observed for $E^B$-10, as compared to the respective comparative T-type device. All H-type and T-type devices using mCBP as $H^P$, $S^B$-1 as $S^B$, and $E^B$-1, $E^B$-3, $E^B$-4, $E^B$-8 or $E^B$-10 as $E^B$ exhibit emission maxima in the desired green wavelength range of 500 nm to 560 nm, even in the more preferred range of 510 nm to 550 nm.

For all H-type devices using PYD2 as $H^P$ and $S^B$-1 as $S^B$, an extension of the relative lifetime of 399% (from 1.00 to 4.99) could be observed for using $E^B$-3, and an extension of the relative lifetime of 590% (from 1.86 to 7.76) could be observed for $E^B$-4, and an extension of the relative lifetime of 120% (from 0.44 to 2.37) could be observed for $E^B$-5, and an extension of the relative lifetime of 136% (from 1.08 to 2.44) could be observed for $E^B$-6, and an extension of the relative lifetime of 781% (from 2.56 to 10.37) could be observed for $E^B$-8, and an extension of the relative lifetime of 981% (from 1.63 to 11.44) could be observed for $E^B$-10, as compared to the respective comparative T-type device, whereas no significant changes in the efficiency (EQE) were observed. Furthermore, for all H-type devices using PYD2 as $H^P$ and $S^B$-1 as $S^B$, a decrease of the full width at half maximum (FWHM) of 47% (from 76 nm to 40 nm) could be observed for $E^B$-3, and a decrease of the FWHM of 48% (from 80 nm to 42 nm) could be observed for $E^B$-4, and a decrease of the FWHM of 46% (from 78 nm to 42 nm) could be observed for $E^B$-5, and a decrease of the FWHM of 47% (from 75 nm to 40 nm) could be observed for $E^B$-6, and a decrease of the FWHM of 45% (from 76 nm to 42 nm) could be observed for $E^B$-8, and a decrease of the FWHM of 47% (from 78 nm to 42 nm) could be observed for $E^B$-10, as compared to the respective comparative T-type device. All H-type and T-type devices using PYD2 as $H^P$, $S^B$-1 as $S^B$, and $E^B$-3, $E^B$-4, $E^B$-5, $E^B$-5, $E^B$-8 or $E^B$-10 as $E^B$ exhibit emission maxima in the desired green wavelength range of 500 nm to 560 nm, even in the more preferred range of 510 nm to 550 nm.

Additional examples of organic electroluminescent devices according to the invention:

Example **D1**

The small FWHM emitter $S^B$-1 was also tested in the OLED **D1,** which was fabricated with the following layer structure:

| Layer # | Thickness | D1 |
|---|---|---|
| **10** | 100 nm | Al |
| **9** | 2 nm | Liq |
| **8** | 20 nm | NBPhen |
| **7** | 10 nm | HBM1 |
| **6** | 50 nm | mCBP (79%): $E^B$-11 (20%) : $S^B$-1 (1%) |
| **5** | 10 nm | mCBP |
| **4** | 10 nm | TCTA |
| **3** | 50 nm | NPB |
| **2** | 5 nm | HAT-CN |
| **1** | 50 nm | ITO |

(continued)

| Layer # | Thickness | D1 |
|---|---|---|
| **Substrate** | | Glass |

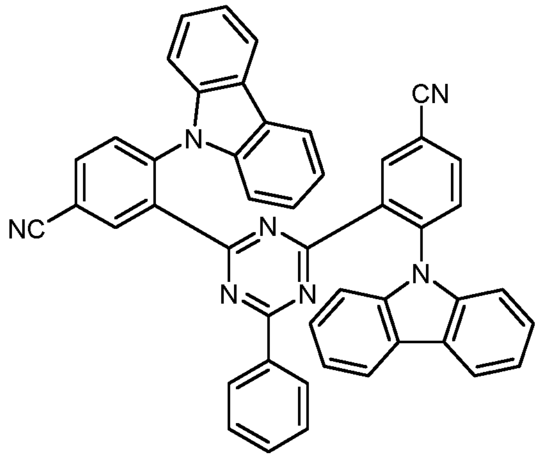

**E^B-11**

OLED **D1** yielded an external quantum efficiency (EQE) at 1000 cd/$m^2$ of 16.1%. The emission maximum is at 532 nm with a FWHM of 38 nm at 7.6 V. The corresponding CIEx value is 0.32 and the CIEy value is 0.65. A LT95-value at 1200 cd/$m^2$ of 1522 h was determined.

Example **D2**

The small FWHM emitter $S^B$-1 was also tested in the OLED **D2,** which was fabricated with the following layer structure:

| Layer # | Thickness | D2 |
|---|---|---|
| **10** | 100 nm | Al |
| **9** | 2 nm | Liq |
| **8** | 20 nm | NBPhen |
| **7** | 10 nm | HBM1 |
| **6** | 50 nm | mCBP (84%): $E^B$-11 (15%) : $S^B$-1 (1%) |
| **5** | 10 nm | PYD2 = $H^P$-2 |
| **4** | 10 nm | TCTA |
| **3** | 50 nm | NPB |
| **2** | 5 nm | HAT-CN |
| **1** | 50 nm | ITO |
| **Substrate** | | Glass |

OLED **D2** yielded an external quantum efficiency (EQE) at 1000 cd/$m^2$ of 17.7%. The emission maximum is at 532 nm with a FWHM of 36 nm at 7.6 V. The corresponding CIEx value is 0.31 and the CIEy value is 0.65. A LT95-value at 1200 cd/$m^2$ of 2006 h was determined.

Example **D3**

The small FWHM emitter $S^B$-1 was also tested in the OLED **D3,** which was fabricated with the following layer structure:

| **Layer #** | **Thickness** | **D2** |
|---|---|---|
| **10** | 100 nm | Al |
| **9** | 2 nm | Liq |
| **8** | 20 nm | NBPhen |
| **7** | 10 nm | HBM1 |
| **6** | 50 nm | mCBP (75%): PYD2 = $H^P$-2 (5%): $E^B$-11 (15%) : $S^B$-1 (1%) |
| **5** | 10 nm | PYD2 = $H^P$-2 |
| **4** | 10 nm | TCTA |
| **3** | 50 nm | NPB |
| **2** | 5 nm | HAT-CN |
| **1** | 50 nm | ITO |
| **Substrate** | | Glass |

OLED **D3** yielded an external quantum efficiency (EQE) at 1000 cd/$m^2$ of 20.2%. The emission maximum is at 532 nm with a FWHM of 38 nm at 7.6 V. The corresponding CIEx value is 0.32 and the CIEy value is 0.65. A LT95-value at 1200 cd/$m^2$ of 1866 h was determined.

Example **D4**

The small FWHM emitter $S^B$-3 was also tested in the OLED **D4,** which was fabricated with the following layer structure:

| **Layer #** | **Thickness** | **D2** |
|---|---|---|
| **10** | 100 nm | Al |
| **9** | 2 nm | Liq |
| **8** | 20 nm | NBPhen |
| **7** | 10 nm | HBM1 |
| **6** | 50 nm | mCBP (84%): $E^B$-11 (15%) : $S^B$ -3 (1%) |
| **5** | 10 nm | mCBP |
| **4** | 10 nm | TCTA |
| **3** | 50 nm | NPB |
| **2** | 5 nm | HAT-CN |
| **1** | 50 nm | ITO |
| **Substrate** | | Glass |

OLED **D4** yielded an external quantum efficiency (EQE) at 1000 cd/$m^2$ of 16.3%. The emission maximum is at

516 nm with a FWHM of 40 nm at 7.0 V. The corresponding CIEx value is 0.27 and the CIEy value is 0.65. A LT95-value at 1200 cd/m$^2$ of 1162 h was determined.

## Claims

**1.** An organic electroluminescent device comprising one or more light-emitting layers B, each comprising independently of each other:

(i) at least one host material $H^B$, which has a lowermost excited singlet state energy level $E(S1^H)$ and a lowermost excited triplet state energy level $E(T1^H)$;
(ii) at least one thermally activated delayed fluorescence (TADF) material $E^B$, which has a lowermost excited singlet state energy level $E(S1^E)$ and a lowermost excited triplet state energy level $E(T1^E)$; and
(iii) at least one small full width at half maximum (FWHM) emitter $S^B$ having an emission spectrum which exhibits an FWHM of less than or equal to 0.25 eV, measured with 1 % by weight of the emitter $S^B$ in poly(methyl methacrylate at 20°C, which has a lowermost excited singlet state energy level $E(S1^S)$ and a lowermost excited triplet state energy level $E(T1^S)$,
wherein each $E^B$ transfers energy to at least one $S^B$ and each $S^B$ emits light with an emission maximum between 500 nm to 560 nm, wherein the relations expressed by the following formulas (1) to (5) apply:

$$E(S1^H) > E(S1^E) \quad (1)$$

$$E(S1^H) > E(S1^S) \quad (2)$$

$$E(S1^E) > E(S1^S) \quad (3)$$

$$E(T1^H) > E(T1^S) \quad (4)$$

$$E(T1^H) > E(T1^E) \quad (5);$$

wherein at least one small full width at half maximum (FWHM) emitter $S^B$ is a boron containing emitter.

**2.** The organic electroluminescent device according to claim 1, wherein a TADF material $E^B$ is **characterized in that** it exhibits a $\Delta E_{ST}$ value, which corresponds to the energy difference between the lowermost excited singlet state $S1^E$ and the lowermost excited triplet state $T1^E$, of less than 0.4 eV.

**3.** The organic electroluminescent device according to any of claims 1 and 2, wherein the at least one small FWHM emitter $S^B$ is **characterized in that** it has an emission spectrum, which exhibits a full width at half maximum (FWHM) of less than or equal to 0.25 eV.

**4.** The organic electroluminescent device according to any of claims 1 to 3, wherein the at least one thermally activated delayed fluorescence (TADF) material $E^B$ has an emission maximum $\lambda_{max}(D)$ in the wavelength range of 500 nm to 560 nm.

**5.** The organic electroluminescent device according to any of claims 1 to 4, wherein the at least one thermally activated delayed fluorescence (TADF) material $E^B$ has a highest occupied molecular orbital $HOMO(E^B)$ having an energy $E^{HOMO}(E^B)$ according to $-6.0\ eV \leq E^{HOMO}(E^B) \leq -5.8\ eV$.

**6.** The organic electroluminescent device according to any of claims 1 to 5, wherein the at least one thermally activated delayed fluorescence (TADF) material $E^B$ has a structure represented by any of the formulas $E^B$-I-1a, $E^B$-I-2a, $E^B$-I-3a, $E^B$-I-4a, $E^B$-I-5a, $E^B$-I-6a, $E^B$-I-7, and $E^B$-I-8,

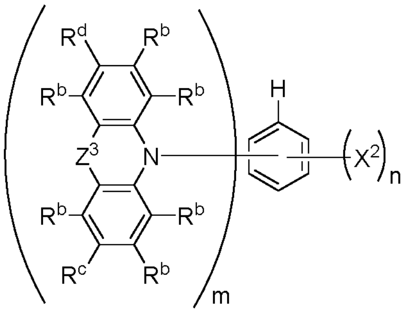

Formula $E^B$-I-1a

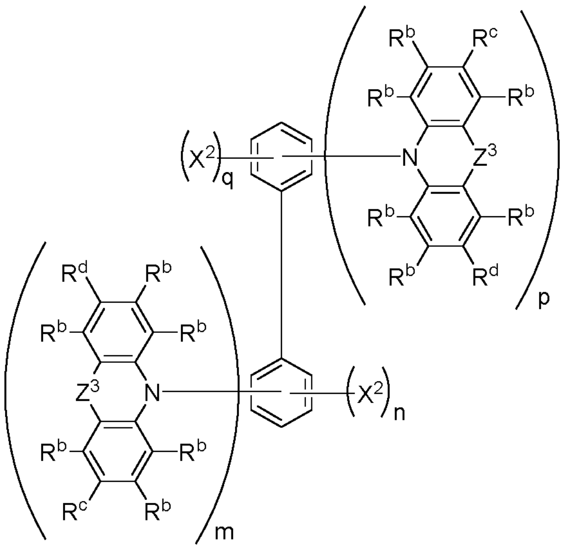

Formula $E^B$-I-2a

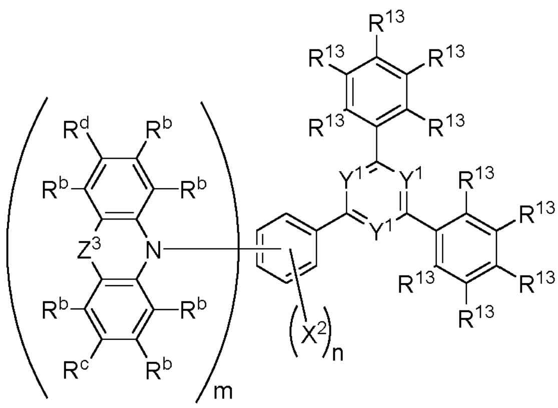

Formula $E^B$-I-3a

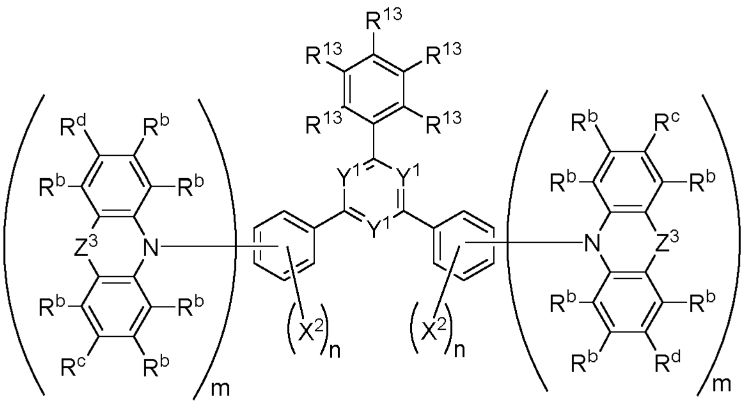

Formula $E^B$-I-4a

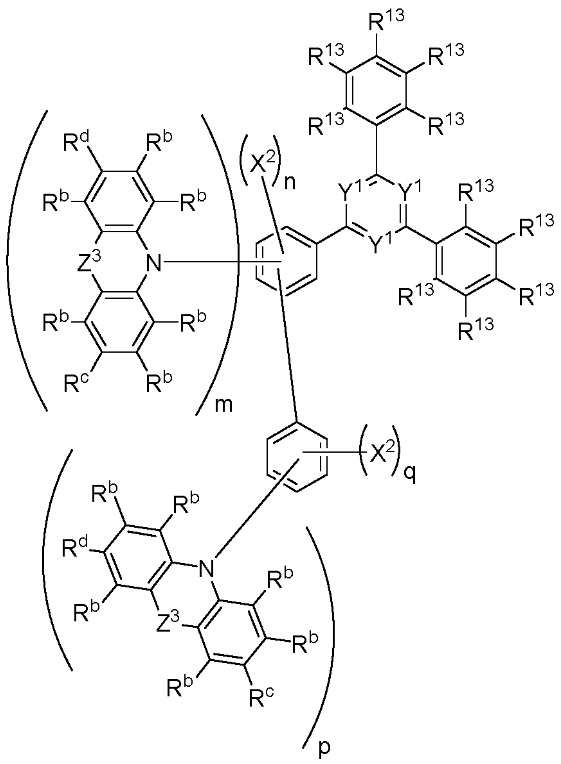

Formula $E^B$-I-5a

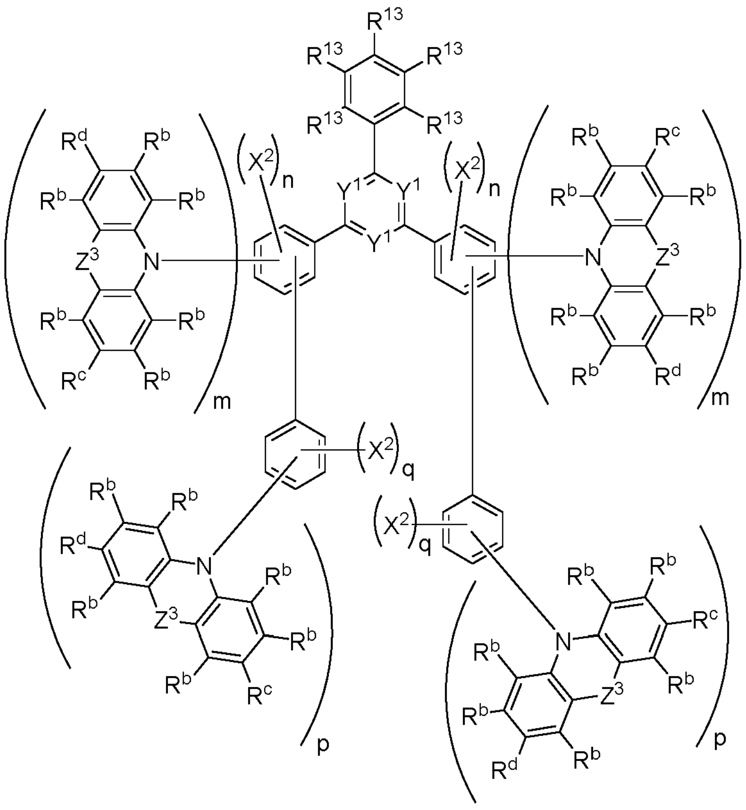

Formula $E^B$-I-6a

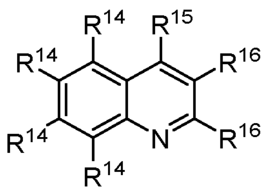

Formula $E^B$-I-7

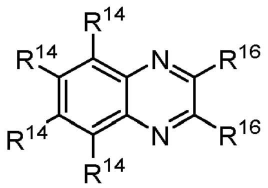

Formula $E^B$-I-8

wherein

$Y^1$ is at each occurrence nitrogen (N) or CH, and at least one $Y^1$ is N;
m is at each occurrence independently of each other 0, 1 or 2;
n is at each occurrence independently of each other 0, 1 or 2;
p is at each occurrence independently of each other 0, 1 or 2;
q is at each occurrence independently of each other 0, 1 or 2;
$X^2$ is at each occurrence independently of each other selected from the group consisting of $Ar^{EWG}$, CN, and $CF_3$,
$Ar^{EWG}$ is at each occurrence independently of each other represented by a structure according to any of the formulas $Ar^{EWG}$-I, $Ar^{EWG}$-II, $Ar^{EWG}$-III, $Ar^{EWG}$-IV, $Ar^{EWG}$-V, $Ar^{EWG}$-VI , $Ar^{EWG}$-VII, $Ar^{EWG}$-VIII, $Ar^{EWG}$-IX, $Ar^{EWG}$-X, $Ar^{EWG}$-XI, $Ar^{EWG}$-XII, $Ar^{EWG}$-XIII, and $Ar^{EWG}$-XIV,

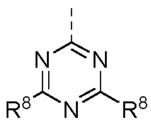

Formula $Ar^{EWG}$-I

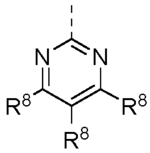

Formula $Ar^{EWG}$-II

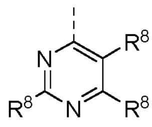

Formula $Ar^{EWG}$-III

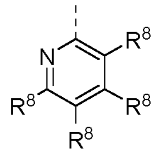

Formula $Ar^{EWG}$-IV

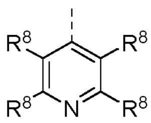

Formula $Ar^{EWG}$-V

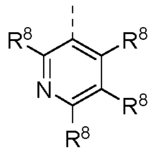

Formula $Ar^{EWG}$-VI

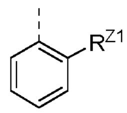

Formula $Ar^{EWG}$-VII

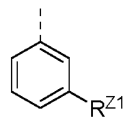

Formula $Ar^{EWG}$-VIII

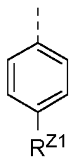

Formula $Ar^{EWG}$-IX

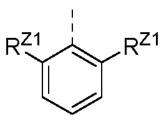

Formula $Ar^{EWG}$-X

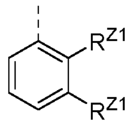

Formula $Ar^{EWG}$-XI

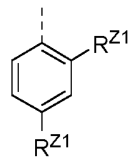

Formula $Ar^{EWG}$-XII

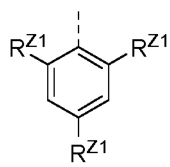

Formula $Ar^{EWG}$-XIII

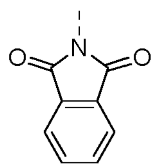

Formula $Ar^{EWG}$-XIV

which are bonded to the core structure via the position marked by the dashed line;
$R^{Z1}$ is at each occurrence independently of each other selected from the group consisting of CN and $CF_3$;
$Z^3$ is at each occurrence independently of each other selected from the group consisting of a direct bond, $CR^9R^{10}$, $C=CR^9R^{10}$, C=O, $C=NR^9$, $NR^9$, O, $SiR^9R^{10}$, S, S(O) and $S(O)_2$;
$R^8$ is at each occurrence independently of each other selected from the group consisting of: hydrogen, deuterium, CN, $CF_3$,
$C_1$-$C_5$-alkyl,
wherein one or more hydrogen atoms are optionally substituted by deuterium;
$C_6$-$C_{18}$-aryl,
which is optionally substituted with one or more substituents $R^{11}$; and
$C_3$-$C_{17}$-heteroaryl,
which is optionally substituted with one or more substituents $R^{11}$;
$R^b$, $R^c$, $R^d$, $R^9$, and $R^{10}$ are at each occurrence independently of each other selected from the group consisting of: hydrogen, deuterium, $N(R^{12})_2$, $OR^{12}$, $Si(R^{12})_3$, $B(OR^{12})_2$, $OSO_2R^{12}$, $CF_3$, CN, F, Br, I,
$C_1$-$C_{40}$-alkyl,

which is optionally substituted with one or more substituents $R^{12}$ and
wherein one or more non-adjacent $CH_2$-groups are optionally C=Se, $C=NR^{12}$, $P(=O)(R^{12})$, SO, $SO_2$, $NR^{12}$, O, S or $CONR^{12}$;

$C_1$-$C_{40}$-alkoxy,

which is optionally substituted with one or more substituents $R^{12}$ and
wherein one or more non-adjacent $CH_2$-groups are optionally substituted by $R^{12}C=CR^{12}$, C=C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_1$-$C_{40}$-thioalkoxy,

which is optionally substituted with one or more substituents $R^{12}$ and
wherein one or more non-adjacent $CH_2$-groups are optionally substituted by $R^{12}C=CR^{12}$, C=C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_2$-$C_{40}$-alkenyl,

which is optionally substituted with one or more substituents $R^{12}$ and
wherein one or more non-adjacent $CH_2$-groups are optionally substituted by $R^{12}C=CR^{12}$, C=C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_2$-$C_{40}$-alkynyl,

which is optionally substituted with one or more substituents $R^{12}$ and
wherein one or more non-adjacent $CH_2$-groups are optionally substituted by $R^{12}C=CR^{12}$, C=C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S or $CONR^{12}$;

$C_6$-$C_{60}$-aryl,
which is optionally substituted with one or more substituents $R^{12}$; and
$C_3$-$C_{57}$-heteroaryl,
which is optionally substituted with one or more substituents $R^{12}$;
$R^{11}$ is at each occurrence independently of each other selected from the group consisting of: hydrogen, deuterium, Me, $^iPr$, $^tBu$, CN, $CF_3$, and
phenyl, which is optionally substituted with one or more substituents independently of each other selected from the group consisting of Me, $^iPr$, $^tBu$, CN, $CF_3$ and Ph;
$R^{12}$ is at each occurrence independently of each other selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
N ($C_3$-$C_{17}$-heteroaryl$)_2$,
$N(C_3$-$C_{17}$-heteroaryl)($C_6$-$C_{18}$-aryl), and
an aliphatic cyclic amino group comprising 5 to 8 carbon atoms (preferably pyrrolidinyl and piperidinyl);
$R^{13}$ is at each occurrence independently of each other selected from the group consisting of: hydrogen, deuterium, CN, $CF_3$, $Ar^{EWG}$,
$C_1$-$C_5$-alkyl,
wherein one or more hydrogen atoms are optionally substituted by deuterium;
$C_6$-$C_{18}$-aryl,
which is optionally substituted with one or more substuítuents independently of each other selected from deuterium, $C_1$-$C_5$-alkyl groups, and $C_6$-$C_{18}$-aryl groups; and
$R^{14}$ and $R^{15}$ are at each occurrence independently of each other selected from the group consisting of C-$Ar^{EWG}$ and $CR^{Q2}$;

$R^{16}$ is at each occurrence independently of each other selected from the group consisting of $CR^6$, C-$Ar^{EWG}$ and $CR^{Q2}$;
$R^{Q2}$ is at is at each occurrence independently of each other selected from the group consisting of: hydrogen, deuterium, Me, $^iPr$, $^tBu$, CN, $CF_3$, Ph, and
carbazolyl, which is optionally substituted with one or more substituents independently from each other selected from the group consisting of Me, $^iPr$, $^tBu$, CN, $CF_3$, Ph, and $N(Ph)_2$;
wherein, optionally, any adjacent substituents $R^b$, $R^c$, $R^d$, $R^9$, and $R^{10}$ independently of each other form a mono- or polycyclic, aliphatic, aromatic and/or benzo-fused ring system; wherein one or more hydrogen atoms of the optionally so formed ring system may be substituted by $R^{12}$; and
wherein not more than two groups $R^{13}$ are CN, $CF_3$ or $Ar^{EWG}$;
wherein:

$$1 \leq (m + p)$$

and

$$1 \leq (n + q).$$

**7.** The organic electroluminescent device according to any of claims 1 to 6, wherein the at least one small FWHM emitters $S^B$ comprises or consists of a structure according to formula $S^B$-I:

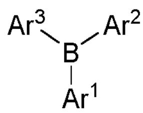

Formula $S^B$-I

wherein B is boron,
$Ar^1$, $Ar^2$, and $Ar^3$ are at each occurrence independently of each other selected from the group consisting of an aromatic ring and a heteroaromatic ring, and $Ar^1$, $Ar^2$, $Ar^3$ may optionally be linked to each other to form one or more additional rings.

**8.** The organic electroluminescent device according to any of claims 1 to 7, wherein the at least one small FWHM emitters $S^B$ comprises or consists of a structure of formula $S^B$-III-3a:

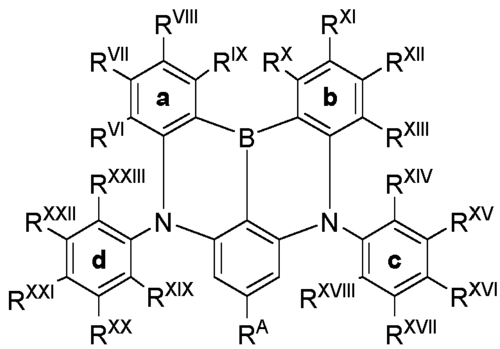

Formula $S^B$-III-3a

wherein $R^{VI}$, $R^{VII}$, $R^{VIII}$, $R^{IX}$, $R^X$, $R^{XI}$, $R^{XII}$, $R^{XIII}$, $R^{XIV}$ $R^{XV}$, $R^{XVI}$, $R^{XVII}$, $R^{XVIII}$ $R^{XIX}$, $R^{XX}$, $R^{XXI}$, $R^{XXII}$, and $R^{XXIII}$ are independently of each other selected from the group consisting of: hydrogen, deuterium, $N(R^{21})_2$, $OR^{21}$, $SR^{21}$, $Si(R^{21})_3$, $B(OR^{21})_2$, $OSO_2R^{21}$, $CF_3$, CN, halogen,
$C_1$-$C_{40}$-alkyl,
which is optionally substituted with one or more substituents $R^{21}$ and wherein one or more non-adjacent $CH_2$-groups are optionally substituted by $R^{21}C$=$CR^{21}$, C=C, $Si(R^{21})_2$, $Ge(R^{21})_2$, $Sn(R^{21})_2$, C=O, C=S, C=Se, C=$NR^{21}$, P(=O)($R^{21}$), SO, $SO_2$, $NR^{21}$, O, S or $CONR^{21}$;
$C_1$-$C_{40}$-alkoxy,

which is optionally substituted with one or more substituents $R^{21}$ and wherein one or more non-adjacent $CH_2$-groups are optionally substituted by $R^{21}C=CR^{21}$, C=C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;
$C_1$-$C_{40}$-thioalkoxy,
which is optionally substituted with one or more substituents $R^{21}$ and wherein one or more non-adjacent $CH_2$-groups are optionally substituted by $R^{21}C=CR^{21}$, C=C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;
$C_2$-$C_{40}$-alkenyl,
which is optionally substituted with one or more substituents $R^{21}$ and wherein one or more non-adjacent $CH_2$-groups are optionally substituted by $R^{21}C=CR^{21}$, C=C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;
$C_2$-$C_{40}$-alkynyl,
which is optionally substituted with one or more substituents $R^{21}$ and wherein one or more non-adjacent $CH_2$-groups are optionally substituted by $R^{21}C=CR^{21}$, C=C, $Si(R^{21})_2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S or $CONR^{21}$;
$C_6$-$C_{60}$-aryl,
which is optionally substituted with one or more substituents $R^{21}$; and
$C_3$-$C_{57}$-heteroaryl,
which is optionally substituted with one or more substituents $R^{21}$;
wherein optionally one or more pair of adjacent groups $R^{VI}$ and $R^{VII}$, $R^{VII}$ and $R^{VIII}$, $R^{VIII}$ and $R^{X}$ $R^{X}$ and $R^{XI}$, $R^{XI}$ and $R^{XII}$, $R^{XII}$ and $R^{XIII}$ , $R^{XIV}$ and $R^{XV}$, $R^{XV}$ and $R^{XVI}$, $R^{XVI}$ and $R^{XVII}$, $R^{XVII}$ and $R^{XVIII}$, $R^{XIX}$ and $R^{XX}$, $R^{XX}$ and $R^{XXI}$, $R^{XXI}$ and $R^{XXII}$, $R^{XXII}$ and $R^{XXIII}$ forms an aromatic ring system which is fused to the adjacent benzene ring a, b, c or d of general formula $S^B$-III-3a and which is optionally substituted with one or more substituents $R^{21}$;
wherein optionally one or both pairs $R^{VI}$ and $R^{XXIII}$, $R^{XIII}$ and $R^{XIV}$ are joint to form a group $Z^4$ which is at each occurrence independently of each other selected from the group consisting of: a direct bond, $CR^{22}R^{23}$, $C=CR^{22}R^{23}$, C=O, $C=NR^{22}$, $NR^{22}$, O, $SiR^{22}R^{23}$, S, S(O), and $S(O)_2$;
$R^{21}$ is at each occurrence independently from another selected from the group consisting of: hydrogen, deuterium, OPh, SPh, $CF_3$, CN, F, $Si(C_1$-$C_5$-alkyl$)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
$N(C_3$-$C_{17}$-heteroaryl$)_2$, and
$N(C_3$-$C_{17}$-heteroaryl$)(C_6$-$C_{18}$-aryl);
$R^{22}$ and $R^{23}$ are at each occurrence independently of each other selected from the group consisting of: hydrogen, deuterium, $N(R^{24})_2$, $OR^{24}$, $Si(R^{24})_3$, $B(OR^{24})_2$, $OSO_2R^{24}$, $CF_3$, CN, F, Br, I,
$C_1$-$C_{40}$-alkyl,
which is optionally substituted with one or more substituents $R^{24}$ and wherein one or more non-adjacent $CH_2$-groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;
$C_1$-$C_{40}$-alkoxy,
which is optionally substituted with one or more substituents $R^{24}$ and wherein one or more non-adjacent $CH_2$-

groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;
$C_1$-$C_{40}$-thioalkoxy,
which is optionally substituted with one or more substituents $R^{24}$ and wherein one or more non-adjacent $CH_2$-groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;
$C_2$-$C_{40}$-alkenyl,
which is optionally substituted with one or more substituents $R^{24}$ and wherein one or more non-adjacent $CH_2$-groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;
$C_2$-$C_{40}$-alkynyl,
which is optionally substituted with one or more substituents $R^{24}$ and wherein one or more non-adjacent $CH_2$-groups are optionally substituted by $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S or $CONR^{24}$;
$C_6$-$C_{60}$-aryl,
which is optionally substituted with one or more substituents $R^{24}$; and
$C_3$-$C_{57}$-heteroaryl,
which is optionally substituted with one or more substituents $R^{24}$;
$R^{24}$ is at each occurrence independently from another selected from the group consisting of: hydrogen, deuterium, OPh, SPh, $CF_3$, CN, F, $Si(C_1$-$C_5$-alkyl$)_3$, $Si(Ph)_3$,
$C_1$-$C_5$-alkyl,
wherein optionally one or more hydrogen atoms are independently of each other substituted by deuterium, CN, $CF_3$, or F;
$C_1$-$C_5$-alkoxy,
wherein optionally one or more hydrogen atoms are independently of each other substituted by deuterium, CN, $CF_3$, or F;
$C_1$-$C_5$-thioalkoxy,
wherein optionally one or more hydrogen atoms are independently of each other substituted by deuterium, CN, $CF_3$, or F;
$C_2$-$C_5$-alkenyl,
wherein optionally one or more hydrogen atoms are independently of each other substituted by deuterium, CN, $CF_3$, or F;
$C_2$-$C_5$-alkynyl,
wherein optionally one or more hydrogen atoms are independently of each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
$N(C_3$-$C_{17}$-heteroaryl$)_2$, and
$N(C_3$-$C_{17}$-heteroaryl$)(C_6$-$C_{18}$-aryl$)$;
$R^A$ is selected from the group consisting of: hydrogen,
$C_3$-$C_{15}$-heteroaryl, wherein optionally one or more hydrogen atoms are independently of each other substituted by deuterium, halogen, $C_1$-$C_5$-alkyl, CN, $CF_3$, $SiMe_3$, $SiPh_3$ (Ph = phenyl), $C_3$-$C_{15}$-heteroaryl, and $C_6$-$C_{18}$-aryl, in which optionally one or more hydrogen atoms are independently from each other substituted by $C_1$-$C_5$-alkyl, CN, $CF_3$ and Ph;
and
$C_6$-$C_{18}$-aryl, wherein optionally one or more hydrogen atoms are independently of each other substituted by a substituent independently of each other selected from the group consisting of: $C_1$-$C_5$-alkyl, CN, $CF_3$, and

Ph, which is optionally substituted with one or more substituents independently of each other selected from the group consisting of Me, $^i$Pr, $^t$Bu, CN, $CF_3$, and Ph,
pyridinyl, which is optionally substituted with one or more substituents independently of each other selected from the group consisting of Me, $^i$Pr, $^t$Bu, CN, $CF_3$, and Ph,
pyrimidinyl, which is optionally substituted with one or more substituents independently of each other selected from the group consisting of Me, $^i$Pr, $^t$Bu, CN, $CF_3$, and Ph, and
triazinyl, which is optionally substituted with one or more substituents independently of each other selected

from the group consisting of Me, $^{i}Pr$, $^{t}Bu$, CN, $CF_3$, and Ph.

**9.** The organic electroluminescent device according to any of claims 1 to 8, wherein $R^A$ is $C_3$-$C_{15}$-heteroaryl,

wherein, optionally, one or more hydrogen atoms are independently of each other substituted by deuterium, halogen, $C_1$-$C_5$-alkyl, CN, $CF_3$, $SiMe_3$, $SiPh_3$ (Ph = phenyl), $C_3$-$C_{15}$-heteroaryl, and $C_6$-$C_{18}$-aryl, in which optionally one or more hydrogen atoms are independently from each other substituted by $C_1$-$C_5$-alkyl, CN, $CF_3$ and Ph;
wherein preferably the binding site of $R^A$ according to formula $S^B$-III-3a is one of the $C_3$-$C_{15}$- carbon atoms of the $C_3$-$C_{15}$-heteroaryl.

**10.** The organic electroluminescent device according to any of claims 1 to 9, comprising one or more p-host material $H^P$ which has a lowest unoccupied molecular orbital LUMO($H^P$) having an energy $E^{LUMO}(H^P)$ according to $-2.6\ eV \leq E^{LUMO}(H^P)$.

**11.** The organic electroluminescent device according to any of claims 1 to 10 comprising one or more p-host materials $H^P$ which comprise or consist of:

- one first chemical moiety, comprising or consisting of a structure according to any of the formulas $H^P$-I, $H^P$-II, $H^P$-III, $H^P$-IV, $H^P$-V, $H^P$-VI, $H^P$-VII, $H^P$-VIII, $H^P$-IX, and $H^P$-X:

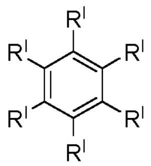

Formula $H^P$-I

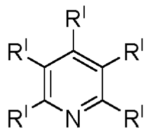

Formula $H^P$-II

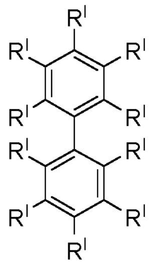

Formula $H^P$-III

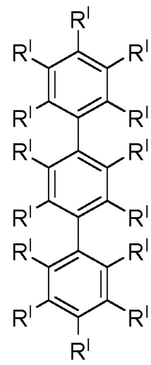

Formula $H^P$-IV

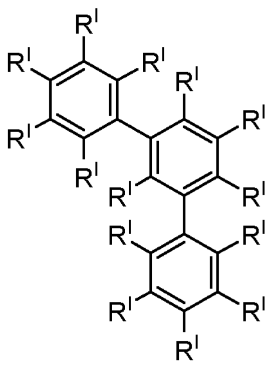

Formula $H^P$-V

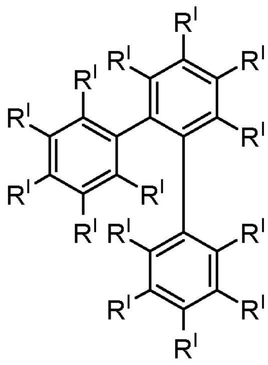

Formula $H^P$-VI

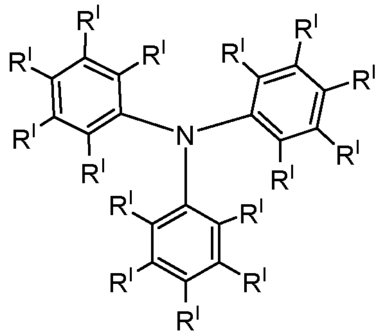

Formula $H^P$-VII

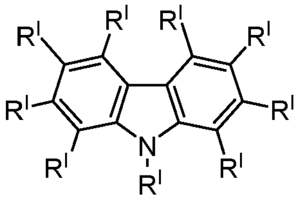

Formula $H^P$-VIII

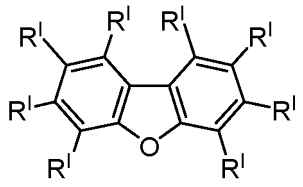

Formula $H^P$-IX

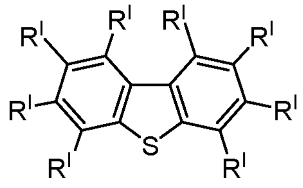

Formula $H^P$-X

and

- at least one second chemical moiety comprising or consisting of a structure according to any of formulas $H^P$-XI, $H^P$-XII, $H^P$-XIII, $H^P$-XIV, $H^P$-XV, $H^P$-XVI, $H^P$-XVII, $H^P$-XVIII, and $H^P$-XIX:

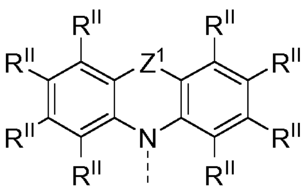

Formula $H^P$-XI

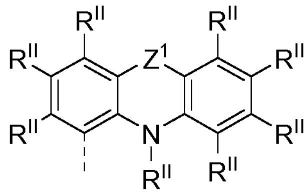

Formula $H^P$-XII

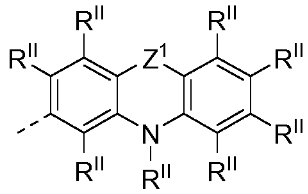

Formula $H^P$-XIII

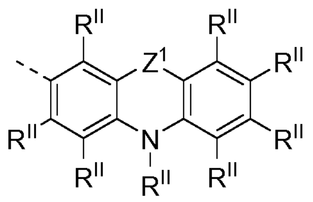

Formula $H^P$-XIV

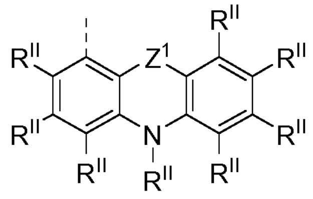

Formula $H^P$-XV

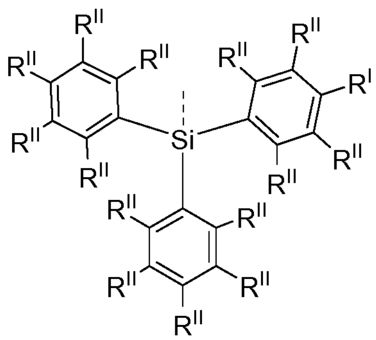

Formula $H^P$-XVI

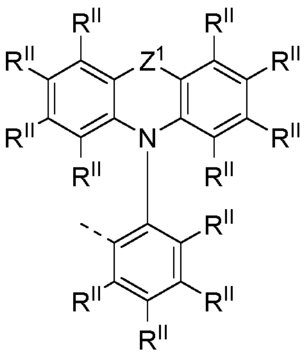

Formula $H^P$-XVII

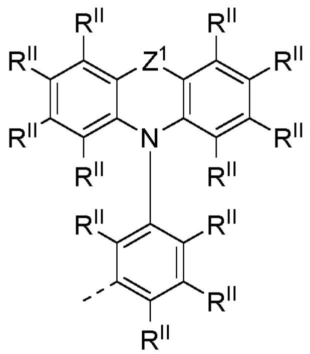

Formula $H^P$-XVIII

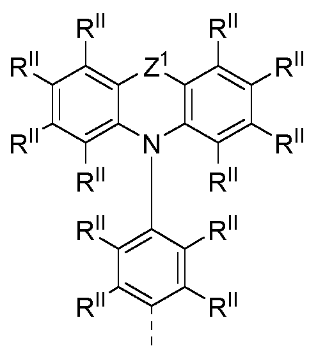

Formula $H^P$-XIX

wherein each of the at least one second chemical moieties which is present in the p-host material $H^P$ is linked to the first chemical moiety via a single bond which is represented in the formulas above by a dashed line;

wherein

$Z^1$ is at each occurrence independently of each other selected from the group consisting of a direct bond, $C(R^{II})_2$, $C=C(R^{II})_2$, C=O, $C=NR^{II}$, $NR^{II}$, O, $Si(R^{II})_2$, S, S(O) and $S(O)_2$;

$R^I$ is at each occurrence independently of each other a binding site of a single bond linking the first chemical moiety to a second chemical moiety or is selected from the group consisting of: hydrogen, deuterium, Me, $^i$Pr, $^t$Bu, wherein at least one $R^I$ is a binding site of a single bond linking the first chemical moiety to a second chemical

moiety, and
Ph, which is optionally substituted with one or more substituents independently of each other selected from the group consisting of: Me, $^{i}$Pr, $^{t}$Bu, and Ph;
$R^{II}$ is at each occurrence independently of each other selected from the group consisting of: hydrogen, deuterium, Me, $^{i}$Pr, $^{t}$Bu, and
Ph, which is optionally substituted with one or more substituents independently of each other selected from the group consisting of: Me, $^{i}$Pr, $^{t}$Bu, and Ph;
wherein two or more adjacent substituents $R^{II}$ may optionally form an aromatic or heteroaromatic ring system with 3-18 carbon atoms.

**12.** The organic electroluminescent device according to any of claims 1 to 11, wherein said organic electroluminescent device is a device selected from the group consisting of an organic light emitting diode, a light emitting electrochemical cell, and a light-emitting transistor.

**13.** The organic electroluminescent device according to any of claims 1 to 12, wherein the at least one light-emitting layer B comprises:

(i) 10-89.9 % by weight of one or more p-host compound $H^P$;
(ii) 0-79.9 % by weight of one or more n-host compound $H^N$;
(iii) 10-50 % by weight of one or more TADF material $E^B$; and
(iv) 0.1-10 % by weight of one or more small FWHM emitter $S^B$; and
(v) 0-72 % by weight of one or more solvents.

**14.** The organic electroluminescent device according to any of claims 1 to 12, wherein the at least one light-emitting layer B comprises:

(i) 22-87.5 % by weight of one or more p-host compound $H^P$;
(ii) optionally 0-65.5 % by weight of one or more n-host compound $H^N$;
(iii) 12-40 % by weight of one or more TADF material $E^B$; and
(iv) 0.5-5 % by weight of one or more small FWHM emitter $S^B$; and
(v) 0-65.5 % by weight of one or more solvents.

**15.** A method for generating green light at a wavelength of from 500 nm to 560 nm, comprising the steps of:

(i) providing an organic electroluminescent device according to any of claims 1 to 14; and
(ii) applying an electrical current to said organic electroluminescent device.

**Patentansprüche**

**1.** Organische elektrolumineszente Vorrichtung umfassend eine oder mehrere lichtemittierende Schichten B, die jeweils unabhängig voneinander umfassen:

(i) mindestens einzeil Wirtsmaterial $H^B$, das ein niedrigstes angeregtes Singulett-Zustands-Energieniveau $E(S1^H)$ und ein niedrigstes angeregtes Triplett-Zustands-Energieniveau $E(T1^H)$ aufweist;
(ii) mindestens ein thermisch aktiviertes verzögertes Fluoreszenz (TADF)-Material $E^B$, das ein niedrigstes angeregtes Singulett-Zustands-Energieniveau $E(S1^E)$ und ein niedrigstes angeregtes Triplett-Zustands-Energieniveau $E(T1^E)$ aufweist; und
(iii) mindestens einen Emitter $S^B$ mit kleiner Halbwertsbreite (FWHM) mit einem Emissionsspektrum, das eine FWHM von weniger als oder gleich 0,25 eV aufweist, gemessen mit 1 Gew.-% des Emitters $S^B$ in Poly(methylmethacrylat bei 20°C, der ein niedrigstes angeregtes Singulett-Zustands-Energieniveau $E(S1^S)$ und ein niedrigstes angeregtes Triplett-Zustands-Energieniveau $E(T1^S)$ aufweist,
wobei jedes $E^B$ Energie auf mindestens ein $S^B$ überträgt und jedes $S^B$ Licht mit einem Emissionsmaximum zwischen 500 nm und 560 nm emittiert, wobei die durch die folgenden Formeln (1) bis (5) ausgedrückten Beziehungen gelten:

$$E(S1^H) > E(S1^E) \qquad (1)$$

$$E(S1^H) > E(S1^S) \quad (2)$$

$$E(S1^E) > E(S1^S) \quad (3)$$

$$E(T1^H) > E(T1^S) \quad (4)$$

$$E(T1^H) > E(T1^E) \quad (5);$$

wobei mindestens ein Emitter $S^B$ mit geringer Halbwertsbreite (FWHM) ein borhaltiger Emitter ist.

**2.** Organische elektrolumineszente Vorrichtung nach Anspruch 1, wobei ein TADF-Material $E^B$ **dadurch gekennzeichnet ist, dass** es einen $\Delta E_{ST}$-Wert, der der Energiedifferenz zwischen dem niedrigsten angeregten Singulett-Zustand $S1^E$ und dem niedrigsten angeregten Triplett-Zustand $T1^E$ entspricht, von weniger als 0,4 eV aufweist.

**3.** Organische elektrolumineszente Vorrichtung nach einem der Ansprüche 1 und 2, wobei der mindestens eine Emitter $S^B$ mit kleiner FWHM **dadurch gekennzeichnet ist, dass** er ein Emissionsspektrum aufweist, das eine Halbwertsbreite (FWHM) von weniger als oder gleich 0,25 eV aufweist.

**4.** Organische elektrolumineszente Vorrichtung nach einem der Ansprüche 1 bis 3, wobei das mindestens eine thermisch aktivierte verzögerte Fluoreszenz (TADF)-Material $E^B$ ein Emissionsmaximum $\lambda_{max}(D)$ im Wellenlängenbereich von 500 nm bis 560 nm aufweist.

**5.** Organische elektrolumineszente Vorrichtung nach einem der Ansprüche 1 bis 4, wobei das mindestens eine thermisch aktivierte verzögerte Fluoreszenz (TADF)-Material $E^B$ ein höchstes besetztes Molekülorbital $HOMO(E^B)$ mit einer Energie $E^{HOMO}(E^B)$ gemäß-6,0 ev $\leq E^{HOMO}(E^B) \leq$ -5,8 eV aufweist.

**6.** Organische elektrolumineszente Vorrichtung nach einem der Ansprüche 1 bis 5, wobei das mindestens eine thermisch aktivierte verzögerte Fluoreszenz (TADF)-Material $E^B$ eine Struktur hat, die durch eine der Formeln $E^B$-I-1a, $E^B$-I-2a, $E^B$-I-3a, $E^B$-I-4a, $E^B$-I-5a, $E^B$-I-6a, $E^B$-I-7 und $E^B$-I-8 dargestellt wird,

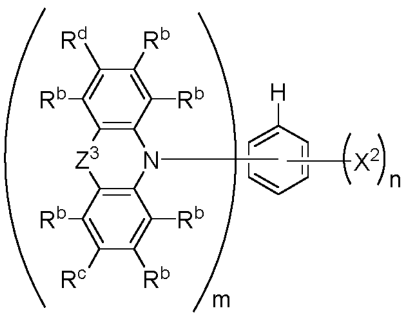

Formel $E^B$-I-1a

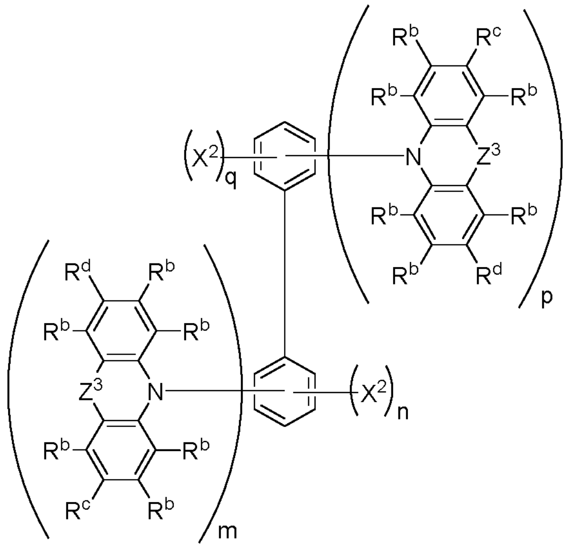

Formel $E^B$-I-2a

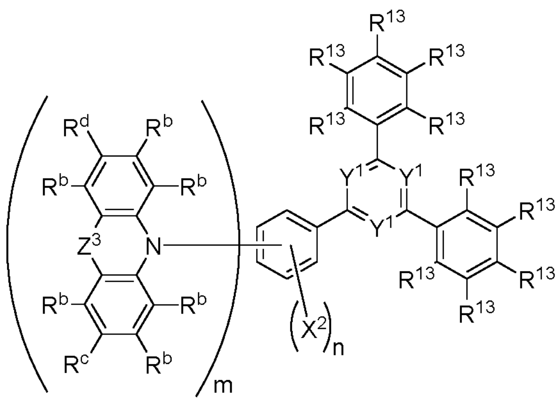

Formel $E^B$-I-3a

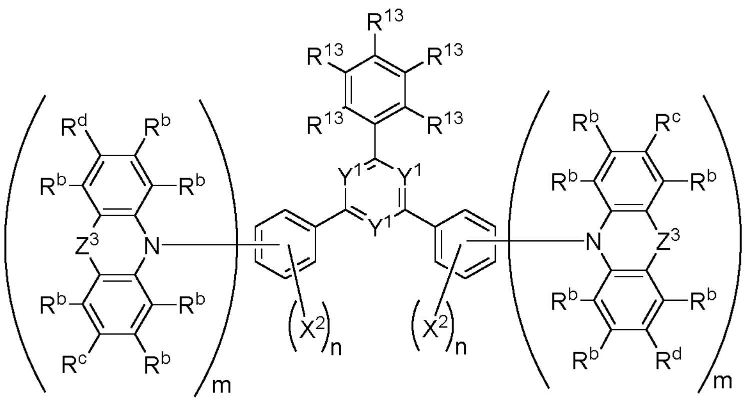

Formel $E^B$-I-4a

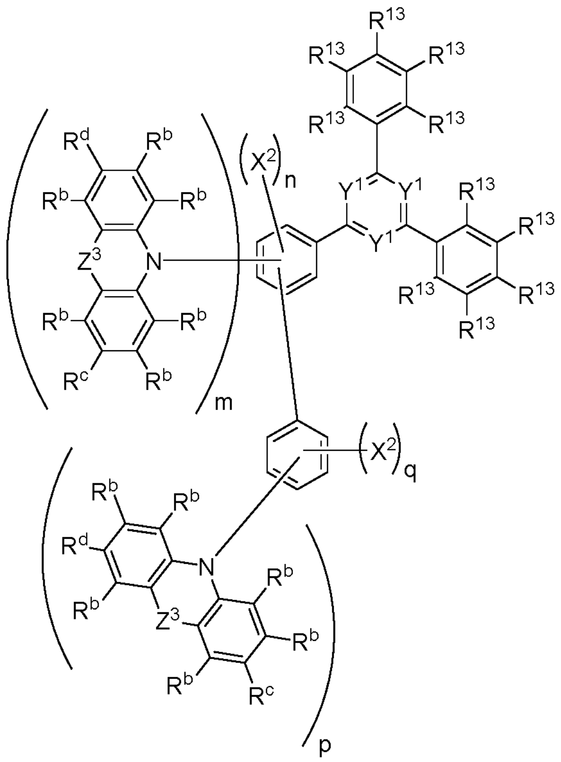

Formel $E^B$-I-5a

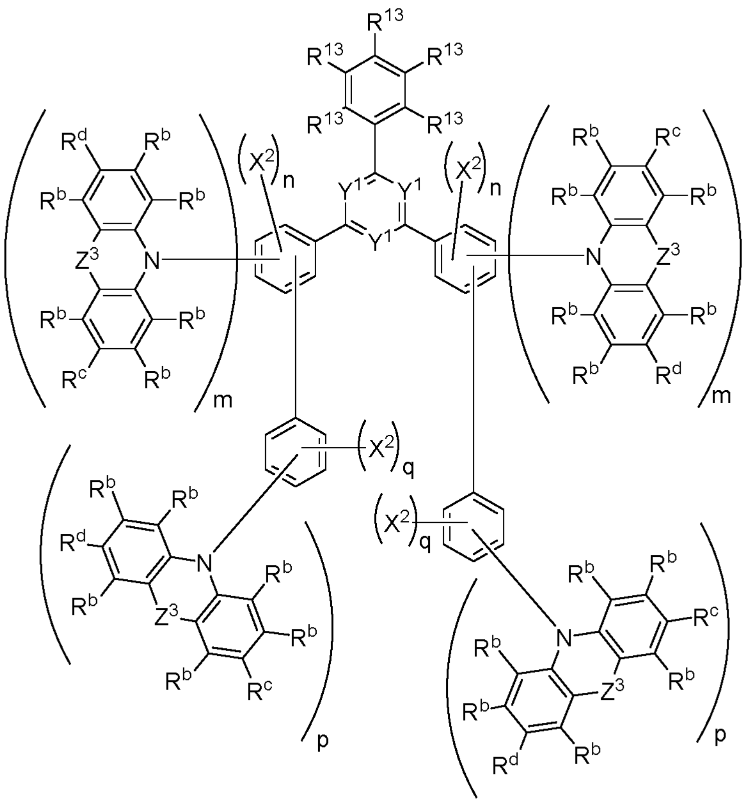

Formel $E^B$-I-6a

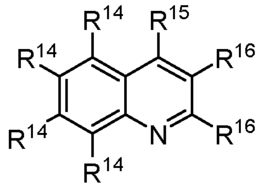

Formel $E^B$-I-7

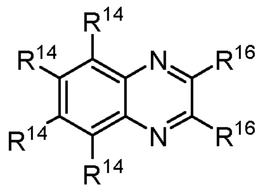

Formel $E^B$-I-8

wobei

$Y^1$ bei jedem Auftreten Stickstoff (N) oder CH ist, und mindestens ein $Y^1$ N ist;
m bei jedem Auftreten unabhängig voneinander 0, 1 oder 2 ist;
n bei jedem Auftreten unabhängig voneinander 0, 1 oder 2 ist;
p bei jedem Auftreten unabhängig voneinander 0, 1 oder 2 ist;
q bei jedem Auftreten unabhängig voneinander 0, 1 oder 2 ist;
$X^2$ bei jedem Auftreten unabhängig voneinander ausgewählt ist aus der Gruppe bestehend aus $Ar^{EWG}$, CN und $CF_3$;
$Ar^{EWG}$ bei jedem Auftreten unabhängig voneinander durch eine Struktur gemäß einer der Formeln $Ar^{EWG}$-I, $Ar^{EWG}$-II, $Ar^{EWG}$-III, $Ar^{EWG}$-IV, $Ar^{EWG}$-V, $Ar^{EWG}$-VI, $Ar^{EWG}$-VII, $Ar^{EWG}$-VIII, $Ar^{EWG}$-IX, $Ar^{EWG}$-X, $Ar^{EWG}$-XI, $Ar^{EWG}$-XII, $Ar^{EWG}$-XIII und $Ar^{EWG}$-XIV dargestellt wird,

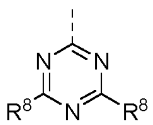

Formel $Ar^{EWG}$-I

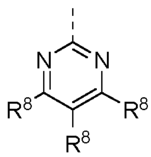

Formel $Ar^{EWG}$-II

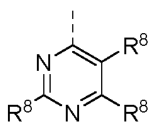

Formel $Ar^{EWG}$-III

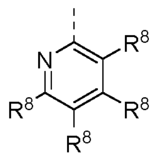

Formel $Ar^{EWG}$-IV

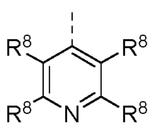

Formel $Ar^{EWG}$-V

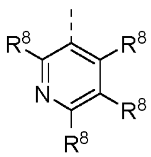

Formel $Ar^{EWG}$-VI

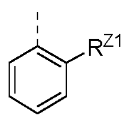

Formel $Ar^{EWG}$-VII

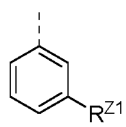

Formel $Ar^{EWG}$-VIII

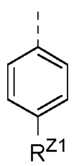

Formel $Ar^{EWG}$-IX

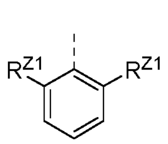

Formel $Ar^{EWG}$-X

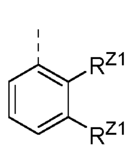

Formel $Ar^{EWG}$-XI

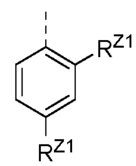

Formel $Ar^{EWG}$-XII

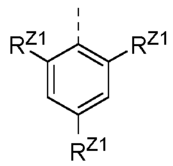
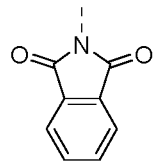

Formel $Ar^{EWG}$-XIII Formel $Ar^{EWG}$-XIV

die an die Kernstruktur über die Position gebunden sind, die durch die gestrichelte Linie markiert ist;
$R^{Z1}$ bei jedem Auftreten unabhängig voneinander ausgewählt ist aus der Gruppe bestehend aus CN und $CF_3$;
$Z^3$ bei jedem Auftreten unabhängig voneinander ausgewählt ist aus der Gruppe bestehend aus einer direkten Bindung, $CR^9R^{10}$, $C{=}CR^9R^{10}$, C=O, $C{=}NR^9$, $NR^9$, O, $SiR^9R^{10}$, S, S(O) und $S(O)_2$;
$R^8$ bei jedem Auftreten unabhängig voneinander ausgewählt ist aus der Gruppe bestehend aus: Wasserstoff, Deuterium, CN, $CF_3$,
$C_1$-$C_5$-Alkyl,
wobei ein oder mehrere Wasserstoffatome optional durch Deuterium ersetzt sind;
$C_6$-$C_{18}$-Aryl,
das optional mit einem oder mehreren Substituenten $R^{11}$ substituiert ist; und
$C_3$-$C_{17}$-Heteroaryl,
das optional mit einem oder mehreren Substituenten $R^{11}$ substituiert ist;
$R^b$, $R^c$, $R^d$, $R^9$ und $R^{10}$ bei jedem Auftreten unabhängig voneinander ausgewählt sind aus der Gruppe bestehend aus: Wasserstoff, Deuterium, $N(R^{12})_2$, $OR^{12}$, $Si(R^{12})_3$, $B(OR^{12})_2$, $OSO_2R^{12}$, $CF_3$, CN, F, Br, I,
$C_1$-$C_{40}$-Alkyl,

das optional mit einem oder mehreren Substituenten $R^{12}$ substituiert ist, und
wobei eine oder mehrere nicht benachbarte $CH_2$-Gruppen optional C=Se, $C{=}NR^{12}$, $P(=O)(R^{12})$, SO, $SO_2$, $NR^{12}$, O, S oder $CONR^{12}$ sind;

$C_1$-$C_{40}$-Alkoxy,

das optional mit einem oder mehreren Substituenten $R^{12}$ substituiert ist, und
wobei eine oder mehrere nicht benachbarte $CH_2$-Gruppen optional durch $R^{12}C{=}CR^{12}$, C≡C, $Si(R^{12})_2$, $Ge(R^{12})_2$, $Sn(R^{12})_2$, C=O, C=S, C=Se, $C{=}NR^{12}$, $P(=O)(R^{12})$, SO, $SO_2$, $NR^{12}$, O, S oder $CONR^{12}$ ersetzt sind;

$C_1$-$C_{40}$-Thioalkoxy,

das optional mit einem oder mehreren Substituenten $R^{12}$ substituiert ist und
wobei eine oder mehrere nicht benachbarte $CH_2$-Gruppen optional durch $R^{12}C{=}CR^{12}$, C=C, $Si(R^{12})_2$, $Ge(R^{12})_2$, $Sn(R^{12})_2$, C=O, C=S, C=Se, $C{=}NR^{12}$, $P(=O)(R^{12})$, SO, $SO_2$, $NR^{12}$, O, S oder $CONR^{12}$ ersetzt sind;

$C_2$-$C_{40}$-Alkenyl,

das optional mit einem oder mehreren Substituenten $R^{12}$ substituiert ist und
wobei eine oder mehrere nicht benachbarte $CH_2$-Gruppen optional durch $R^{12}C{=}CR^{12}$, C=C, $Si(R^{12})_2$, $Ge(R^{12})_2$, $Sn(R^{12})_2$, C=O, C=S, C=Se, $C{=}NR^{12}$, $P(=O)(R^{12})$, SO, $SO_2$, $NR^{12}$, O, S oder $CONR^{12}$ ersetzt sind;

$C_2$-$C_{40}$-Alkinyl,

die optional mit einem oder mehreren Substituenten $R^{12}$ substituiert ist, und
wobei eine oder mehrere nicht benachbarte $CH_2$-Gruppen optional durch $R^{12}C{=}CR^{12}$, C=C, $Si(R^{12})_2$, $Ge(R^{12})_2$, $Sn(R^{12})_2$, C=O, C=S, C=Se, $C{=}NR^{12}$, $P(=O)(R^{12})$, SO, $SO_2$, $NR^{12}$, O, S oder $CONR^{12}$ ersetzt sind;

$C_6$-$C_{60}$-Aryl,
das optional mit einem oder mehreren Substituenten $R^{12}$ substituiert ist; und
$C_3$-$C_{57}$-Heteroaryl,
das optional mit einem oder mehreren Substituenten $R^{12}$ substituiert ist;
$R^{11}$ bei jedem Auftreten unabhängig voneinander ausgewählt ist aus der Gruppe bestehend aus: Wasserstoff, Deuterium, Me, $^i$Pr, $^t$Bu, CN, $CF_3$, und

Phenyl, das optional mit einem oder mehreren Substituenten substituiert ist, die unabhängig voneinander aus der Gruppe ausgewählt sind, die aus Me, $^{i}$Pr, $^{t}$Bu, CN, $CF_3$ und Ph besteht;
$R^{12}$ bei jedem Auftreten unabhängig voneinander ausgewählt ist aus der Gruppe, bestehend aus: Wasserstoff, Deuterium, OPh, $CF_3$, CN, F,
$C_1$-$C_5$-Alkyl,
wobei ein oder mehrere Wasserstoffatome optional, unabhängig voneinander, durch Deuterium, CN, $CF_3$ oder F ersetzt sind;
$C_1$-$C_5$-Alkoxy,
wobei ein oder mehrere Wasserstoffatome optional unabhängig voneinander durch Deuterium, CN, $CF_3$ oder F ersetzt sind;
$C_1$-$C_5$-Thioalkoxy,
wobei ein oder mehrere Wasserstoffatome optional unabhängig voneinander durch Deuterium, CN, $CF_3$ oder F ersetzt sind;
$C_2$-$C_5$-Alkenyl,
wobei ein oder mehrere Wasserstoffatome optional unabhängig voneinander durch Deuterium, CN, $CF_3$ oder F ersetzt sind;
$C_2$-$C_5$-Alkinyl,
wobei ein oder mehrere Wasserstoffatome optional unabhängig voneinander durch Deuterium, CN, $CF_3$ oder F ersetzt sind;
$C_6$-$C_{18}$-Aryl,
das optional mit einem oder mehreren $C_1$-$C_5$-Alkylsubstituenten substituiert ist;
$C_3$-$C_{17}$-Heteroaryl,
das optional mit einem oder mehreren $C_1$-$C_5$-Alkylsubstituenten substituiert ist;
N($C_6$-$C_{18}$-Aryl)$_2$,
N($C_3$-$C_{17}$-Heteroaryl)$_2$,
N($C_3$-$C_{17}$-Heteroaryl)($C_6$-$C_{18}$-Aryl), und
einer aliphatischen cyclischen Aminogruppe umfassend 5 bis 8 Kohlenstoffatome (vorzugsweise Pyrrolidinyl und Piperidinyl);
$R^{13}$ bei jedem Auftreten unabhängig voneinander ausgewählt ist aus der Gruppe bestehend aus: Wasserstoff, Deuterium, CN, $CF_3$, $Ar^{EWG}$,
$C_1$-$C_5$-Alkyl,
wobei ein oder mehrere Wasserstoffatome optional durch Deuterium ersetzt sind;
$C_6$-$C_{18}$-Aryl,
das optional mit einem oder mehreren Substituenten unabhängig voneinander ausgewählt aus Deuterium, $C_1$-$C_5$-Alkylgruppen und $C_6$-$C_{18}$-Arylgruppen substituiert ist; und
$R^{14}$ und $R^{15}$ bei jedem Auftreten unabhängig voneinander ausgewählt sind aus der Gruppe bestehend aus C-$Ar^{EWG}$ und $CR^{Q2}$;
$R^{16}$ bei jedem Auftreten unabhängig voneinander ausgewählt ist aus der Gruppe bestehend aus $CR^6$, C-$Ar^{EWG}$ und $CR^{Q2}$;
$R^{Q2}$ bei jedem Auftreten unabhängig voneinander ausgewählt ist aus der Gruppe, bestehend aus: Wasserstoff, Deuterium, Me, $^{i}$Pr, $^{t}$Bu, CN, $CF_3$, Ph, und
Carbazolyl, das optional mit einem oder mehreren Substituenten unabhängig voneinander ausgewählt aus der Gruppe bestehend aus Me, $^{i}$Pr, $^{t}$Bu, CN, $CF_3$, Ph und $N(Ph)_2$ substituiert ist;
wobei optional beliebige benachbarte Substituenten $R^b$, $R^c$, $R^d$, $R^9$ und $R^{10}$ unabhängig voneinander ein mono- oder polycyclisches, aliphatisches, aromatisches und/oder benzokondensiertes Ringsystem bilden; wobei ein oder mehrere Wasserstoffatome des optional so gebildeten Ringsystems durch $R^{12}$ ersetzt sein können; und wobei nicht mehr als zwei Gruppen $R^{13}$ CN, $CF_3$ oder $Ar^{EWG}$ sind;
wobei:

$$1 \leq (m + p)$$

und

$$1 \leq (n + q).$$

**7.** Organische elektrolumineszente Vorrichtung nach einem der Ansprüche 1 bis 6, wobei der mindestens eine Emitter $S^B$ mit kleiner FWHM eine Struktur gemäß der Formel $S^B$-I umfasst oder daraus besteht:

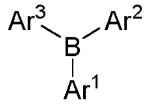

Formel $S^B$-I

wobei B Bor ist,
$Ar^1$, $Ar^2$ und $Ar^3$ bei jedem Auftreten unabhängig voneinander ausgewählt sind aus der Gruppe bestehend aus einem aromatischen Ring und einem heteroaromatischen Ring, und $Ar^1$, $Ar^2$, $Ar^3$ optional miteinander verbunden sein können, um einen oder mehrere zusätzliche Ringe zu bilden.

**8.** Organische elektrolumineszente Vorrichtung nach einem der Ansprüche 1 bis 7, wobei der mindestens eine Emitter $S^B$ mit kleiner FWHM eine Struktur der Formel $S^B$-III-3a umfasst oder daraus besteht:

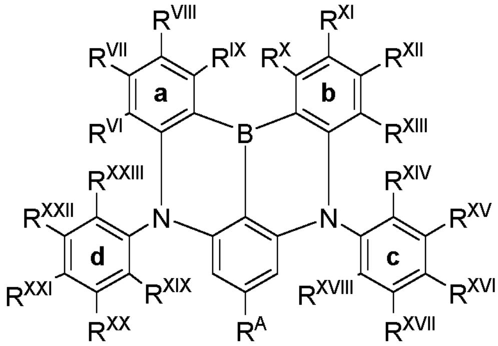

Formel $S^B$-III-3a

wobei $R^{VI}$, $R^{VII}$, $R^{VIII}$, $R^{IX}$, $R^{X}$, $R^{XI}$, $R^{XII}$, $R^{XIII}$, $R^{XIV}$, $R^{XV}$, $R^{XVI}$, $R^{XVII}$, $R^{XVIII}$, $R^{XIX}$, $R^{XX}$, $R^{XXI}$, $R^{XXII}$ und $R^{XXIII}$ unabhängig voneinander ausgewählt sind aus der Gruppe, bestehend aus: Wasserstoff, Deuterium, $N(R^{21})_2$, $OR^{21}$, $SR^{21}$, $Si(R^{21})_3$, $B(OR^{21})_2$, $OSO_2R^{21}$, $CF_3$, CN, Halogen,
$C_1$-$C_{40}$-Alkyl,

das optional mit einem oder mehreren Substituenten $R^{21}$ substituiert ist, und
wobei eine oder mehrere nicht benachbarte $CH_2$-Gruppen optional durch $R^{21}C{=}CR^{21}$, C≡C, $Si(R^{21})_2$, $Ge(R^{21})_2$, $Sn(R^{21})_2$, C=O, C=S, C=Se, $C{=}NR^{21}$, $P(=O)(R^{21})$, SO, $SO_2$, $NR^{21}$, O, S oder $CONR^{21}$ ersetzt sind;

$C_1$-$C_{40}$-Alkoxy,

das optional mit einem oder mehreren Substituenten $R^{21}$ substituiert ist, und
wobei eine oder mehrere nicht benachbarte $CH_2$-Gruppen optional durch $R^{21}C{=}CR^{21}$, C≡C, $Si(R^{21})2$, $Ge(R^{21})_2$, $Sn(R^{21})_2$, C=O, C=S, C=Se, $C{=}NR^{21}$, $P(=O)(R^{21})$, SO, $SO_2$, $NR^{21}$, O, S oder $CONR^{21}$ ersetzt sind;

$C_1$-$C_{40}$-Thioalkoxy,

das optional mit einem oder mehreren Substituenten $R^{21}$ substituiert ist, und
wobei eine oder mehrere nicht benachbarte $CH_2$-Gruppen optional durch $R^{21}C{=}CR^{21}$, C≡C, $Si(R^{21})2$, $Ge(R^{21})_2$, $Sn(R^{21})_2$, C=O, C=S, C=Se, $C{=}NR^{21}$, $P(=O)(R^{21})$, SO, $SO_2$, $NR^{21}$, O, S oder $CONR^{21}$ ersetzt sind;

$C_2$-$C_{40}$-Alkenyl,

das optional mit einem oder mehreren Substituenten $R^{21}$ substituiert ist und
wobei eine oder mehrere nicht benachbarte $CH_2$-Gruppen optional durch $R^{21}C{=}CR^{21}$, C≡C, $Si(R^{21})2$, $Ge(R^{21})_2$, $Sn(R^{21})_2$, C=O, C=S, C=Se, $C{=}NR^{21}$, $P(=O)(R^{21})$, SO, $SO_2$, $NR^{21}$, O, S oder $CONR^{21}$ ersetzt sind;

$C_2$-$C_{40}$-Alkinyl,

das optional mit einem oder mehreren Substituenten $R^{21}$ substituiert ist und

wobei eine oder mehrere nicht benachbarte $CH_2$-Gruppen optional durch $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S oder $CONR^{21}$ ersetzt sind;

$C_6$-$C_{60}$-Aryl,
das optional mit einem oder mehreren Substituenten $R^{21}$ substituiert ist; und
$C_3$-$C_{57}$-Heteroaryl,
das optional mit einem oder mehreren Substituenten $R^{21}$ substituiert ist;
wobei optional ein oder mehrere Paare von benachbarten Gruppen $R^{VI}$ und $R^{VII}$, $R^{VII}$ und $R^{VIII}$, $R^{VIII}$ und $R^{IX}$, $R^{X}$ und $R^{XI}$, $R^{XI}$ und $R^{XII}$, $R^{XII}$ und $R^{XIII}$, $R^{XIV}$ und $R^{XV}$, $R^{XV}$ und $R^{XVI}$, $R^{XVI}$ und $R^{XVII}$, $R^{XVII}$, und $R^{XVIII}$, $R^{XIX}$ und $R^{XX}$, $R^{XX}$ und $R^{XXI}$ $R^{XXI}$ und $R^{XXII}$, $R^{XXII}$ und $R^{XXIII}$ ein aromatisches Ringsystem bildet, das mit dem benachbarten Benzolring a, b, c oder d der allgemeinen Formel $S^{B}$-III-3a kondensiert ist und das optional mit einem oder mehreren Substituenten $R^{21}$ substituiert ist;
wobei optional ein oder beide Paare $R^{VI}$ und $R^{XXIII}$, $R^{XIII}$ und $R^{XIV}$ verbunden sind, um eine Gruppe $Z^4$ zu bilden, die bei jedem Auftreten unabhängig voneinander ausgewählt ist aus der Gruppe bestehend aus: einer direkten Bindung, $CR^{22}R^{23}$, $C=CR^{22}R^{23}$, C=O, $C=NR^{22}$, $NR^{22}$, O, $SiR^{22}R^{23}$, S, S(O) und $S(O)_2$;
$R^{21}$ bei jedem Auftreten unabhängig von einem anderen ausgewählt ist aus der Gruppe bestehend aus: Wasserstoff, Deuterium, OPh, SPh, $CF_3$, CN, F, $Si(C_1$-$C_5$-$Alkyl)_3$, $Si(Ph)_3$,
$C_1$-$C_5$-Alkyl,
wobei optional ein oder mehrere Wasserstoffatome unabhängig voneinander durch Deuterium, CN, $CF_3$ oder F ersetzt sind;
$C_1$-$C_5$-Alkoxy,
wobei optional ein oder mehrere Wasserstoffatome unabhängig voneinander durch Deuterium, CN, $CF_3$ oder F ersetzt sind;
$C_1$-$C_5$-Thioalkoxy,
wobei optional ein oder mehrere Wasserstoffatome unabhängig voneinander durch Deuterium, CN, $CF_3$ oder F ersetzt sind;
$C_2$-$C_5$-Alkenyl,
wobei optional ein oder mehrere Wasserstoffatome unabhängig voneinander durch Deuterium, CN, $CF_3$ oder F ersetzt sind;
$C_2$-$C_5$-Alkinyl,
wobei optional ein oder mehrere Wasserstoffatome unabhängig voneinander durch Deuterium, CN, $CF_3$ oder F ersetzt sind;
$C_6$-$C_{18}$-Aryl,
das optional mit einem oder mehreren $C_1$-$C_5$-Alkylsubstituenten substituiert ist;
$C_3$-$C_{17}$-Heteroaryl,
das optional mit einem oder mehreren $C_1$-$C_5$-Alkylsubstituenten substituiert ist;
$N(C_6$-$C_{18}$-$Aryl)_2$,
$N(C_3$-$C_{17}$-$Heteroaryl)_2$, und
$N(C_3$-$C_{17}$-Heteroaryl)$(C_6$-$C_{18}$-Aryl);
$R^{22}$ und $R^{23}$ bei jedem Auftreten unabhängig voneinander ausgewählt sind aus der Gruppe bestehend aus: Wasserstoff, Deuterium, $N(R^{24})_2$, $OR^{24}$, $Si(R^{24})_3$, $B(OR^{24})_2$, $OSO_2R^{24}$, $CF_3$, CN, F, Br, I,
$C_1$-$C_{40}$-Alkyl,

das optional mit einem oder mehreren Substituenten $R^{24}$ substituiert ist, und
wobei eine oder mehrere nicht benachbarte $CH_2$-Gruppen optional durch $R^{24}C=CR^{24}$, C≡C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S oder $CONR^{24}$ ersetzt sind;

$C_1$-$C_{40}$-Alkoxy,

die optional mit einem oder mehreren Substituenten $R^{24}$ substituiert ist, und
wobei eine oder mehrere nicht benachbarte $CH_2$-Gruppen optional durch $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S oder $CONR^{24}$ ersetzt sind;

$C_1$-$C_{40}$-Thioalkoxy,

das optional mit einem oder mehreren Substituenten $R^{24}$ substituiert ist und
wobei eine oder mehrere nicht benachbarte $CH_2$-Gruppen optional durch $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S oder $CONR^{24}$ ersetzt sind;

$C_2$-$C_{40}$-Alkenyl,

das optional mit einem oder mehreren Substituenten $R^{24}$ substituiert ist und
wobei eine oder mehrere nicht benachbarte $CH_2$-Gruppen optional durch $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S oder $CONR^{24}$ ersetzt sind;

$C_2$-$C_{40}$-Alkinyl,

das optional mit einem oder mehreren Substituenten $R^{24}$ substituiert ist, und
wobei eine oder mehrere nicht benachbarte $CH_2$-Gruppen optional durch $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S oder $CONR^{24}$ ersetzt sind;

$C_6$-$C_{60}$-Aryl,
das optional mit einem oder mehreren Substituenten $R^{24}$ substituiert ist; und
$C_3$-$C_{57}$-Heteroaryl,
das optional mit einem oder mehreren Substituenten $R^{24}$ substituiert ist;
$R^{24}$ bei jedem Auftreten unabhängig von einem anderen ausgewählt ist aus der Gruppe bestehend aus: Wasserstoff, Deuterium, OPh, SPh, $CF_3$, CN, F, $Si(C_1$-$C_5$-$Alkyl)_3$, $Si(Ph)_3$,
$C_1$-$C_5$-Alkyl,
wobei optional ein oder mehrere Wasserstoffatome unabhängig voneinander durch Deuterium, CN, $CF_3$ oder F ersetzt sind;
$C_1$-$C_5$-Alkoxy,
wobei optional ein oder mehrere Wasserstoffatome unabhängig voneinander durch Deuterium, CN, $CF_3$ oder F ersetzt sind;
$C_1$-$C_5$-Thioalkoxy,
wobei optional ein oder mehrere Wasserstoffatome unabhängig voneinander durch Deuterium, CN, $CF_3$ oder F ersetzt sind;
$C_2$-$C_5$-Alkenyl,
wobei optional ein oder mehrere Wasserstoffatome unabhängig voneinander durch Deuterium, CN, $CF_3$ oder F ersetzt sind;
$C_2$-$C_5$-Alkinyl,
wobei optional ein oder mehrere Wasserstoffatome unabhängig voneinander durch Deuterium, CN, $CF_3$ oder F ersetzt sind;
$C_6$-$C_{18}$-Aryl,
das optional mit einem oder mehreren $C_1$-$C_5$-Alkylsubstituenten substituiert ist;
$C_3$-$C_{17}$-Heteroaryl,
das optional mit einem oder mehreren $C_1$-$C_5$-Alkylsubstituenten substituiert ist;
$N(C_6$-$C_{18}$-$Aryl)_2$,
$N(C_3$-$C_{17}$-$Heteroaryl)_2$, und
$N(C_3$-$C_{17}$-Heteroaryl)$(C_6$-$C_{18}$-Aryl);
$R^A$ ausgewählt ist aus der Gruppe bestehend aus: Wasserstoff,
$C_3$-$C_{15}$-Heteroaryl, wobei optional ein oder mehrere Wasserstoffatome unabhängig voneinander durch Deuterium, Halogen, $C_1$-$C_5$-Alkyl, CN, $CF_3$, $SiMe_3$, $SiPh_3$ (Ph = Phenyl), $C_3$-$C_{15}$-Heteroaryl und
$C_6$-$C_{18}$-Aryl, in denen optional ein oder mehrere Wasserstoffatome unabhängig voneinander durch $C_1$-$C_5$-Alkyl, CN, $CF_3$ und Ph ersetzt sind;
und
$C_6$-$C_{18}$-Aryl, wobei optional ein oder mehrere Wasserstoffatome unabhängig voneinander durch einen Substituenten ersetzt sind, der unabhängig voneinander ausgewählt ist aus der Gruppe bestehend aus: $C_1$-$C_5$-Alkyl, CN, $CF_3$, und

Ph, das optional mit einem oder mehreren Substituenten substituiert ist, unabhängig voneinander ausgewählt aus der Gruppe bestehend aus Me, $^i$Pr, $^t$Bu, CN, $CF_3$ und Ph,
Pyridinyl, das optional mit einem oder mehreren Substituenten substituiert ist, unabhängig voneinander ausgewählt aus der Gruppe bestehend aus Me, $^i$Pr, $^t$Bu, CN, $CF_3$ und Ph,
Pyrimidinyl, das optional mit einem oder mehreren Substituenten substituiert ist, unabhängig voneinander ausgewählt aus der Gruppe bestehend aus Me, $^i$Pr, $^t$Bu, CN, $CF_3$ und Ph, und
Triazinyl, das optional mit einem oder mehreren Substituenten substituiert ist, unabhängig voneinander ausgewählt aus der Gruppe bestehend aus Me, $^i$Pr, $^t$Bu, CN, $CF_3$ und Ph.

**9.** Organische elektrolumineszente Vorrichtung nach einem der Ansprüche 1 bis 8, wobei $R^A$ $C_3$-$C_{15}$-Heteroaryl ist,

wobei optional ein oder mehrere Wasserstoffatome unabhängig voneinander durch Deuterium, Halogen, $C_1$-$C_5$-Alkyl, CN, $CF_3$, $SiMe_3$, $SiPh_3$ (Ph = Phenyl), $C_3$-$C_{15}$-Heteroaryl und $C_6$-$C_{18}$-Aryl ersetzt sind, wobei optional ein oder mehrere Wasserstoffatome unabhängig voneinander durch $C_1$-$C_5$-Alkyl, CN, $CF_3$ und Ph ersetzt sind;
wobei vorzugsweise die Bindungsstelle von $R^A$ gemäß Formel $S^B$-III-3a eines der $C_3$-$C_{15}$-Kohlenstoffatome des $C_3$-$C_{15}$-Heteroaryls ist.

**10.** Organische elektrolumineszente Vorrichtung nach einem der Ansprüche 1 bis 9, umfassend ein oder mehrere p-Wirtsmaterialien $H^P$, die ein niedrigstes unbesetztes Molekülorbital LUMO($H^P$) mit einer Energie $E^{LUMO}(H^P)$ gemäß $-2{,}6\ eV \leq E^{LUMO}(H^P)$ aufweisen.

**11.** Organische elektrolumineszente Vorrichtung nach einem der Ansprüche 1 bis 10, umfassend ein oder mehrere p-Wirtsmaterialien $H^P$, die umfassen oder bestehen aus:

- einer ersten chemischen Einheit, die eine Struktur gemäß einer der Formeln $H^P$-I, $H^P$-II, $H^P$-III, $H^P$-IV, $H^P$-V, $H^P$-VI, $H^P$-VII, $H^P$-VIII, $H^P$-IX und $H^P$-X umfasst oder daraus besteht:

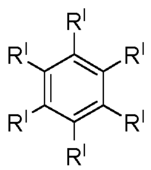
Formel $H^P$-I

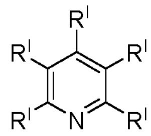
Formel $H^P$-II

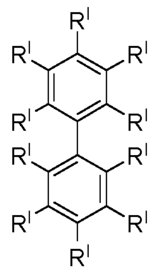
Formel $H^P$-III

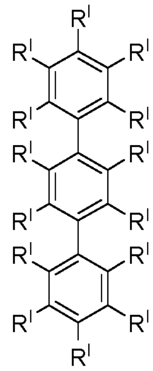
Formel $H^P$-IV

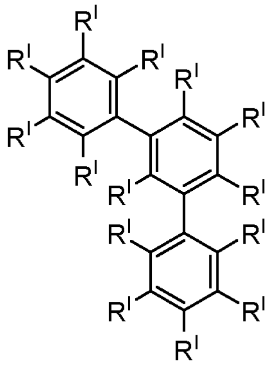
Formel $H^P$-V

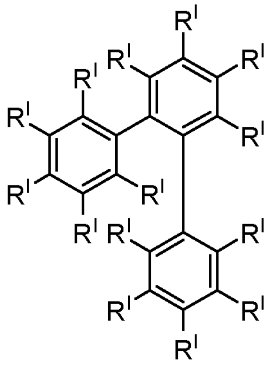
Formel $H^P$-VI

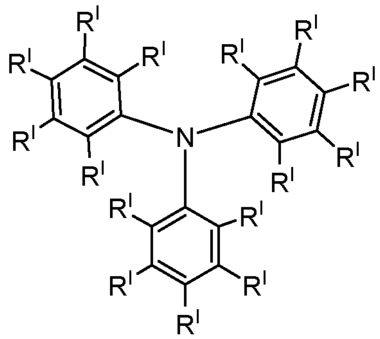
Formel $H^P$-VII

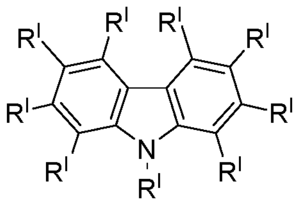
Formel $H^P$-VIII

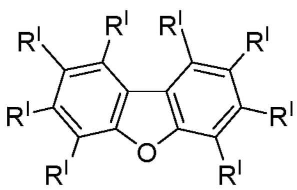
Formel $H^P$-IX

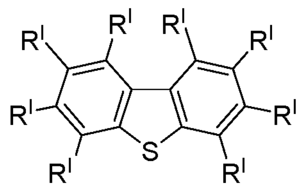
Formel $H^P$-X

- mindestens eine zweite chemische Einheit, die eine Struktur gemäß einer der Formeln $H^P$-XI, $H^P$-XII, $H^P$-XIII,

$H^P$-XIV, $H^P$-XV, $H^P$-XVI, $H^P$-XVII, $H^P$-XVIII und $H^P$-XIX umfasst oder daraus besteht:

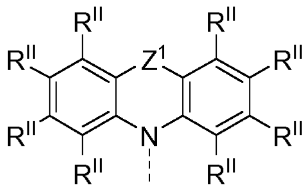
Formel $H^P$-XI

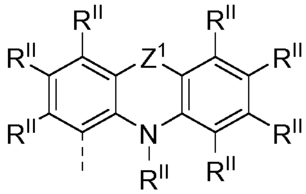
Formel $H^P$-XII

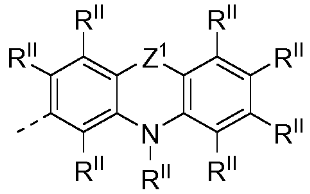
Formel $H^P$-XIII

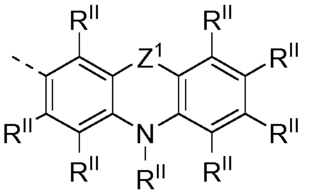
Formel $H^P$-XIV

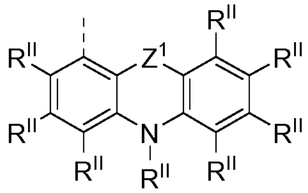
Formel $H^P$-XV

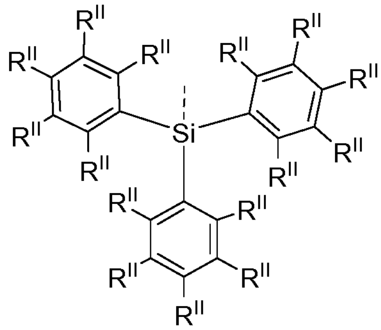
Formel $H^P$-XVI

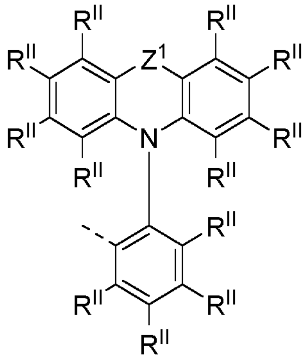
Formel $H^P$-XVII

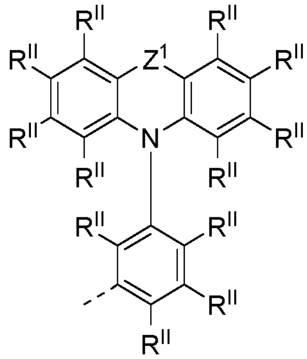
Formel $H^P$-XVIII

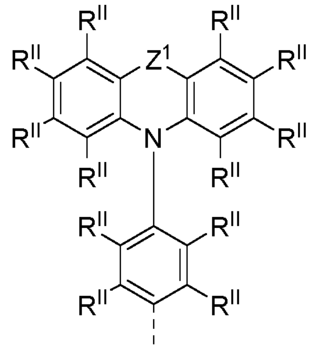
Formel $H^P$-XIX

wobei jede der mindestens einen zweiten chemischen Einheiten, die in dem p-Wirtsmaterial $H^P$ vorhanden ist, mit der ersten chemischen Einheit über eine Einfachbindung verbunden ist, die in den obigen Formeln durch eine gestrichelte Linie dargestellt ist;
wobei
$Z^1$ bei jedem Auftreten unabhängig voneinander ausgewählt ist aus der Gruppe bestehend aus einer direkten Bindung, $C(R^{II})_2$, $C=C(R^{II})_2$, C=O, $C=NR^{II}$, $NR^{II}$, O, $Si(R^{II})_2$, S, S(O) und $S(O)_2$;
$R^I$ bei jedem Auftreten unabhängig voneinander eine Bindungsstelle einer Einfachbindung ist, die die erste chemische Einheit mit einer zweiten chemischen Einheit verbindet, oder ausgewählt ist aus der Gruppe bestehend aus: Wasserstoff, Deuterium, Me, $^i$Pr, $^t$Bu, wobei mindestens ein $R^I$ eine Bindungsstelle einer Einfachbindung ist, die die erste chemische Einheit mit einer zweiten chemischen Einheit verbindet, und
Ph, das optional mit einem oder mehreren Substituenten unabhängig voneinander substituiert ist, ausgewählt aus der Gruppe bestehend aus: Me, $^i$Pr, $^t$Bu, und Ph;
$R^{II}$ bei jedem Auftreten unabhängig voneinander ausgewählt ist aus der Gruppe, bestehend aus: Wasserstoff, Deuterium, Me, $^i$Pr, $^t$Bu, und
Ph, das optional mit einem oder mehreren Substituenten unabhängig voneinander substituiert ist, ausgewählt aus der Gruppe bestehend aus: Me, $^i$Pr, $^t$Bu, und Ph;
wobei zwei oder mehr benachbarte Substituenten $R^{II}$ optional ein aromatisches oder heteroaromatisches Ringsystem mit 3-18 Kohlenstoffatomen bilden können.

**12.** Organische elektrolumineszente Vorrichtung nach einem der Ansprüche 1 bis 11, wobei die organische elektrolumineszente Vorrichtung eine Vorrichtung ist ausgewählt aus der Gruppe bestehend aus einer organischen lichtemittierenden Diode, einer lichtemittierenden elektrochemischen Zelle und einem lichtemittierenden Transistor.

**13.** Organische elektrolumineszente Vorrichtung nach einem der Ansprüche 1 bis 12, wobei die mindestens eine lichtemittierende Schicht B umfasst:

(i) 10-89,9 Gew.-% einer oder mehrerer p-Wirtsverbindungen $H^P$;
(ii) 0-79,9 Gew.-% einer oder mehrerer n-Wirtsverbindungen $H^N$;
(iii) 10-50 Gew.-% eines oder mehrerer TADF-Materialien $E^B$; und
(iv) 0,1-10 Gew.-% eines oder mehrerer Emitter $S^B$ mit kleiner FWHM; und
(v) 0-72 Gew.-% eines oder mehrerer Lösungsmittel.

**14.** Organische elektrolumineszente Vorrichtung nach einem der Ansprüche 1 bis 12, wobei die mindestens eine lichtemittierende Schicht B umfasst:

(i) 22-87,5 Gew.-% einer oder mehrerer p-Wirtsverbindungen $H^P$;
(ii) optional 0-65,5 Gew.-% einer oder mehrerer n-Wirtsverbindungen $H^N$;
(iii) 12-40 Gew.-% eines oder mehrerer TADF-Materialien $E^B$; und
(iv) 0,5-5 Gew.-% eines oder mehrerer Emitter $S^B$ mit kleiner FWHM; und
(v) 0-65,5 Gew.-% eines oder mehrerer Lösungsmittel.

**15.** Verfahren zur Erzeugung von grünem Licht mit einer Wellenlänge von 500 nm bis 560 nm, das die folgenden Schritte umfasst:

(i) Bereitstellen einer organischen elektrolumineszenten Vorrichtung nach einem der Ansprüche 1 bis 14; und
(ii) Anlegen eines elektrischen Stroms an die organische elektrolumineszente Vorrichtung.

## Revendications

**1.** Dispositif électroluminescent organique comprenant une ou plusieurs couches émettrices de lumière B, chacune comprenant indépendamment les unes des autres :

(i) au moins un matériau hôte $H^B$, qui a un niveau d'énergie d'état singulet excité le plus bas $E(S1^H)$ et un niveau d'énergie d'état triplet excité le plus bas $E(T1^H)$;
(ii) au moins un matériau à fluorescence retardée activée thermiquement (TADF) $E^B$, qui a un niveau d'énergie d'état singulet excité le plus bas $E(S1^E)$ et un niveau d'énergie d'état triplet excité le plus bas $E(T1^E)$ ; et
(iii) au moins un émetteur $S^B$ de faible largeur totale à mi-hauteur (FWHM) ayant un spectre d'émission qui présente une FWHM inférieure ou égale à 0,25 eV, mesurée avec 1 % en poids de l'émetteur $S^B$ dans du poly(méthacrylate de méthyle) à 20 °C, qui a un niveau d'énergie d'état singulet excité le plus bas $E(S1^s)$ et un niveau d'énergie d'état triplet excité le plus bas $E(T1^s)$,
dans lequel chaque $E^B$ transfère de l'énergie à au moins un $S^B$ et chaque $S^B$ émet de la lumière avec un maximum d'émission compris entre 500 nm et 560 nm, dans lequel les relations exprimées par les formules (1) à (5) suivantes s'appliquent :

$$E(S1^H) > E(S1^E) \quad (1)$$

$$E(S1^H) > E(S1^S) \quad (2)$$

$$E(S1^E) > E(S1^S) \quad (3)$$

$$E(T1^H) > E(T1^S) \quad (4)$$

$$E(T1^H) > E(T1^E) \quad (5) ;$$

dans lequel au moins un émetteur $S^B$ de faible largeur totale à mi-hauteur (FWHM) est un émetteur contenant du bore.

2. Dispositif électroluminescent organique selon la revendication 1, dans lequel un matériau TADF $E^B$ est **caractérisé en ce qu'**il présente une valeur $\Delta E_{ST}$, qui correspond à la différence d'énergie entre l'état singulet excité le plus bas $S1^E$ et l'état triplet excité le plus bas $T1^E$, inférieure à 0,4 eV.

3. Dispositif électroluminescent organique selon l'une quelconque des revendications 1 et 2, dans lequel l'au moins un émetteur $S^B$ de faible FWHM est **caractérisé en ce qu'**il a un spectre d'émission, qui présente une largeur totale à mi-hauteur (FWHM) inférieure ou égale à 0,25 eV.

4. Dispositif électroluminescent organique selon l'une quelconque des revendications 1 à 3, dans lequel l'au moins un matériau à fluorescence retardée activée thermiquement (TADF) $E^B$ a un maximum d'émission $\lambda_{max}(D)$ dans la plage de longueurs d'onde de 500 nm à 560 nm.

5. Dispositif électroluminescent organique selon l'une quelconque des revendications 1 à 4, dans lequel l'au moins un matériau à fluorescence retardée activée thermiquement (TADF) $E^B$ a une orbitale moléculaire occupée la plus élevée HOMO($E^B$) ayant une énergie $E^{HOMO}(E^B)$ selon -6,0 ev $\leq E^{HOMO}(E^B) \leq$ -5,8 eV.

6. Dispositif électroluminescent organique selon l'une quelconque des revendications 1 à 5, dans lequel l'au moins un matériau à fluorescence retardée activée thermiquement (TADF) $E^B$ a une structure représentée par l'une quelconque des formules $E^B$-I-1a, $E^B$-I-2a, $E^B$-I-3a, $E^B$-I-4a, $E^B$-I-5a, $E^B$-I-6a, $E^B$-I-7 et $E^B$-I-8,

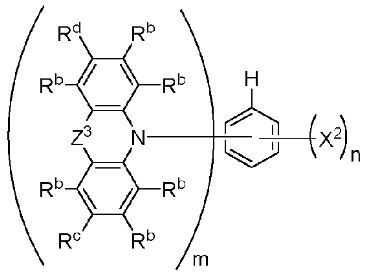

formule $E^B$-I-1a

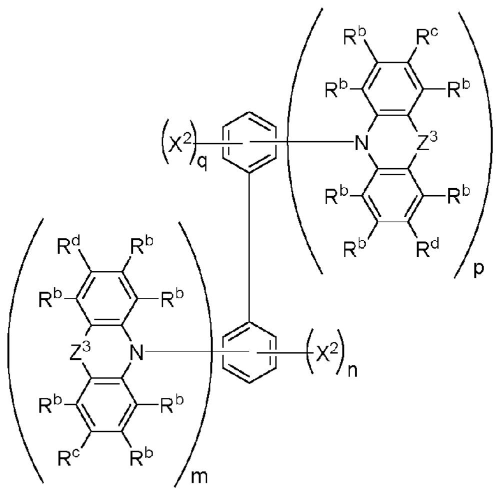

formule $E^B$-I-2a

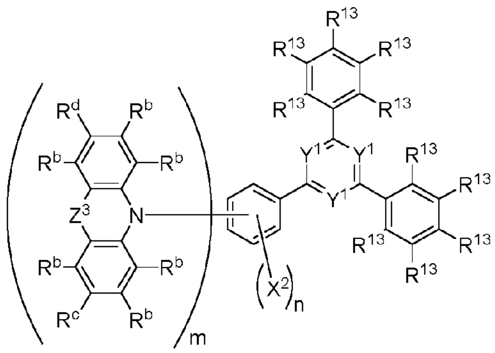

formule $E^B$-I-3a

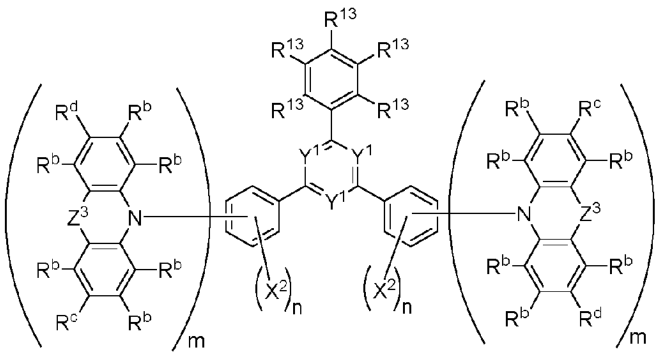

formule $E^B$-I-4a

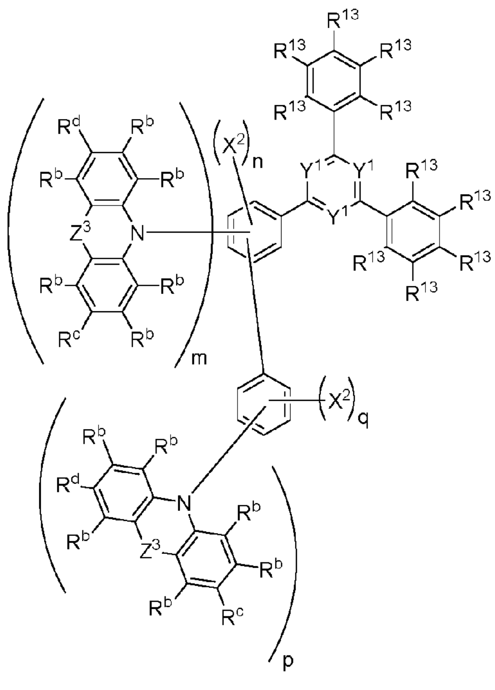

formule $E^B$-I-5a

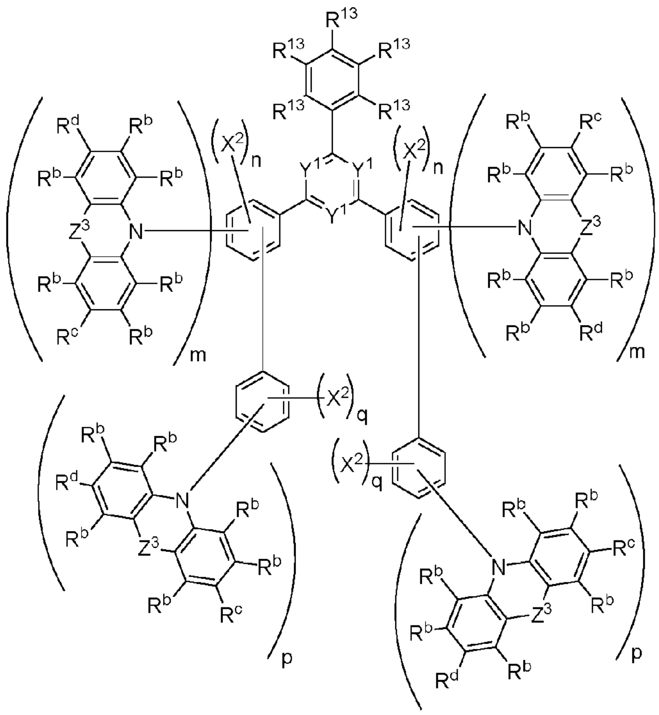

formule $E^B$-I-6a

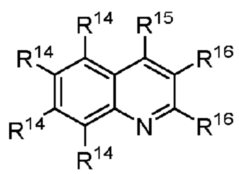

formule $E^B$-I-7

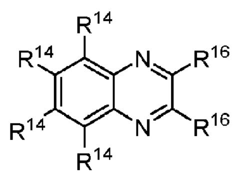

formule $E^B$-I-8

$Y^1$ étant en chaque occurrence azote (N) ou CH, et au moins un $Y^1$ étant N ;
m étant en chaque occurrence indépendamment l'un de l'autre 0, 1 ou 2 ;
n étant en chaque occurrence indépendamment l'un de l'autre 0, 1 ou 2 ;
p étant en chaque occurrence indépendamment l'un de l'autre 0, 1 ou 2 ;
q étant en chaque occurrence indépendamment l'un de l'autre 0, 1 ou 2 ;
$X^2$ étant en chaque occurrence indépendamment l'un de l'autre choisi dans le groupe constitué par $Ar^{EWG}$, CN, et $CF_3$ ;
$Ar^{EWG}$ étant en chaque occurrence indépendamment l'un de l'autre représenté par une structure selon l'une quelconque des formules $Ar^{EWG}$-I, $Ar^{EWG}$-II, $Ar^{EWG}$-III, $Ar^{EWG}$-IV, $Ar^{EWG}$-V, $Ar^{EWG}$-VI, $Ar^{EWG}$-VII, $Ar^{EWG}$-VIII, $Ar^{EWG}$-IX, $Ar^{EWG}$-X, $Ar^{EWG}$-XI, $Ar^{EWG}$-XII, $Ar^{EWG}$-XIII, et $Ar^{EWG}$-XIV,

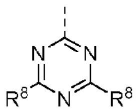 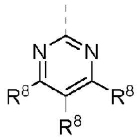 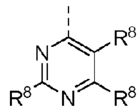 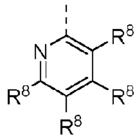

formule $Ar^{EWG}$-I formule $Ar^{EWG}$-II formule $Ar^{EWG}$-III formule $Ar^{EWG}$-IV

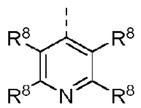 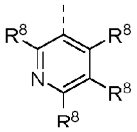 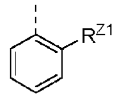 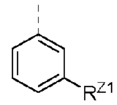

formule $Ar^{EWG}$-V formule $Ar^{EWG}$-VI formule $Ar^{EWG}$-VII formule $Ar^{EWG}$-VIII

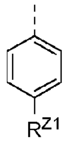 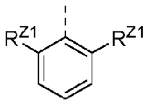 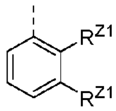 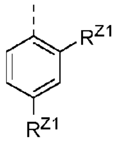

formule $Ar^{EWG}$-IX formule $Ar^{EWG}$-X formule $Ar^{EWG}$-XI formule $Ar^{EWG}$-XII

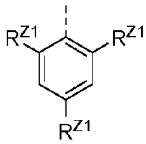 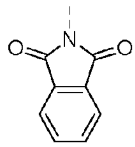

formule $Ar^{EWG}$-XIII formule $Ar^{EWG}$-XIV

qui sont liées à la structure centrale via la position marquée par la ligne en pointillés ;

$R^{Z1}$ étant en chaque occurrence indépendamment l'un de l'autre choisi dans le groupe constitué par CN et $CF_3$ ;

$Z^3$ étant en chaque occurrence indépendamment l'un de l'autre choisi dans le groupe constitué par une liaison directe, $CR^9R^{10}$, $C=CR^9R^{10}$, C=O, $C=NR^9$, $NR^9$, O, $SiR^9R^{10}$, S, S(O) et $S(O)_2$ ;

$R^8$ étant en chaque occurrence indépendamment l'un de l'autre choisi dans le groupe constitué par : hydrogène, deutérium, CN, $CF_3$,

$C_1$-$C_5$-alkyle,

un ou plusieurs atomes d'hydrogène étant éventuellement substitués par deutérium ;

$C_6$-$C_{18}$-aryle,

qui est éventuellement substitué par un ou plusieurs substituants $R^{11}$; et $C_3$-$C_{17}$-hétéroaryle,

qui est éventuellement substitué par un ou plusieurs substituants $R^{11}$ ;

$R^b$, $R^c$, $R^d$, $R^9$, et $R^{10}$ étant en chaque occurrence indépendamment l'un de l'autre choisis dans le groupe constitué par : hydrogène, deutérium, $N(R^{12})_2$, $OR^{12}$, $Si(R^{12})_3$, $B(OR^{12})_2$, $OSO_2R^{12}$, $CF_3$, CN, F, Br, I,

$C_1$-$C_{40}$-alkyle,

qui est éventuellement substitué par un ou plusieurs substituants $R^{12}$, et un ou plusieurs groupes $CH_2$ non adjacents étant éventuellement C=Se, $C=NR^{12}$, $P(=O)(R^{12})$, SO, $SO_2$, $NR^{12}$, O, S ou $CONR^{12}$ ;

$C_1$-$C_{40}$-alcoxy,

qui est éventuellement substitué par un ou plusieurs substituants $R^{12}$, et un ou plusieurs groupes $CH_2$ non adjacents étant éventuellement substitués par $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S ou $CONR^{12}$ ;

$C_1$-$C_{40}$-thioalcoxy,

qui est éventuellement substitué par un ou plusieurs substituants $R^{12}$, et un ou plusieurs groupes $CH_2$ non adjacents étant éventuellement substitués par $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S ou $CONR^{12}$ ;

$C_2$-$C_{40}$-alcényle,

qui est éventuellement substitué par un ou plusieurs substituants $R^{12}$, et un ou plusieurs groupes $CH_2$ non adjacents étant éventuellement substitués par $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S ou $CONR^{12}$ ;

$C_2$-$C_{40}$-alcynyle,

qui est éventuellement substitué par un ou plusieurs substituants $R^{12}$, et un ou plusieurs groupes $CH_2$ non adjacents étant éventuellement substitués par $R^{12}C=CR^{12}$, C≡C, $Si(R^{12})_2$, $Ge(R^{12})_2$, $Sn(R^{12})_2$, C=O, C=S, C=Se, $C=NR^{12}$, $P(=O)(R^{12})$, SO, $SO_2$, $NR^{12}$, O, S ou $CONR^{12}$ ;

$C_6$-$C_{60}$-aryle,

qui est éventuellement substitué par un ou plusieurs substituants $R^{12}$ ; et $C_3$-$C_{57}$-hétéroaryle,

qui est éventuellement substitué par un ou plusieurs substituants $R^{12}$ ;

$R^{11}$ étant en chaque occurrence indépendamment l'un de l'autre choisi dans le groupe constitué par : hydrogène, deutérium, Me, $^i$Pr, $^t$Bu, CN, $CF_3$, et

phényle, qui est éventuellement substitué par un ou plusieurs substituants indépendamment les uns des autres choisis dans le groupe constitué par Me, $^i$Pr, $^t$Bu, CN, $CF_3$ et Ph ;

$R^{12}$ étant en chaque occurrence indépendamment l'un de l'autre choisi dans le groupe constitué par : hydrogène, deutérium, OPh, $CF_3$, CN, F, $C_1$-$C_5$-alkyle,

un ou plusieurs atomes d'hydrogène étant éventuellement, indépendamment les uns des autres, substitués par deutérium, CN, $CF_3$, ou F ;

$C_1$-$C_5$-alcoxy,

un ou plusieurs atomes d'hydrogène étant éventuellement, indépendamment les uns des autres, substitués par deutérium, CN, $CF_3$, ou F ;

$C_1$-$C_5$-thioalcoxy,

un ou plusieurs atomes d'hydrogène étant éventuellement, indépendamment les uns des autres, substitués par deutérium, CN, $CF_3$, ou F ;

$C_2$-$C_5$-alcényle,

un ou plusieurs atomes d'hydrogène étant éventuellement, indépendamment les uns des autres, substitués par deutérium, CN, $CF_3$, ou F ;

$C_2$-$C_5$-alcynyle,

un ou plusieurs atomes d'hydrogène étant éventuellement, indépendamment les uns des autres, substitués par deutérium, CN, $CF_3$, ou F ;

$C_6$-$C_{18}$-aryle,

qui est éventuellement substitué par un ou plusieurs substituants $C_1$-$C_5$-alkyle ;

$C_3$-$C_{17}$-hétéroaryle,

qui est éventuellement substitué par un ou plusieurs substituants $C_1$-$C_5$-alkyle ;

$N(C_6$-$C_{18}$-aryle$)_2$,

$N(C_3$-$C_{17}$-hétéroaryle$)_2$,

$N(C_3$-$C_{17}$-hétéroaryl$)(C_6$-$C_{18}$-aryle), et

un groupe amino cyclique aliphatique comprenant 5 à 8 atomes de carbone (préférablement pyrrolidinyle et pipéridinyle) ;

$R^{13}$ étant en chaque occurrence indépendamment l'un de l'autre choisi dans le groupe constitué par : hydrogène, deutérium, CN, $CF_3$, $Ar^{EWG}$

$C_1$-$C_5$-alkyle,

un ou plusieurs atomes d'hydrogène étant éventuellement substitués par deutérium ;

$C_6$-$C_{18}$-aryle,

qui est éventuellement substitué par un ou plusieurs substituants indépendamment les uns des autres choisis parmi deutérium, des groupes $C_1$-$C_5$-alkyle, et des groupes $C_6$-$C_{18}$-aryle ; et

$R^{14}$ et $R^{15}$ étant en chaque occurrence indépendamment l'un de l'autre choisis dans le groupe constitué par C-$Ar^{EWG}$ et $CR^{Q2}$ ;

$R^{16}$ étant en chaque occurrence indépendamment l'un de l'autre choisi dans le groupe constitué par $CR^6$, C-$Ar^{EWG}$ et $cR^{\alpha 2}$ ;

$R^{Q2}$ étant en chaque occurrence indépendamment l'un de l'autre choisi dans le groupe constitué par : hydrogène, deutérium, Me, $^i$Pr, $^t$Bu, CN, $CF_3$, Ph, et

carbazolyle, qui est éventuellement substitué par un ou plusieurs substituants indépendamment les uns des autres choisis dans le groupe constitué par Me, $^i$Pr, $^t$Bu, CN, $CF_3$, Ph, et $N(Ph)_2$ ;

éventuellement, de quelconques substituants adjacents $R^b$, $R^c$, $R^d$, $R^9$, et $R^{10}$ indépendamment les uns des autres formant un système cyclique monocyclique ou polycyclique, aliphatique, aromatique et/ou benzocondensé ; un ou plusieurs atomes d'hydrogène du système cyclique éventuellement ainsi formé pouvant être substitués par $R^{12}$ ; et

pas plus de deux groupes $R^{13}$ étant CN, $CF_3$ ou $Ar^{EWG}$ ;

$$1 \leq (m + p)$$

et

$$1 \leq (n + q).$$

**7.** Dispositif électroluminescent organique selon l'une quelconque des revendications 1 à 6, l'au moins un émetteur $S^B$ de faible FWHM comprenant ou étant constitué d'une structure selon la formule $S^B$-I :

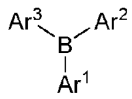

formule $S^B$-I

B étant le bore,
$Ar^1$, $Ar^2$, et $Ar^3$ étant en chaque occurrence indépendamment les uns des autres choisis dans le groupe constitué par un cycle aromatique et un cycle hétéroaromatique, et $Ar^1$, $Ar^2$, $Ar^3$ pouvant éventuellement être liés les uns aux autres pour former un ou plusieurs cycles supplémentaires.

**8.** Dispositif électroluminescent organique selon l'une quelconque des revendications 1 à 7, l'au moins un émetteur $S^B$ de faible FWHM comprenant ou étant constitué d'une structure of formule $S^B$-III-3a :

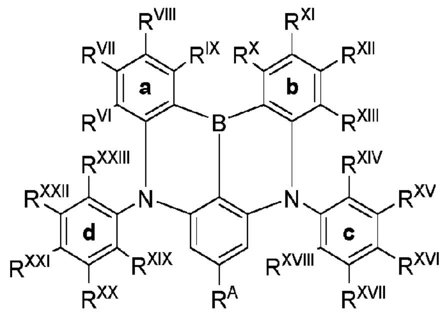

formule $S^B$-III-3a

$R^{VI}$, $R^{VII}$, $R^{VIII}$, $R^{IX}$, $R^X$, $R^{XI}$, $R^{XII}$, $R^{XIII}$, $R^{XIV}$, $R^{XV}$, $R^{XVI}$, $R^{XVII}$, $R^{XVII}$, $R^{XIX}$ $R^{XX}$, $R^{XXI}$, $R^{XXII}$, et $R^{XXIII}$ étant indépendamment les uns des autres choisis dans le groupe constitué par : hydrogène, deutérium, $N(R^{21})_2$,$OR^{21}$,$SR^{21}$, $Si(R^{21})_3$, $B(OR^{21})_2$,$OSO_2R^{21}$, $CF_3$, CN, halogène,
$C_1$-$C_{40}$-alkyle,
qui est éventuellement substitué par un ou plusieurs substituants $R^{21}$, et un ou plusieurs groupes $CH_2$ non adjacents étant éventuellement substitués par $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S ou $CONR^{21}$ ;
$C_1$-$C_{40}$-alcoxy,
qui est éventuellement substitué par un ou plusieurs substituants $R^{21}$, et un ou plusieurs groupes $CH_2$ non adjacents étant éventuellement substitués par $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S ou $CONR^{21}$ ;
$C_1$-$C_{40}$-thioalcoxy,
qui est éventuellement substitué par un ou plusieurs substituants $R^{21}$, et un ou plusieurs groupes $CH_2$ non adjacents étant éventuellement substitués par $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S ou $CONR^{21}$ ;
$C_2$-$C_{40}$-alcényle,
qui est éventuellement substitué par un ou plusieurs substituants $R^{21}$, et un ou plusieurs groupes $CH_2$ non adjacents étant éventuellement substitués par $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S ou $CONR^{21}$ ;
$C_2$-$C_{40}$-alcynyle,
qui est éventuellement substitué par un ou plusieurs substituants $R^{21}$, et un ou plusieurs groupes $CH_2$ non adjacents étant éventuellement substitués par $R^{21}C=CR^{21}$, C≡C, $Si(R^{21})2$, $Ge(R^{21})_2$, $Sn(R^{21})_2$, C=O, C=S, C=Se, $C=NR^{21}$, $P(=O)(R^{21})$, SO, $SO_2$, $NR^{21}$, O, S ou $CONR^{21}$ ;

$C_6$-$C_{60}$-aryle,
qui est éventuellement substitué par un ou plusieurs substituants $R^{21}$; et $C_3$-$C_{57}$-hétéroaryle,
qui est éventuellement substitué par un ou plusieurs substituants $R^{21}$ ;
éventuellement une ou plusieurs paires de groupes adjacents $R^{VI}$ et $R^{VII}$, $R^{VII}$ et $R^{VIII}$, $R^{VIII}$ et $R^{IX}$, $R^{X}$ et $R^{XI}$, $R^{XI}$ et $R^{XII}$, $R^{XII}$ et $R^{XIII}$, $R^{XIV}$ et $R^{XV}$, $R^{XV}$ et $R^{XVI}$, $R^{XVI}$, et $R^{XVII}$, $R^{XVII}$, et $R^{XVIII}$, $R^{XIX}$ et $R^{XX}$, $R^{XX}$ et $R^{XXI}$, $R^{XXI}$ et $R^{XXII}$, $R^{XXII}$ et $R^{XXIII}$ formant un système cyclique aromatique qui est condensé au cycle benzène adjacent a, b, c ou d de la formule générale $S^B$-III-3a et qui est éventuellement substitué par un ou plusieurs substituants $R^{21}$ ;
éventuellement l'une ou les deux paires $R^{VI}$ et $R^{XXIII}$, $R^{XIII}$ et $R^{XIV}$ étant jointes pour former un groupe $Z^4$ qui est en chaque occurrence indépendamment l'un de l'autre choisi dans le groupe constitué par : une liaison directe, $CR^{22}R^{23}$, $C=CR^{22}R^{23}$, C=O, $C=NR^{22}$, $NR^{22}$, O, $SiR^{22}R^{23}$, S, S(O), et $S(O)_2$ ;
$R^{21}$ étant en chaque occurrence indépendamment l'un de l'autre choisi dans le groupe constitué par : hydrogène, deutérium, OPh, SPh, $CF_3$, CN, F, $Si(C_1$-$C_5$- alkyl$)_3$, $Si(Ph)_3$,
$C_1$-$C_5$-alkyle,
éventuellement un ou plusieurs atomes d'hydrogène étant indépendamment les uns des autres substitués par deutérium, CN, $CF_3$, ou F ;
$C_1$-$C_5$-alcoxy,
éventuellement un ou plusieurs atomes d'hydrogène étant indépendamment les uns des autres substitués par deutérium, CN, $CF_3$, ou F ;
$C_1$-$C_5$-thioalcoxy,
éventuellement un ou plusieurs atomes d'hydrogène étant indépendamment les uns des autres substitués par deutérium, CN, $CF_3$, ou F ;
$C_2$-$C_5$-alcényle,
éventuellement un ou plusieurs atomes d'hydrogène étant indépendamment les uns des autres substitués par deutérium, CN, $CF_3$, ou F ;
$C_2$-$C_5$-alcynyle,
éventuellement un ou plusieurs atomes d'hydrogène étant indépendamment les uns des autres substitués par deutérium, CN, $CF_3$, ou F ;
$C_6$-$C_{18}$-aryle,
qui est éventuellement substitué par un ou plusieurs substituants $C_1$-$C_5$-alkyle ;
$C_3$-$C_{17}$-hétéroaryle,
qui est éventuellement substitué par un ou plusieurs substituants $C_1$-$C_5$-alkyle ;
$N(C_6$-$C_{18}$-aryle$)_2$,
$N(C_3$-$C_{17}$-hétéroaryle$)_2$, et
$N(C_3$-$C_{17}$-hétéroaryl$)(C_6$-$C_{18}$-aryle) ;
$R^{22}$ et $R^{23}$ étant en chaque occurrence indépendamment l'un de l'autre choisis dans le groupe constitué par : hydrogène, deutérium, $N(R^{24})_2$, $OR^{24}$, $Si(R^{24})_3$, $B(OR^{24})_2$, $OSO_2R^{24}$, $CF_3$, CN, F, Br, I,
$C_1$-$C_{40}$-alkyle,
qui est éventuellement substitué par un ou plusieurs substituants $R^{24}$, et un ou plusieurs groupes $CH_2$ non adjacents étant éventuellement substitués par $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S ou $CONR^{24}$ ;
$C_1$-$C_{40}$-alcoxy,
qui est éventuellement substitué par un ou plusieurs substituants $R^{24}$, et un ou plusieurs groupes $CH_2$ non adjacents étant éventuellement substitués par $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S ou $CONR^{24}$ ;
$C_1$-$C_{40}$-thioalcoxy,
qui est éventuellement substitué par un ou plusieurs substituants $R^{24}$, et un ou plusieurs groupes $CH_2$ non adjacents étant éventuellement substitués par $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S ou $CONR^{24}$ ;
$C_2$-$C_{40}$-alcényle,
qui est éventuellement substitué par un ou plusieurs substituants $R^{24}$, et un ou plusieurs groupes $CH_2$ non adjacents étant éventuellement substitués par $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S ou $CONR^{24}$ ;
$C_2$-$C_{40}$-alcynyle,
qui est éventuellement substitué par un ou plusieurs substituants $R^{24}$, et un ou plusieurs groupes $CH_2$ non adjacents étant éventuellement substitués par $R^{24}C=CR^{24}$, C=C, $Si(R^{24})_2$, $Ge(R^{24})_2$, $Sn(R^{24})_2$, C=O, C=S, C=Se, $C=NR^{24}$, $P(=O)(R^{24})$, SO, $SO_2$, $NR^{24}$, O, S ou $CONR^{24}$ ;
$C_6$-$C_{60}$-aryle,
qui est éventuellement substitué par un ou plusieurs substituants $R^{24}$ ; et

$C_3$-$C_{57}$-hétéroaryle,
qui est éventuellement substitué par un ou plusieurs substituants $R^{24}$ ;
$R^{24}$ étant en chaque occurrence indépendamment l'un de l'autre choisi dans le groupe constitué par : hydrogène, deutérium, OPh, SPh, $CF_3$, CN, F, $Si(C_1$-$C_5$-alkyl$)_3$, $Si(Ph)_3$,
$C_1$-$C_5$-alkyle,
éventuellement un ou plusieurs atomes d'hydrogène étant indépendamment les uns des autres substitués par deutérium, CN, $CF_3$, ou F ;
$C_1$-$C_5$-alcoxy,
éventuellement un ou plusieurs atomes d'hydrogène étant indépendamment les uns des autres substitués par deutérium, CN, $CF_3$, ou F ;
$C_1$-$C_5$-thioalcoxy,
éventuellement un ou plusieurs atomes d'hydrogène étant indépendamment les uns des autres substitués par deutérium, CN, $CF_3$, ou F ;
$C_2$-$C_5$-alcényle,
éventuellement un ou plusieurs atomes d'hydrogène étant indépendamment les uns des autres substitués par deutérium, CN, $CF_3$, ou F ;
$C_2$-$C_5$-alcynyle,
éventuellement un ou plusieurs atomes d'hydrogène étant indépendamment les uns des autres substitués par deutérium, CN, $CF_3$, ou F ;
$C_6$-$C_{18}$-aryle,
qui est éventuellement substitué par un ou plusieurs substituants $C_1$-$C_5$-alkyle ;
$C_3$-$C_{17}$-hétéroaryle,
qui est éventuellement substitué par un ou plusieurs substituants $C_1$-$C_5$-alkyle ;
$N(C_6$-$C_{18}$-aryle$)_2$,
$N(C_3$-$C_{17}$-hétéroaryle$)_2$, et
$N(C_3$-$C_{17}$-hétéroaryl$)(C_6$-$C_{18}$-aryle$)$ ;
$R^A$ étant choisi dans le groupe constitué par : hydrogène,
$C_3$-$C_{15}$-hétéroaryle, éventuellement un ou plusieurs atomes d'hydrogène étant indépendamment les uns des autres substitués par deutérium, halogène, $C_1$-$C_5$-alkyle, CN, $CF_3$, $SiMe_3$, $SiPh_3$ (Ph = phényle), $C_3$-$C_{15}$-hétéroaryle, et
$C_6$-$C_{18}$-aryle, dans lequel éventuellement un ou plusieurs atomes d'hydrogène sont indépendamment les uns des autres substitués par $C_1$-$C_5$-alkyle, CN, $CF_3$ et Ph ;
et
$C_6$-$C_{18}$-aryle, éventuellement un ou plusieurs atomes d'hydrogène étant indépendamment les uns des autres substitués par un substituant indépendamment l'un de l'autre choisi dans le groupe constitué par : $C_1$-$C_5$-alkyle, CN, $CF_3$, et
Ph, qui est éventuellement substitué par un ou plusieurs substituants indépendamment les uns des autres choisis dans le groupe constitué par Me, $^i$Pr, $^t$Bu, CN, $CF_3$, et Ph,
pyridinyle, qui est éventuellement substitué par un ou plusieurs substituants indépendamment les uns des autres choisis dans le groupe constitué par Me, $^i$Pr, $^t$Bu, CN, $CF_3$, et Ph,
pyrimidinyle, qui est éventuellement substitué par un ou plusieurs substituants indépendamment les uns des autres choisis dans le groupe constitué par Me, $^i$Pr, $^t$Bu, CN, $CF_3$, et Ph, et
triazinyle, qui est éventuellement substitué par un ou plusieurs substituants indépendamment les uns des autres choisis dans le groupe constitué par Me, $^i$Pr, $^t$Bu, CN, $CF_3$, et Ph.

**9.** Dispositif électroluminescent organique selon l'une quelconque des revendications 1 à 8, $R^A$ étant $C_3$-$C_{15}$-hétéroaryle,

éventuellement, un ou plusieurs atomes d'hydrogène étant indépendamment les uns des autres substitués par deutérium, halogène, $C_1$-$C_5$-alkyle, CN, $CF_3$, $SiMe_3$, $SiPh_3$ (Ph = phényle), $C_3$-$C_{15}$-hétéroaryle, et $C_6$-$C_{18}$-aryle, dans lequel éventuellement un ou plusieurs atomes d'hydrogène sont indépendamment les uns des autres substitués par $C_1$-$C_5$-alkyle, CN, $CF_3$ et Ph ;
préférablement le site de liaison de $R^A$ selon la formule $S^B$-III-3a étant l'un des atomes de carbone $C_3$-$C_{15}$- du $C_3$-$C_{15}$-hétéroaryle.

**10.** Dispositif électroluminescent organique selon l'une quelconque des revendications 1 à 9, comprenant un ou plusieurs matériaux hôtes p $H^P$ qui possèdent une orbitale moléculaire non occupée la plus basse $LUMO(H^P)$ ayant une énergie $E^{LUMO}(H^P)$ selon $-2{,}6\ eV \leq E^{LUMO}(H^P)$.

**11.** Dispositif électroluminescent organique selon l'une quelconque des revendications 1 à 10 comprenant un ou plusieurs matériaux hôtes p $H^P$ qui comprennent ou sont constitués de :

- un premier groupement chimique, comprenant ou constitué d'une structure selon l'une quelconque des formules $H^P$-I, $H^P$-II, $H^P$-III, $H^P$-IV, $H^P$-V, $H^P$-VI, $H^P$-VII, $H^P$-VIII, $H^P$-IX, et $H^P$-X :

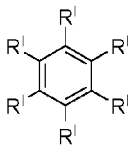
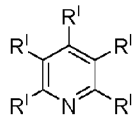
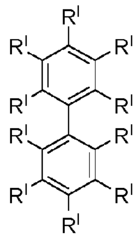
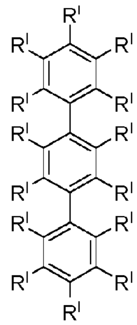

formule $H^P$-I formule $H^P$-II formule $H^P$-III formule $H^P$-IV

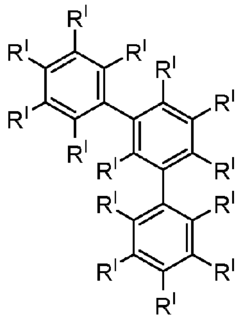
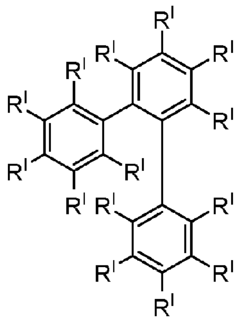
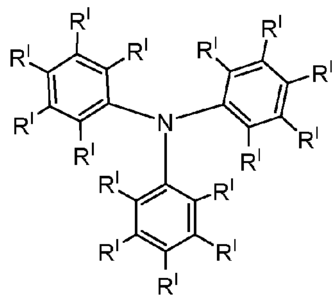

formule $H^P$-V formule $H^P$-VI formule $H^P$-VII

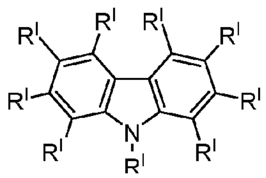
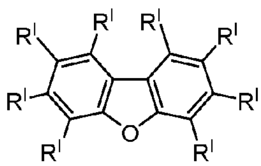
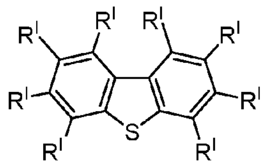

formule $H^P$-VIII formule $H^P$-IX formule $H^P$-X

et

- au moins un deuxième groupement chimique comprenant ou constitué d'une structure selon l'une quelconque des formules $H^P$-XI, $H^P$-XII, $H^P$-XIII, $H^P$-XIV, $H^P$-XV, $H^P$-XVI, $H^P$-XVII, $H^P$-XVIII, et $H^P$-XIX:

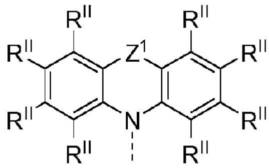
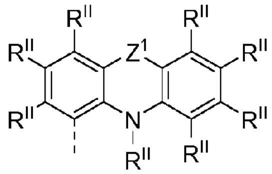
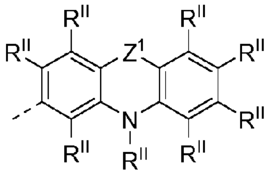

formule $H^P$-XI formule $H^P$-XII formule $H^P$-XIII

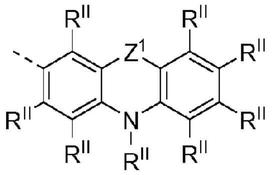
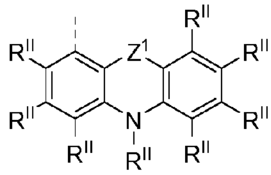
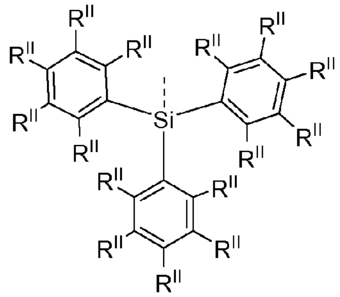

formule $H^P$-XIV formule $H^P$-XVformule $H^P$-XVI

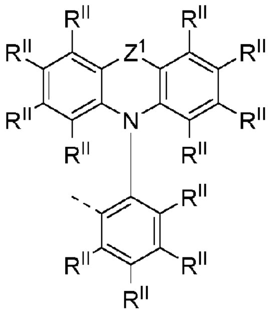
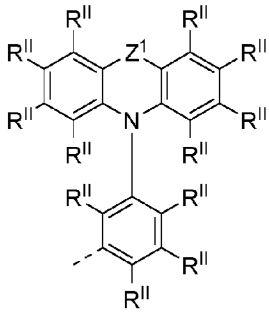
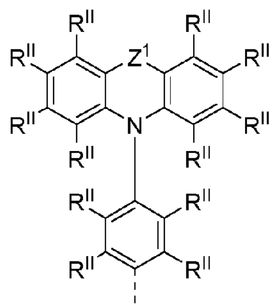

formule $H^P$-XVII formule $H^P$-XVIIIformule $H^P$-XIX

chacun parmi l'au moins un deuxième groupement chimique qui est présent dans le matériau hôte p $H^P$ étant lié au premier groupement chimique via une simple liaison qui est représentée dans les formules ci-dessus par une ligne en pointillés ;
$Z^1$ étant en chaque occurrence indépendamment l'un de l'autre choisi dans le groupe constitué par une liaison directe, $C(R^{II})_2$, $C=C(R^{II})_2$, C=O, $C=NR^{II}$, $NR^{II}$, O, $Si(R^{II})_2$, S, S(O) et $S(O)_2$ ;
$R^I$ étant en chaque occurrence indépendamment l'un de l'autre un site de liaison d'une simple liaison liant le premier groupement chimique au deuxième groupement chimique ou étant choisi dans le groupe constitué par : hydrogène, deutérium, Me, $^i$Pr, $^t$Bu,
au moins un $R^I$ étant un site de liaison d'une simple liaison liant le premier groupement chimique au deuxième groupement chimique, et
Ph, qui est éventuellement substitué par un ou plusieurs substituants indépendamment les uns des autres choisis dans le groupe constitué par : Me, $^i$Pr, $^t$Bu, et Ph ;
$R^{II}$ étant en chaque occurrence indépendamment l'un de l'autre choisi dans le groupe constitué par : hydrogène, deutérium, Me, $^i$Pr, $^t$Bu, et
Ph, qui est éventuellement substitué par un ou plusieurs substituants indépendamment les uns des autres choisis dans le groupe constitué par : Me, $^i$Pr, $^t$Bu, et Ph ;
deux substituants adjacents ou plus $R^{II}$ pouvant éventuellement former un système cyclique aromatique ou hétéroaromatique comportant 3 à 18 atomes de carbone.

**12.** Dispositif électroluminescent organique selon l'une quelconque des revendications 1 à 11, dans lequel ledit dispositif électroluminescent organique est un dispositif choisi dans le groupe constitué par une diode émettrice de lumière organique, une cellule électrochimique émettrice de lumière et un transistor émetteur de lumière.

**13.** Dispositif électroluminescent organique selon l'une quelconque des revendications 1 à 12, dans lequel l'au moins une couche émettrice de lumière B comprend :

(i) 10-89,9 % en poids d'un ou de plusieurs composés hôtes p $H^P$ ;
(ii) 0-79,9 % en poids d'un ou de plusieurs composés hôtes n $H^N$ ;
(iii) 10-50 % en poids d'un ou de plusieurs matériaux TADF $E^B$ ; et
(iv) 0,1-10 % en poids d'un ou de plusieurs émetteurs $S^B$ de faible FWHM ; et
(v) 0-72 % en poids d'un ou de plusieurs solvants.

**14.** Dispositif électroluminescent organique selon l'une quelconque des revendications 1 à 12, dans lequel l'au moins une

couche émettrice de lumière B comprend :

(i) 22-87,5 % en poids d'un ou de plusieurs composés hôtes p $H^P$ ;
(ii) éventuellement 0-65,5 % en poids d'un ou de plusieurs composés hôtes n $H^N$ ;
(iii) 12-40 % en poids d'un ou de plusieurs matériaux TADF $E^B$ ; et
(iv) 0,5-5 % en poids d'un ou de plusieurs émetteurs $S^B$ de faible FWHM ; et
(v) 0-65,5 % en poids d'un ou de plusieurs solvants.

**15.** Procédé pour générer de la lumière verte à une longueur d'onde de 500 nm à 560 nm, comprenant les étapes de :

(i) fourniture d'un dispositif électroluminescent organique selon l'une quelconque des revendications 1 à 14 ; et
(ii) application d'un courant électrique audit dispositif électroluminescent organique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3490023 A1
- WO 2017005699 A


### Non-patent literature cited in the description


- *CHEMICAL ABSTRACTS*, 73183-34-3
- **HATAKEYAMA et al.** *Advanced Materials*, 2016, vol. 28 (14), 2777-2781
- *CHEMICAL ABSTRACTS*, 81067-41-6
- *CHEMICAL ABSTRACTS*, 51364-51-3
- *CHEMICAL ABSTRACTS*, 13716-12-6
- *CHEMICAL ABSTRACTS*, 594-19-4
- *CHEMICAL ABSTRACTS*, 61676-62-8
- *CHEMICAL ABSTRACTS*, 7087-68-5
- *CHEMICAL ABSTRACTS*, 7446-70-0
- *CHEMICAL ABSTRACTS*, 3375-31-3
- *CHEMICAL ABSTRACTS*, 657408-07-6